# EUROPEAN PATENT APPLICATION

(11) **EP 4 245 374 A2**
(43) Date of publication of application: **20.09.2023**
(21) Application number: 23162663.1
(22) Date of filing: 17.03.2023
(51) Int. Cl.: A61P 35/00, C07K 16/28, G01N 33/574

(54) **PVRL2 AND/OR PVRIG AS BIOMARKERS FOR TREATMENT**

(30) Priority: 18.03.2022 US 202263321564 P
(71) Applicant: Compugen Ltd., 5885849 Holon (IL)
(72) Inventor: Granit, Roy Z., 5885849 Holon (IL); Cojocaru, Gad S., 5885849 Holon (IL); Ophir, Eran, 5885849 Holon (IL); Novik, Amit, 5885849 Holon (IL); Sabath, Niv, 5885849 Holon (IL); Alteber, Zoya, 5885849 Holon (IL)
(74) Representative: Fuchs Patentanwälte Partnerschaft mbB

(57) **Abstract**

The present invention provides biomarkers for use in determining populations for treatment with anti-PVRIG antibodies and such biomarkers include, for example PVRIG and/or PVRL2 expression.

## Description

### CROSS-REFERENCE TO RELATED APPLICATIONS

This application claims priority to U.S. Provisional Patent Application 63/321,564, filed on March 18, 2022, which is hereby incorporated by reference in its entirety.

### BACKGROUND OF THE INVENTION

Naive T cells must receive two independent signals from antigen-presenting cells (APC) in order to become productively activated. The first, Signal 1, is antigen-specific and occurs when T cell antigen receptors encounter the appropriate antigen-MHC complex on the APC. The fate of the immune response is determined by a second, antigen-independent signal (Signal 2) which is delivered through a T cell costimulatory molecule that engages its APC-expressed ligand. This second signal could be either stimulatory (positive costimulation) or inhibitory (negative costimulation or coinhibition). In the absence of a costimulatory signal, or in the presence of a coinhibitory signal, T-cell activation is impaired or aborted, which may lead to a state of antigen-specific unresponsiveness (known as T-cell anergy), or may result in T-cell apoptotic death.

Costimulatory molecule pairs usually consist of ligands expressed on APCs and their cognate receptors expressed on T cells. The prototype ligand/receptor pairs of costimulatory molecules are B7/CD28 and CD40/CD40L. The B7 family consists of structurally related, cell-surface protein ligands, which may provide stimulatory or inhibitory input to an immune response. Members of the B7 family are structurally related, with the extracellular domain containing at least one variable or constant immunoglobulin domain.

Both positive and negative costimulatory signals play critical roles in the regulation of cell-mediated immune responses, and molecules that mediate these signals have proven to be effective targets for immunomodulation. Based on this knowledge, several therapeutic approaches that involve targeting of costimulatory molecules have been developed, and were shown to be useful for prevention and treatment of cancer by turning on, or preventing the turning off, of immune responses in cancer patients and for prevention and treatment of autoimmune diseases and inflammatory diseases, as well as rejection of allogenic transplantation, each by turning off uncontrolled immune responses, or by induction of "off signal" by negative costimulation (or coinhibition) in subjects with these pathological conditions.

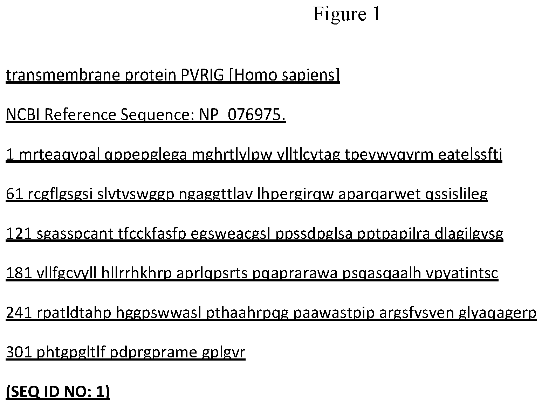

With regard treatment therapies, a particular pathway and target of interest is PVRIG. PVRIG is a transmembrane domain protein of 326 amino acids in length, with a signal peptide (spanning from amino acid 1 to 40), an extracellular domain (spanning from amino acid 41 to 171), a transmembrane domain (spanning from amino acid 172 to 190) and a cytoplasmic domain (spanning from amino acid 191 to 326). The full length human PVRIG protein is shown in Figure 1. There are two methionines that can be start codons, but the mature proteins are identical.

The PVRIG proteins contain an immunoglobulin (Ig) domain within the extracellular domain, which is a PVR-like Ig fold domain. The PVR-like Ig fold domain may be responsible for functional counterpart binding, by analogy to the other B7 family members. The PVR-like Ig fold domain of the extracellular domain includes one disulfide bond formed between intra domain cysteine residues, as is typical for this fold and may be important for structure-function. These cysteines are located at residues 22 and 93 (or 94). In one embodiment, there is provided a soluble fragment of PVRIG that can be used in testing of PVRIG antibodies. Included within the definition of PVRIG proteins are PVRIG ECD fragments, including know ECD fragments such as those described in U.S. Patent No. 9,714, 289.

PVRIG has also been identified as an inhibitory receptor which recognizes CD112 but not CD155, and it may be involved in negative regulation of the anti-tumor functions mediated by DNAM-1. PVRL2 was identified as the ligand for PVRIG, placing PVRIG in the DNAM-1/TIGIT immunoreceptor axis (see, Liang et al., Journal of Clinical Oncology 2017 35:15_suppl, 3074-3074).

Anti-PVRIG antibodies (including antigen-binding fragments) that both bind to PVRIG and prevent activation by PVRL2 have been identified, however, there remains a need in the art to develop biomarkers for determining which patient populations for which anti-PVRIG antibodies might find the most beneficial use in treatment. As such, biomarkers for use in identifying these populations are needed.

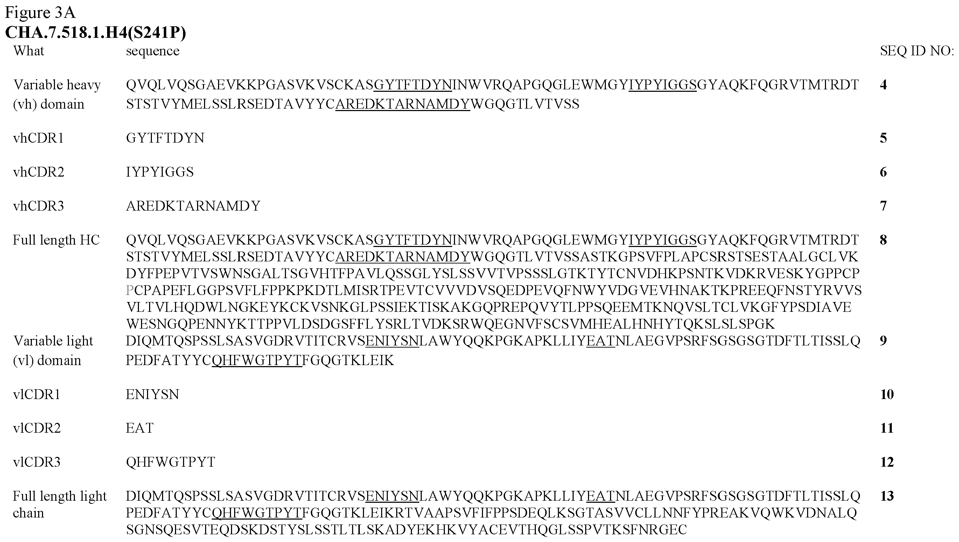

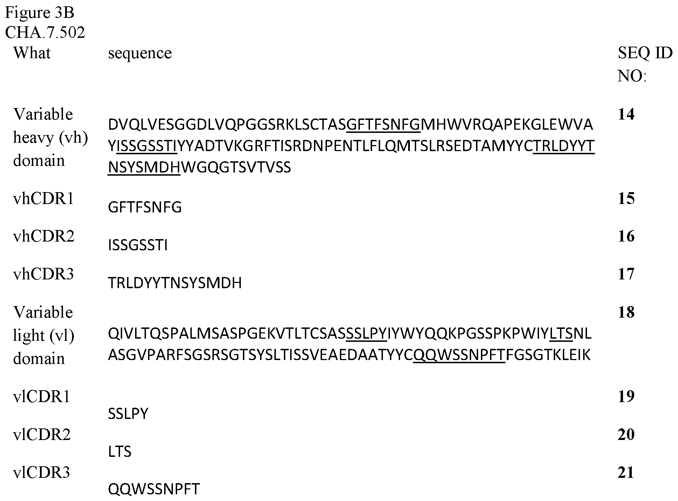

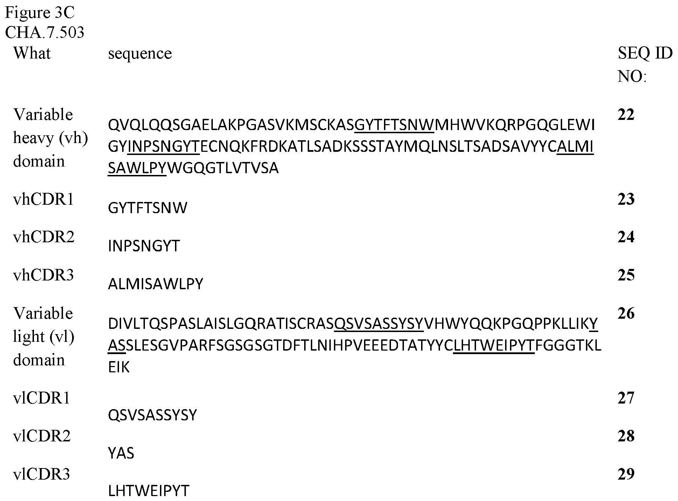

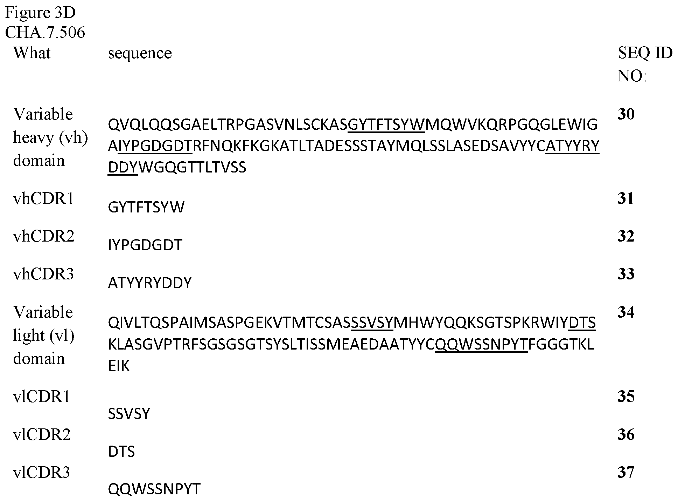

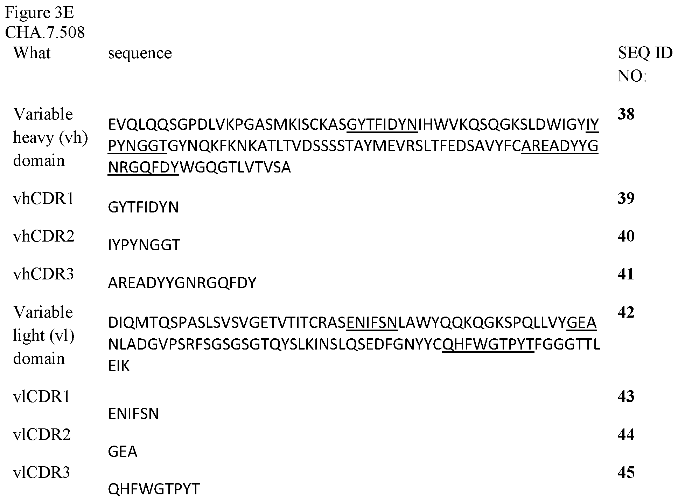

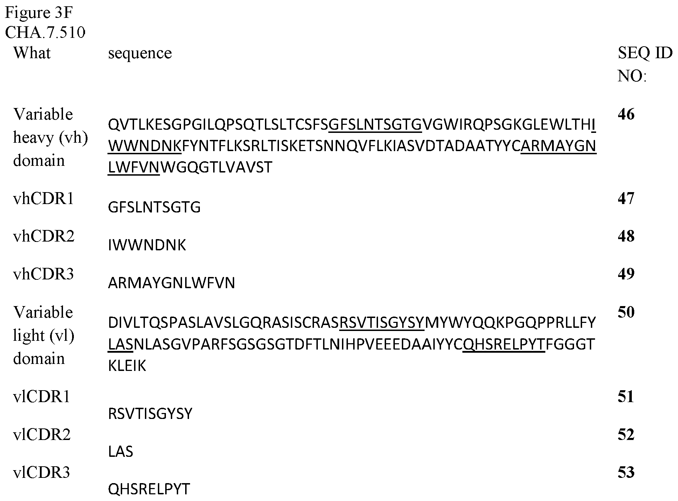

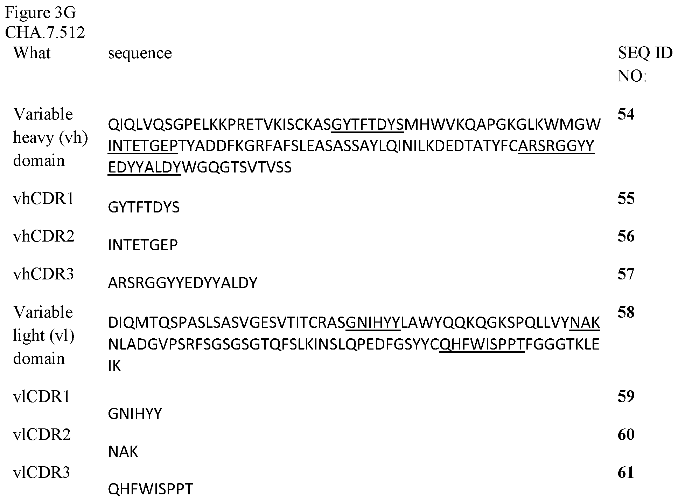

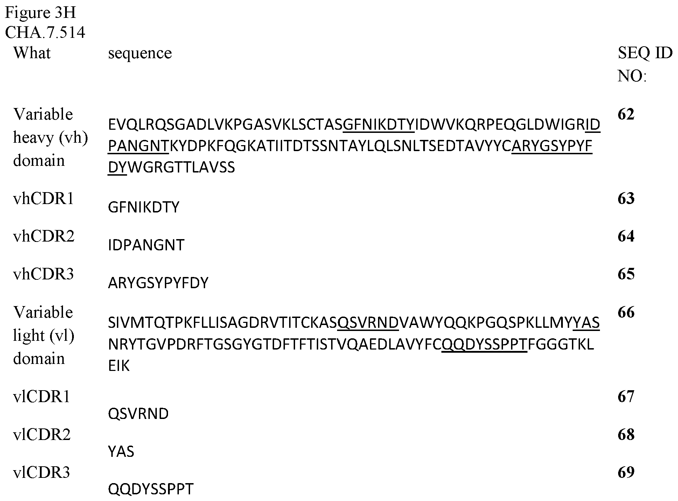

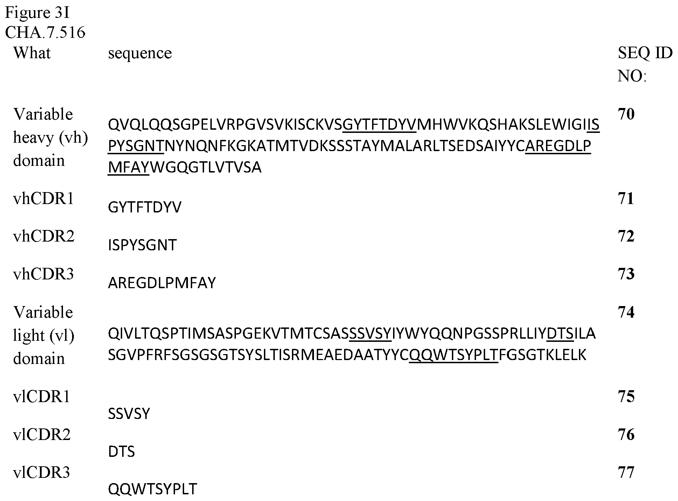

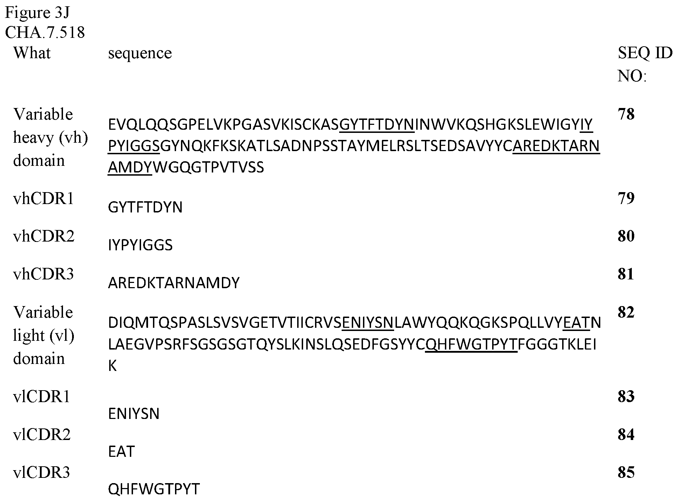

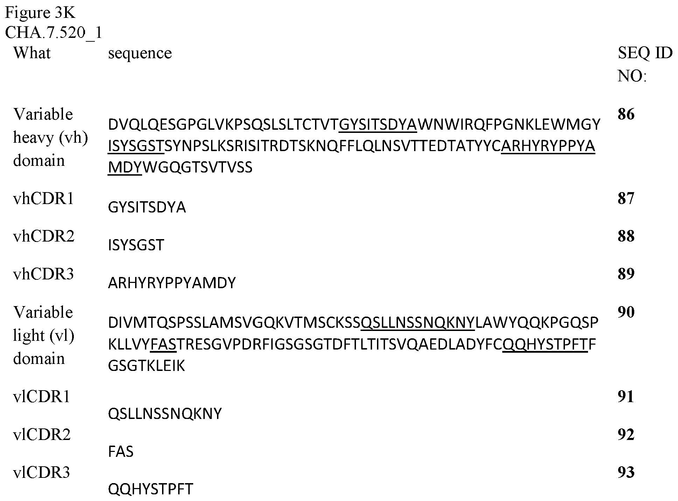

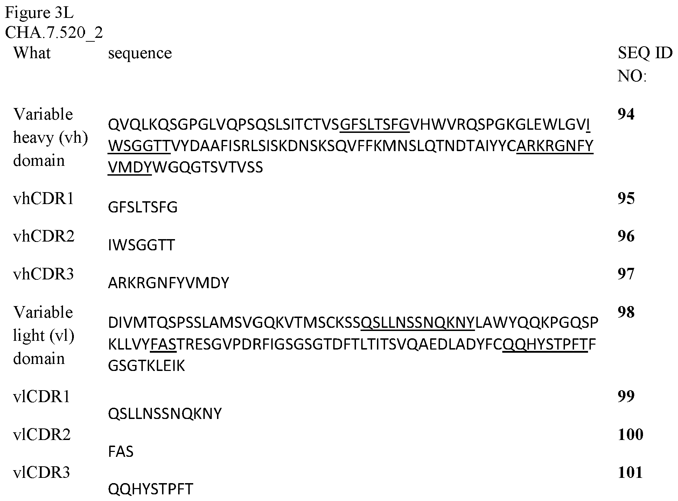

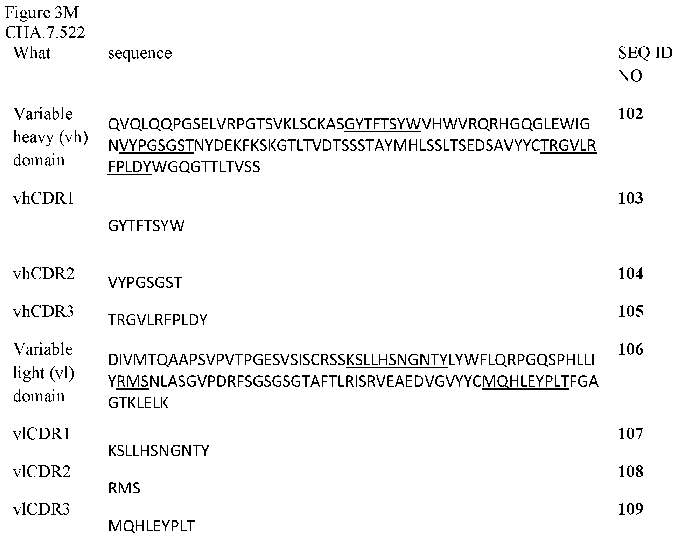

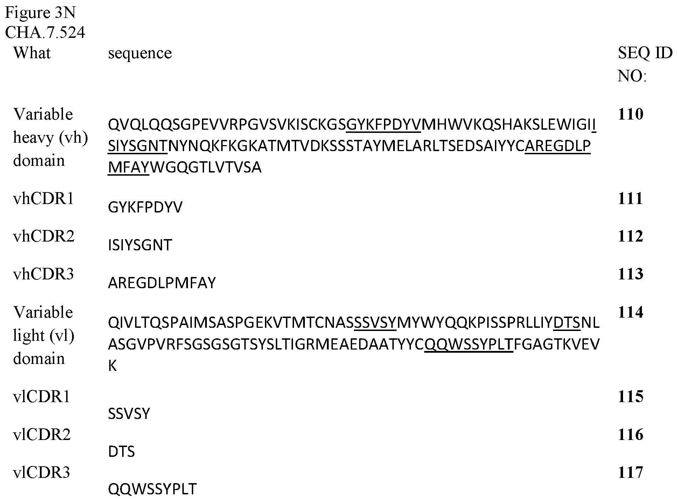

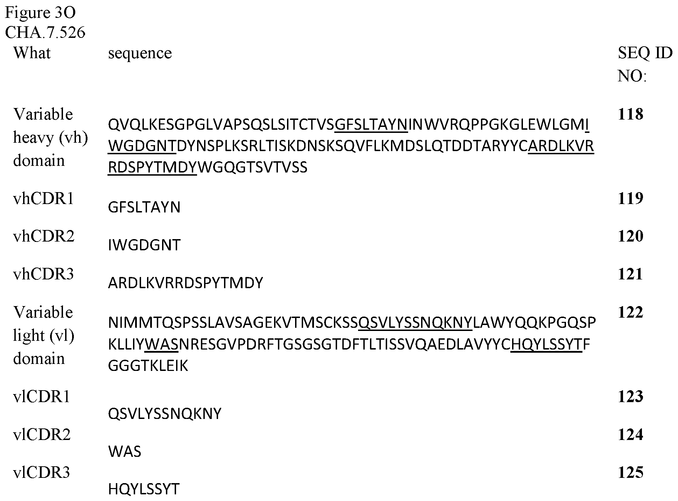

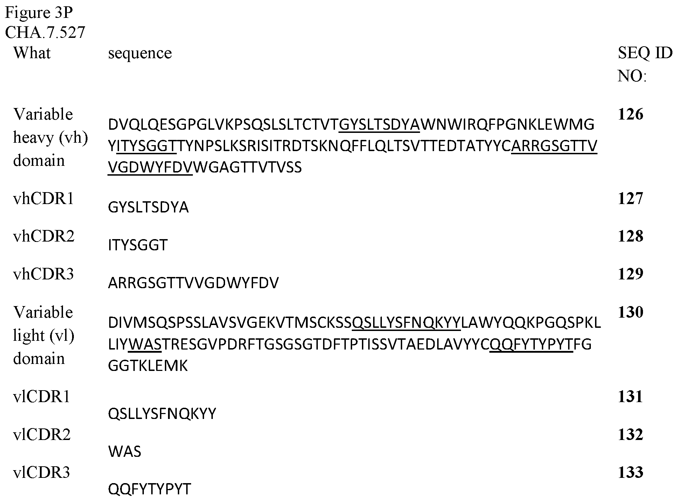

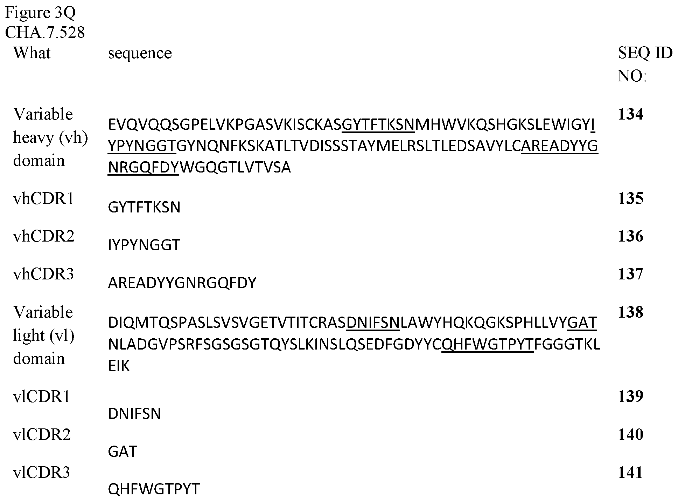

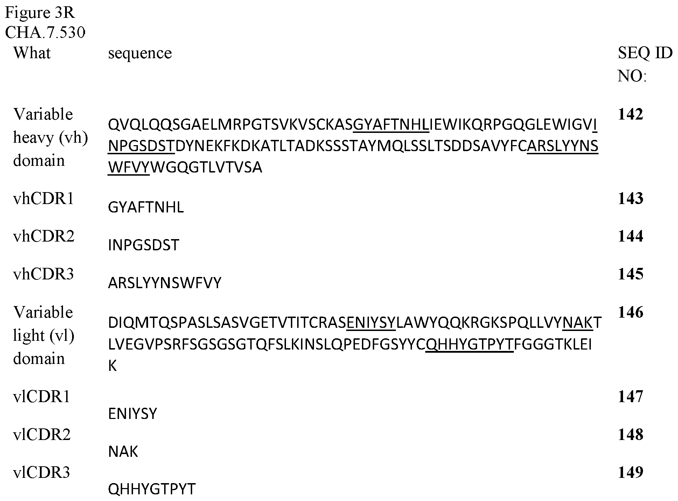

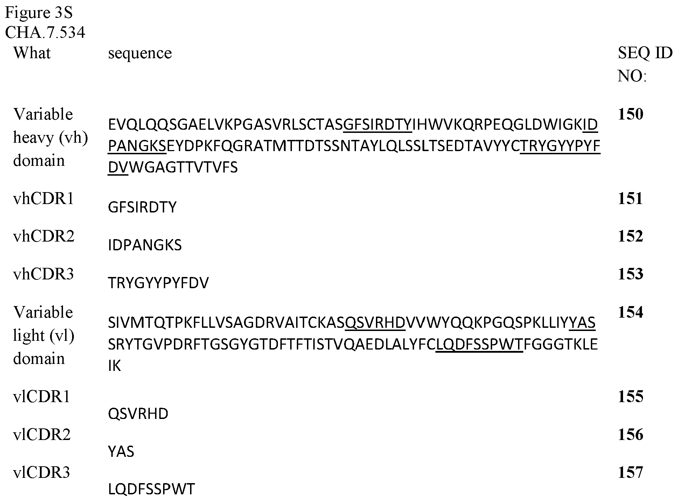

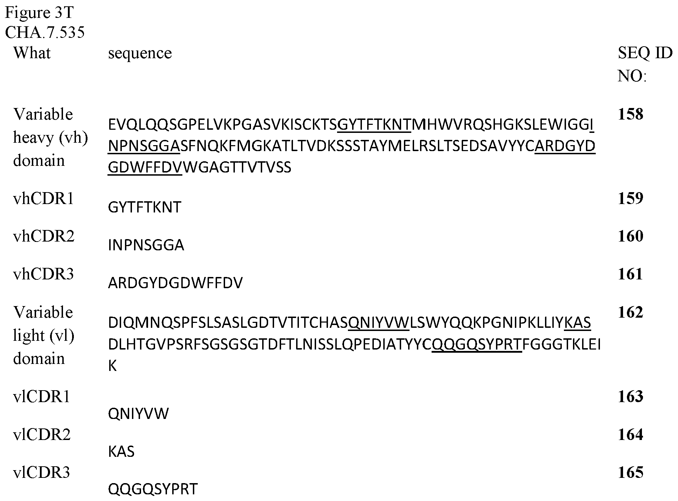

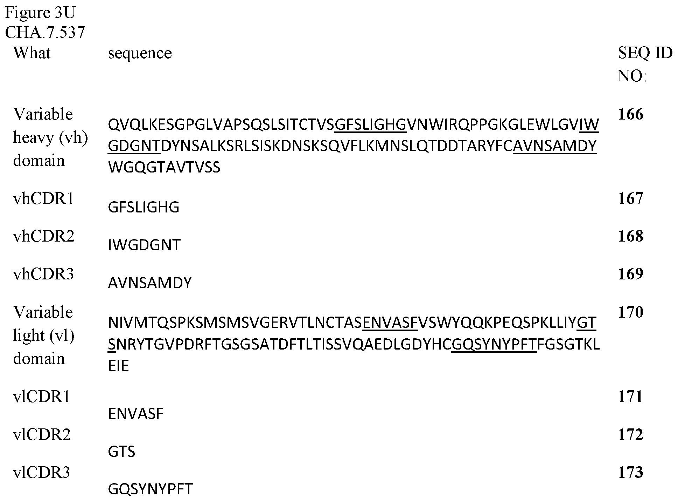

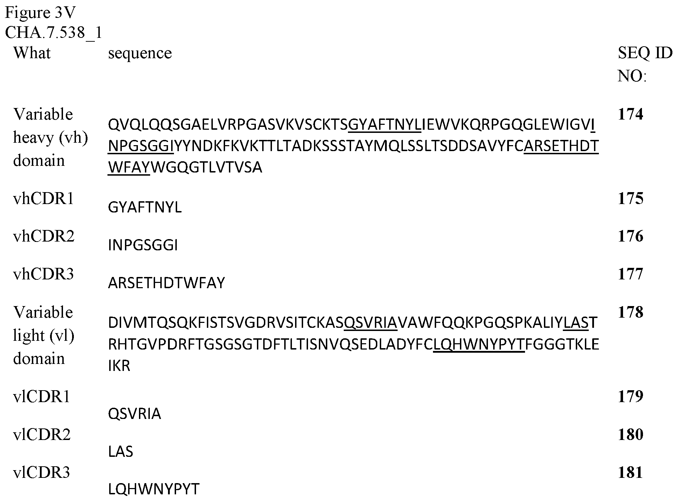

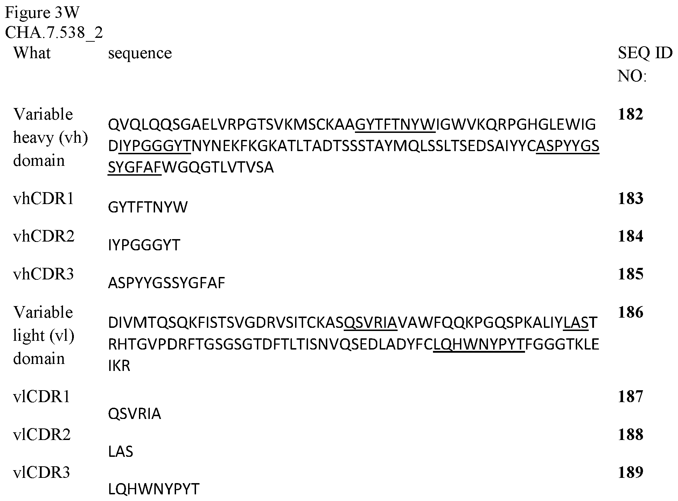

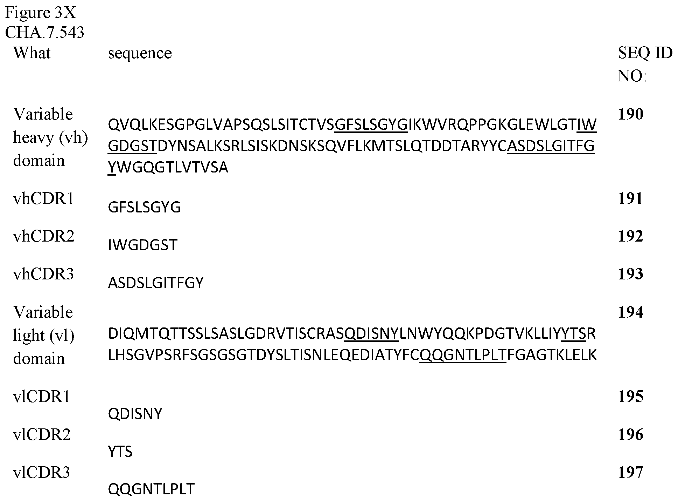

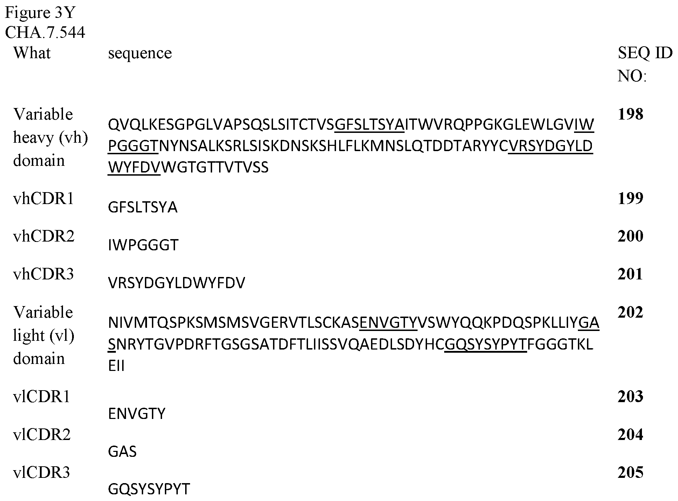

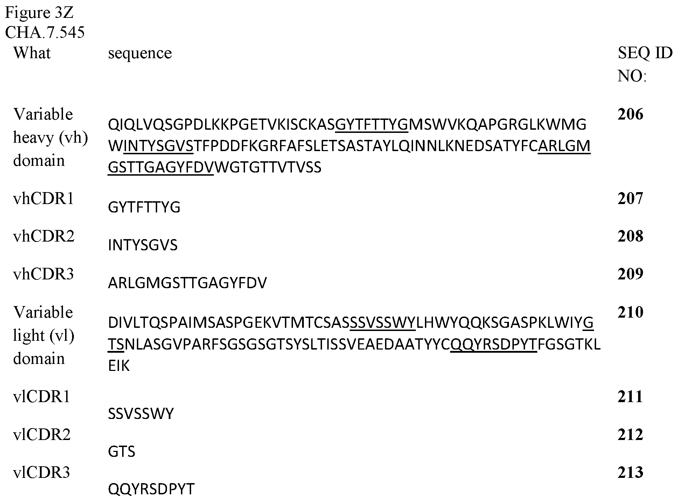

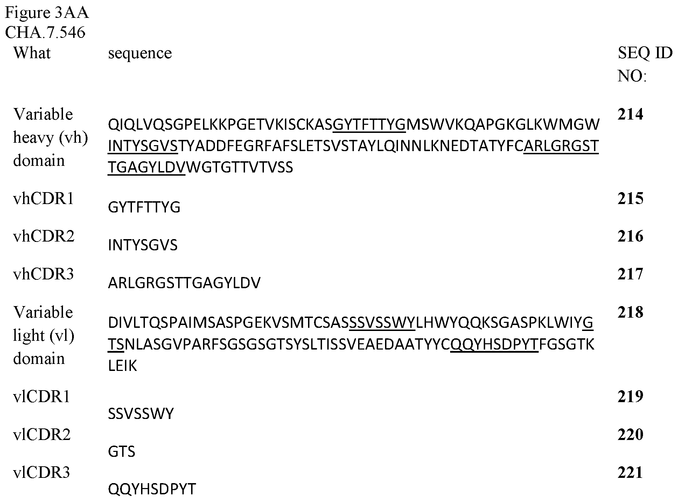

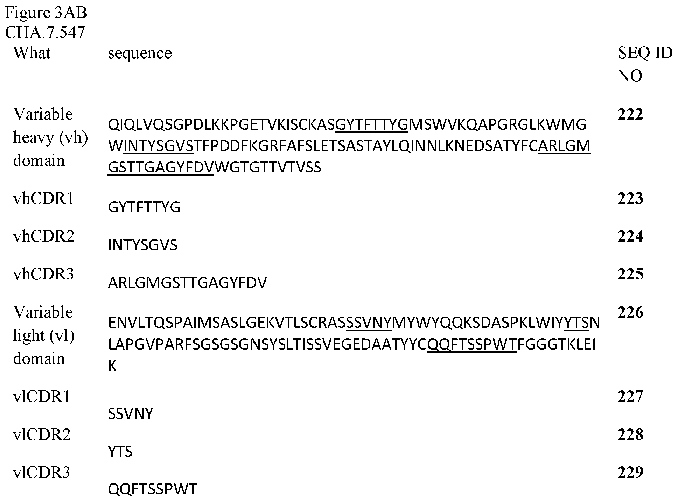

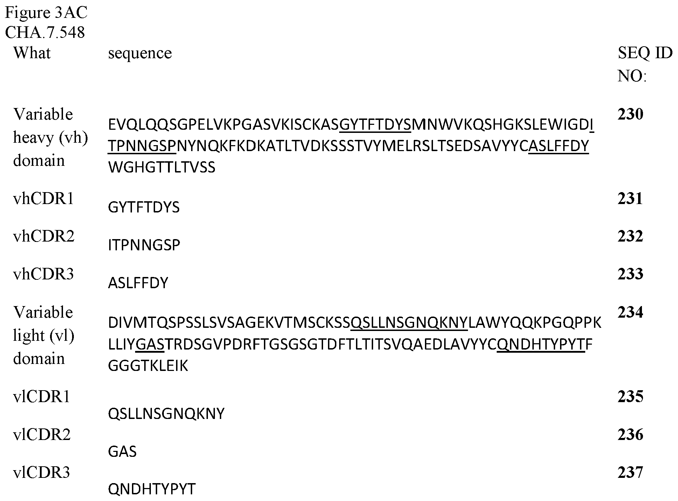

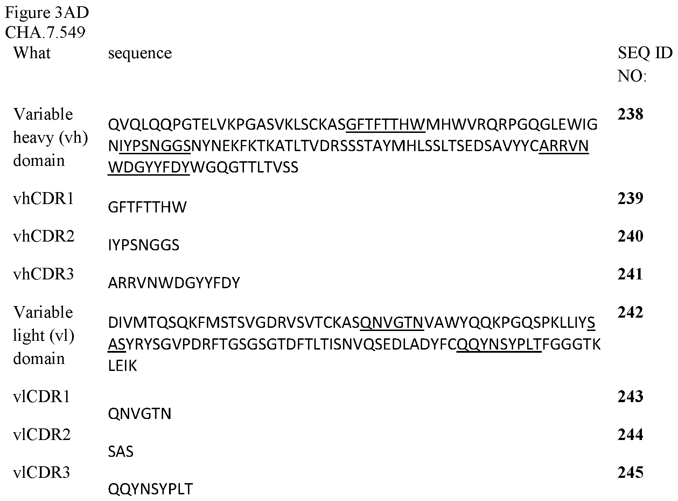

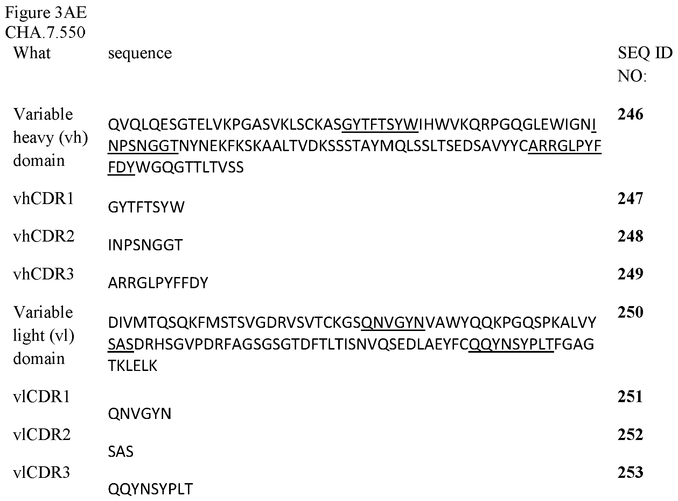

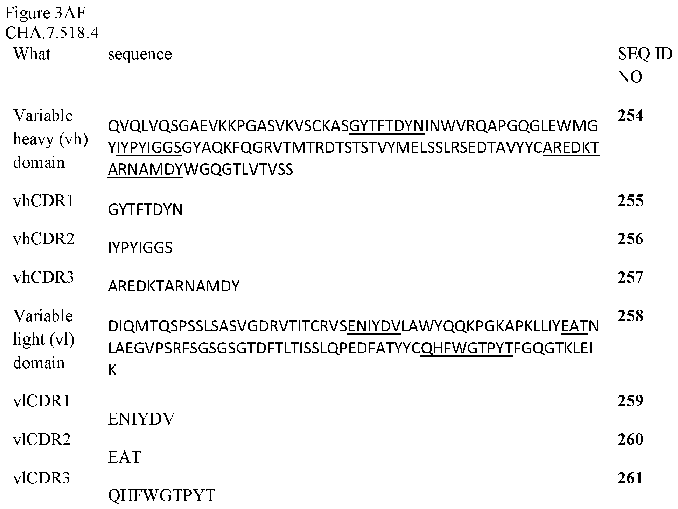

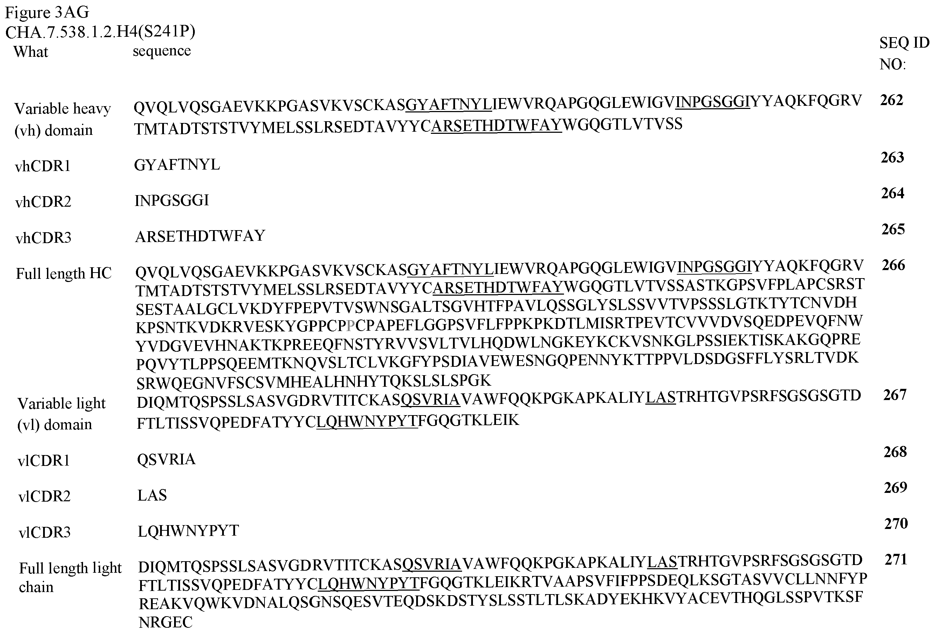

Accordingly, it is an object of the invention to provide biomarkers for use in determining populations for treatment with anti-PVRIG antibodies (e.g., anti-PVRIG antibodies including those with CDRs identical to those shown in Figure 3, also referred to herein as anti-PVRIG treatment antibodies). Such biomarkers include, for example PVRIG and/or PVRL2 expression, as described herein.

### BRIEF SUMMARY OF THE INVENTION

Accordingly, it is an object of the invention to provide biomarkers for use in determining populations for treatment with anti-PVRIG antibodies including and such biomarkers include, for example PVRIG and/or PVRL2 expression.

The present invention provides a method for determining a cancer patient population for treatment with an anti-PVRIG antibody, the method comprising:
(a) detecting the presence in a biological sample from the cancer patient one or more cellular components selected from the group consisting of:
   (i) TSCM, TRM, naive, exhausted, cycling, and effector CD8 positive T cells, that express PVRIG; and/or
   (ii) activated DC cells, DC1, and/or DC2 that express PVRL2,
(b) quantitating the measurement of the level of components selected from the group consisting of:
   (i) TSCM, TRM, naive, exhausted, cycling, and effector CD8 positive T cells, that express PVRIG; and/or
   (ii) activated DC cells, DC1, and/or DC2 that express PVRL2; and
(c) treating the cancer patient with an anti-PVRIG antibody when one or more cellular components in (a) as quantitated in step (b) are present at an increased level as compared to a control or a patient that does not have detectable levels of the cells.

In some embodiments, the present invention provides a method for predicting or determining the efficacy of treatment with an anti-PVRIG treatment antibody, the method comprising:
(a) measuring the level of one or more cellular components selected from the group consisting of:
   (i) TSCM, TRM, naive, exhausted, cycling, and effector CD8 positive T cells that express PVRIG; and/or
   (ii) activated DC cells, DC1, and DC2 that express PVRL2;
(b) quantitating the measurement of the level of one or more cellular components selected from the group consisting of:
   (i) TSCM, TRM, naive, exhausted, cycling, and effector CD8 positive T cells, expressing PVRIG; and/or
   (ii) activated DC cells, DC1, and DC2 that express PVRL2; and
(c) correlating the level of the cellular components with the efficacy of treatment, wherein any of the cellular components being present and/or being present at an increased level as compared to a control or a patient that does not have detectable levels of the cells, is indicative of treatment efficacy for treatment with an anti-PVRIG treatment antibody.

In some embodiments, the method further comprises:
(d) treating the cancer patient with an anti-PVRIG antibody when any of the cellular components in (a) are present at an increased level as compared to a control or a patient that does not have detectable levels of the cells.

The present invention provides a method for predicting or determining the efficacy of treatment or determining a population for treatment with an anti-PVRIG treatment antibody when any of TSCM, TRM, naive, exhausted, cycling, and effector CD8 positive T cells, that express PVRIG comprise at least 0.5%, at least 1%, at least 1.5%, at least 2%, at least 2.5%, at least 3%, at least 3.5%, at least 4%, at least 4.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, of the total CD8 cells in the biological sample is indicative of treatment efficacy and/or indicative for treatment with an anti-PVRIG antibody.

The present invention provides a method for predicting or determining the efficacy of treatment or determining a population for treatment with an anti-PVRIG treatment antibody when any of activated DC cells, DC1, and DC2 that express the PVRL2 comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in the biological sample.

In some embodiments, the presence of and/or an increased level as compared to an untreated control and/or to a control prior to treatment, of one more cellular components, is indicative of anti-PVRIG treatment efficacy:
(i) TSCM, TRM, naive, exhausted, cycling, and effector CD8 positive T cells, that express PVRIG comprise at least 0.5%, at least 1%, at least 1.5%, at least 2%, at least 2.5%, at least 3%, at least 3.5%, at least 4%, at least 4.5%, or at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, out of total CD8 cells in the biological sample; and/or
(ii) DC cells, DC1, and DC2 that express PVRL2 comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in the biological sample.

In some embodiments, the biological sample is obtained from a tumor, tumor microenvironment, and/or peripheral blood from the cancer patient.

The present invention provides a method for determining a cancer patient population for treatment with an anti-PVRIG treatment antibody, the method comprising:
(a) measuring the level of PVRIG and/or PVRL2 expression in a biological sample obtained from a cancer and/or tumor microenvironment and/or peripheral blood in the cancer patient,
(b) quantitating the measurement of the level of PVRIG and/or PVRL2 expression in the biological sample, and
(c) treating the cancer patient with an anti-PVRIG antibody wherein PVRIG and/or PVRL2 is expressed and/or expressed at an increased level as compared to a control or a patient that does not have detectable levels of the PVRIG and/or PVRL2 in the biological sample.

The present invention provides a method for predicting or determining the efficacy of treatment with an anti-PVRIG treatment antibody, the method comprising:
(a) measuring the level of PVRIG and/or PVRL2 expression in a biological sample obtained from a cancer and/or tumor microenvironment and/or peripheral blood in the cancer patient,
(b) quantitating the measurement of the level of PVRIG and/or PVRL2 expression in the biological sample, and
(c) correlating the level of PVRIG and/or PVRL2 with the efficacy of treatment, wherein PVRIG and/or PVRL2 is expressed and/or expressed at an increased level as compared to a control or a patient that does not have detectable levels of the PVRIG and/or PVRL2, is indicative of treatment efficacy for treatment with an anti-PVRIG treatment antibody.

The present invention provides a method for altering the regimen of treatment for a patient with cancer, the method comprising:
(a) measuring the level of PVRIG and/or PVRL2 expression in a biological sample obtained from a cancer and/or tumor microenvironment and/or peripheral blood in the cancer patient,
(b) quantitating the measurement of the level of PVRIG and/or PVRL2 expression in the biological sample, wherein PVRIG and/or PVRL2 being expressed and/or being expressed at an increased level as compared to a control or a patient that does not have detectable levels of the PVRIG and/or PVRL2 is indicative of treatment efficacy,
(c) correlating the level of PVRIG and/or PVRL2 expression with the efficacy of treatment, wherein a high level of PVRIG and/or PVRL2 expression is indicative of altering the treatment regimen for treatment with anti-PVRIG treatment antibody, and
(d) altering the treatment regimen to include an anti-PVRIG antibody when a moderate or high level of PVRIG and/or PVRL2 expression is measured.

In some embodiments, the anti-PVRIG antibody comprises the vhCDR1, vhCDR2, vhCDR3, vlCDR1, vlCDR2, and vlCDR3 sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.520.1, CHA.7.520.2, CHA.7.522, CHA.7.524, CHA.7.526, CHA.7.527, CHA.7.528, CHA.7.530, CHA.7.534, CHA.7.535, CHA.7.537, CHA.7.538.1.2.H4(S241P), CHA.7.538.1, CHA.7.538.2, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.

In some embodiments, the anti-PVRIG antibody comprises the variable heavy domain and the variable light domain sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.518.1, CHA.7.518.2, CHA.7.518.3, CHA.7.518.4, CHA.7.518.5, CHA.7.520.1, CHA.7.520.2, CHA.7.522, CHA.7.524, CHA.7.524.1, CHA.7.524.2, CHA.7.524.3, CHA.7.524.4, CHA.7.526, CHA.7.527, CHA.7.528, CHA.7.530, CHA.7.530.1, CHA.7.530.2, CHA.7.530.3, CHA.7.530.4, CHA.7.530.5, CHA.7.534, CHA.7.535, CHA.7.537, CHA.7.538.1.2.H4(S241P), CHA.7.538.1, CHA.7.538.1.1, CHA.7.538.1.2, CHA.7.538.1.3, CHA.7.538.1.4, CHA.7.538.2, CHA.7.538.2.1, CHA.7.538.2.2, CHA.7.538.2.3, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.

In some embodiments, the anti-PVRIG treatment antibody comprises a heavy chain variable domain from the heavy chain of CHA.7.518.1.H4(S241P) (SEQ ID NO:8) and a light chain variable domain from the light chain of CHA.7.518.1.H4(S241P) (SEQ ID NO:13).

In some embodiments, the anti-PVRIG treatment antibody comprises a heavy chain variable domain from the heavy chain of CHA.7.538.1.2.H4(S241P) (SEQ ID NO:266) and a light chain variable domain from the light chain of CHA.7.538.1.2.H4(S241P) (SEQ ID NO:271).

In some embodiments, the PVRIG and/or PVRL2 expression is determined using single-cell resolution analysis.

In some embodiments, the single-cell resolution analysis includes RNAseq, immunohistochemistry (IHC), multiplex immunohistochemistry (mIHC) and/or immunofluorescence (IF), flow cytometry (e.g., FACS) and mass cytometry (e.g., CyTOF), as well as combinations thereof.

In some embodiments, the PVRIG and/or PVRL2 expression is determined using immunohistochemistry.

In some embodiments, the PVRIG antibody used for the single-cell resolution analysis and/or immunohistochemistry is AB-635 PVRIG Ab (6D8-1 clone).

In some embodiments, the PVRIG antibody used for the single-cell resolution analysis and/or immunohistochemistry is 6D8-1 (heavy chain SEQ ID NO:659 and light chain SEQ ID NO:663).

In some embodiments, the PVRIG antibody used for the single-cell resolution analysis and/or immunohistochemistry comprises:
i) a heavy chain variable domain comprising the vhCDR1, vhCDR2, and vhCDR3 from the heavy chain of 6D8-1 (SEQ ID NO:659), and
ii) a light chain variable domain comprising the vlCDR1, vlCDR2, and vlCDR3 from the light chain of 6D8-1 (SEQ ID NO:663).

In some embodiments, the PVRL2 antibody used for the immunohistochemistry and/or single-cell resolution analysis is CST PVRL2 Ab (Nectin-2/CD112 (D8D3F) XP^{®} Rabbit mAb).

In some embodiments, the PVRL2 expression level is categorized as strong, moderate, weak, or negative.

In some embodiments, the PVRL2 expression is categorized as strong or moderate.

In some embodiments, the PVRL2 expression level is categorized as 0-no signal, 1-low, 2-medium, 3- high.

In some embodiments, the PVRL2 expression is categorized as 2-medium or 3- high.

In some embodiments, the PVRL2 expression is 0% membrane staining there is predicted to be no response or a minimal response to an anti-PVRIG antibody.

In some embodiments, the PVRL2 expression is under 20% tumor membrane staining at +1 staining score there is predicted to be no response or a minimal response to an anti-PVRIG antibody.

In some embodiments, the PVRL2 expression is >20% tumor membrane staining at +1 staining score there is predicted to be a response to an anti-PVRIG antibody.

In some embodiments, the PVRL2 expression is >20% immune infiltrating cells at any intensity membrane or cytoplasmatic staining there is predicted to be a response to an anti-PVRIG antibody.

In some embodiments, the PVRL2 expression is characterized by at least two of the following
i. >20% tumor membrane staining at +1 staining score there is predicted to be no response or a minimal response to an anti-PVRIG antibody,
ii. >20% tumor membrane staining at +1 staining score there is predicted to be a response to an anti-PVRIG antibody, or
iii. >20% immune infiltrating cells at any intensity membrane or cytoplasmatic staining,
there is an increased prediction of a response to an anti-PVRIG antibody.

In some embodiments, the staining score is an IHC score.

In some embodiments, the staining score is an mIHC/IF score.

In some embodiments, a cancer patient having dendritic cells in the tumor, tumor microenvironment, and/or peripheral blood wherein at least 10%, 20%, 30%, 40%, 50%, 60%, 70%, 80%, 90%, or 100% PVRL2 expression exhibits a higher probability to respond the anti-PVRIG antibody treatment as compared to cancer patient with lower PVRL2 expression.

In some embodiments, a cancer patient having activated dendritic cells in the tumor, tumor microenvironment, and/or peripheral blood wherein at least 10%, 20%, 30%, 40%, 50%, 60%, 70%, 80%, 90%, or 100% PVRL2 expression exhibits a higher probability to respond the anti-PVRIG antibody treatment as compared to cancer patient with lower PVRL2 expression.

In some embodiments, a cancer patient having DC1 in the tumor, tumor microenvironment, and/or peripheral blood with at least 5%, 10%, 20%, 30%, 40%, 50%, 60%, 70%, 80%, 90%, or 100% PVRL2 expression exhibits a higher probability to respond the anti-PVRIG antibody treatment as compared to cancer patient with lower PVRL2 expression.

In some embodiments, a cancer patient having DC2 in the tumor, tumor microenvironment, and/or peripheral blood wherein at least 10%, 20%, 30%, 40%, 50%, 60%, 70%, 80%, 90%, or 100% PVRL2 expression exhibits a higher probability to respond the anti-PVRIG antibody treatment as compared to cancer patient with lower PVRL2 expression.

In some embodiments, a cancer patient having at least 10%, 20%, 30%, 40%, 50%, 60%, 70%, 80%, 90%, or 100% of CD8 cells in the tumor, tumor microenvironment, and/or peripheral blood expressing PVRIG exhibits a higher probability to respond to the anti-PVRIG antibody treatment as compared to cancer patient with lower PVRIG expression.

In some embodiments, a cancer patient having at least 10%, 20%, 30%, 40%, 50%, 60%, 70%, 80%, 90%, or 100% of TSCM cells in the tumor, tumor microenvironment, and/or peripheral blood expressing PVRIG exhibits a higher probability to respond to the anti-PVRIG antibody treatment as compared to cancer patient with lower PVRIG expression.

In some embodiments, a cancer patient having at least 10%, 20%, 30%, 40%, 50%, 60%, 70%, 80%, 90%, or 100% of Naïve CD8 cells in the tumor, tumor microenvironment, and/or peripheral blood expressing PVRIG exhibits a higher probability to respond to the anti-PVRIG antibody treatment as compared to cancer patient with lower PVRIG expression.

In some embodiments, a cancer patient having at least 10%, 20%, 30%, 40%, 50%, 60%, 70%, 80%, 90%, or 100% of exhausted CD8 cells in the tumor, tumor microenvironment, and/or peripheral blood expressing PVRIG exhibits a higher probability to respond to the anti-PVRIG antibody treatment as compared to cancer patient with lower PVRIG expression.

In some embodiments, a cancer patient having at least 5%, 10%, 20%, 30%, 40%, 50%, 60%, 70%, 80%, 90%, or 100% of TRM cells in the tumor, tumor microenvironment, and/or peripheral blood expressing PVRIG exhibits a higher probability to respond to the anti-PVRIG antibody treatment as compared to cancer patient with lower PVRIG expression.

In some embodiments, a cancer patient having at least 5%, 10%, 20%, 30%, 40%, 50%, 60%, 70%, 80%, 90%, or 100% of effector cells in the tumor, tumor microenvironment, and/or peripheral blood expressing PVRIG exhibits a higher probability to respond to the anti-PVRIG antibody treatment as compared to cancer patient with lower PVRIG expression.

In some embodiments, a cancer patient having more than 2 copies of PVRL2 in cells obtained from the tumor, tumor microenvironment, and/or peripheral blood exhibits a higher probability to respond to the anti-PVRIG antibody treatment as compared to cancer patient with only 2 copies of PVRL2 in cells obtained from the tumor, tumor microenvironment, and/or peripheral blood.

In some embodiments, the anti-PVRIG treatment antibody is administered as a stable liquid pharmaceutical formulation of the anti-PVRIG antibody comprising:
(a) an anti-PVRIG antibody, wherein the anti-PVRIG antibody comprises:
   i) a heavy chain variable domain comprising the vhCDR1, vhCDR2, and vhCDR3 from the heavy chain of CHA.7.518.1.H4(S241P) (SEQ ID NO:8), and
   ii) a light chain variable domain comprising the vlCDR1, vlCDR2, and vlCDR3 from the light chain of CHA.7.518.1.H4(S241P) (SEQ ID NO:13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the anti-PVRIG treatment antibody comprises a CH1-hinge-CH2-CH3 sequence of IgG4 (SEQ ID NO:657 or SEQ ID NO:658), wherein the hinge region optionally comprises mutations.

In some embodiments, the anti-PVRIG antibody comprises the CH1-hinge-CH2-CH3 region from IgG1, IgG2, IgG3, or IgG4, wherein the hinge region optionally comprises mutations.

In some embodiments, the heavy chain variable domain is from the heavy chain of CHA.7.518.1.H4(S241P) (SEQ ID NO:8) and the light chain variable domain is from the light chain of CHA.7.518.1.H4(S241P) (SEQ ID NO: 13).

In some embodiments, the anti-PVRIG antibody comprises a CL region of human kappa 2 light chain.

In some embodiments, the stable liquid formulation comprises from 10 mM to 80 mM histidine, from 15 mM to 70 mM histidine, from 20 mM to 60 mM histidine, from 20 mM to 50 mM histidine, or from 20 mM to 30 mM histidine.

In some embodiments, the pharmaceutical formulation comprises about 25 mM histidine.

In some embodiments, the pharmaceutical formulation comprises from 30 mM to 100 mM NaCl, from 30 mM to 90 mM NaCl, from 40 mM to 80 mM NaCl, from 30 mM to 70 mM histidine, or from 45 mM to 70 mM NaCl.

In some embodiments, the pharmaceutical formulation comprises about 60 mM NaCl.

In some embodiments, the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine.

In some embodiments, the pharmaceutical formulation comprises about 100 mM L-arginine.

In some embodiments, the pharmaceutical formulation comprises from 0.006% to 0.1% w/v polysorbate 80, from 0.007% to 0.09% w/v polysorbate 80, from 0.008% to 0.08% w/v polysorbate 80, from 0.009% to 0.09% w/v polysorbate 80, from 0.01% to 0.08% w/v polysorbate 80, from 0.01% to 0.07% w/v polysorbate 80, from 0.01% to 0.07% w/v polysorbate 80, or from 0.01% to 0.06% w/v polysorbate 80, or from 0.009% to 0.05% w/v polysorbate 80.

In some embodiments, the pharmaceutical formulation comprises about 0.01% polysorbate 80.

In some embodiments, the pH is from 6 to 7.0.

In some embodiments, the pH is from 6.3 to 6.8.

In some embodiments, the pH is 6.5 +/- 0.2.

In some embodiments, the anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL.

In some embodiments, the anti-PVRIG antibody is at a concentration of about 20 mg/mL.

In some embodiments, the anti-PVRIG antibody formulation comprises:
a) a heavy chain comprising:
   i) a VH-CH1-hinge-CH2-CH3, wherein the VH is from CHA.7.518.1.H4(S241P) (SEQ ID NO:4) and wherein the CH1-hinge-CH2-CH3 region is from IgG4; and
b) a light chain comprising:
   i) a VL-CL, wherein the VL from CHA.7.518.1.H4(S241P) (SEQ ID NO:9) and wherein the CL region is from human kappa 2 light chain.

In some embodiments, the hinge region optionally comprises mutations.

In some embodiments, the hinge region optionally comprises mutations.

In some embodiments, the anti-PVRIG antibody formulation comprises:
i) a heavy chain comprising the heavy chain from CHA.7.518.1.H4(S241P) (SEQ ID NO:8); and
ii) a light chain comprising the light chain from CHA.7.518.1.H4(S241P) (SEQ ID NO: 13).

In some embodiments, the anti-PVRIG antibody formulation comprising:
(a) an anti-PVRIG antibody, wherein the anti-PVRIG antibody comprises:
   i) a heavy chain variable domain comprising the vhCDR1, vhCDR2, and vhCDR3 from the heavy chain of CHA.7.518.1.H4(S241P) (SEQ ID NO:8), and
   ii) a light chain variable domain comprising the vlCDR1, vlCDR2, and vlCDR3 from the light chain of CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the anti-PVRIG antibody formulation comprising:
(a) an anti-PVRIG antibody, wherein the anti-PVRIG antibody comprises:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the anti-PVRIG treatment antibody is administered at a dosage of about 0.01 mg/kg to about 20 mg/kg of the anti-PVRIG antibody or about 0.01 mg/kg to about 10 mg/kg of the anti-PVRIG antibody.

In some embodiments, the anti-PVRIG treatment antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg of the anti-PVRIG antibody.

In some embodiments, the anti-PVRIG treatment antibody is administered 20 mg/kg every 4 weeks.

In some embodiments, the cancer selected from the group consisting of prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, and/or fallopian tube cancer.

In some embodiments, the anti-PVRIG antibody is administered in combination with an anti-PD-1 antibody.

In some embodiments, the anti-PVRIG antibody is administered in combination with an anti-PD-L1 antibody.

In some embodiments, the anti-PVRIG antibody is administered in combination with an anti-TIGIT antibody.

In some embodiments, the anti-PVRIG antibody is administered in combination with an anti-PD-1 antibody and an anti-TIGIT antibody.

In some embodiments, the anti-PVRIG antibody is administered in combination with an anti-PD-L1 antibody and an anti-TIGIT antibody.

In some embodiments, the anti-PD1 antibody is selected from the group consisting of nivolumab and pembrolizumab.

In some embodiments, the anti-PD-L1 antibody is selected from the group consisting of atezolizumab, avelumab, durvalumab, KN035(Envafolimab), and CK-301 (Cosibelimab).

In some embodiments, the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.

In some embodiments, the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.

In some embodiments, the anti-TIGIT antibody is selected from the group consisting of CPA.9.083.H4(S241P) and CPA.9.086.H4(S241P).

In some embodiments, the anti-TIGIT antibody comprises:
a) a heavy chain comprising VH-CH1-hinge-CH2-CH3; and
b) a light chain comprising VL-VC, wherein VC is either kappa or lambda.

In some embodiments, the CH1-hinge-CH2-CH3 is selected from human IgG1, IgG2 and IgG4, and variants thereof.

In some embodiments, the anti-PVRIG antibody is CHA.7.518.1.H4(S241P), the anti-TIGIT antibody is CPA.9.086.H4(S241P) and the anti-PD-1 antibody is nivolumab.

In some embodiments, the anti-PVRIG antibody is CHA.7.518.1.H4(S241P), the anti-TIGIT antibody is CPA.9.083.H4(S241P) and the anti-PD-1 antibody is nivolumab.

In some embodiments, the anti-PVRIG antibody is CHA.7.518.1.H4(S241P), the anti-TIGIT antibody is CPA.9.086.H4(S241P) and the anti-PD-1 antibody is pembrolizumab.

In some embodiments, the anti-PVRIG antibody is CHA.7.518.1.H4(S241P), the anti-TIGIT antibody is CPA.9.083.H4(S241P) and the anti-PD-1 antibody is pembrolizumab.

In some embodiments, the anti-PVRIG antibody is CHA.7.538.1.2.H4(S241P), the anti-TIGIT antibody is CPA.9.086.H4(S241P) and the anti-PD-1 antibody is nivolumab.

In some embodiments, the anti-PVRIG antibody is CHA.7.538.1.2.H4(S241P), the anti-TIGIT antibody is CPA.9.083.H4(S241P) and the anti-PD-1 antibody is nivolumab.

In some embodiments, the anti-PVRIG antibody is CHA.7.538.1.2.H4(S241P), the anti-TIGIT antibody is CPA.9.086.H4(S241P) and the anti-PD-1 antibody is pembrolizumab.

In some embodiments, the anti-PVRIG antibody is CHA.7.538.1.2.H4(S241P), the anti-TIGIT antibody is CPA.9.083.H4(S241P) and the anti-PD-1 antibody is pembrolizumab.

In some embodiments, the anti-PVRIG antibody is CHA.7.518.1.H4(S241P), the anti-TIGIT antibody is CPA.9.086.H4(S241P) and the anti-PD-L1 antibody is atezolizumab.

In some embodiments, the anti-PVRIG antibody is CHA.7.518.1.H4(S241P), the anti-TIGIT antibody is CPA.9.086.H4(S241P) and the anti-PD-L1 antibody is avelumab.

In some embodiments, the anti-PVRIG antibody is CHA.7.518.1.H4(S241P), the anti-TIGIT antibody is CPA.9.086.H4(S241P) and the anti-PD-L1 antibody is durvalumab.

In some embodiments, the anti-PVRIG antibody is CHA.7.518.1.H4(S241P), the anti-TIGIT antibody is CPA.9.086.H4(S241P) and the anti-PD-L1 antibody is KN035(Envafolimab).

In some embodiments, the anti-PVRIG antibody is CHA.7.518.1.H4(S241P), the anti-TIGIT antibody is CPA.9.086.H4(S241P) and the anti-PD-L1 antibody is CK-301 (Cosibelimab).

In some embodiments, the anti-PVRIG antibody is CHA.7.538.1.2.H4(S241P), the anti-TIGIT antibody is CPA.9.086.H4(S241P) and the anti-PD-L1 antibody is atezolizumab.

In some embodiments, the anti-PVRIG antibody is CHA.7.538.1.2.H4(S241P), the anti-TIGIT antibody is CPA.9.086.H4(S241P) and the anti-PD-L1 antibody is avelumab.

In some embodiments, the anti-PVRIG antibody is CHA.7.538.1.2.H4(S241P), the anti-TIGIT antibody is CPA.9.086.H4(S241P) and the anti-PD-L1 antibody is durvalumab.

In some embodiments, the anti-PVRIG antibody is CHA.7.538.1.2.H4(S241P), the anti-TIGIT antibody is CPA.9.086.H4(S241P) and the anti-PD-L1 antibody is KN035(Envafolimab).

In some embodiments, the anti-PVRIG antibody is CHA.7.538.1.2.H4(S241P), the anti-TIGIT antibody is CPA.9.086.H4(S241P) and the anti-PD-L1 antibody is CK-301 (Cosibelimab).

In some embodiments, the anti-PVRIG antibody is CHA.7.518.1.H4(S241P), the anti-TIGIT antibody is CPA.9.083.H4(S241P) and the anti-PD-L1 antibody is atezolizumab.

In some embodiments, the anti-PVRIG antibody is CHA.7.518.1.H4(S241P), the anti-TIGIT antibody is CPA.9.083.H4(S241P) and the anti-PD-L1 antibody is avelumab.

In some embodiments, the anti-PVRIG antibody is CHA.7.518.1.H4(S241P), the anti-TIGIT antibody is CPA.9.083.H4(S241P) and the anti-PD-L1 antibody is durvalumab.

In some embodiments, the anti-PVRIG antibody is CHA.7.518.1.H4(S241P), the anti-TIGIT antibody is CPA.9.083.H4(S241P) and the anti-PD-L1 antibody is KN035(Envafolimab).

In some embodiments, the anti-PVRIG antibody is CHA.7.518.1.H4(S241P), the anti-TIGIT antibody is CPA.9.083.H4(S241P) and the anti-PD-L1 antibody is CK-301 (Cosibelimab).

In some embodiments, the anti-PVRIG antibody is CHA.7.538.1.2.H4(S241P), the anti-TIGIT antibody is CPA.9.083.H4(S241P) and the anti-PD-L1 antibody is atezolizumab.

In some embodiments, the anti-PVRIG antibody is CHA.7.538.1.2.H4(S241P), the anti-TIGIT antibody is CPA.9.083.H4(S241P) and the anti-PD-L1 antibody is avelumab.

In some embodiments, the anti-PVRIG antibody is CHA.7.538.1.2.H4(S241P), the anti-TIGIT antibody is CPA.9.083.H4(S241P) and the anti-PD-L1 antibody is durvalumab.

In some embodiments, the anti-PVRIG antibody is CHA.7.538.1.2.H4(S241P), the anti-TIGIT antibody is CPA.9.083.H4(S241P) and the anti-PD-L1 antibody is KN035(Envafolimab).

In some embodiments, the anti-PVRIG antibody is CHA.7.538.1.2.H4(S241P), the anti-TIGIT antibody is CPA.9.083.H4(S241P) and the anti-PD-L1 antibody is CK-301 (Cosibelimab).

The present invention also provides an agent for use in a method, including a method of treatment, wherein the agent is capable of blocking the interaction between PVRIG and PVRL2.

In some embodiments, the agent capable of blocking the interaction between PVRIG and PVRL2 comprises an anti-PVRIG antibody.

In some embodiments, the agent capable of blocking the interaction between PVRIG and PVRL2 increases the interaction between PVRL2 with DNAM-1 by:
i) reducing the competition between PVRIG and DNAM-1 for binding to PVRL2 by between about 1-fold to about 10-fold.

In some embodiments, the blocking reduces the competition between PVRIG and DNAM-1 for binding to PVRL2 and/or increases the interaction between PVRL2 and DNAM-1, as compared to a control or a patient that is not treated with the agent capable of blocking the interaction between PVRIG and PVRL2.

The present invention also provides for a method for determining a cancer patient population for treatment with an anti-PVRIG antibody, the method comprising:
(d) detecting the presence of DNAM-1 and/or PVRIG in a biological sample from the cancer patient;
(e) quantitating the measurement of the level of DNAM-1 and/or PVRIG; and
(f) treating the cancer patient with an anti-PVRIG antibody when PVRIG in (a) as quantitated in step (b) are present and/or present at an increased level as compared to a control or a patient that does not have detectable levels of DNAM-1 and/or PVRIG.

The present invention also provides for a method for predicting or determining the efficacy of treatment for a cancer patient with an anti-PVRIG treatment antibody, the method comprising:
(a) measuring the level of DNAM-1 and/or PVRIG;
(b) quantitating the measurement of the level of DNAM-1 and/or PVRIG; and
(c) correlating the level of the DNAM-1 and/or PVRIG with the efficacy of treatment, wherein any of DNAM-1 and/or PVRIG being present and/or being present at an increased level as compared to a control or a patient that does not have detectable levels of DNAM-1 and/or PVRIG, is indicative of treatment efficacy for treatment with an anti-PVRIG treatment antibody.

In some embodiments, the method further comprises:
(d) treating the cancer patient with an anti-PVRIG antibody when DNAM-1 and/or PVRIG in (a) are present at an increased level as compared to a control or a patient that does not have detectable levels of the cells.

In some embodiments, the biological sample comprises immune cells.

In some embodiments, the immune cells are selected from the group consisting of NK cells, NKT cells, gamma-delta T cells, T cells, CD4 positive T cells, and CD8 positive T cells.

In some embodiments, the immune cells are NK cells, NKT cells, and/or gamma-delta T cells.

In some embodiments, the immune cells are selected from the group consisting of CD4 positive T cells and CD8 positive T cells.

In some embodiments, the T cells are selected from the group consisting of TSCM, TRM, naive, exhausted, cycling, memory, effector CD8 positive cells, and effector CD4 positive T cells.

In some embodiments, the immune cells co-express PVRIG and DNAM-1.

The present invention also provides for a method for predicting or determining the efficacy of treatment or determining a population for treatment with an anti-PVRIG treatment antibody when the immune cells that express DNAM-1 and/or PVRIG comprise at least 0.1%, at least 0.5%, at least 1%, at least 1.5%, at least 2%, at least 2.5%, at least 3%, at least 3.5%, at least 4%, at least 4.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85% of the total immune cells in the biological sample is indicative of treatment efficacy and/or indicative for treatment with an anti-PVRIG antibody.

The present invention also provides for a method for predicting or determining the efficacy of treatment or determining a population for treatment with an anti-PVRIG treatment antibody when the T cells, NK cells, and NKT cells in the immune cells that express DNAM-1 and/or PVRIG comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total T cells, NK cells, and NKT cells in the biological sample is indicative of treatment efficacy and/or indicative for treatment with an anti-PVRIG antibody.

In some embodiments, the presence of and/or an increased level as compared to an untreated control and/or to a control prior to treatment of DNAM-1 and/or PVRIG, is indicative and/or predictive of anti-PVRIG treatment efficacy.

In some embodiments, the biological sample is obtained from a tumor, tumor microenvironment, and/or peripheral blood from the cancer patient.

The present invention also provides for a method for determining a cancer patient population for treatment with an anti-PVRIG treatment antibody, the method comprising:
(a) measuring the level of PVRIG and/or DNAM-1 expression in a biological sample obtained from a cancer and/or tumor microenvironment and/or peripheral blood in the cancer patient,
(b) quantitating the measurement of the level of PVRIG and/or DNAM-1 expression in the biological sample, and
(c) treating the cancer patient with an anti-PVRIG antibody wherein PVRIG and/or DNAM-1 is expressed and/or expressed at an increased level as compared to a control or a patient that does not have detectable levels of the PVRIG and/or DNAM-1 in the biological sample.

The present invention also provides for a method for predicting or determining the efficacy of treatment for a cancer patient with an anti-PVRIG treatment antibody, the method comprising:
(a) measuring the level of PVRIG and/or DNAM-1 expression in a biological sample obtained from a cancer and/or tumor microenvironment and/or peripheral blood in the cancer patient,
(b) quantitating the measurement of the level of PVRIG and/or DNAM-1 expression in the biological sample, and
(c) correlating the level of PVRIG and/or DNAM-1 with the efficacy of treatment, wherein PVRIG and/or DNAM-1 is expressed and/or expressed at an increased level as compared to a control or a patient that does not have detectable levels of the PVRIG and/or DNAM-1, is indicative of treatment efficacy for treatment with an anti-PVRIG treatment antibody.

The present invention also provides for a method for altering the regimen of treatment for a patient with cancer, the method comprising:
(a) measuring the level of PVRIG and/or DNAM-1 expression in a biological sample obtained from a cancer and/or tumor microenvironment and/or peripheral blood in the cancer patient,
(b) quantitating the measurement of the level of PVRIG and/or DNAM-1 expression in the biological sample, wherein PVRIG and/or DNAM-1 being expressed and/or being expressed at an increased level as compared to a control or a patient that does not have detectable levels of the PVRIG and/or DNAM-1 is indicative of treatment efficacy,
(c) correlating the level of PVRIG and/or DNAM-1 expression with the efficacy of treatment, wherein a high level of PVRIG and/or DNAM-1 expression is indicative of altering the treatment regimen for treatment with anti-PVRIG treatment antibody, and
(d) altering the treatment regimen to include an anti-PVRIG antibody when a moderate or high level of PVRIG and/or DNAM-1 expression is measured.

In some embodiments, the anti-PVRIG antibody comprises the vhCDR1, vhCDR2, vhCDR3, vlCDR1, vlCDR2, and vlCDR3 sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.520.1, CHA.7.520.2, CHA.7.522, CHA.7.524, CHA.7.526, CHA.7.527, CHA.7.528, CHA.7.530, CHA.7.534, CHA.7.535, CHA.7.537, CHA.7.538.1.2.H4(S241P), CHA.7.538.1, CHA.7.538.2, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.

In some embodiments, the anti-PVRIG antibody comprises the variable heavy domain and the variable light domain sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.518.1, CHA.7.518.2, CHA.7.518.3, CHA.7.518.4, CHA.7.518.5, CHA.7.520.1, CHA.7.520.2, CHA.7.522, CHA.7.524, CHA.7.524.1, CHA.7.524.2, CHA.7.524.3, CHA.7.524.4, CHA.7.526, CHA.7.527, CHA.7.528, CHA.7.530, CHA.7.530.1, CHA.7.530.2, CHA.7.530.3, CHA.7.530.4, CHA.7.530.5, CHA.7.534, CHA.7.535, CHA.7.537, CHA.7.538.1.2.H4(S241P), CHA.7.538.1, CHA.7.538.1.1, CHA.7.538.1.2, CHA.7.538.1.3, CHA.7.538.1.4, CHA.7.538.2, CHA.7.538.2.1, CHA.7.538.2.2, CHA.7.538.2.3, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.

In some embodiments, the anti-PVRIG treatment antibody comprises a heavy chain variable domain from the heavy chain of CHA.7.518.1.H4(S241P) (SEQ ID NO:8) and a light chain variable domain from the light chain of CHA.7.518.1.H4(S241P) (SEQ ID NO:13).

In some embodiments, the anti-PVRIG treatment antibody comprises a heavy chain variable domain from the heavy chain of CHA.7.538.1.2.H4(S241P) (SEQ ID NO:266) and a light chain variable domain from the light chain of CHA.7.538.1.2.H4(S241P) (SEQ ID NO:271).

In some embodiments, the PVRIG and/or DNAM-1 expression is determined using single-cell resolution analysis.

In some embodiments, the single-cell resolution analysis includes RNAseq, immunohistochemistry (IHC), multiplex immunohistochemistry (mIHC) and/or immunofluorescence (IF), flow cytometry (e.g., FACS) and mass cytometry (e.g., CyTOF), as well as combinations thereof.

In some embodiments, the PVRIG and/or DNAM-1 expression is determined using immunohistochemistry.

In some embodiments, the anti-PVRIG antibody is used for the single-cell resolution analysis and/or immunohistochemistry, and wherein the anti-PVRIG antibody is AB-635 PVRIG Ab (6D8-1 clone).

In some embodiments, the anti-PVRIG antibody is used for the single-cell resolution analysis and/or immunohistochemistry, and wherein the anti-PVRIG antibody is 6D8-1 (heavy chain SEQ ID NO:659 and light chain SEQ ID NO:663).

In some embodiments, the anti-PVRIG antibody is used for the single-cell resolution analysis and/or immunohistochemistry, and wherein the PVRIG antibody used for the single-cell resolution analysis and/or immunohistochemistry comprises:
i) a heavy chain variable domain comprising the vhCDR1, vhCDR2, and vhCDR3 from the heavy chain of 6D8-1 (SEQ ID NO:659), and
ii) a light chain variable domain comprising the vlCDR1, vlCDR2, and vlCDR3 from the light chain of 6D8-1 (SEQ ID NO:663).

In some embodiments, the anti-DNAM-1 antibody is used for the single-cell resolution analysis and/or immunohistochemistry, and wherein the DNAM-1 antibody used for the immunohistochemistry analysis is selected from the group consisting of DNAM-1/CD226 (E8L9G) XP^{®} Rabbit mAb #66631, Mouse Anti-CD226 Recombinant Antibody (clone MM0248-1X20), Cluster of Differentiation 226 (CD226) Antibody abx171784, and Recombinant Anti-CD226 antibody [EPR20710] (ab212077).

In some embodiments, the DNAM-1 expression level is categorized as strong, moderate, weak, or negative.

In some embodiments, the DNAM-1 expression is categorized as strong or moderate.

In some embodiments, the DNAM-1 expression level is categorized as 0-no signal, 1-low, 2-medium, 3- high.

In some embodiments, the DNAM-1 expression is categorized as 2-medium or 3-high.

In some embodiments, the cancer patient having immune cells in the tumor, tumor microenvironment, and/or peripheral blood having at least 3%, 5%, 10%, 20%, 30%, 40%, 50%, 60%, 70%, 80%, 90%, or 100% DNAM-1 expression exhibits a higher probability to respond the anti-PVRIG antibody treatment as compared to a cancer patient with lower DNAM-1 expression.

In some embodiments, the cancer patient having CD4 positive T cells and/or CD8 positive T cells in the tumor, tumor microenvironment, and/or peripheral blood having at least 3%, 5%, 10%, 20%, 30%, 40%, 50%, 60%, 70%, 80%, 90%, or 100% DNAM-1 expression exhibits a higher probability to respond the anti-PVRIG antibody treatment as compared to a cancer patient with lower DNAM-1 expression

In some embodiments, the cancer patient having at least 3%, 5%, 10%, 20%, 30%, 40%, 50%, 60%, 70%, 80%, 90%, or 100% of CD4 positive T cells and/or CD8 positive T cells in the tumor, tumor microenvironment, and/or peripheral blood expressing PVRIG and DNAM-1 exhibits a higher probability to respond to the anti-PVRIG antibody treatment as compared to a cancer patient with lower PVRIG and/or DNAM-1 expression.

In some embodiments, the cancer patient having at least 3%, 5%, 10%, 20%, 30%, 40%, 50%, 60%, 70%, 80%, 90%, or 100% of effector CD8 and/or CD4 positive T cells in the tumor, tumor microenvironment, and/or peripheral blood expressing PVRIG and DNAM-1 exhibits a higher probability to respond to the anti-PVRIG antibody treatment as compared to cancer patient with lower PVRIG and/or DNAM-1 expression.

In some embodiments, the cancer patient having more than 2 copies of DNAM-1 in cells obtained from the tumor, tumor microenvironment, and/or peripheral blood exhibits a higher probability to respond to the anti-PVRIG antibody treatment as compared to cancer patient with only 2 copies of DNAM-1 in cells obtained from the tumor, tumor microenvironment, and/or peripheral blood.

In some embodiments, the immune cells are NK cells, NKT cells, and/or gamma-delta T cells.

In some embodiments, the immune cells are CD4 positive T cells and/or CD8 positive T cells.

In some embodiments, the T cells are selected from the group consisting of TSCM, TRM, naive, exhausted, cycling, memory, effector CD8 positive T cells, and effector CD4 positive T cells.

In some embodiments, the anti-PVRIG treatment antibody is administered as a stable liquid pharmaceutical formulation of the anti-PVRIG antibody comprising:
(a) an anti-PVRIG antibody, wherein the anti-PVRIG antibody comprises:
   i) a heavy chain variable domain comprising the vhCDR1, vhCDR2, and vhCDR3 from the heavy chain of CHA.7.518.1.H4(S241P) (SEQ ID NO:8), and
   ii) a light chain variable domain comprising the vlCDR1, vlCDR2, and vlCDR3 from the light chain of CHA.7.518.1.H4(S241P) (SEQ ID NO:13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the anti-PVRIG treatment antibody comprises a CH1-hinge-CH2-CH3 sequence of IgG4 (SEQ ID NO:657 or SEQ ID NO:658), wherein the hinge region optionally comprises mutations.

In some embodiments, the anti-PVRIG antibody comprises a CH1-hinge-CH2-CH3 region from IgG1, IgG2, IgG3, or IgG4, wherein the hinge region optionally comprises mutations.

In some embodiments, the heavy chain variable domain of the anti-PVRIG antibody is from the heavy chain of CHA.7.518.1.H4(S241P) (SEQ ID NO:8) and the light chain variable domain of the anti-PVRIG antibody is from the light chain of CHA.7.518.1.H4(S241P) (SEQ ID NO: 13).

In some embodiments, the anti-PVRIG antibody comprises a CL region of human kappa 2 light chain.

In some embodiments, the stable liquid formulation comprises from 10 mM to 80 mM histidine, from 15 mM to 70 mM histidine, from 20 mM to 60 mM histidine, from 20 mM to 50 mM histidine, or from 20 mM to 30 mM histidine.

In some embodiments, the pharmaceutical formulation comprises about 25 mM histidine.

In some embodiments, the pharmaceutical formulation comprises from 30 mM to 100 mM NaCl, from 30 mM to 90 mM NaCl, from 40 mM to 80 mM NaCl, from 30 mM to 70 mM histidine, or from 45 mM to 70 mM NaCl.

In some embodiments, the pharmaceutical formulation comprises about 60 mM NaCl.

T In some embodiments, the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine.

In some embodiments, the pharmaceutical formulation comprises about 100 mM L-arginine.

In some embodiments, the pharmaceutical formulation comprises from 0.006% to 0.1% w/v polysorbate 80, from 0.007% to 0.09% w/v polysorbate 80, from 0.008% to 0.08% w/v polysorbate 80, from 0.009% to 0.09% w/v polysorbate 80, from 0.01% to 0.08% w/v polysorbate 80, from 0.01% to 0.07% w/v polysorbate 80, from 0.01% to 0.07% w/v polysorbate 80, or from 0.01% to 0.06% w/v polysorbate 80, or from 0.009% to 0.05% w/v polysorbate 80.

In some embodiments, the pharmaceutical formulation comprises about 0.01% polysorbate 80.

In some embodiments, the pH is from 6 to 7.0.

In some embodiments, the pH is from 6.3 to 6.8.

In some embodiments, the pH is 6.5 +/- 0.2.

In some embodiments, the anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL.

In some embodiments, the anti-PVRIG antibody is at a concentration of about 20 mg/mL.

In some embodiments, the anti-PVRIG antibody formulation comprises:
a) a heavy chain comprising:
   i) a VH-CH1-hinge-CH2-CH3, wherein the VH is from CHA.7.518.1.H4(S241P) (SEQ ID NO:4) and wherein the CH1-hinge-CH2-CH3 region is from IgG4; and
b) a light chain comprising:
   i) a VL-CL, wherein the VL from CHA.7.518.1.H4(S241P) (SEQ ID NO:9) and wherein the CL region is from human kappa 2 light chain.

In some embodiments, the hinge region optionally comprises mutations.

In some embodiments, the hinge region optionally comprises mutations.

In some embodiments, the anti-PVRIG antibody formulation comprises:
i) a heavy chain comprising the heavy chain from CHA.7.518.1.H4(S241P) (SEQ ID NO:8); and
ii) a light chain comprising the light chain from CHA.7.518.1.H4(S241P) (SEQ ID NO:13).

In some embodiments, the anti-PVRIG antibody formulation comprising:
(a) an anti-PVRIG antibody, wherein the anti-PVRIG antibody comprises:
   i) a heavy chain variable domain comprising the vhCDR1, vhCDR2, and vhCDR3 from the heavy chain of CHA.7.518.1.H4(S241P) (SEQ ID NO:8), and
   ii) a light chain variable domain comprising the vlCDR1, vlCDR2, and vlCDR3 from the light chain of CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the anti-PVRIG antibody formulation comprising:
(a) an anti-PVRIG antibody, wherein the anti-PVRIG antibody comprises:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the anti-PVRIG treatment antibody is administered at a dosage of about 0.01 mg/kg to about 20 mg/kg of the anti-PVRIG antibody or about 0.01 mg/kg to about 10 mg/kg of the anti-PVRIG antibody.

In some embodiments, the anti-PVRIG treatment antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg of the anti-PVRIG antibody.

In some embodiments, the anti-PVRIG treatment antibody is administered 20 mg/kg every 4 weeks.

In some embodiments, the cancer is selected from the group consisting of prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, and/or fallopian tube cancer.

In some embodiments, the anti-PVRIG antibody is administered in combination with an anti-PD-1 antibody.

In some embodiments, the anti-PVRIG antibody is administered in combination with an anti-PD-L1 antibody.

In some embodiments, the anti-PVRIG antibody is administered in combination with an anti-TIGIT antibody.

In some embodiments, the anti-PVRIG antibody is administered in combination with an anti-PD-1 antibody and an anti-TIGIT antibody.

In some embodiments, the anti-PVRIG antibody is administered in combination with an anti-PD-L1 antibody and an anti-TIGIT antibody.

In some embodiments, the anti-PD1 antibody is selected from the group consisting of nivolumab and pembrolizumab.

In some embodiments, the anti-PD-L1 antibody is selected from the group consisting of atezolizumab, avelumab, durvalumab, KN035(Envafolimab), and CK-301 (Cosibelimab).

In some embodiments, the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.

In some embodiments, the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.

In some embodiments, the anti-TIGIT antibody is selected from the group consisting of CPA.9.083.H4(S241P) and CPA.9.086.H4(S241P).

In some embodiments, the anti-TIGIT antibody comprises:
a) a heavy chain comprising VH-CH1-hinge-CH2-CH3; and
b) a light chain comprising VL-VC, wherein VC is either kappa or lambda.

In some embodiments, the sequence of the CH1-hinge-CH2-CH3 is selected from human IgG1, IgG2 and IgG4, and variants thereof.

In some embodiments, the anti-PVRIG antibody is CHA.7.518.1.H4(S241P), the anti-TIGIT antibody is CPA.9.086.H4(S241P) and the anti-PD-1 antibody is nivolumab.

In some embodiments, the anti-PVRIG antibody is CHA.7.518.1.H4(S241P), the anti-TIGIT antibody is CPA.9.083.H4(S241P) and the anti-PD-1 antibody is nivolumab.

In some embodiments, the anti-PVRIG antibody is CHA.7.518.1.H4(S241P), the anti-TIGIT antibody is CPA.9.086.H4(S241P) and the anti-PD-1 antibody is pembrolizumab.

In some embodiments, the anti-PVRIG antibody is CHA.7.518.1.H4(S241P), the anti-TIGIT antibody is CPA.9.083.H4(S241P) and the anti-PD-1 antibody is pembrolizumab.

In some embodiments, the anti-PVRIG antibody is CHA.7.538.1.2.H4(S241P), the anti-TIGIT antibody is CPA.9.086.H4(S241P) and the anti-PD-1 antibody is nivolumab.

In some embodiments, the anti-PVRIG antibody is CHA.7.538.1.2.H4(S241P), the anti-TIGIT antibody is CPA.9.083.H4(S241P) and the anti-PD-1 antibody is nivolumab.

In some embodiments, the anti-PVRIG antibody is CHA.7.538.1.2.H4(S241P), the anti-TIGIT antibody is CPA.9.086.H4(S241P) and the anti-PD-1 antibody is pembrolizumab.

In some embodiments, the anti-PVRIG antibody is CHA.7.538.1.2.H4(S241P), the anti-TIGIT antibody is CPA.9.083.H4(S241P) and the anti-PD-1 antibody is pembrolizumab.

In some embodiments, the anti-PVRIG antibody is CHA.7.518.1.H4(S241P), the anti-TIGIT antibody is CPA.9.086.H4(S241P) and the anti-PD-L1 antibody is selected from the group consisting of atezolizumab, avelumab, durvalumab, KN035(Envafolimab), and CK-301 (Cosibelimab).

In some embodiments, the anti-PVRIG antibody is CHA.7.518.1.H4(S241P), the anti-TIGIT antibody is CPA.9.083.H4(S241P) and the anti-PD-L1 antibody is selected from the group consisting of atezolizumab, avelumab, durvalumab, KN035(Envafolimab), and CK-301 (Cosibelimab).

In some embodiments, the anti-PVRIG antibody is CHA.7.538.1.2.H4(S241P), the anti-TIGIT antibody is CPA.9.086.H4(S241P) and the anti-PD-L1 antibody is selected from the group consisting of atezolizumab, avelumab, durvalumab, KN035(Envafolimab), and CK-301 (Cosibelimab).

In some embodiments, the anti-PVRIG antibody is CHA.7.538.1.2.H4(S241P), the anti-TIGIT antibody is CPA.9.083.H4(S241P) and the anti-PD-L1 antibody is selected from the group consisting of atezolizumab, avelumab, durvalumab, KN035(Envafolimab), and CK-301 (Cosibelimab).

In some embodiments, the activated DC cells, DC1, and/or DC2 disclosed herein express CXCL10.

In some embodiments, the early memory CD8 T cells disclosed herein include CD8 T cells expressing CXCR3.

### BRIEF DESCRIPTION OF THE DRAWINGS

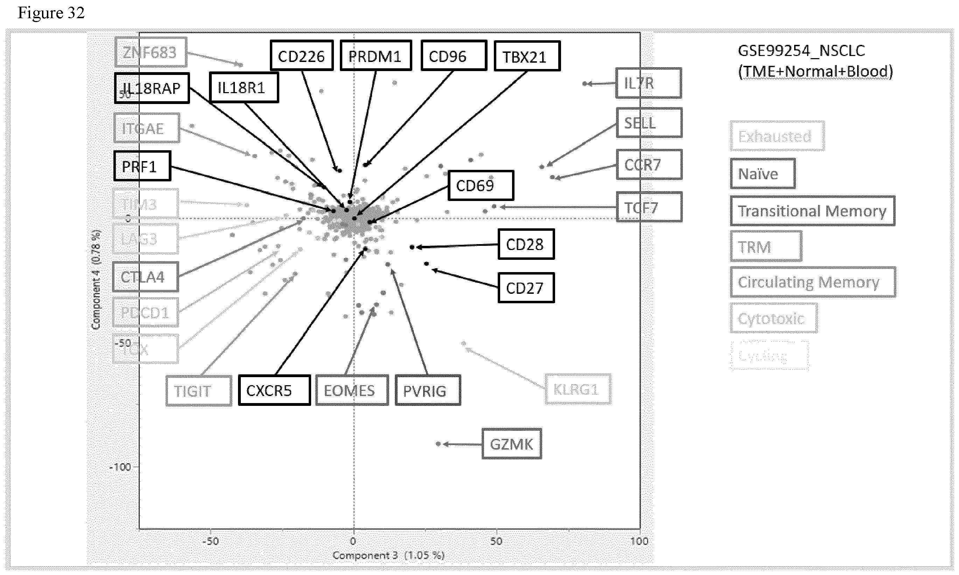

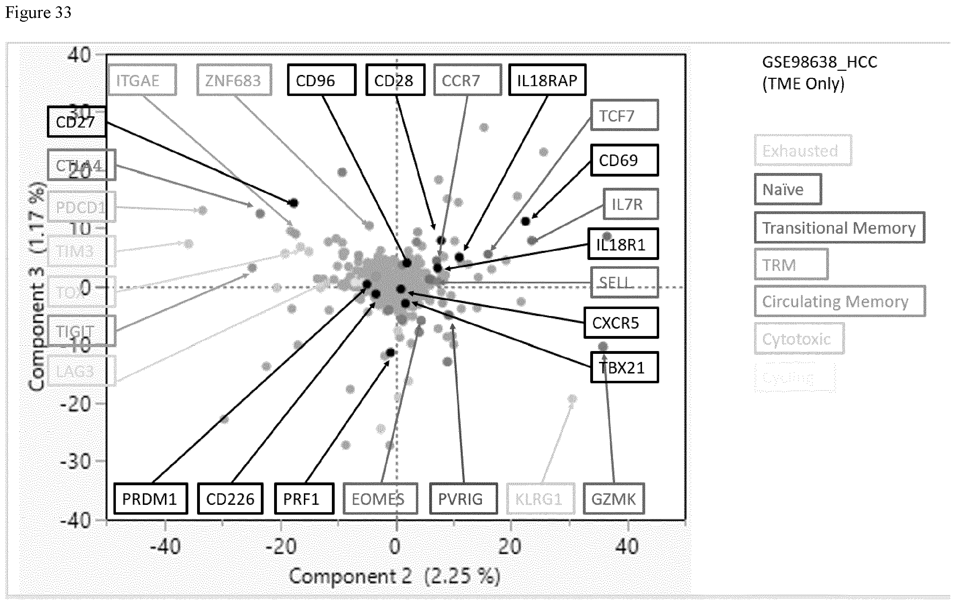

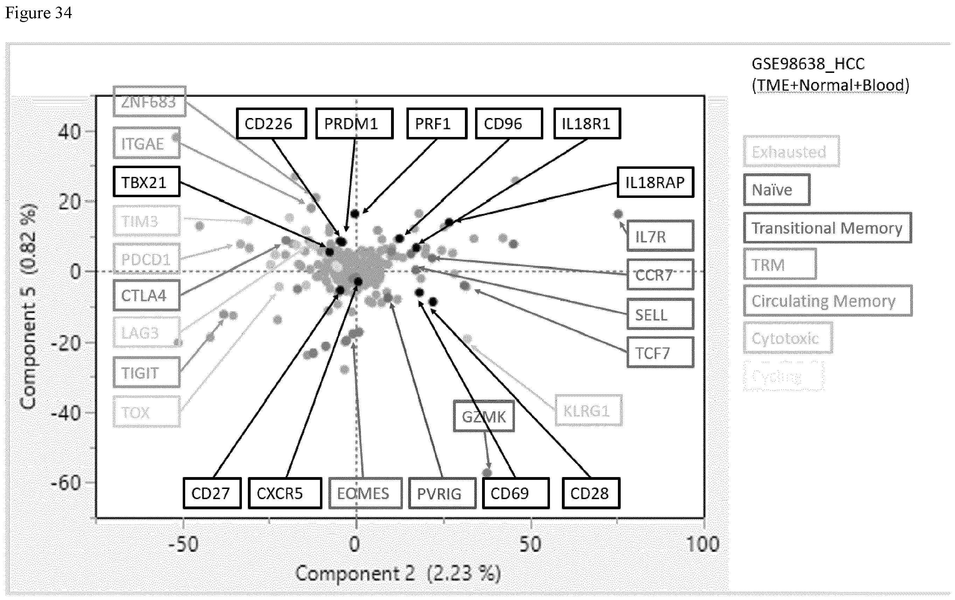

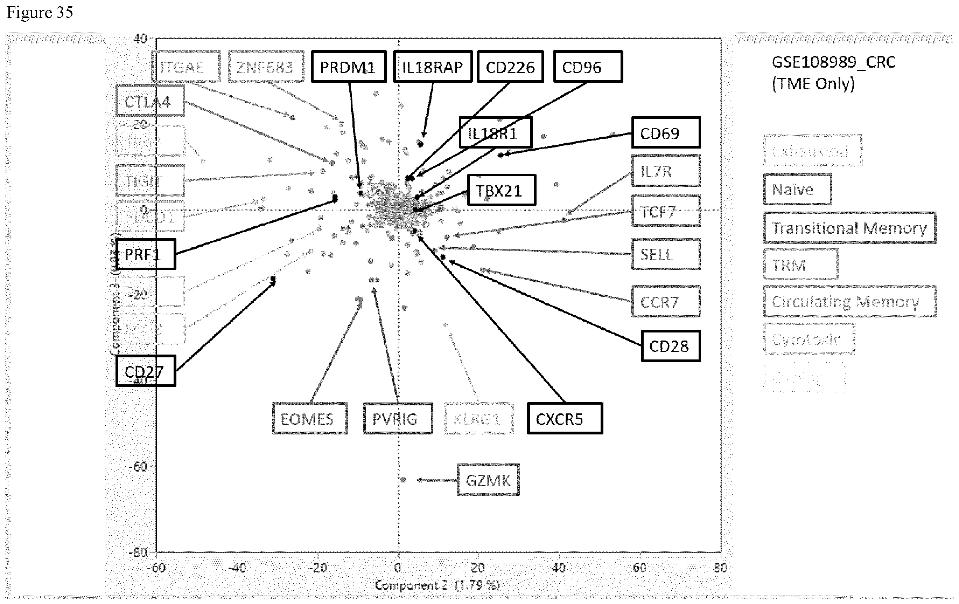

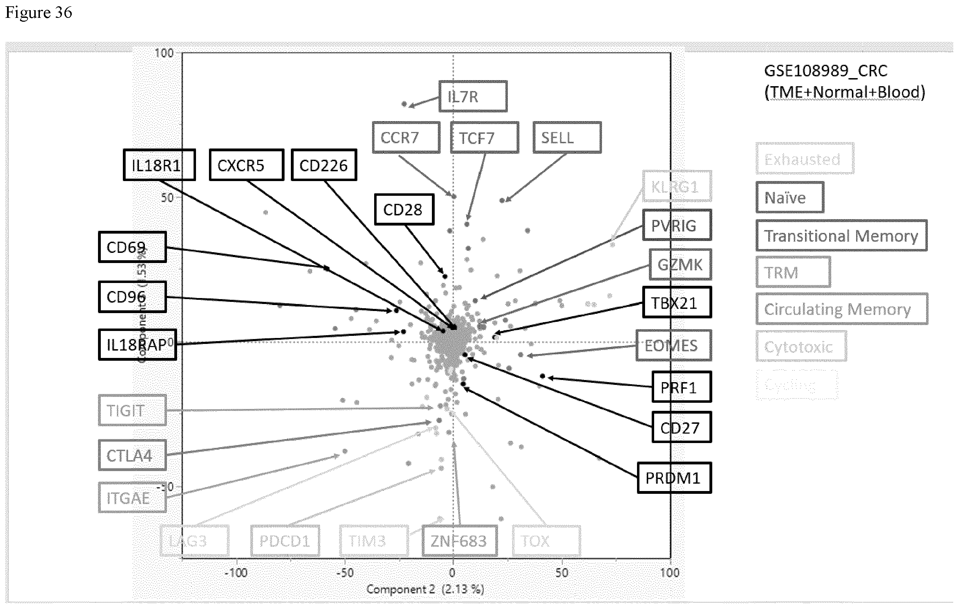

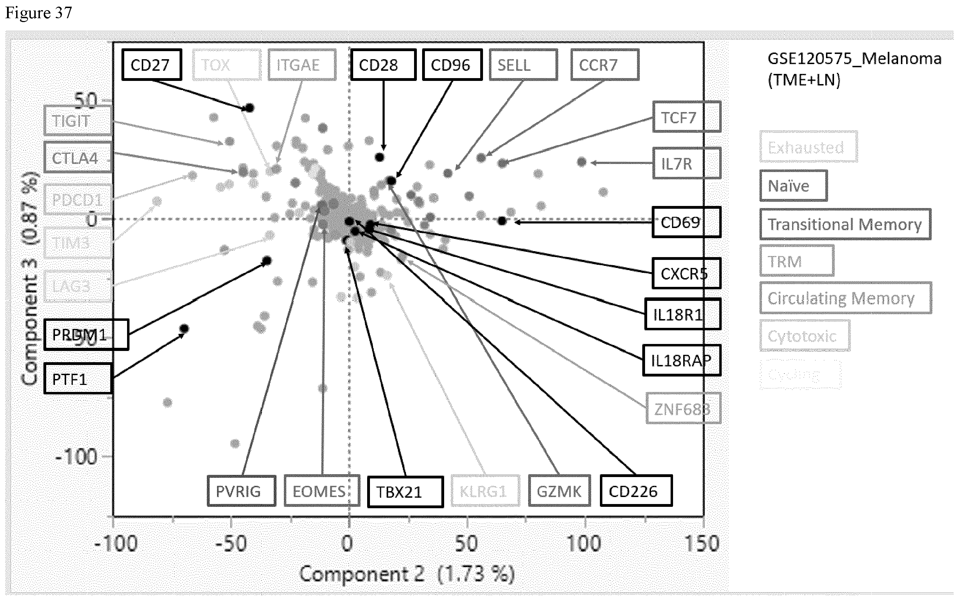

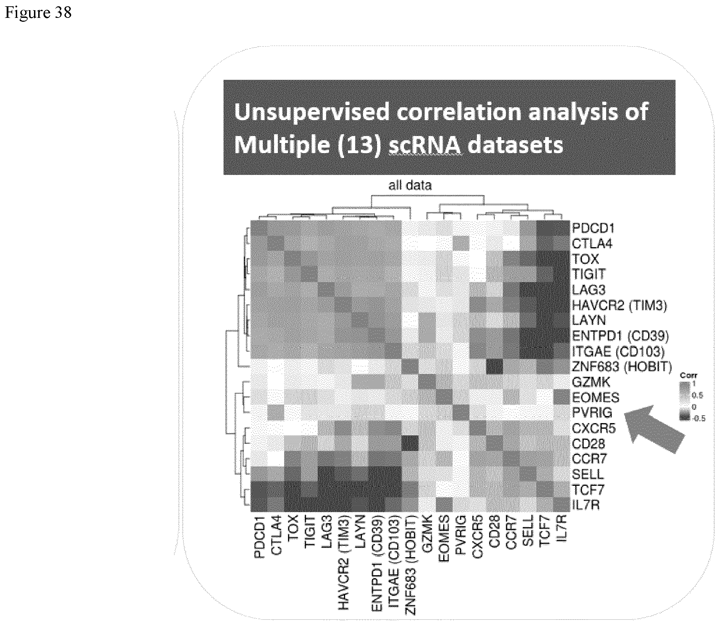

Figure 1 depicts the full-length sequence of human PVRIG.

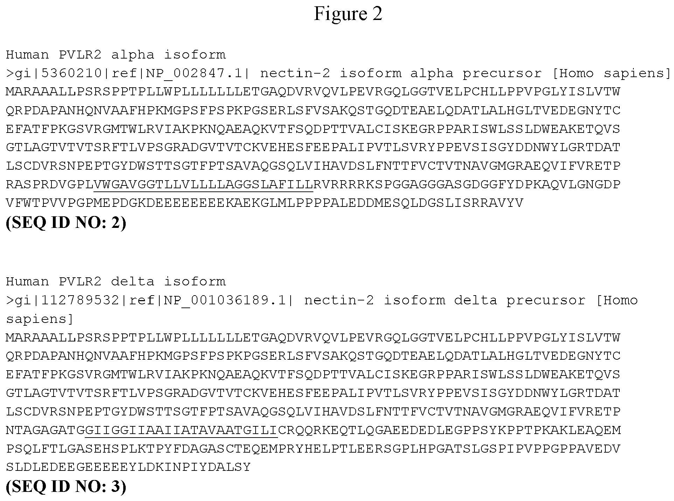

Figure 2 depicts the sequence of the human Poliovirus receptor-related 2 protein (PVLR2, also known as nectin-2, CD112 or herpesvirus entry mediator B, (HVEB)), the binding partner of PVRIG. PVLR2 is a human plasma membrane glycoprotein.
Figure 3A-3AG depicts the variable heavy and light chains as well as the vhCDR1, vhCDR2, vhCDR3, vlCDR1, vlCDR2 and vlCDR3 sequences of the PVRIG antibodies of the invention, including CHA.7.518.1.H4(S241P).
Figures 4A-4AA shows the amino acid sequences of the variable heavy and light domains, the full length heavy and light chains, and the variable heavy and variable light CDRs for the enumerated human CPA anti-PVRIG sequences of the invention that both bind PVRIG and block binding of PVRIG and PVLR2.
Figures 5A-5H depicts the amino acid sequences of the variable heavy and light domains, the full length heavy and light chains, and the variable heavy and variable light CDRs for eight human CPA anti-PVRIG sequences of the invention that bind PVRIG and but do not block binding of PVRIG and PVLR2.

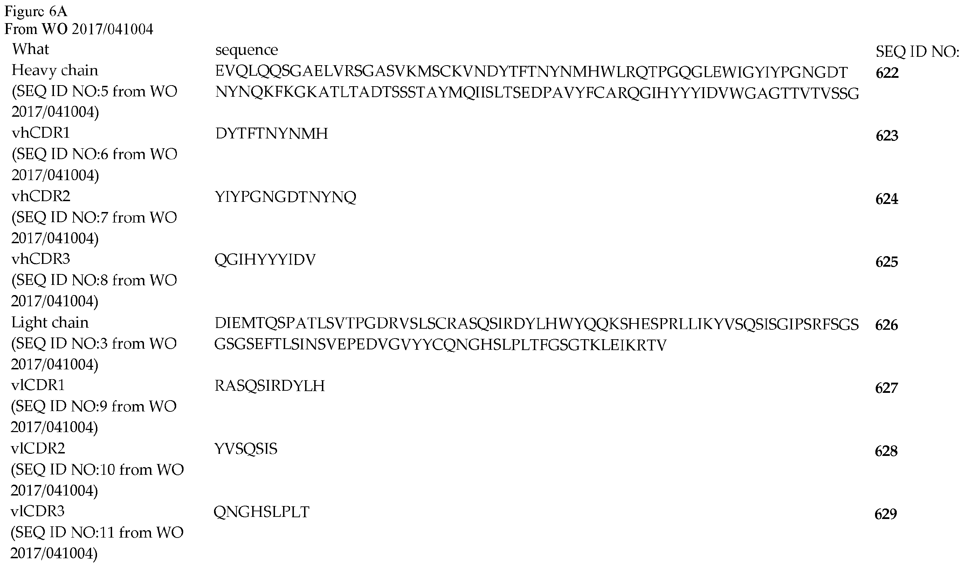

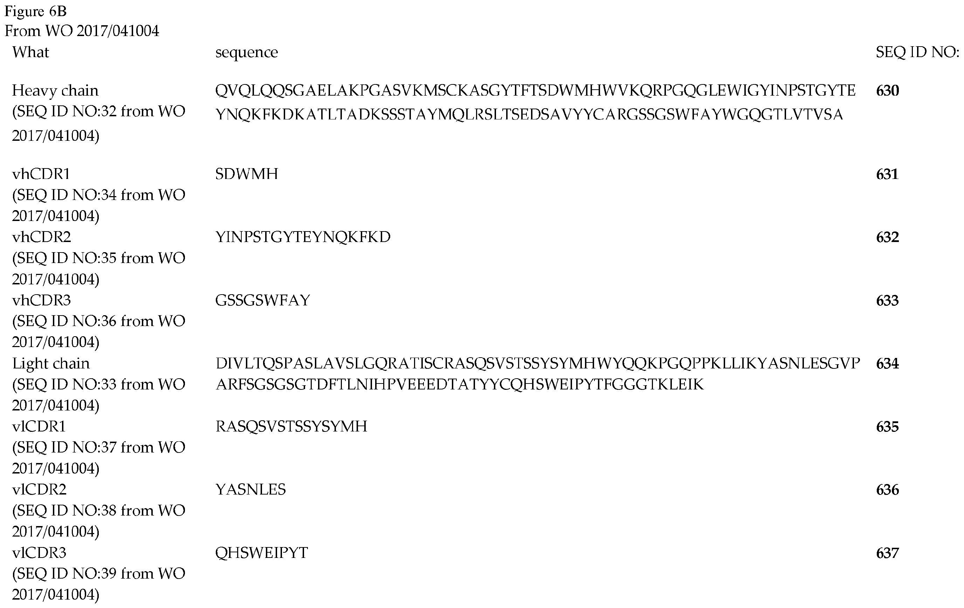

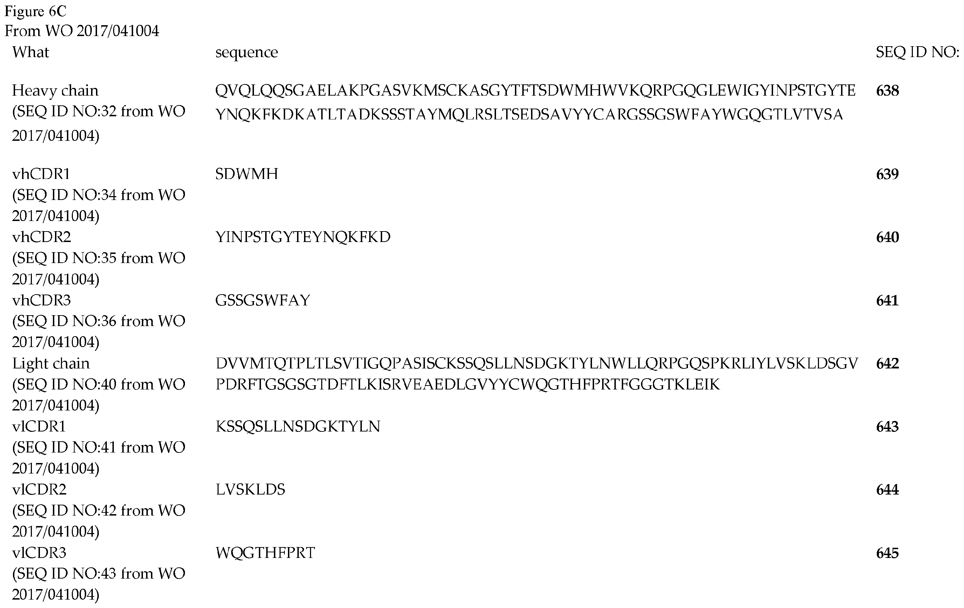

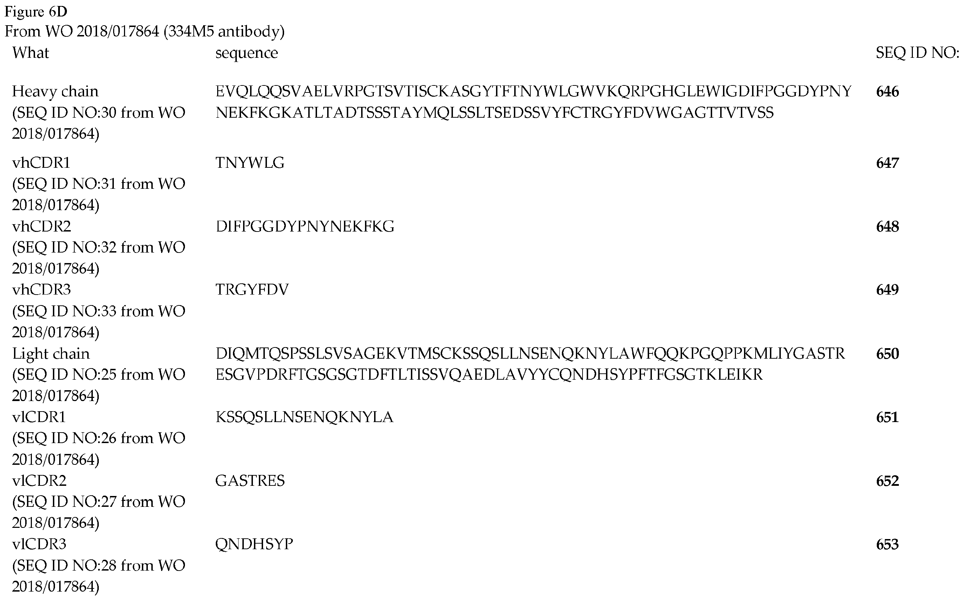

Figures 6A-6D depicts the sequences of other PVRIG antibodies that can be formulated according to stable liquid formulations of an anti-PVRIG antibody of the present invention and can be used as anti-PVRIG treatment antibodies.
Figure 7 depicts the sequences of human IgG1, IgG2, IgG3 and IgG4.
Figure 8. Image of immunohistochemistry staining of tumor samples using PVRL2 antibody.
Figure 9. Expression of +/- markers in CD8+ T cells in NSCLC patient sample.
Figure 10. Expression of inhibitory receptors in CD8+ T cells in NSCLC patient sample.
Figure 11. Frequencies of TSCM in CD8+ T cells in lymph node and tumors.
Figure 12. Frequencies of CD8+ T cell in responders and non-responders to pretreatment.
Figure 13. Expression profiles of PVRIG among CD8+ T cells in tumors.
Figure 14A-14C. Expression of scRNAs of PVR, PVRL2, and PD-L1 in subpopulations of dendritic cells.
Figure 15. Microscopy characterization of PVR- and PVRL2-expressing cells in human lymph nodes.
Figure 16. Expression level of PVR and PVRL2 in dendritic cells by disease.
Figure 17. Percentage of altered samples for PVRL2 and with altered mutations and GISTIC2 by disease.
Figure 18. Frequencies of altered samples for PVRL2 (DNA-sequence mutation and GISTIC2 calls) by cancer types.
Figure 19. Putative copy-number alterations for PVRL2 from GISTIC2.
Figure 20A-20B. A) IFNγ secretion of MEL624 parental tumor cells and MEL624 cells stably transduced with a human PVRL2 (MEL624 PVRL2 OE). MEL624 PVRL2 OE tumor cells were treated with CHA.7.518.1.H4(S241P) anti-PVRIG antibody. B) PVRL2 expression in MEL624 tumor cells and MEL624 PVRL2 OE tumor cells.

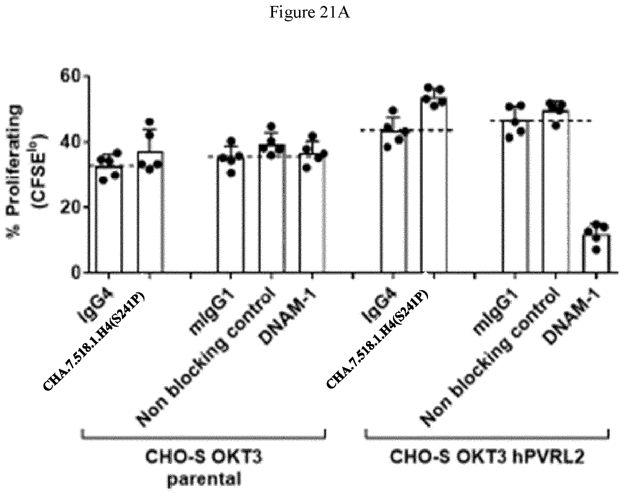

Figure 21A-21B. CHO-S OKT3 assay. A) The effect of CHA.7.518.1.H4(S241P), a non-blocking control antibody, and anti-DNAM-1 on CD4+ T cells from a healthy donor activated by CHO-S OKT3 cells or CHO-S OKT3 hPVRL2 cells is shown. The dashed line indicates the percentage of CFSEl0 CD4+ T cells after treatment with either the human IgG4 or the mouse IgG1 isotype antibodies. Each dot represents a technical replicate, average plus standard deviation is shown, data are representative of at least 2 batches of tests. B) shows IFN-γ production by three different human CD8⁺ T cell donors (Donors 231, 232, and 234) when co-cultured with the CHO-S OKT3 hPVRL2 cells and the indicated antibody.
Figure 22 Expression of PVRL2 and OKT3 in CD4+ T cells.
Figure 23. AB-635 PVRIG (6D8-1 clone) antibody sequences.
Figure 24A-24B. Expression of PVRIG in ovarian and CRC cancers. A. expression of PVRIG in CD8⁺ T cells, in Tscm defined by TIM3⁻CXCR5⁺ or TIM3-PD1⁺CD28⁺ and CD4⁺ T cells. B. matched comparison of PVRIG expression between the colorectal (CRC) tumor sample and a matched normal adjacent tissue (NAT), in Tscm defined by TIM3⁻CXCR5⁺ or TIM3-PD1⁺CD28⁺ and CD4⁺ T cells.
Figures 25A-25I depicts a collation of the humanized sequences of five CHA antibodies.
Figures 26A-26E depicts a collation of the humanized sequences of five CHA antibodies.
Figure 27 depicts schemes for combining the humanized VH and VL CHA antibodies of Figures 25A-25I and Figures 26A-26E. The "chimVH" and "chimVL" are the mouse variable heavy and light sequences attached to a human IgG constant domain.

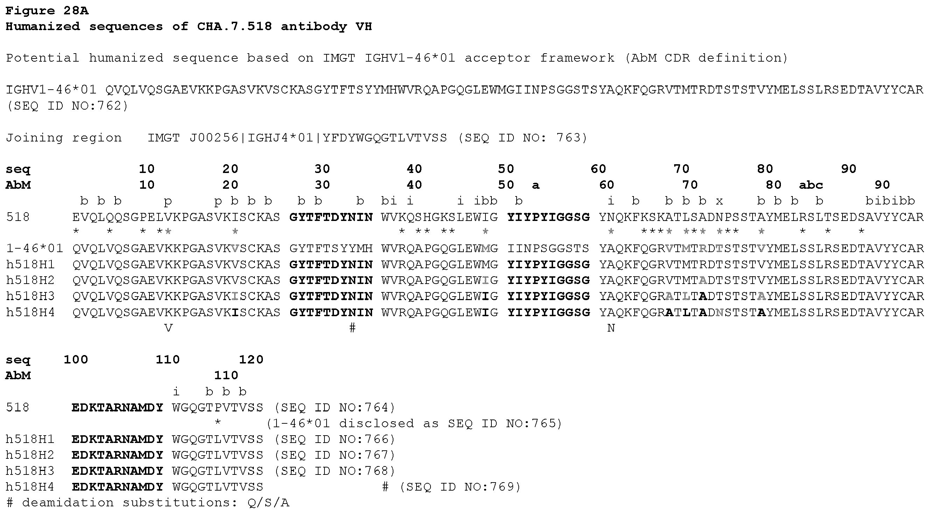

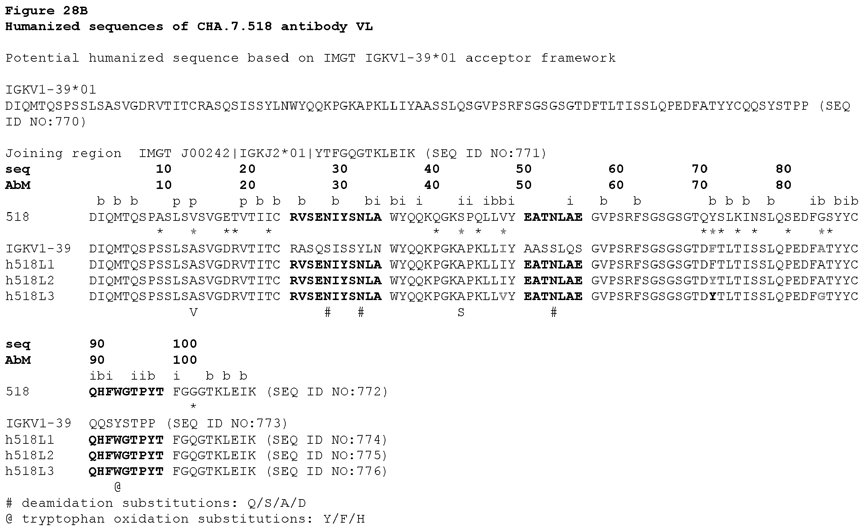

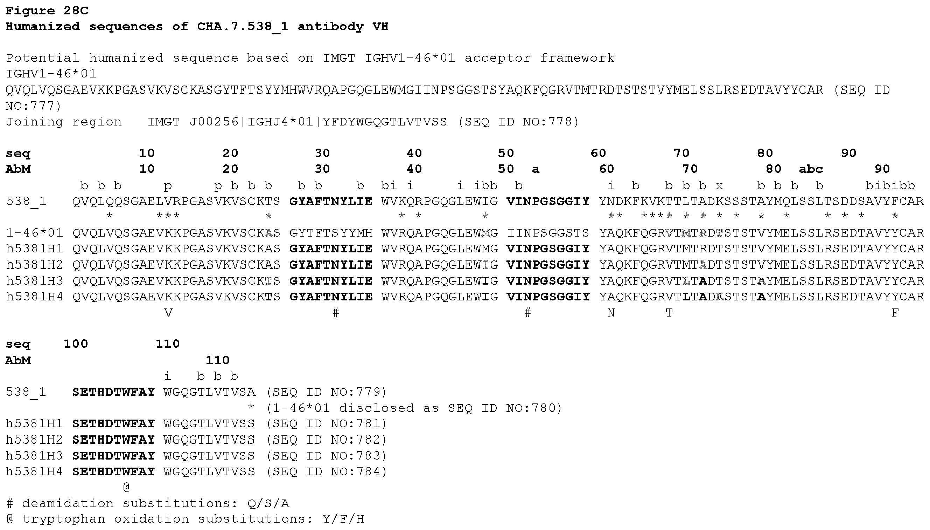

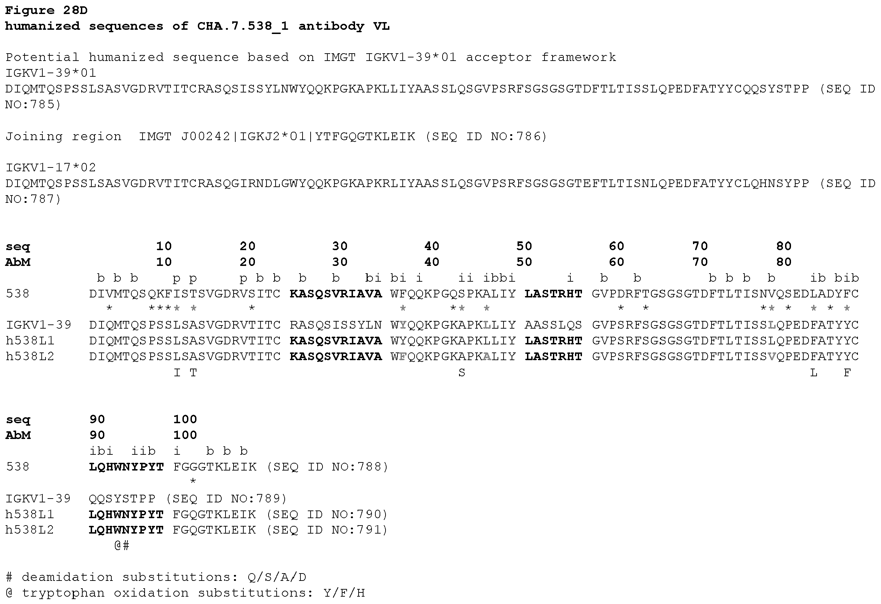

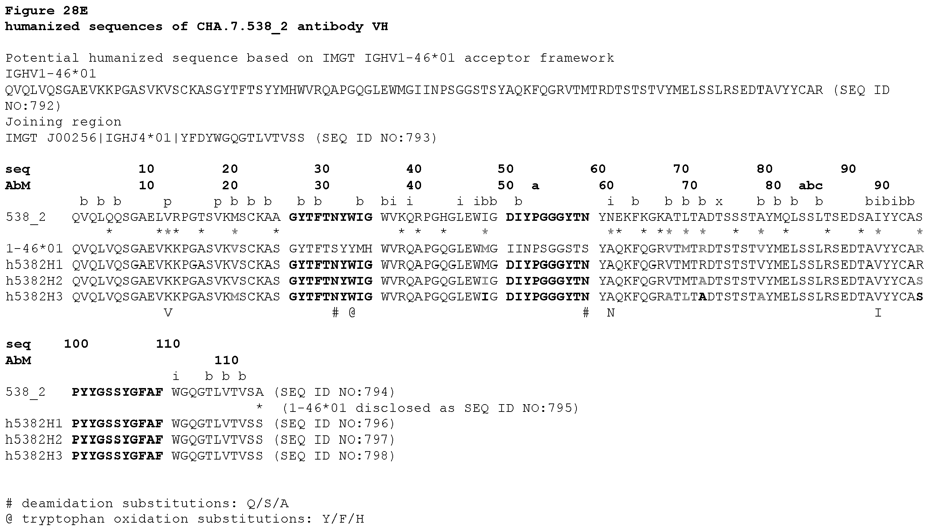

Figure 28A-28E depict four humanized sequences for each of CHA.7.518, CHA.7.524, CHA.7.530, CHA.7.538_1 and CHA.7.538_2. All humanized antibodies comprise the H4(S241P) substitution. Note that the light chain for CHA.7.538_2 is the same as for CHA. 7.53 S _1. The "H1" of each is a "CDR swap" with no changes to the human framework. Subsequent sequences alter framework changes shown in larger bold font. CDR sequences are noted in bold. CDR definitions are AbM from website www.bioinf.org.uk/abs/. Human germline and joining sequences from IMGT^{®} the international ImMunoGeneTics^{®} information system www.imgt.org (founder and director: Marie-Paule Lefranc, Montpellier, France). Residue numbering shown as sequential (seq) or according to Chothia from website www.bioinf.org.uk/abs/ (AbM). "b" notes buried sidechain; "p" notes partially buried; "i" notes sidechain at interface between VH and VL domains. Sequence differences between human and murine germlines noted by asterisk (*). Potential additional mutations in frameworks are noted below sequence. Potential changes in CDR sequences noted below each CDR sequence as noted on the figure (# deamidation substitutions: Q/S/A; these may prevent asparagine (N) deamidation. @ tryptophan oxidation substitutions: Y/F/H; these may prevent tryptophan oxidation; @ methionine oxidation substitutions: L/F/A).
Figure 29A to 29C depicts a collation of the humanized sequences of three CHA antibodies: CHA.7.518, CHA.7.538.1, and CHA.7.538.2.
Figure 30A-30AX depict the sequences of anti-TIGIT antibodies. Unless otherwise noted, the CDRs utilize the IMGT numbering (including the antibodies of the sequence listing.

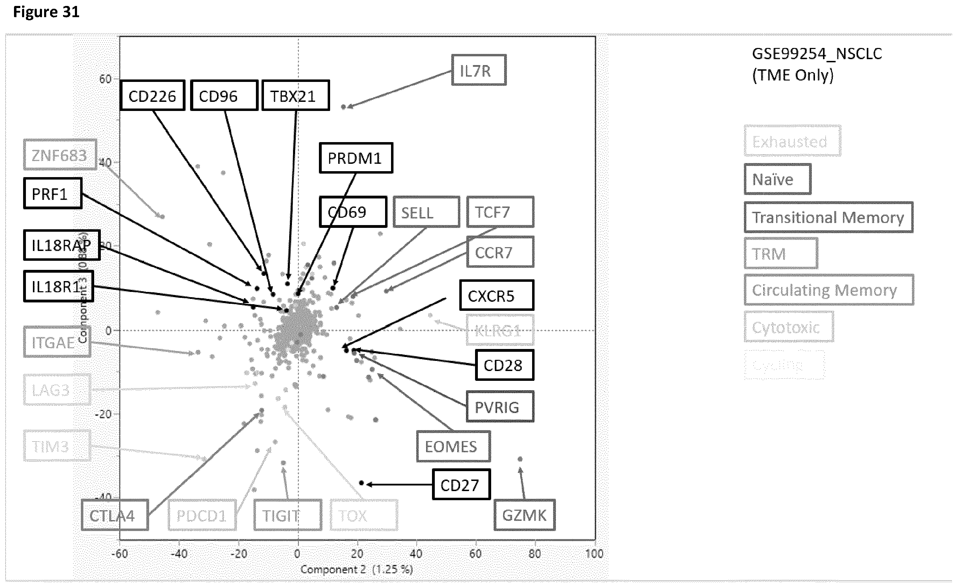

Figure 31 diagram showing genes that are markers for particular CD8 cell types:
   - Naive: TCF7, CCR7, IL7R, LEF1, SELL, LTB
   - Exhausted: TIM3, ENTPD1, LAYN, CD38, LAG3, VCAM1, TOX
   - Transitional Memory (early memory): GZMK, SH2D1A, EOMES, DTHD1, SLAMF7, FCRL3, CD28
   - Tissue resident Memory: XCL1, XCL2, ITGAE, ITGA1, ZNF683, CXCR6
   - Circulating memory: NR4A1, NR4A2, NR4A3, KLF2
   - Cytotoxic: FCGR3A, FGFBP2, CX3CR1, KLRG1, LILRB1
   - Cycling: TOP2A, MKI67
Figure 32 diagram showing genes that are markers for particular CD8 cell types:
   - Naive: TCF7, CCR7, IL7R, LEF1, SELL, LTB
   - Exhausted: TIM3, ENTPD1, LAYN, CD38, LAG3, VCAM1, TOX
   - Transitional Memory (early memory): GZMK, SH2D1A, EOMES, DTHD1, SLAMF7, FCRL3, CD28
   - Tissue resident Memory: XCL1, XCL2, ITGAE, ITGA1, ZNF683, CXCR6
   - Circulating memory: NR4A1, NR4A2, NR4A3, KLF2
   - Cytotoxic: FCGR3A, FGFBP2, CX3CR1, KLRG1, LII,RB1
   - Cycling: TOP2A, MKI67
Figure 33 diagram showing genes that are markers for particular CD8 cell types:
   - Naive: TCF7, CCR7, IL7R, LEF1, SELL, LTB
   - Exhausted: TIM3, ENTPD1, LAYN, CD38, LAG3, VCAM1, TOX
   - Transitional Memory (early memory): GZMK, SH2D1A, EOMES, DTHD1, SLAMF7, FCRL3, CD28
   - Tissue resident Memory: XCL1, XCL2, ITGAE, ITGA1, ZNF683, CXCR6
   - Circulating memory: NR4A1, NR4A2, NR4A3, KLF2
   - Cytotoxic: FCGR3A, FGFBP2, CX3CR1, KLRG1, LILRB1
   - Cycling: TOP2A, MKI67
Figure 34 diagram showing genes that are markers for particular CD8 cell types:
   - Naive: TCF7, CCR7, IL7R, LEF1, SELL, LTB
   - Exhausted: TIM3, ENTPD1, LAYN, CD38, LAG3, VCAM1, TOX
   - Transitional Memory (early memory): GZMK, SH2D1A, EOMES, DTHD1, SLAMF7, FCRL3, CD28
   - Tissue resident Memory: XCL1, XCL2, ITGAE, ITGA1, ZNF683, CXCR6
   - Circulating memory: NR4A1, NR4A2, NR4A3, KLF2
   - Cytotoxic: FCGR3A, FGFBP2, CX3CR1, KLRG1, LII,RB1
   - Cycling: TOP2A, MKI67
Figure 35 diagram showing genes that are markers for particular CD8 cell types:
   - Naive: TCF7, CCR7, IL7R, LEF1, SELL, LTB
   - Exhausted: TIM3, ENTPD1, LAYN, CD38, LAG3, VCAM1, TOX
   - Transitional Memory (early memory): GZMK, SH2D1A, EOMES, DTHD1, SLAMF7, FCRL3, CD28
   - Tissue resident Memory: XCL1, XCL2, ITGAE, ITGA1, ZNF683, CXCR6
   - Circulating memory: NR4A1, NR4A2, NR4A3, KLF2
   - Cytotoxic: FCGR3A, FGFBP2, CX3CR1, KLRG1, LII,RB1
   - Cycling: TOP2A, MKI67
Figure 36 diagram showing genes that are markers for particular CD8 cell types:
   - Naive: TCF7, CCR7, IL7R, LEF1, SELL, LTB
   - Exhausted: TIM3, ENTPD1, LAYN, CD38, LAG3, VCAM1, TOX
   - Transitional Memory (early memory): GZMK, SH2D1A, EOMES, DTHD1, SLAMF7, FCRL3, CD28
   - Tissue resident Memory: XCL1, XCL2, ITGAE, ITGA1, ZNF683, CXCR6
   - Circulating memory: NR4A1, NR4A2, NR4A3, KLF2
   - Cytotoxic: FCGR3A, FGFBP2, CX3CR1, KLRG1, LII,RB1
   - Cycling: TOP2A, MKI67
Figure 37 diagram showing genes that are markers for particular CD8 cell types:
   - Naive: TCF7, CCR7, IL7R, LEF1, SELL, LTB
   - Exhausted: TIM3, ENTPD1, LAYN, CD38, LAG3, VCAM1, TOX
   - Transitional Memory (early memory): GZMK, SH2D1A, EOMES, DTHD1, SLAMF7, FCRL3, CD28
   - Tissue resident Memory: XCL1, XCL2, ITGAE, ITGA1, ZNF683, CXCR6
   - Circulating memory: NR4A1, NR4A2, NR4A3, KLF2
   - Cytotoxic: FCGR3A, FGFBP2, CX3CR1, KLRG1, LILRB1
   - Cycling: TOP2A, MKI67
Figure 38 shows unsupervised correlation analysis of 13 data sets.

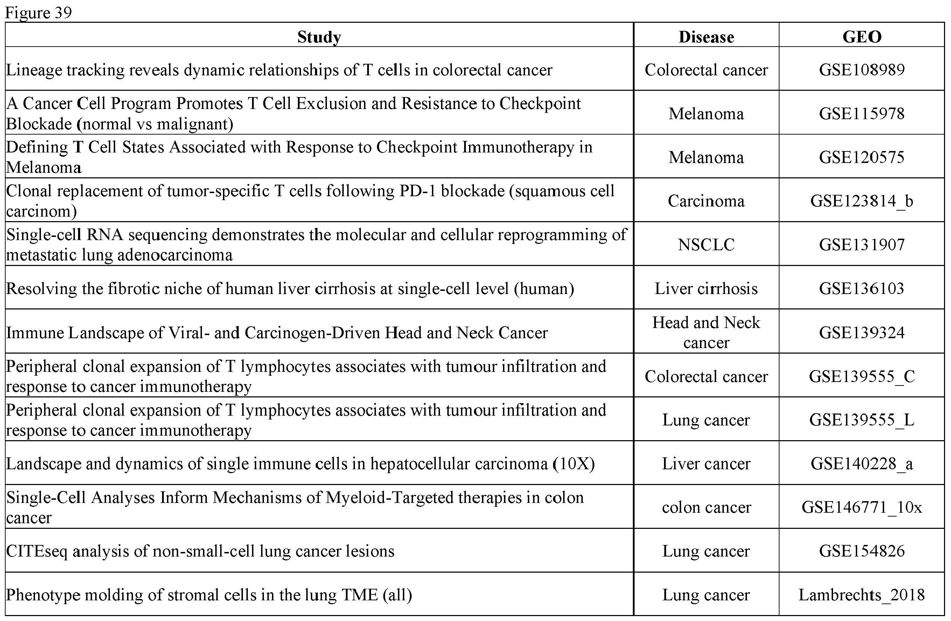

Figure 39 provides data sets analyzed in Figure 38.

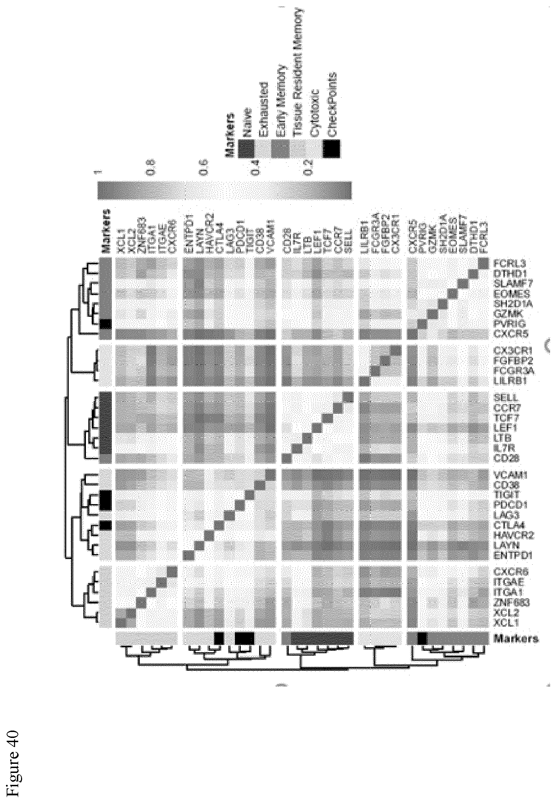

Figure 40 shows Cosine similarity analysis and calculation heat map.

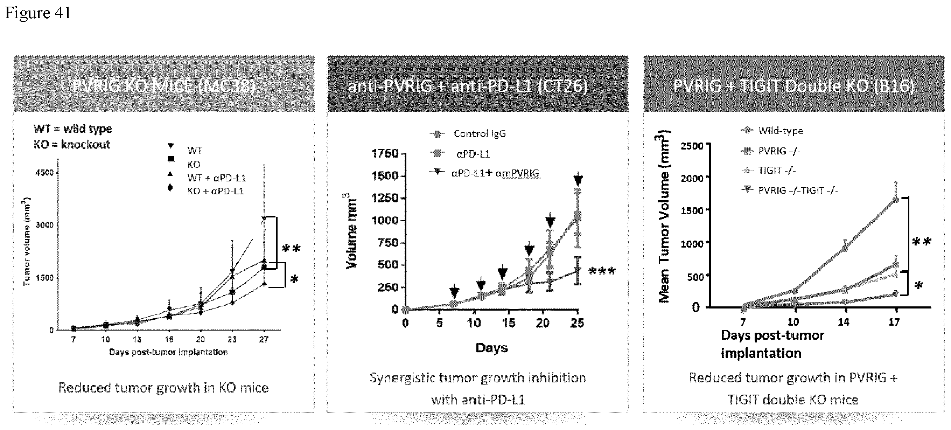

Figure 41: PVRIG Knockout or Inhibition Reduces Tumor Growth. Combination data shown with PD-L1 or TIGIT in mouse models.

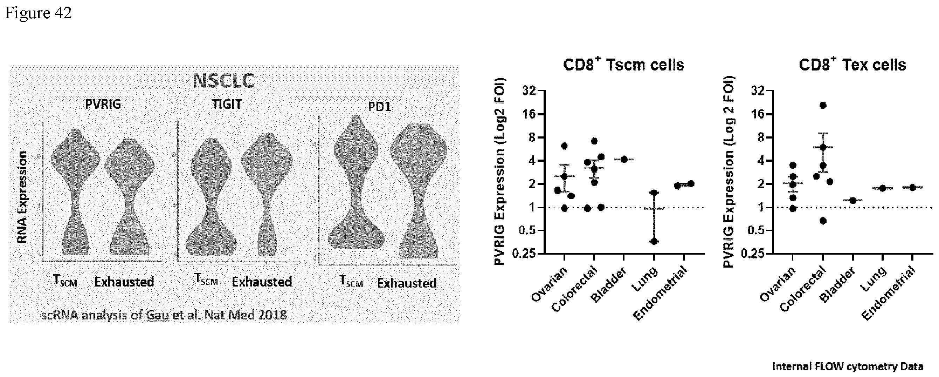

Figure 42: PVRIG is Expressed by T Stem-like Cells. Left panel shows scRNA analysis of Gau *et al*., *Nat Med.* (2018). Right panel shows flow cytometry data. Potential for optimal T_{SCM} activation, expansion, and generation of effector T cells.

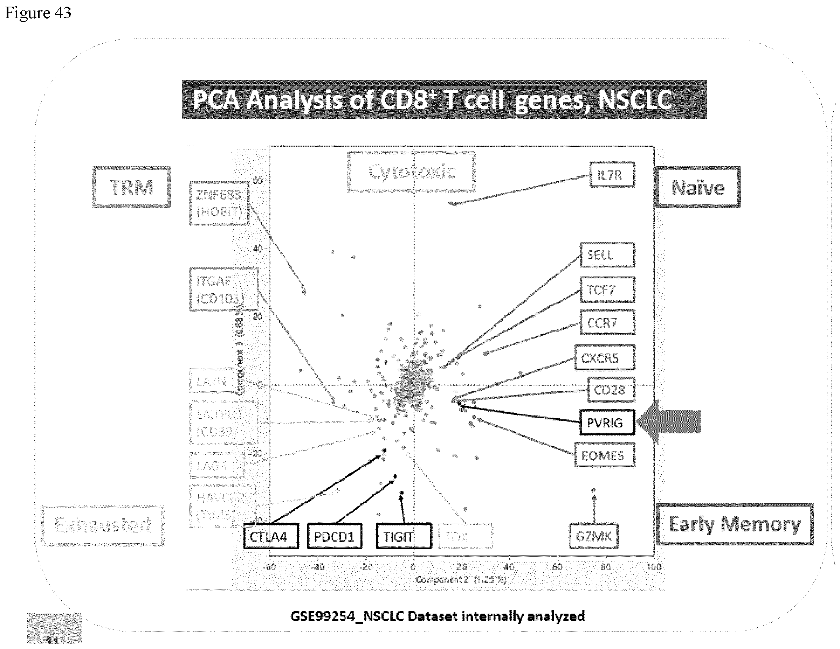

Figure 43: PVRIG uniquely clusters with early memory differentiation/ stem-like genes: PCA analysis of CD8+ T cell genes - NSCLC.

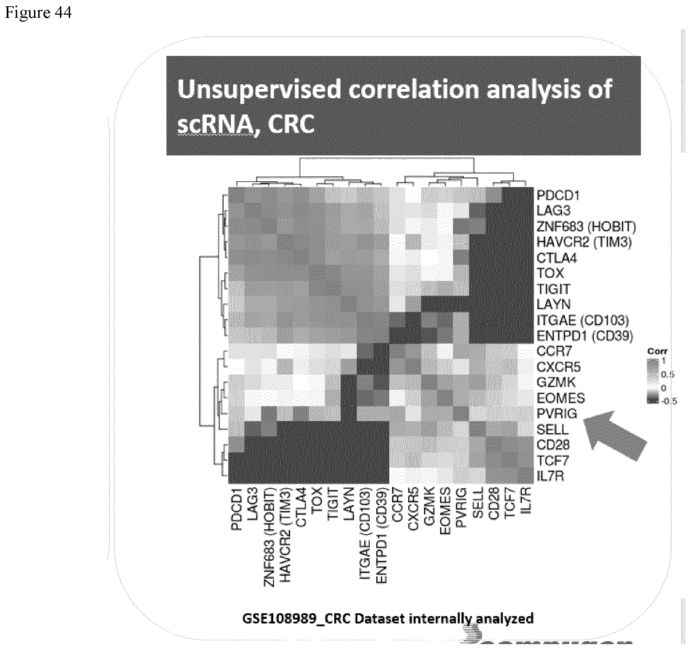

Figure 44: PVRIG uniquely clusters with early memory differentiation/ stem-like genes: unsupervised correlation analysis of scRNA - CRC.

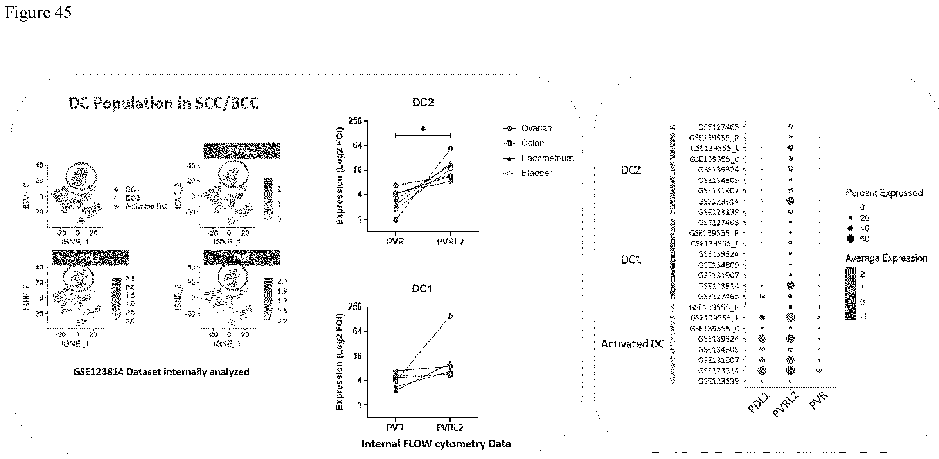

Figure 45: PVRL2 has dominant expression on dendritic cells. CHA.7.518.1.H4(S241P) may enhance interaction and priming of T_{SCM} by DCs in PD-L1^{low} non-inflamed indications.

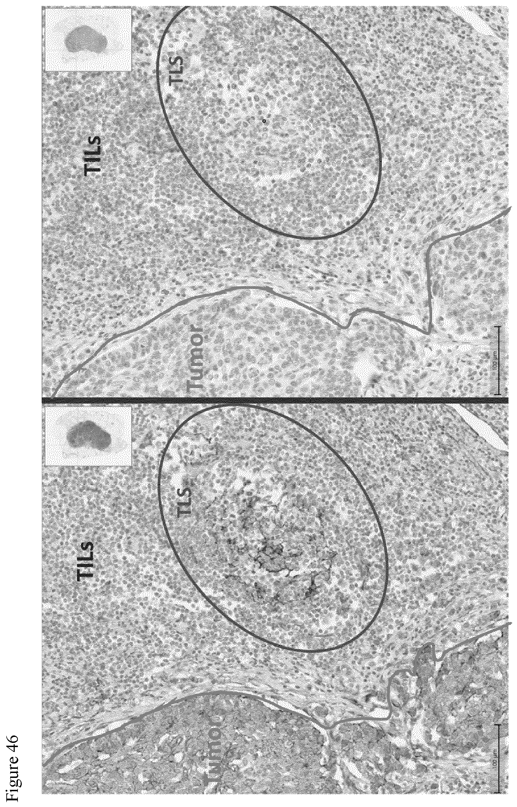

Figure 46: PVRL2 and PVRIG expression in tertiary lymphoid structures (tertiary lymphoid structures are lymphoid structures in the tumor bed in which local T cell priming occur), which is predictive of PD1 response. Supports the potential of CHA.7.518.1.H4(S241P) to enhance T cell proliferation at the tumor bed.

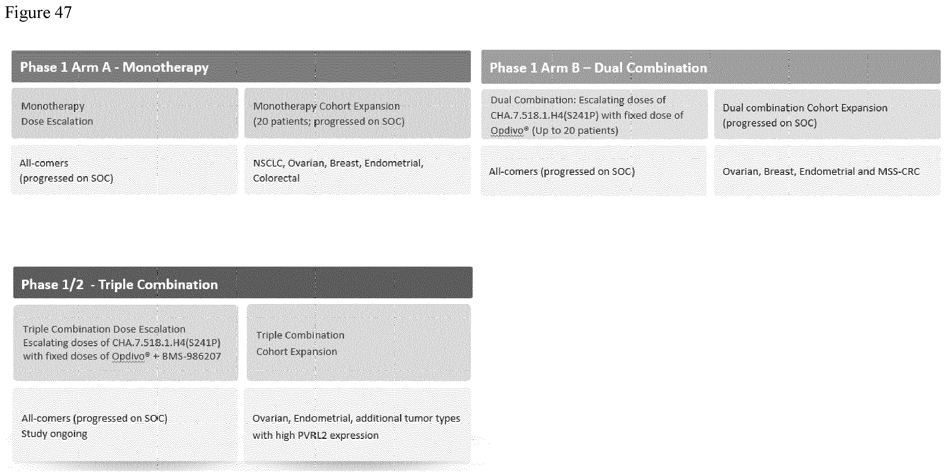

Figure 47 provides a schematic of the clinical study design. CHA.7.518.1.H4(S241P), which is the first PVRIG blocker tested clinically.

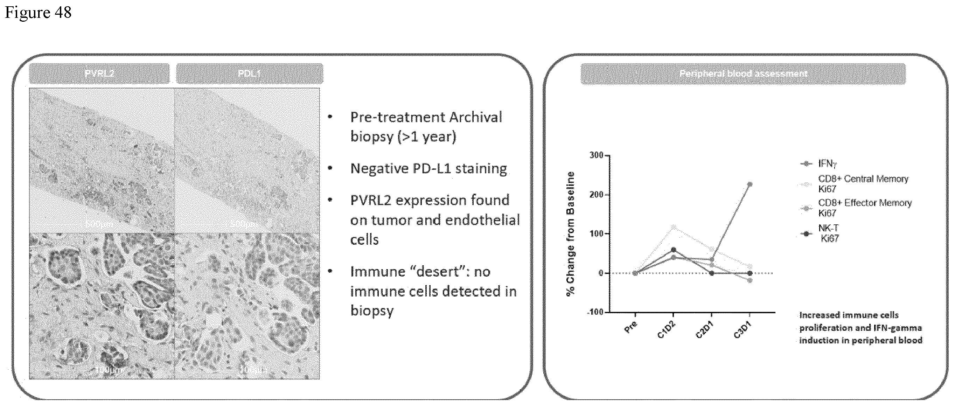

Figure 48 provides data related to confirmed partial response (PR) in patient with primary peritoneal PD-L1 negative cancer treated with CHA.7.518.1.H4(S241P) monotherapy. PR in patient with non-inflamed TME demonstrating immune activation in peripheral blood following CHA.7.518.1.H4(S241P) monotherapy.

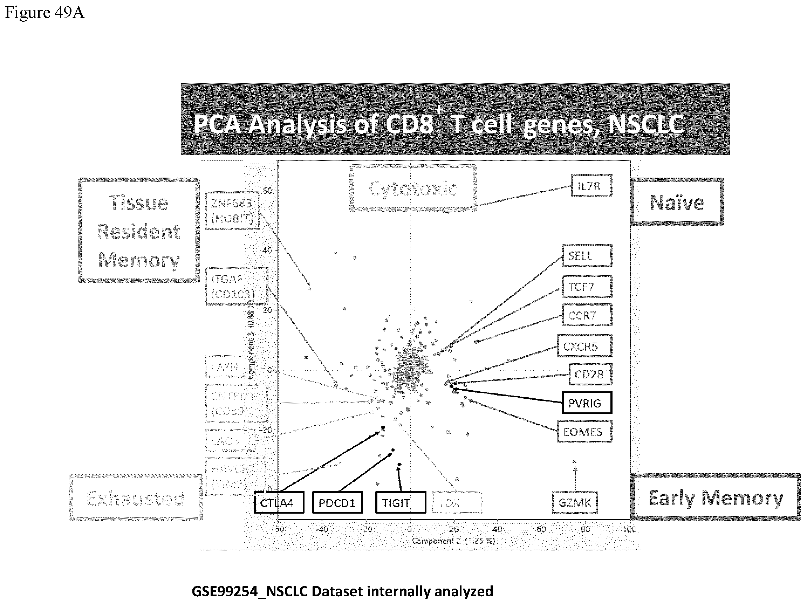

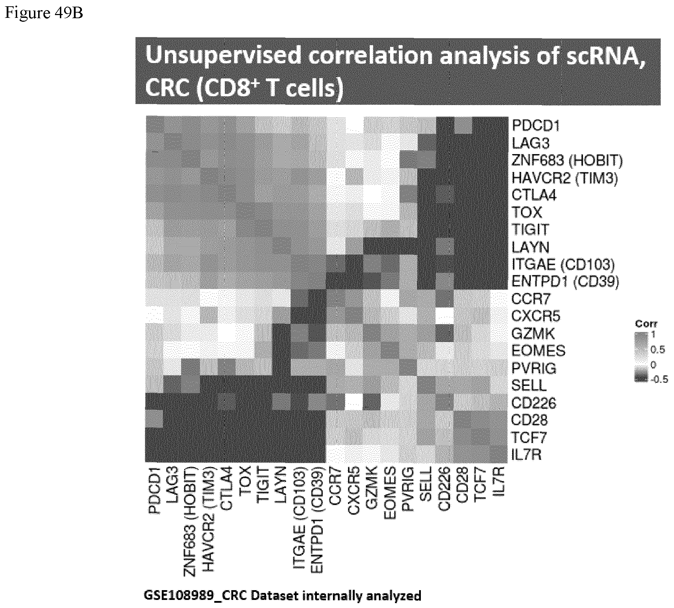

Figure 49A-49B: PVRIG clusters with early differentiation/memory genes, unlike other immune checkpoints that cluster with exhausted genes, in CD8+ T cells.

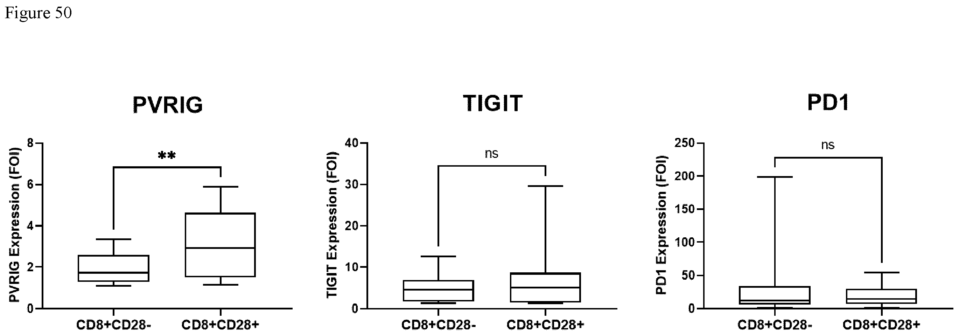

Figure 50: PVRIG is expressed by early memory CD8+ T cells in multiple cancer indications.

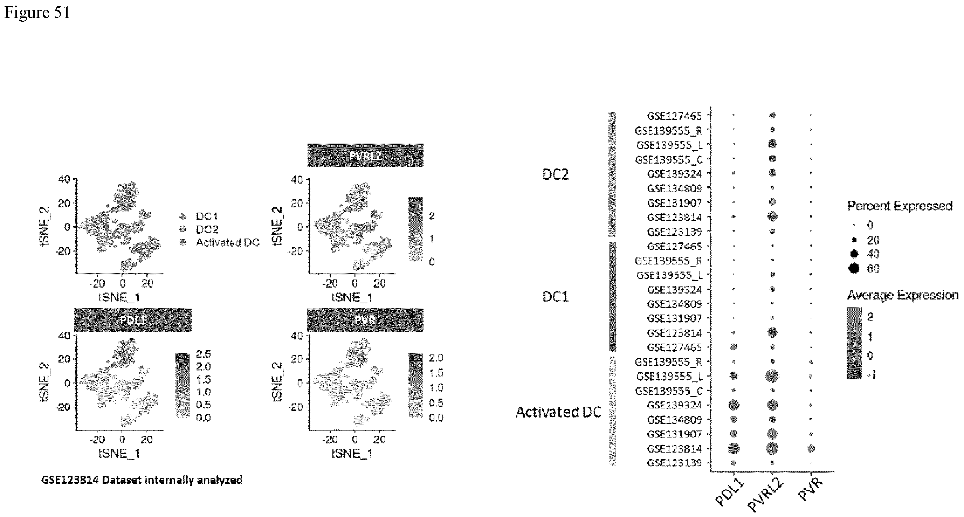

Figure 51: PVRL2 is dominantly expressed on dendritic cells.

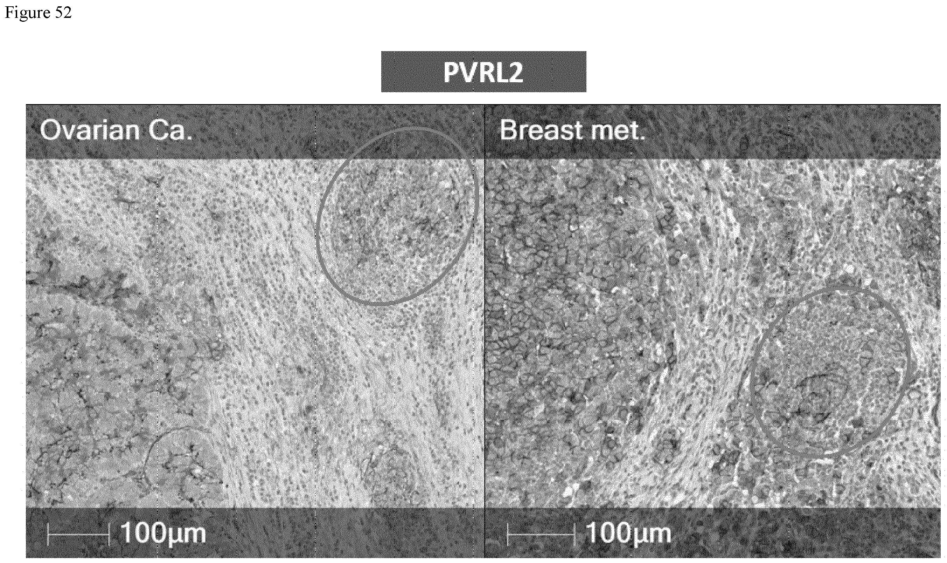

Figure 52: PVRL2 is expressed in Tertiary Lymphoid Structures in the tumor bed. TLSs were identified in subsets of samples across all tumors tested (NSCLC, CRC primary and metastasis, ovarian cancer, endometrial cancer, breast primary TNBC and breast metastasis) and for most cases TLSs were positive for PVRL2. Tertiary lymphoid structures are Lymphoid Structures in the tumor bed in which local T cell activation occur. In addition to tumor cells and endothelial cells, PVRL2 is also expressed on TLSs and immune aggregates formed in tumors (arrows). Staining was performed using a proprietary rabbit mAb raised against the ECD of PVRL2, staining was performed on a Dako Autostainer. Moreover, immunohistochemistry analysis across multiple tumor types, identified PVRL2 expression in Tertiary Lymphoid Structures, local structures in the TME in which in-situ T cell priming occurs. This further support PVRIG-PVRL2 interaction as a potential dominant interaction important for T cell activation in TME.

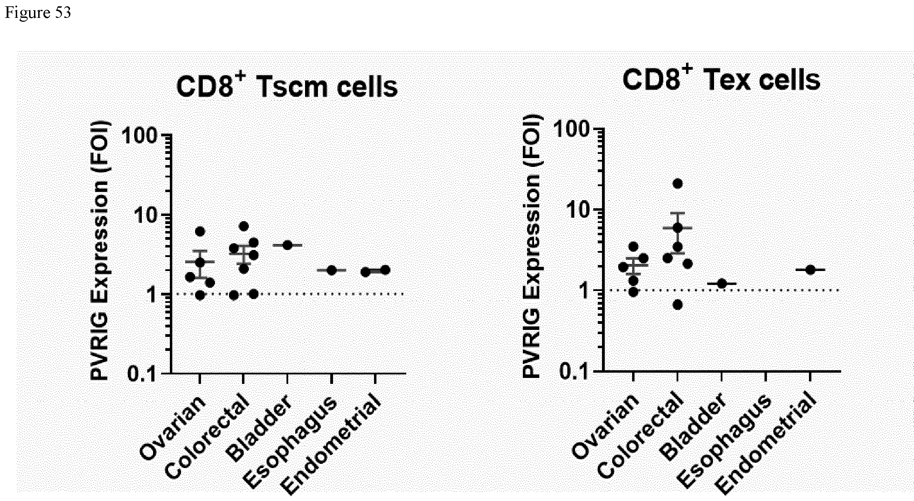

Figure 53: PVRIG is Expressed by Stem-like and Exhausted CD8+ T Cells in the TME. PVRIG is expressed by both stem-like (TIM3⁻CD28⁺PD-1⁺) and exhausted (TIM3⁺CD28⁻PD-1⁺) CD8+ T cells. When looking at PVRIG expression by flow cytometry in various cancer samples, PVRIG, as previously described for TIGIT and PD-1 is expressed on both stem-cell-memory and exhausted CD8 T cells. Indicated cancer samples (n=16) were dissociated to single cell suspensions and analyzed for PVRIG expression by flow-cytometry. PVRIG is expressed by both stem-like (TIM3⁻CD28⁺PD-1⁺) and exhausted (TIM3⁺CD28⁻PD-1⁺) CD8⁺ T cells B.

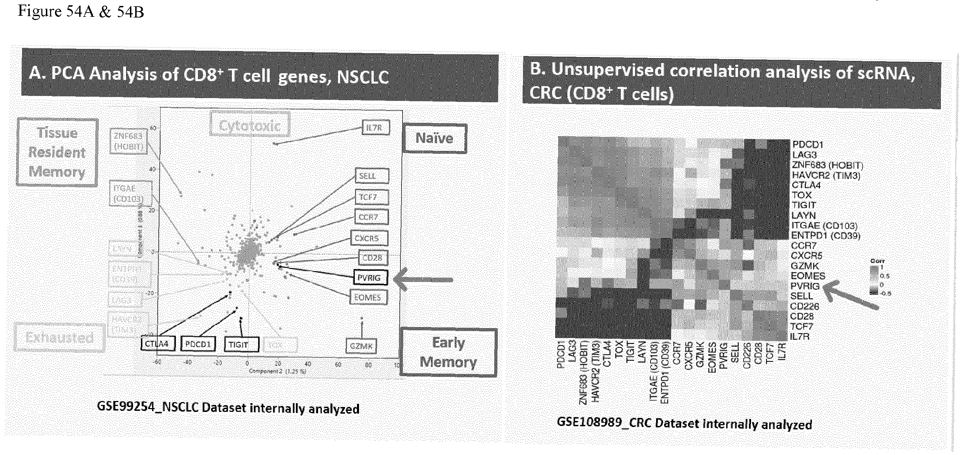

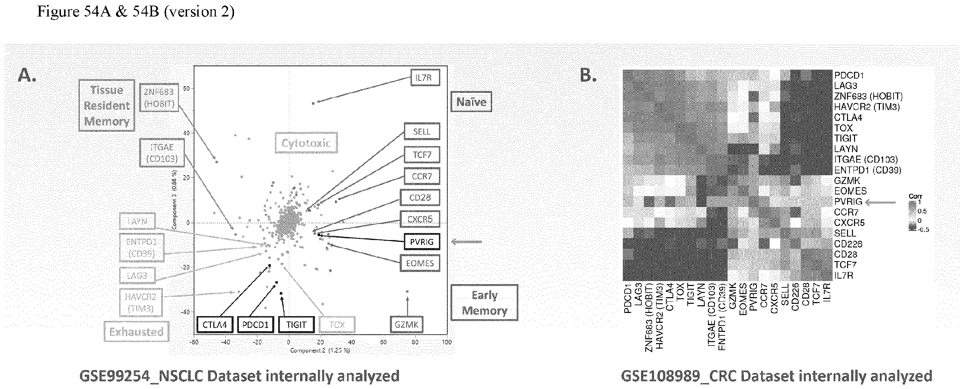

Figure 54A-54B: PVRIG Uniquely Clusters with Markers of Early Memory (Stem-like) CD8+ T Cells. By performing unsupervised principal component analysis on scRNA expression matrix of CD8+ T cells across multiple tumor types, we discovered PVRIG unique expression pattern: while TIGIT is strongly correlated with PD-1, CTLA-4, and other markers of exhausted T-cells, PVRIG uniquely clusters with markers of early memory T-cells, indicating that PVRIG has more dominant expression on these early differentiated cells, which have higher proliferative potential. Additional un-supervised clustering approach yielded similar results as shown in the matrix on the right side.
Figure 54A-54B (version 2): A. Unsupervised PCA was performed on scRNA expression matrix of TME CD8+ T cells, which includes all variable genes. Using cells as features and genes as entries, co-expression pattern among genes known to be expressed on naive, memory, and exhausted CD8+ T cells is shown. Nine publicly available datasets were analyzed, representative NSCLC data set is shown. B. Publicly available scRNA-seq datasets (CRC, NSCLC, HNSCC, Melanoma, Liver cancer, n=13) were analyzed for co-expression pattern among 19 genes, including genes known to be expressed on naive (TCF7, IL7R, SELL), memory (GZMK, EOMES), and exhausted (PDCD1, LAG3, HAVCR2) CD8+ T cells. Representative dataset of CRC is presented.

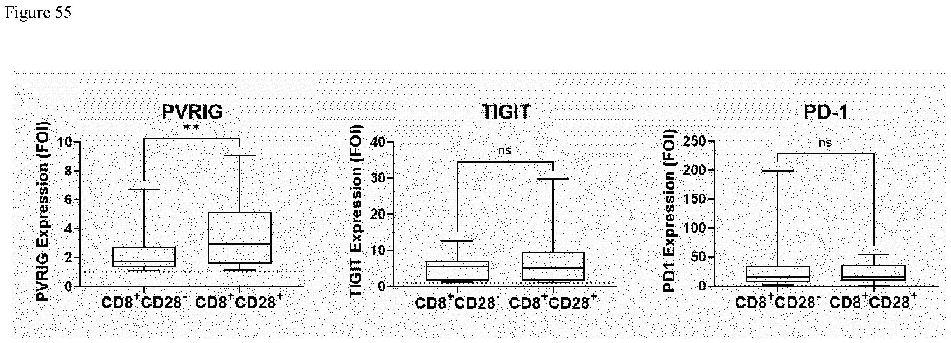

Figure 55: PVRIG Shows Significantly Higher Expression on Early Memory (CD28+) CD8+ T cells Differentiating it from TIGIT and PD-1. CRC, stomach, ovarian, endometrial and bladder cancer (n=13). Accordingly, PVRIG is shown to have a significantly higher expression on early memory (CD28+) CD8+ T cells, in contrast to TIGIT and PD-1. Samples of CRC, stomach, ovarian, endometrial and bladder cancer (n=13) were dissociated to single cell suspensions and analyzed for protein expression by flow-cytometry. Paired t-test was used to compare between PVRIG, TIGIT and PD-1 expression among cell populations. PVRIG is shown to have significant higher expression on early memory (CD28⁺) T cells, in contrast to TIGIT and PD-1 which have comparable expression on CD28⁺ and CD28⁻ T cells.

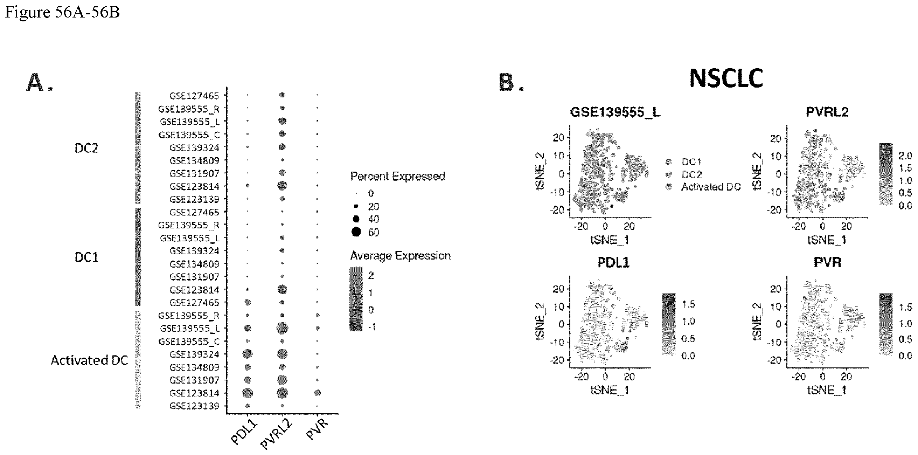

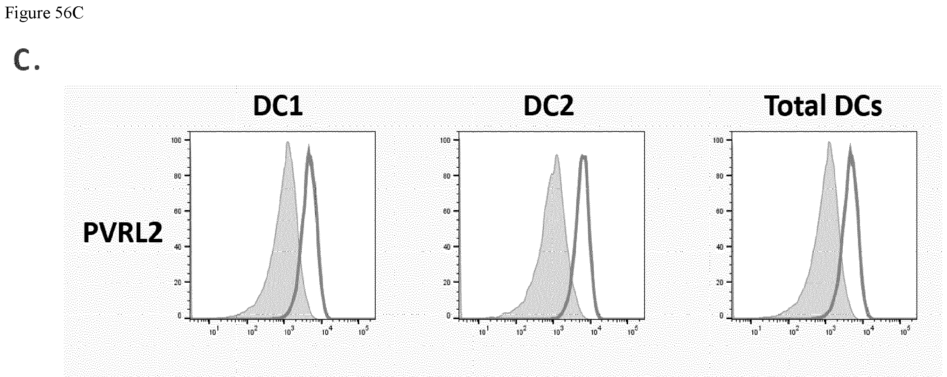

Figure 56A-56C: A)/B) PVRL2 is Dominantly Expressed on Dendritic Cells. When looking at PVRL2, the ligand of PVRIG, RNA expression in multiple datasets, reveals that PVRL2 is more abundantly expressed across DC subtypes compared to PD-L1 and PVR (ligand of TIGIT). A representative NSCLC sample on right, shows the dominant PVRL2 expression on DC1, DC2 and activated DCs. C) PVRL2 Protein is Expressed across Dendritic Cell Sub-Types (Representative ovarian sample shown). Flow cytometry analysis validated RNA results, confirming PVRL2 protein expression across tumor types. A representative plot of ovarian tumor shows dominant PVRL2 expression on DC subtypes.

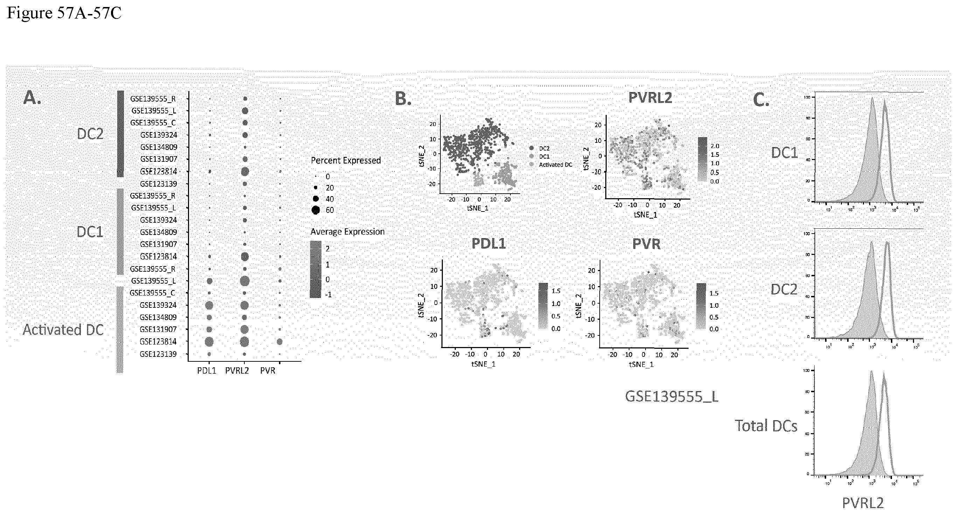

Figure 57A-57C: A) Dot plots showing the percent of cells and average level of expression of PVR/PVRL2/PD-L1 in major dendritic cell subsets across multiple publicly available scRNA-seq cancer datasets. B) Representative tSNE map depicting the expression profile of PVR/PVRL2/PD-L1 in major dendritic cell subsets in NSCLC. C) PVRL2 protein expression across DC subsets in a representative ovarian cancer sample analyzed by flow-cytometry.

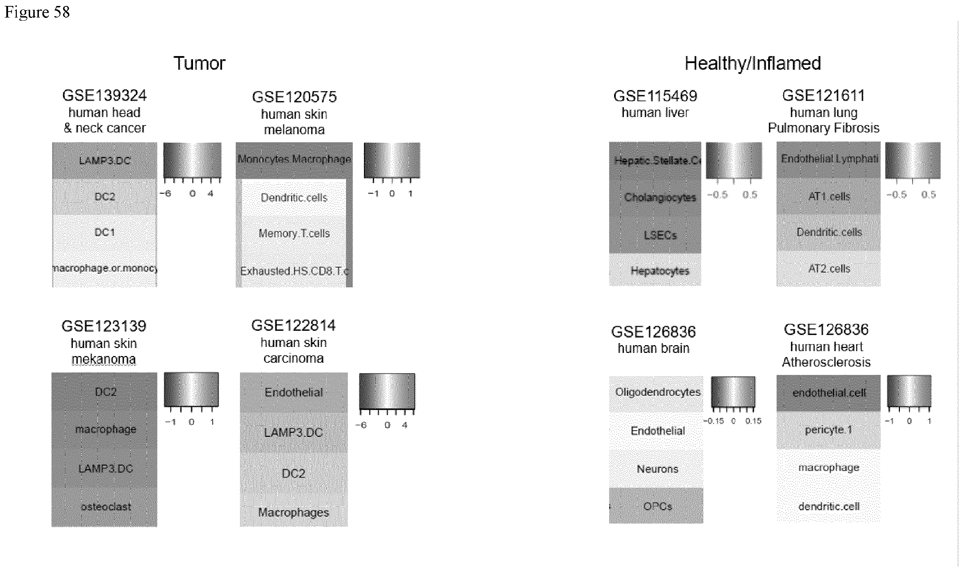

Figure 58 provides PVRL2 expression from multiple scRNA data sets: tumor, healthy, and inflamed.

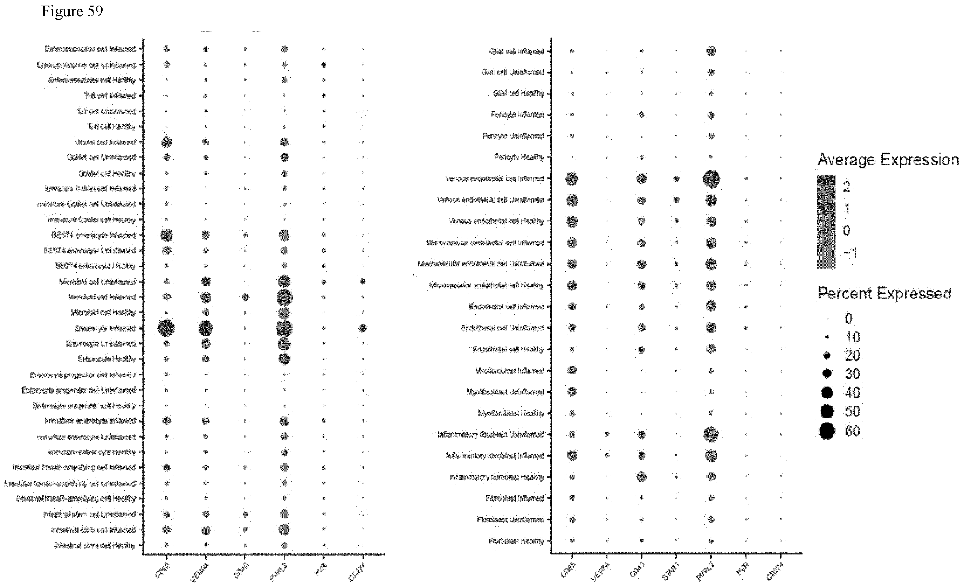

Figure 59 provides PVRL2 is overexpressed by endothelial cells and/or enterocytes in healthy and/or inflamed gastrointestinal (GI) tracts.

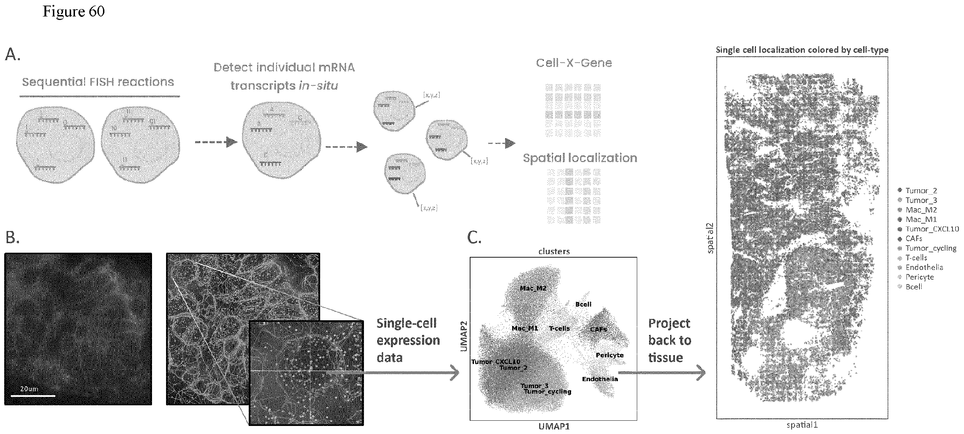

Figure 60A-60C: Technology. A) Illustration of the MERFISH technology, using sequential FISH reactions each transcript is detected by series of binary barcodes which are later translated into distinct mRNA transcripts. Transcripts are then quantified per cell as detected by cell-border stain to obtain single-cell expression matrix and spatial localization map. B) Image showing cell boundary stain in CRC tumor (left) and the overlayed boundaries as detected algorithmically (gray line in the middle image) individual mRNA transcripts appear as colored dots. Example of individual cell and its associated transcripts (bottom left). C) (left) UMAP showing the clustering of cells based on the extracted single-cell data and later projected back onto the spatial localization (right). M1 - inflamatory macrophages. M2 - anti-inflamatory macrophages.

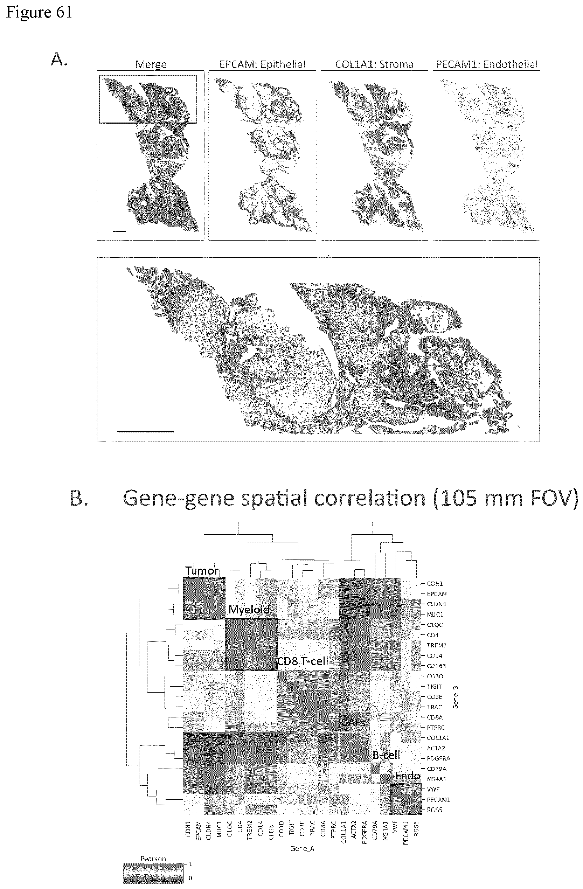

Figure 61A-61B: Spatial mRNA correlation provides means to study gene association. A) Images showing the spatial distribution in-situ of selected mRNA probes, representing different lineages, in Ovarian tumor sample. Bottom panel shows enlarged region of the overlayed image. Scale bar 1mm. B) Spatial correlation of selected genes representing different lineages based on Pearson correlation in window size of 105 mm. It is observed that genes specific to different cell type spatially co-localize and thus cluster together.

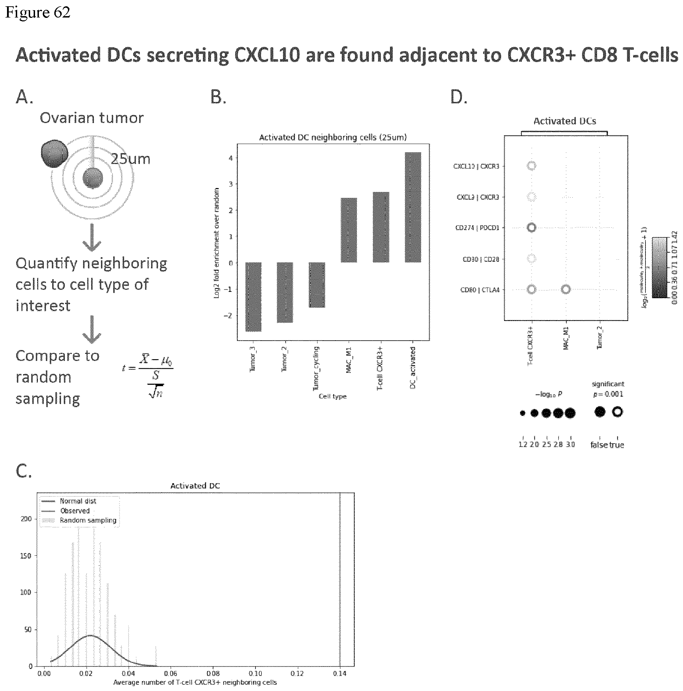

Figure 62A-62B: Profiling cellular neighborhood at the single cell level. A) Schematic of cellular neighborhood profiling methodology. B) Enrichment of cell populations next to activated DCs vs random sampling. C) Average number of CXCR3+ T-cell within the neighborhood of activated DCs, comparing random sampling to observed distribution. P-value 2.95E-32, T-Test. D) Predicted cell-cell interactions between activated DCs and the listed cell populations, considering both expression levels of receptor-ligand and cell-proximity it is suggested that Activated DCs interact with neigboring CXCR3+ T-cells.

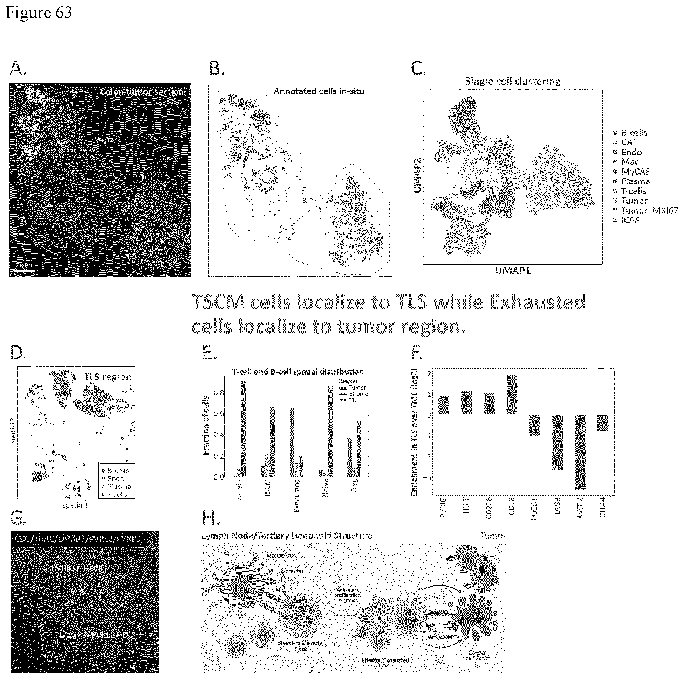

Figure 63A-63H: DNAM-1 axis shows dominant presence in tertiary lymphoid structures (TLS). A) DAPI pseudo-coloring of colorectal tumor section highlighting tumor, stroma and TLS regions. B) Spatial localization of single cells corresponding to (A). C) UMAP showing single-cell clustering of single cells. D) Localization of selected cell types in the TLS region showing the unique co-localization. E) Relative abundance of types of T-cells and B-cells in the three regions noted in (A). F) Fold enrichment of selected checkpoints shows preferential expression of PVRIG, TIGIT and DNAM-1 at the TLS region versus TME. G) Image showing individual PVRIG+ T-cell adjacent to LMAP3+PVRL2+ DC in the TLS region. H) Summarizing illustration showcasing the activity of PVRIG in the TLS regions and potential contribution of CHA.7.518.1.H4(S241P) (anti-PVRIG) treatment to activating immune response. (*Zoya Alteber, et al., SITC 2021.)

### DETAILED DESCRIPTION OF THE INVENTION

### I. INTRODUCTION

Cancer can be considered as an inability of the patient to recognize and eliminate cancerous cells. In many instances, these transformed (e.g., cancerous) cells counteract immunosurveillance. There are natural control mechanisms that limit T-cell activation in the body to prevent unrestrained T-cell activity, which can be exploited by cancerous cells to evade or suppress the immune response. Restoring the capacity of immune effector cells-especially T cells-to recognize and eliminate cancer is the goal of immunotherapy. The field of immuno-oncology, sometimes referred to as "immunotherapy" is rapidly evolving, with several recent approvals of T cell checkpoint inhibitory antibodies such as Yervoy, Keytruda and Opdivo. These antibodies are generally referred to as "checkpoint inhibitors" because they block normally negative regulators of T cell immunity. It is generally understood that a variety of immunomodulatory signals, both costimulatory and coinhibitory, can be used to orchestrate an optimal antigen-specific immune response. Generally, these antibodies bind to checkpoint inhibitor proteins such as CTLA-4 and PD-1, which under normal circumstances prevent or suppress activation of cytotoxic T cells (CTLs). By inhibiting the checkpoint protein, for example through the use of antibodies that bind these proteins, an increased T cell response against tumors can be achieved. That is, these cancer checkpoint proteins suppress the immune response; when the proteins are blocked, for example using antibodies to the checkpoint protein, the immune system is activated, leading to immune stimulation, resulting in treatment of conditions such as cancer and infectious disease. PVRIG is expressed on the cell surface of NK and T-cells and shares several similarities to other known immune checkpoints. As indicated about, PVRL2 has been identified as the ligand for PVRIG.

The present invention is directed to biomarkers for use in determining populations for treatment with antibodies to human Poliovirus Receptor Related Immunoglobulin Domain Containing Protein, or "PVRIG", sometimes also referred to herein as "PV protein" (e.g., anti-PVRIG antibodies including those with CDRs identical to those shown in Figure 3). Such biomarkers include, for example PVRIG and/or PVRL2 expression, as described herein, as well as DNAM-1 expression. In some embodiments, the biomarker is PVRIG expression. In some embodiments, the biomarker is PVRL2 expression. In some embodiments, the biomarker is DNAM-1 expression. In some embodiments, the biomarker is PVRIG expression. In some embodiments, the biomarker is PVRL2 expression.

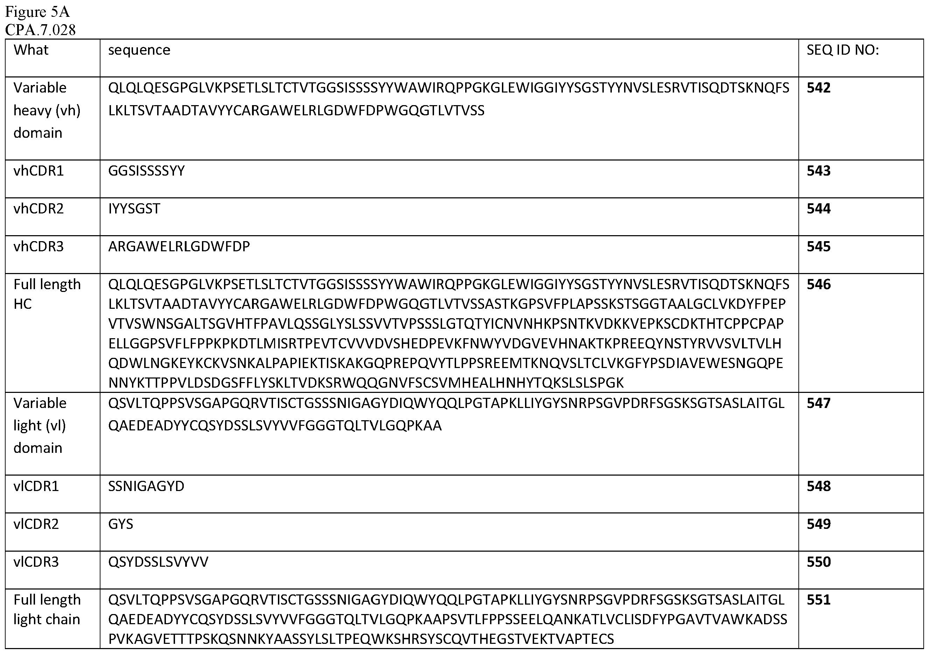

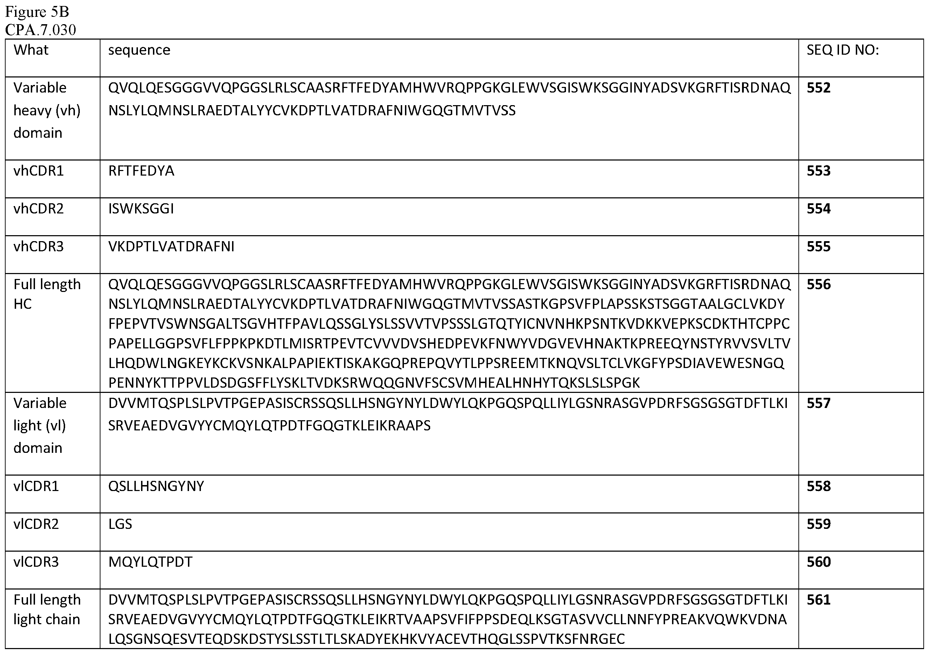

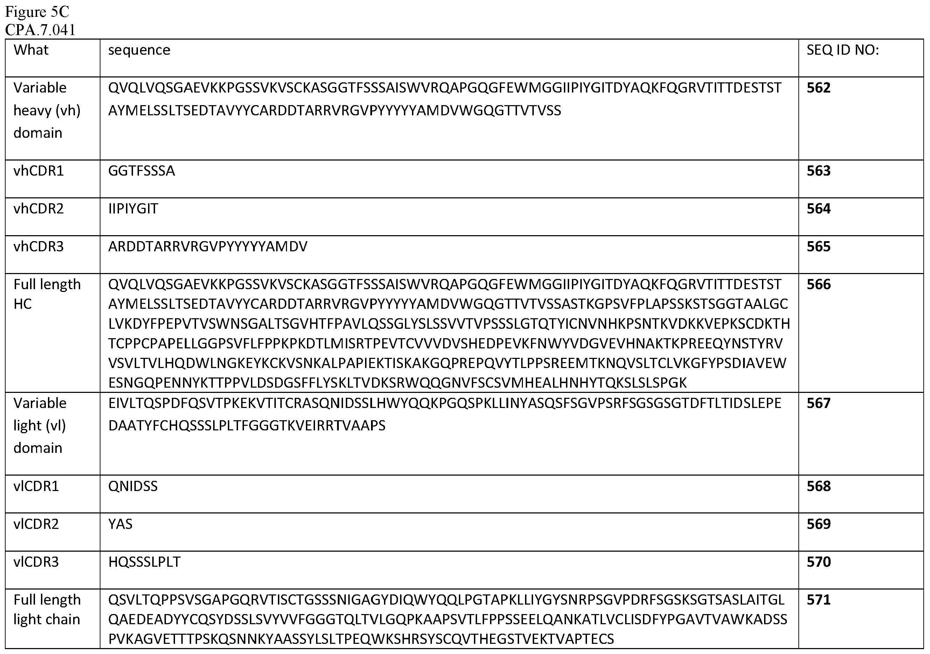

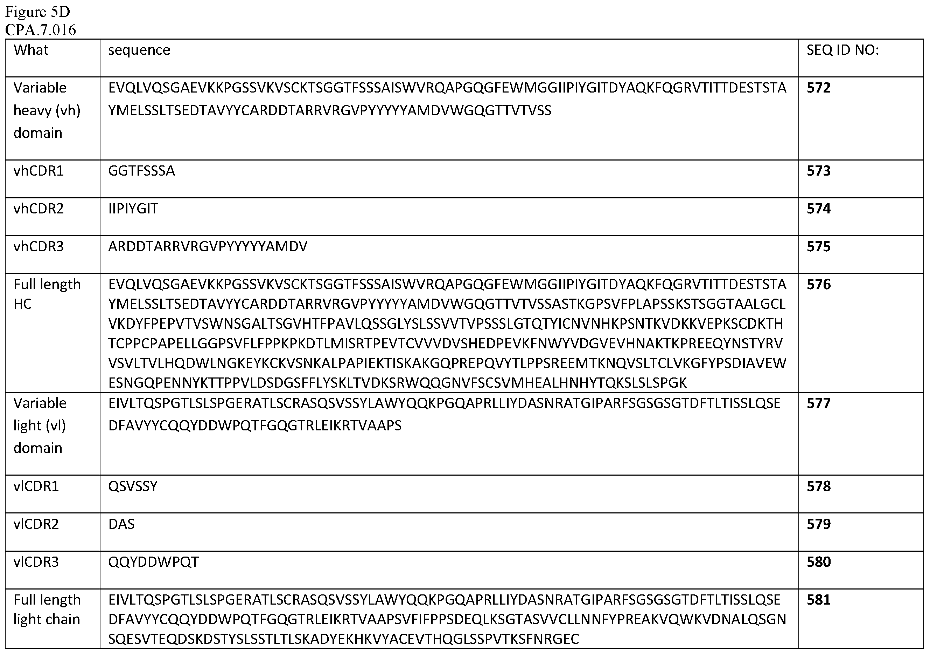

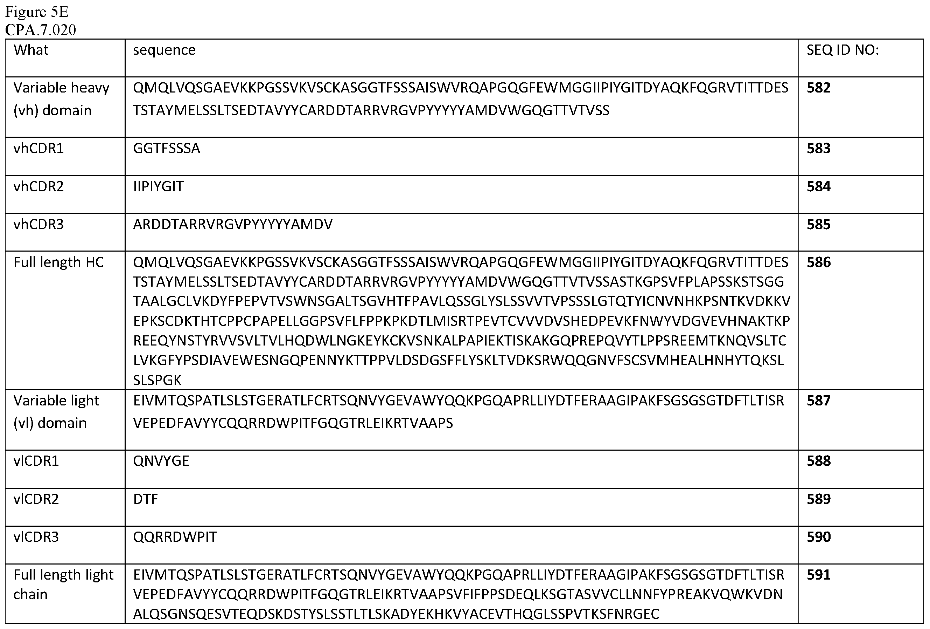

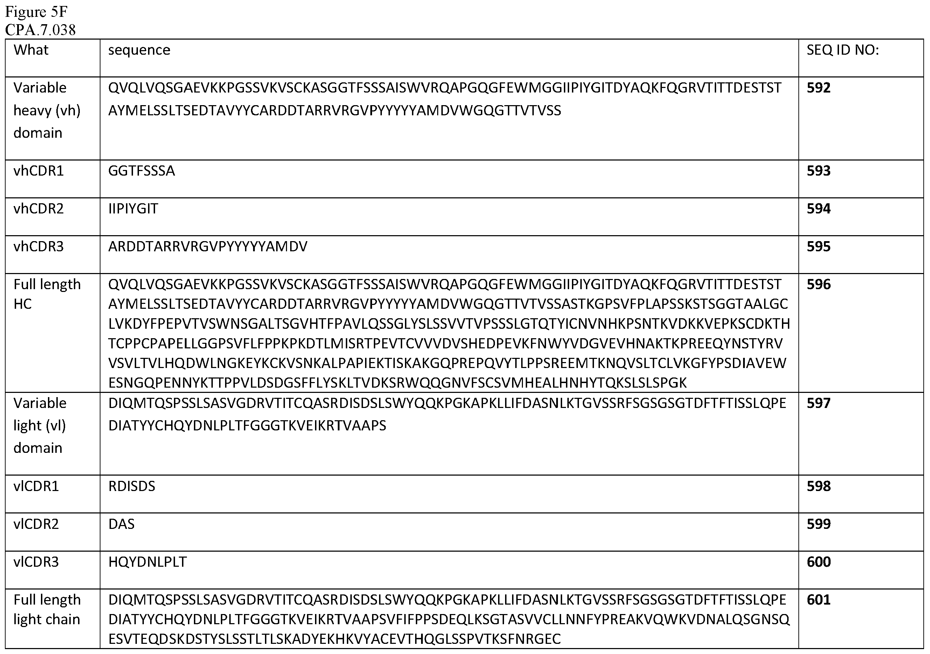

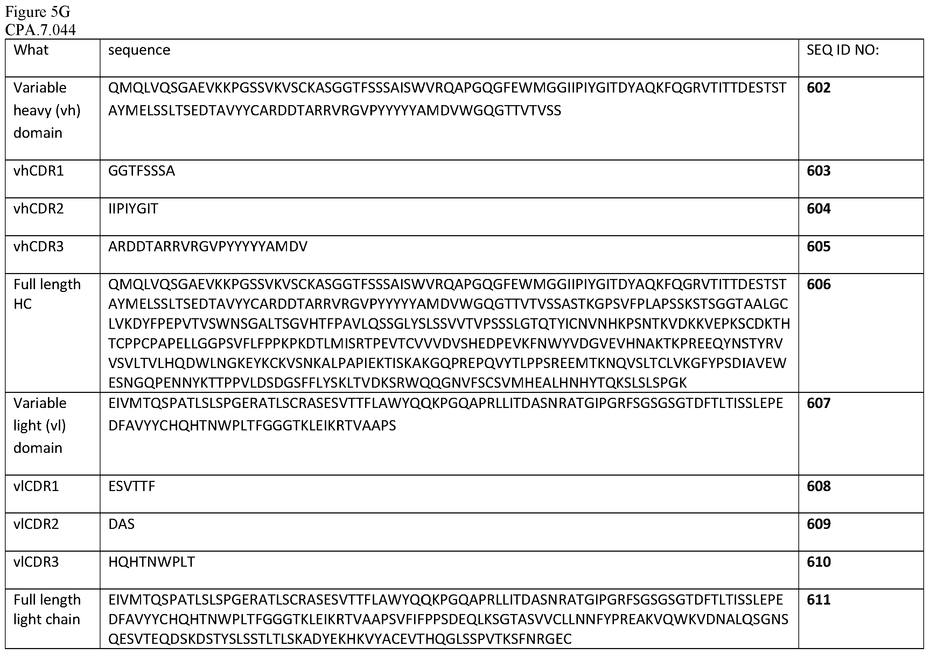

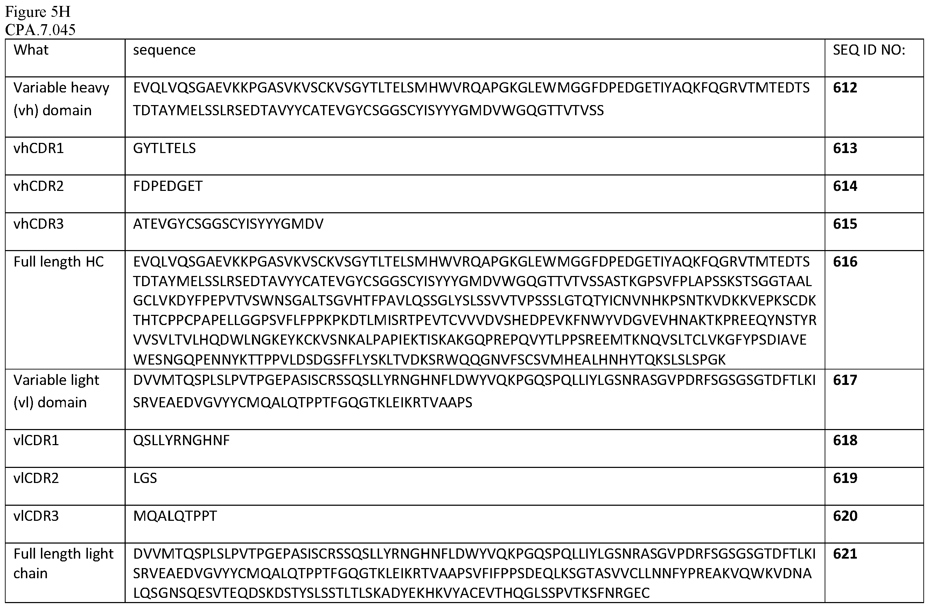

The present invention also provides biomarkers for use with formulations comprising anti-PVRIG antibodies, including antigen binding domains, that bind to the human PVRIG and peptides thereof and methods of activating T cells and/or NK cells to treat diseases such as cancer and infectious diseases, and other conditions where increased immune activity results in treatment. In particular, these formulations comprise antibodies comprising heavy and light chains as well as the vhCDR1, vhCDR2, vhCDR3, vlCDR1, vlCDR2 and vlCDR3 sequences from CHA.7.518.1.H4(S241P). In some embodiments, anti-PVRIG antibodies include those with CDRs identical to those shown in Figure 3. In some embodiments, anti-PVRIG antibodies include those with CDRs identical to those shown in Figures 5A-5D, as well as anti-PVRIG antibodies comprising the heavy and light chains as provided in Figures 5A-5D.

### II. PVRIG PROTEINS

The present invention provides formulations comprising antibodies that specifically bind to PVRIG proteins. "Protein" in this context is used interchangeably with "polypeptide", and includes peptides as well. The present invention provides antibodies that specifically bind to PVRIG proteins. PVRIG is a transmembrane domain protein of 326 amino acids in length, with a signal peptide (spanning from amino acid 1 to 40), an extracellular domain (spanning from amino acid 41 to 171), a transmembrane domain (spanning from amino acid 172 to 190) and a cytoplasmic domain (spanning from amino acid 191 to 326). The full length human PVRIG protein is shown in Figure 1. There are two methionines that can be start codons, but the mature proteins are identical.

Accordingly, as used herein, the term "PVRIG" or "PVRIG protein" or "PVRIG polypeptide" may optionally include any such protein, or variants, conjugates, or fragments thereof, including but not limited to known or wild type PVRIG, as described herein, as well as any naturally occurring splice variants, amino acid variants or isoforms, and in particular the ECD fragment of PVRIG. The term "soluble" form of PVRIG is also used interchangeably with the terms "soluble ectodomain (ECD)" or "ectodomain" or "extracellular domain (ECD) as well as "fragments of PVRIG polypeptides", which may refer broadly to one or more of the following optional polypeptides:

The PVRIG proteins contain an immunoglobulin (Ig) domain within the extracellular domain, which is a PVR-like Ig fold domain. The PVR-like Ig fold domain may be responsible for functional counterpart binding, by analogy to the other B7 family members. The PVR-like Ig fold domain of the extracellular domain includes one disulfide bond formed between intra domain cysteine residues, as is typical for this fold and may be important for structure-function. These cysteines are located at residues 22 and 93 (or 94). In one embodiment, there is provided a soluble fragment of PVRIG that can be used in testing of PVRIG antibodies. Included within the definition of PVRIG proteins are PVRIG ECD fragments, including know ECD fragments such as those described in U.S. Patent No. 9,714, 289, incorporate by reference herein in its entirety for all purposes.

As noted herein and more fully described below, the anti-PVRIG antibodies (including antigen-binding fragments) that both bind to PVRIG and prevent activation by PVRL2 (e.g., most commonly by blocking the interaction of PVRIG and PVLR2), are used to enhance T cell and/or NK cell activation and be used in treating diseases such as cancer and pathogen infection.

### III. TIGIT PROTEINS

The present invention provides antibodies that specifically bind to TIGIT proteins and prevent activation by its ligand protein, PVR, poliovirus receptor (*aka*, CD155) a human plasma membrane glycoprotein. TIGIT, or T cell immunoreceptor with Ig and ITIM domains, is a coinhibitory receptor protein also known as WUCAM, Vstm3 or Vsig9. TIGIT has an immunoglobulin variable domain, a transmembrane domain, and an immunoreceptor tyrosine-based inhibitory motif (ITIM) and contains signature sequence elements of the PVR protein family. The extracellular domain (ECD) sequences of TIGIT and of PVR are shown in Figure 1B. The antibodies of the invention are specific for the TIGIT ECD such that the binding of TIGIT and PVR is blocked

Accordingly, as used herein, the term "TIGIT" or "TIGIT protein" or "TIGIT polypeptide" may optionally include any such protein, or variants, conjugates, or fragments thereof, including but not limited to known or wild type TIGIT, as described herein, as well as any naturally occurring splice variants, amino acid variants or isoforms, and in particular the ECD fragment of TIGIT.

As noted herein and more fully described below, anti-TIGIT antibodies (including antigen-binding fragments) that both bind to TIGIT and prevent activation by PVR (e.g., most commonly by blocking the interaction of TIGIT and PVR), are used to enhance T cell and/or NK cell activation and be used in treating diseases such as cancer and pathogen infection.

### IV. ANTIBODIES

Accordingly, the invention provides anti-PVRIG antibodies that can be formulated according to the formulations and invention described herein and which are provided in Figure 3 (*e*.*g*., including anti-PVRIG antibodies including those with CDRs identical to those shown in Figure 3). PVRIG, also called Poliovirus Receptor Related Immunoglobulin Domain Containing Protein, Q6DKI7 or C7orf15, relates to amino acid and nucleic acid sequences shown in RefSeq accession identifier NP_076975, shown in Figure 1. The antibodies of the invention are specific for the PVRIG extracellular domain.

As is discussed below, the term "antibody" is used generally. Antibodies that find use in the present invention can take on a number of formats as described herein, including traditional antibodies as well as antibody derivatives, fragments and mimetics, described below. In general, the term "antibody" includes any polypeptide that includes at least one antigen binding domain, as more fully described below. Antibodies may be polyclonal, monoclonal, xenogeneic, allogeneic, syngeneic, or modified forms thereof, as described herein, with monoclonal antibodies finding particular use in many embodiments. In some embodiments, antibodies of the invention bind specifically or substantially specifically to PVRIG molecules. The terms "monoclonal antibodies" and "monoclonal antibody composition", as used herein, refer to a population of antibody molecules that contain only one species of an antigen-binding site capable of immunoreacting with a particular epitope of an antigen, whereas the term "polyclonal antibodies" and "polyclonal antibody composition" refer to a population of antibody molecules that contain multiple species of antigen-binding sites capable of interacting with a particular antigen. A monoclonal antibody composition, typically displays a single binding affinity for a particular antigen with which it immunoreacts.

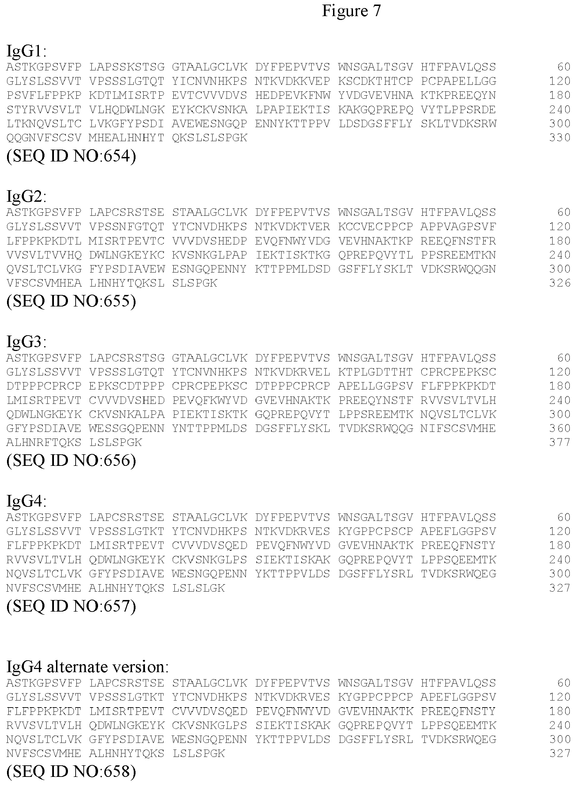

Traditional full length antibody structural units typically comprise a tetramer. Each tetramer is typically composed of two identical pairs of polypeptide chains, each pair having one "light" (typically having a molecular weight of about 25 kDa) and one "heavy" chain (typically having a molecular weight of about 50-70 kDa). Human light chains are classified as kappa and lambda light chains. The present invention is directed to the IgG class, which has several subclasses, including, but not limited to IgG1, IgG2, IgG3, and IgG4. Thus, "isotype" as used herein is meant any of the subclasses of immunoglobulins defined by the chemical and antigenic characteristics of their constant regions. While the exemplary antibodies herein designated "CPA" are based on IgG1 heavy constant regions, as shown in Figure 7, the anti-PVRIG antibodies of the invention include those using IgG2, IgG3 and IgG4 sequences, or combinations thereof. For example, as is known in the art, different IgG isotypes have different effector functions which may or may not be desirable. Accordingly, the CPA antibodies of the invention can also swap out the IgG1 constant domains for IgG2, IgG3 or IgG4 constant domains (depicted in Figure 7), with IgG2 and IgG4 finding particular use in a number of situations, for example for ease of manufacture or when reduced effector function is desired, the latter being desired in some situations.

The amino-terminal portion of each chain includes a variable region of about 100 to 110 or more amino acids primarily responsible for antigen recognition, generally referred to in the art and herein as the "Fv domain" or "Fv region". In the variable region, three loops are gathered for each of the V domains of the heavy chain and light chain to form an antigen-binding site. Each of the loops is referred to as a complementarity-determining region (hereinafter referred to as a "CDR"), in which the variation in the amino acid sequence is most significant. "Variable" refers to the fact that certain segments of the variable region differ extensively in sequence among antibodies. Variability within the variable region is not evenly distributed. Instead, the V regions consist of relatively invariant stretches called framework regions (FRs) of 15-30 amino acids separated by shorter regions of extreme variability called "hypervariable regions".

Each VH and VL is composed of three hypervariable regions ("complementary determining regions," "CDRs") and four FRs, arranged from amino-terminus to carboxy-terminus in the following order: FR1-CDR1-FR2-CDR2-FR3-CDR3-FR4.

The hypervariable region generally encompasses amino acid residues from about amino acid residues 24-34 (LCDR1; "L" denotes light chain), 50-56 (LCDR2) and 89-97 (LCDR3) in the light chain variable region and around about 31-35B (HCDR1; "H" denotes heavy chain), 50-65 (HCDR2), and 95-102 (HCDR3) in the heavy chain variable region, although sometimes the numbering is shifted slightly as will be appreciated by those in the art; Kabat et al., SEQUENCES OF PROTEINS OF IMMUNOLOGICAL INTEREST, 5 th Ed. Public Health Service, National Institutes of Health, Bethesda, Md. (1991) and/or those residues forming a hypervariable loop (*e*.*g*., residues 26-32 (LCDR1), 50-52 (LCDR2) and 91-96 (LCDR3) in the light chain variable region and 26-32 (HCDR1), 53-55 (HCDR2) and 96-101 (HCDR3) in the heavy chain variable region; Chothia and Lesk (1987) J. Mol. Biol. 196:901-917. Specific CDRs of the invention are described below and shown in Figure 3.

The carboxy-terminal portion of each chain defines a constant region primarily responsible for effector function. Kabat et al. collected numerous primary sequences of the variable regions of heavy chains and light chains. Based on the degree of conservation of the sequences, they classified individual primary sequences into the CDR and the framework and made a list thereof (see SEQUENCES OF IMMUNOLOGICAL INTEREST, 5 th edition, NIH publication, No. 91-3242, E. A. Kabat et al., entirely incorporated by reference).

In the IgG subclass of immunoglobulins, there are several immunoglobulin domains in the heavy chain. By "immunoglobulin (Ig) domain" herein is meant a region of an immunoglobulin having a distinct tertiary structure. Of interest in the present invention are the heavy chain domains, including, the constant heavy (CH) domains and the hinge domains. In the context of IgG antibodies, the IgG isotypes each have three CH regions. Accordingly, "CH" domains in the context of IgG are as follows: "CH1" refers to positions 118-220 according to the EU index as in Kabat. "CH2" refers to positions 237-340 according to the EU index as in Kabat, and "CH3" refers to positions 341-447 according to the EU index as in Kabat.

Accordingly, the invention provides variable heavy domains, variable light domains, heavy constant domains, light constant domains and Fc domains to be used as outlined herein. By "variable region" as used herein is meant the region of an immunoglobulin that comprises one or more Ig domains substantially encoded by any of the Vκ or Vλ, and/or VH genes that make up the kappa, lambda, and heavy chain immunoglobulin genetic loci respectively. Accordingly, the variable heavy domain comprises vhFR1-vhCDR1-vhFR2-vhCDR2-vhFR3-vhCDR3-vhFR4, and the variable light domain comprises vlFR1-vlCDR1-vlFR2-vlCDR2-vlFR3-vlCDR3-vlFR4. By "heavy constant region" herein is meant the CH1-hinge-CH2-CH3 portion of an antibody. By "Fc" or "Fc region" or "Fc domain" as used herein is meant the polypeptide comprising the constant region of an antibody excluding the first constant region immunoglobulin domain and in some cases, part of the hinge. Thus Fc refers to the last two constant region immunoglobulin domains of IgA, IgD, and IgG, the last three constant region immunoglobulin domains of IgE and IgM, and the flexible hinge N-terminal to these domains. For IgA and IgM, Fc may include the J chain. For IgG, the Fc domain comprises immunoglobulin domains Cγ2 and Cγ3 (Cγ2 and Cγ3) and the lower hinge region between Cγ1 (Cγ1) and Cγ2 (Cγ2). Although the boundaries of the Fc region may vary, the human IgG heavy chain Fc region is usually defined to include residues C226 or P230 to its carboxyl-terminus, wherein the numbering is according to the EU index as in Kabat. In some embodiments, as is more fully described below, amino acid modifications are made to the Fc region, for example to alter binding to one or more FcyR receptors or to the FcRn receptor.

Thus, "Fc variant" or "variant Fc" as used herein is meant a protein comprising an amino acid modification in an Fc domain. The Fc variants of the present invention are defined according to the amino acid modifications that compose them. Thus, for example, N434S or 434S is an Fc variant with the substitution serine at position 434 relative to the parent Fc polypeptide, wherein the numbering is according to the EU index. Likewise, M428L/N434S defines an Fc variant with the substitutions M428L and N434S relative to the parent Fc polypeptide. The identity of the WT amino acid may be unspecified, in which case the aforementioned variant is referred to as 428L/434S. It is noted that the order in which substitutions are provided is arbitrary, that is to say that, for example, 428L/434S is the same Fc variant as M428L/N434S, and so on. For all positions discussed in the present invention that relate to antibodies, unless otherwise noted, amino acid position numbering is according to the EU index.

By "Fab" or "Fab region" as used herein is meant the polypeptide that comprises the VH, CH1, VL, and CL immunoglobulin domains. Fab may refer to this region in isolation, or this region in the context of a full length antibody, antibody fragment or Fab fusion protein. By "Fv" or "Fv fragment" or "Fv region" as used herein is meant a polypeptide that comprises the VL and VH domains of a single antibody. As will be appreciated by those in the art, these generally are made up of two chains.

Throughout the present specification, either the IMTG numbering system or the Kabat numbering system is generally used when referring to a residue in the variable domain (approximately, residues 1-107 of the light chain variable region and residues 1-113 of the heavy chain variable region) (e.g., Kabat et al., supra (1991)). EU numbering as in Kabat is generally used for constant domains and/or the Fc domains.

The CDRs contribute to the formation of the antigen-binding, or more specifically, epitope binding site of antibodies. "Epitope" refers to a determinant that interacts with a specific antigen binding site in the variable region of an antibody molecule known as a paratope. Epitopes are groupings of molecules such as amino acids or sugar side chains and usually have specific structural characteristics, as well as specific charge characteristics. A single antigen may have more than one epitope.

The epitope may comprise amino acid residues directly involved in the binding (also called immunodominant component of the epitope) and other amino acid residues, which are not directly involved in the binding, such as amino acid residues which are effectively blocked by the specifically antigen binding peptide; in other words, the amino acid residue is within the footprint of the specifically antigen binding peptide.

Epitopes may be either conformational or linear. A conformational epitope is produced by spatially juxtaposed amino acids from different segments of the linear polypeptide chain. A linear epitope is one produced by adjacent amino acid residues in a polypeptide chain. Conformational and nonconformational epitopes may be distinguished in that the binding to the former but not the latter is lost in the presence of denaturing solvents.

An epitope typically includes at least 3, and more usually, at least 5 or 8-10 amino acids in a unique spatial conformation. Antibodies that recognize the same epitope can be verified in a simple immunoassay showing the ability of one antibody to block the binding of another antibody to a target antigen, for example "binning". Specific bins are described below.

Included within the definition of "antibody" is an "antigen-binding portion" of an antibody (also used interchangeably with "antigen-binding fragment", "antibody fragment" and "antibody derivative"). That is, for the purposes of the invention, an antibody of the invention has a minimum functional requirement that it bind to a PVRIG antigen. As will be appreciated by those in the art, there are a large number of antigen fragments and derivatives that retain the ability to bind an antigen and yet have alternative structures, including, but not limited to, (i) the Fab fragment consisting of VL, VH, CL and CH1 domains, (ii) the Fd fragment consisting of the VH and CH1 domains, (iii) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., 1988, Science 242:423-426, Huston et al., 1988, Proc. Natl. Acad. Sci. U.S.A. 85:5879-5883, entirely incorporated by reference), (iv) "diabodies" or "triabodies", multivalent or multispecific fragments constructed by gene fusion (Tomlinson et. al., 2000, Methods Enzymol. 326:461-479; WO94/13804; Holliger et al., 1993, Proc. Natl. Acad. Sci. U.S.A. 90:6444-6448, all entirely incorporated by reference), (v) "domain antibodies" or "dAb" (sometimes referred to as an "immunoglobulin single variable domain", including single antibody variable domains from other species such as rodent (for example, as disclosed in WO 00/29004), nurse shark and Camelid V-HH dAbs, (vi) SMIPs (small molecule immunopharmaceuticals), camelbodies, nanobodies and IgNAR. The present invention is directed to monoclonal antibodies that generally are based on the IgG class, which has several subclasses, including, but not limited to IgG1, IgG2, IgG3, and IgG4. In general, IgG1, IgG2 and IgG4 are used more frequently than IgG3. It should be noted that IgG1 has different allotypes with polymorphisms at 356 (D or E) and 358 (L or M). The sequences depicted herein use the 356D/358M allotype, however the other allotype is included herein. That is, any sequence inclusive of an IgG1 Fc domain included herein can have 356E/358L replacing the 356D/358M allotype.

The amino-terminal portion of each chain includes a variable region of about 100 to 110 or more amino acids primarily responsible for antigen recognition, generally referred to in the art and herein as the "Fv domain" or "Fv region". In the variable region, three loops are gathered for each of the V domains of the heavy chain and light chain to form an antigen-binding site. Each of the loops is referred to as a complementarity-determining region (hereinafter referred to as a "CDR"), in which the variation in the amino acid sequence is most significant. "Variable" refers to the fact that certain segments of the variable region differ extensively in sequence among antibodies. Variability within the variable region is not evenly distributed. Instead, the V regions consist of relatively invariant stretches called framework regions (FRs) of 15-30 amino acids separated by shorter regions of extreme variability called "hypervariable regions" that are each 9-15 amino acids long or longer.

Each VH and VL is composed of three hypervariable regions ("complementary determining regions," "CDRs") and four FRs, arranged from amino-terminus to carboxy-terminus in the following order: FR1-CDR1-FR2-CDR2-FR3-CDR3-FR4.

The hypervariable region generally encompasses amino acid residues from about amino acid residues 24-34 (LCDR1; "L" denotes light chain), 50-56 (LCDR2) and 89-97 (LCDR3) in the light chain variable region and around about 31-35B (HCDR1; "H" denotes heavy chain), 50-65 (HCDR2), and 95-102 (HCDR3) in the heavy chain variable region; Kabat et al., SEQUENCES OF PROTEINS OF IMMUNOLOGICAL INTEREST, 5th Ed. Public Health Service, National Institutes of Health, Bethesda, Md. (1991) and/or those residues forming a hypervariable loop (e.g. residues 26-32 (LCDR1), 50-52 (LCDR2) and 91-96 (LCDR3) in the light chain variable region and 26-32 (HCDR1), 53-55 (HCDR2) and 96-101 (HCDR3) in the heavy chain variable region; Chothia and Lesk (1987) J. Mol. Biol. 196:901-917. Specific CDRs of the invention are described below.

As will be appreciated by those in the art, the exact numbering and placement of the CDRs can be different among different numbering systems. However, it should be understood that the disclosure of a variable heavy and/or variable light sequence includes the disclosure of the associated (inherent) CDRs. Accordingly, the disclosure of each variable heavy region is a disclosure of the vhCDRs (e.g. vhCDR1, vhCDR2 and vhCDR3) and the disclosure of each variable light region is a disclosure of the vlCDRs (e.g. vlCDR1, vlCDR2 and vlCDR3). A useful comparison of CDR numbering is as below, see Lafranc et al., Dev. Comp. Immunol. 27(1):55-77 (2003):

| | Kabat+Clothia | IMGT | Kabat | AbM | Chothia | Contact |
|---|---|---|---|---|---|---|
| vhCDR1 | 26-35 | 27-38 | 31-35 | 26-35 | 26-32 | 30-35 |
| vhCDR2 | 50-65 | 56-65 | 50-65 | 50-58 | 53-55 | 47-58 |
| vhCDR3 | 95-102 | 105-117 | 95-102 | 95-102 | 96-101 | 93-101 |
| vlCDR1 | 24-34 | 27-38 | 24-34 | 24-34 | 26-32 | 30-36 |
| vlCDR2 | 50-56 | 56-65 | 50-56 | 50-56 | 50-52 | 46-55 |
| vlCDR3 | 89-97 | 105-117 | 89-97 | 89-97 | 91-96 | 89-96 |

Throughout the present specification, the Kabat numbering system is generally used when referring to a residue in the variable domain (approximately, residues 1-107 of the light chain variable region and residues 1-113 of the heavy chain variable region) and the hinge and the EU numbering system for Fc regions (e.g, Kabat et al., supra (1991)).

The present invention provides a large number of different CDR sets. In this case, a "full CDR set" comprises the three variable light and three variable heavy CDRs, e.g. a vlCDR1, vlCDR2, vlCDR3, vhCDR1, vhCDR2 and vhCDR3. These can be part of a larger variable light or variable heavy domain, respectfully. In addition, as more fully outlined herein, the variable heavy and variable light domains can be on separate polypeptide chains, when a heavy and light chain is used, or on a single polypeptide chain in the case of scFv sequences.

The CDRs contribute to the formation of the antigen-binding, or more specifically, epitope binding site of antibodies. "Epitope" refers to a determinant that interacts with a specific antigen binding site in the variable region of an antibody molecule known as a paratope. Epitopes are groupings of molecules such as amino acids or sugar side chains and usually have specific structural characteristics, as well as specific charge characteristics. A single antigen may have more than one epitope.

The epitope may comprise amino acid residues directly involved in the binding (also called immunodominant component of the epitope) and other amino acid residues, which are not directly involved in the binding, such as amino acid residues which are effectively blocked by the specifically antigen binding peptide; in other words, the amino acid residue is within the footprint of the specifically antigen binding peptide.

Epitopes may be either conformational or linear. A conformational epitope is produced by spatially juxtaposed amino acids from different segments of the linear polypeptide chain. A linear epitope is one produced by adjacent amino acid residues in a polypeptide chain. Conformational and non-conformational epitopes may be distinguished in that the binding to the former but not the latter is lost in the presence of denaturing solvents.

An epitope typically includes at least 3, and more usually, at least 5 or 8-10 amino acids in a unique spatial conformation. Antibodies that recognize the same epitope can be verified in a simple immunoassay showing the ability of one antibody to block the binding of another antibody to a target antigen, for example "binning." As outlined below, the invention not only includes the enumerated antigen binding domains and antibodies herein, but those that compete for binding with the epitopes bound by the enumerated antigen binding domains.

The carboxy-terminal portion of each chain defines a constant region primarily responsible for effector function. Kabat et al. collected numerous primary sequences of the variable regions of heavy chains and light chains. Based on the degree of conservation of the sequences, they classified individual primary sequences into the CDR and the framework and made a list thereof (see SEQUENCES OF IMMUNOLOGICAL INTEREST, 5th edition, NIH publication, No. 91-3242, E.A. Kabat et al., entirely incorporated by reference).

In the IgG subclass of immunoglobulins, there are several immunoglobulin domains in the heavy chain. By "immunoglobulin (Ig) domain" herein is meant a region of an immunoglobulin having a distinct tertiary structure. Of interest in the present invention are the heavy chain domains, including, the constant heavy (CH) domains and the hinge domains. In the context of IgG antibodies, the IgG isotypes each have three CH regions. Accordingly, "CH" domains in the context of IgG are as follows: "CH1" refers to positions 118-220 according to the EU index as in Kabat. "CH2" refers to positions 237-340 according to the EU index as in Kabat, and "CH3" refers to positions 341-447 according to the EU index as in Kabat.

Another type of Ig domain of the heavy chain is the hinge region. By "hinge" or "hinge region" or "antibody hinge region" or "immunoglobulin hinge region" herein is meant the flexible polypeptide comprising the amino acids between the first and second constant domains of an antibody. Structurally, the IgG CH1 domain ends at EU position 220, and the IgG CH2 domain begins at residue EU position 237. Thus for IgG the antibody hinge is herein defined to include positions 221 (D221 in IgG1) to 236 (G236 in IgG1), wherein the numbering is according to the EU index as in Kabat.

The light chain generally comprises two domains, the variable light domain (containing the light chain CDRs and together with the variable heavy domains forming the Fv region), and a constant light chain region (often referred to as CL or Cκ). In general, either the constant lambda or constant kappa domain can be used, with lambda generally finding use in the invention.

Another region of interest for additional substitutions, outlined below, is the Fc region.

Still further, an antibody or antigen-binding portion thereof (antigen-binding fragment, antibody fragment, antibody portion) may be part of a larger immunoadhesion molecules (sometimes also referred to as "fusion proteins"), formed by covalent or noncovalent association of the antibody or antibody portion with one or more other proteins or peptides. Examples of immunoadhesion molecules include use of the streptavidin core region to make a tetrameric scFv molecule and use of a cysteine residue, a marker peptide and a C-terminal polyhistidine tag to make bivalent and biotinylated scFv molecules. Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

In general, the anti-PVRIG antibodies of the invention are recombinant. "Recombinant" as used herein, refers broadly with reference to a product, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

The term "recombinant antibody", as used herein, includes all antibodies that are prepared, expressed, created or isolated by recombinant means, such as (a) antibodies isolated from an animal (*e*.*g*., a mouse) that is transgenic or transchromosomal for human immunoglobulin genes or a hybridoma prepared therefrom (described further below), (b) antibodies isolated from a host cell transformed to express the human antibody, *e*.*g*., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, in vivo somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

### A. Chimeric and Humanized Antibodies

In some embodiments, the anti-PVRIG antibpdies and/or anti-TIGIT antibodies herein can be derived from a mixture from different species, e.g. a chimeric antibody and/or a humanized antibody. In general, both "chimeric antibodies" and "humanized antibodies" refer to antibodies that combine regions from more than one species. For example, "chimeric antibodies" traditionally comprise variable region(s) from a mouse (or rat, in some cases) and the constant region(s) from a human. "Humanized antibodies" generally refer to non-human antibodies that have had the variable-domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies is described in, e.g., WO 92/11018, Jones, 1986, Nature 321:522-525, Verhoeyen et al., 1988, Science 239:1534-1536, all entirely incorporated by reference. "Backmutation" of selected acceptor framework residues to the corresponding donor residues is often required to regain affinity that is lost in the initial grafted construct (US 5530101; US 5585089; US 5693761; US 5693762; US 6180370; US 5859205; US 5821337; US 6054297; US 6407213, all entirely incorporated by reference). The humanized antibody optimally also will comprise at least a portion, and usually all, of an immunoglobulin constant region, typically that of a human immunoglobulin, and thus will typically comprise a human Fc region. Humanized antibodies can also be generated using mice with a genetically engineered immune system. Roque et al., 2004, Biotechnol. Prog. 20:639-654, entirely incorporated by reference. A variety of techniques and methods for humanizing and reshaping non-human antibodies are well known in the art (See Tsurushita & Vasquez, 2004, Humanization of Monoclonal Antibodies, Molecular Biology of B Cells, 533-545, Elsevier Science (USA), and references cited therein, all entirely incorporated by reference). Humanization methods include but are not limited to methods described in Jones et al., 1986, Nature 321:522-525; Riechmann et al.,1988; Nature 332:323-329; Verhoeyen et al., 1988, Science, 239:1534-1536; Queen et al., 1989, Proc Natl Acad Sci, USA 86:10029-33; He et al., 1998, J. Immunol. 160: 1029-1035; Carter et al., 1992, Proc Natl Acad Sci USA 89:4285-9, Presta et al., 1997, Cancer Res. 57(20):4593-9; Gorman et al., 1991, Proc. Natl. Acad. Sci. USA 88:4181-4185; O'Connor et al., 1998, Protein Eng 11:321-8, all entirely incorporated by reference. Humanization or other methods of reducing the immunogenicity of nonhuman antibody variable regions may include resurfacing methods, as described for example in Roguska et al., 1994, Proc. Natl. Acad. Sci. USA 91:969-973, entirely incorporated by reference.

Thus, the vhCDRs and vlCDRs from any of the enumerated antibodies herein may be humanized (or "rehumanized", for those that were already humanized).

In certain embodiments, the antibodies of the invention comprise a heavy chain variable region from a particular germline heavy chain immunoglobulin gene and/or a light chain variable region from a particular germline light chain immunoglobulin gene. For example, such antibodies may comprise or consist of a human antibody comprising heavy or light chain variable regions that are "the product of" or "derived from" a particular germline sequence. A human antibody that is "the product of" or "derived from" a human germline immunoglobulin sequence can be identified as such by comparing the amino acid sequence of the human antibody to the amino acid sequences of human germline immunoglobulins and selecting the human germline immunoglobulin sequence that is closest in sequence (i.e., greatest % identity) to the sequence of the human antibody. A human antibody that is "the product of" or "derived from" a particular human germline immunoglobulin sequence may contain amino acid differences as compared to the germline sequence, due to, for example, naturally-occurring somatic mutations or intentional introduction of site-directed mutation. However, a humanized antibody typically is at least 90% identical in amino acids sequence to an amino acid sequence encoded by a human germline immunoglobulin gene and contains amino acid residues that identify the antibody as being derived from human sequences when compared to the germline immunoglobulin amino acid sequences of other species (e.g., murine germline sequences). In certain cases, a humanized antibody may be at least 95, 96, 97, 98 or 99%, or even at least 96%, 97%, 98%, or 99% identical in amino acid sequence to the amino acid sequence encoded by the germline immunoglobulin gene excluding the CDRs. That is, the CDRs may be murine, but the framework regions of the variable region (either heavy or light) can be at least 96%, 97%, 98%, or 99% identical in amino acid sequence to the framework amino acids encoded by one human germline immunoglobulin gene.

Typically, a humanized antibody derived from a particular human germline sequence will display no more than 10-20 amino acid differences from the amino acid sequence encoded by the human germline immunoglobulin gene. In certain cases, the humanized antibody may display no more than 5, or even no more than 4, 3, 2, or 1 amino acid difference from the amino acid sequence encoded by the germline immunoglobulin gene (again, prior to the introduction of any variants herein; that is, the number of variants is generally low).

In one embodiment, the parent antibody has been affinity matured, as is known in the art. Structure-based methods may be employed for humanization and affinity maturation, for example as described in USSN 11/004,590. Selection based methods may be employed to humanize and/or affinity mature antibody variable regions, including but not limited to methods described in Wu et al., 1999, J. Mol. Biol. 294:151-162; Baca et al., 1997, J. Biol. Chem. 272(16):10678-10684; Rosok et al., 1996, J. Biol. Chem. 271(37): 22611-22618; Rader et al., 1998, Proc. Natl. Acad. Sci. USA 95: 8910-8915; Krauss et al., 2003, Protein Engineering 16(10):753-759, all entirely incorporated by reference. Other humanization methods may involve the grafting of only parts of the CDRs, including but not limited to methods described in USSN 09/810,510; Tan et al., 2002, J. Immunol. 169:1119-1125; De Pascalis et al., 2002, J. Immunol. 169:3076-3084, all entirely incorporated by reference.

### B. PVRIG Antibodies

The present invention provides anti-PVRIG antibodies. (For convenience, "anti-PVRIG antibodies" and "PVRIG antibodies" are used interchangeably). The anti-PVRIG antibodies of the invention specifically bind to human PVRIG, and preferably the ECD of human PVRIG, as depicted in Figure 1.

Specific binding for PVRIG or a PVRIG epitope can be exhibited, for example, by an antibody having a KD of at least about 10⁻⁴ M, at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, or greater, where KD refers to a dissociation rate of a particular antibody-antigen interaction. Typically, an antibody that specifically binds an antigen will have a KD that is 20-, 50-, 100-, 500-, 1000-, 5,000-, 10,000- or more times greater for a control molecule relative to the PVRIG antigen or epitope.

However, as shown in the Examples, for optimal binding to PVRIG expressed on the surface of NK and T-cells, the antibodies preferably have a KD less 50 nM and most preferably less than 1 nM, with less than 0.1 nM and less than 1 pM and 0.1 pM finding use in the methods of the invention.

Also, specific binding for a particular antigen or an epitope can be exhibited, for example, by an antibody having a KA or Ka for a PVRIG, antigen or epitope of at least 20-, 50-, 100-, 500-, 1000-, 5,000-, 10,000- or more times greater for the epitope relative to a control, where KA or Ka refers to an association rate of a particular antibody-antigen interaction.

In some embodiments, the anti-PVRIG antibodies of the invention bind to human PVRIG with a K_{D} of 100 nM or less, 50 nM or less, 10 nM or less, or 1 nM or less (that is, higher binding affinity), or 1pM or less, wherein K_{D} is determined by known methods, e.g. surface plasmon resonance (SPR, e.g. Biacore assays), ELISA, KINEXA, and most typically SPR at 25°C or 37°C.

### C. Specific anti-PVRIG antibodies

The invention provides antigen binding domains, including full length antibodies, which contain a number of specific, enumerated sets of 6 CDRs.

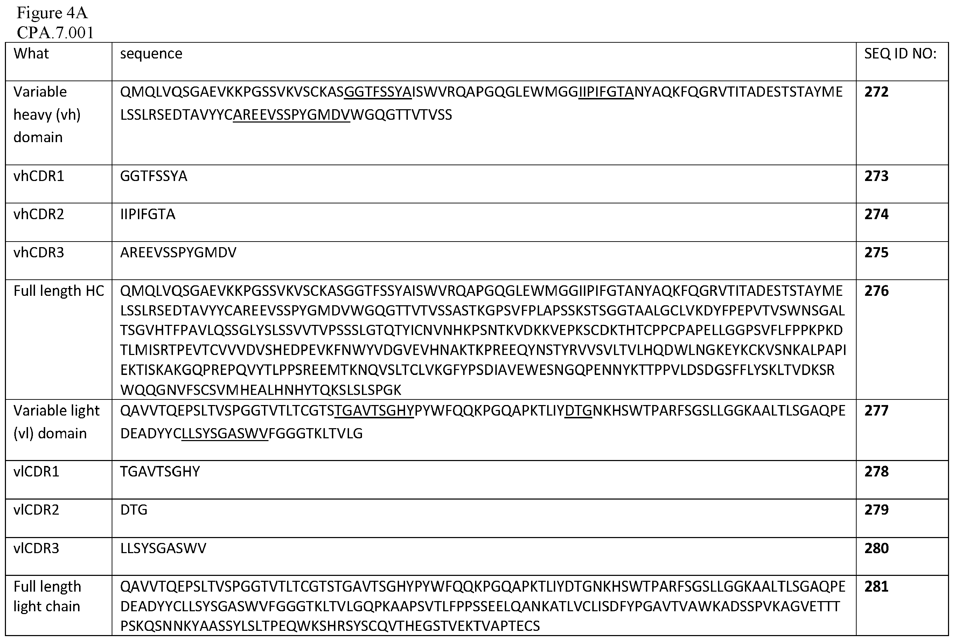

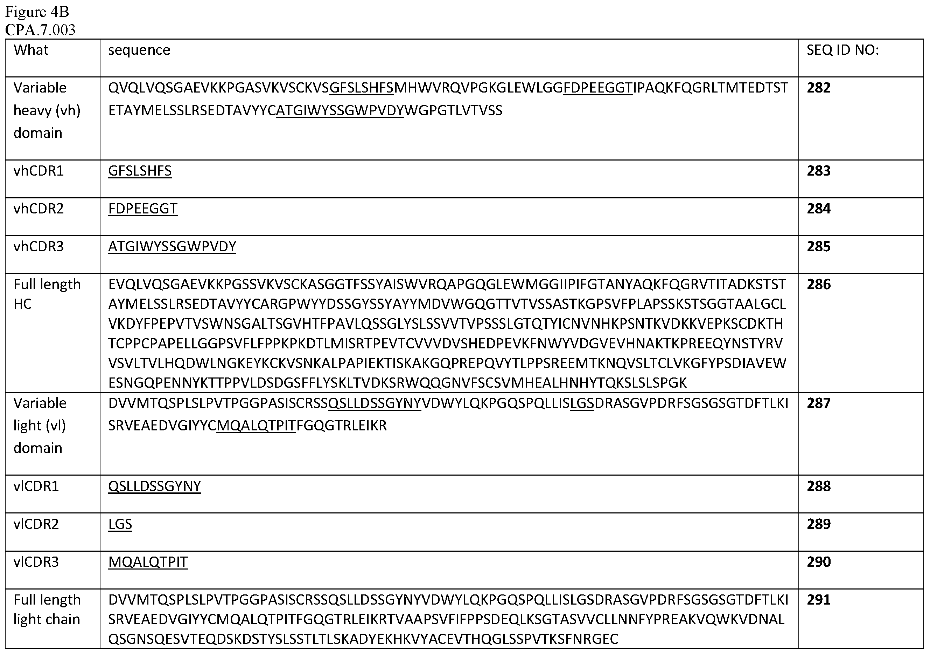

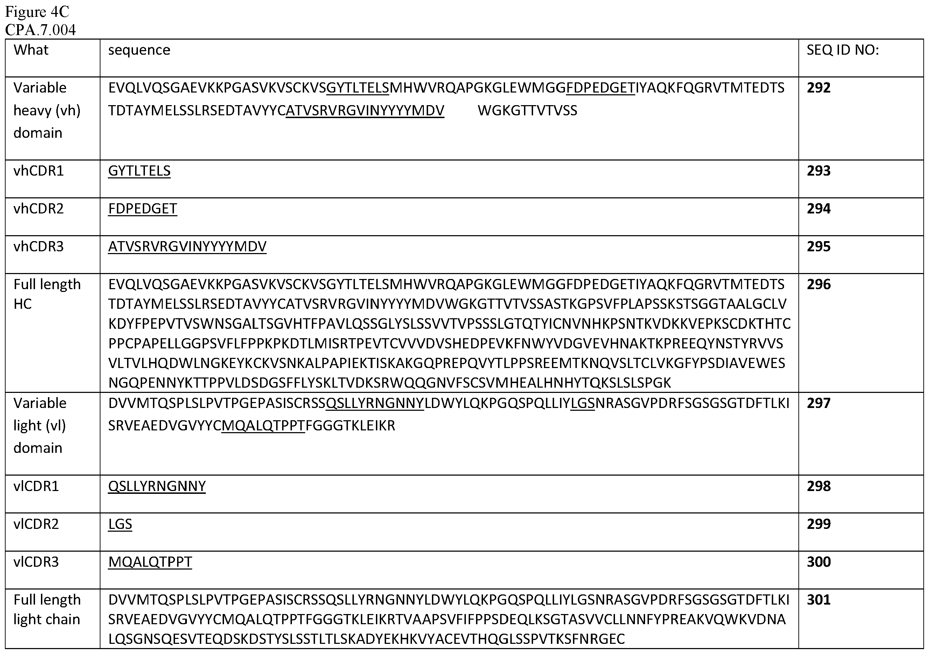

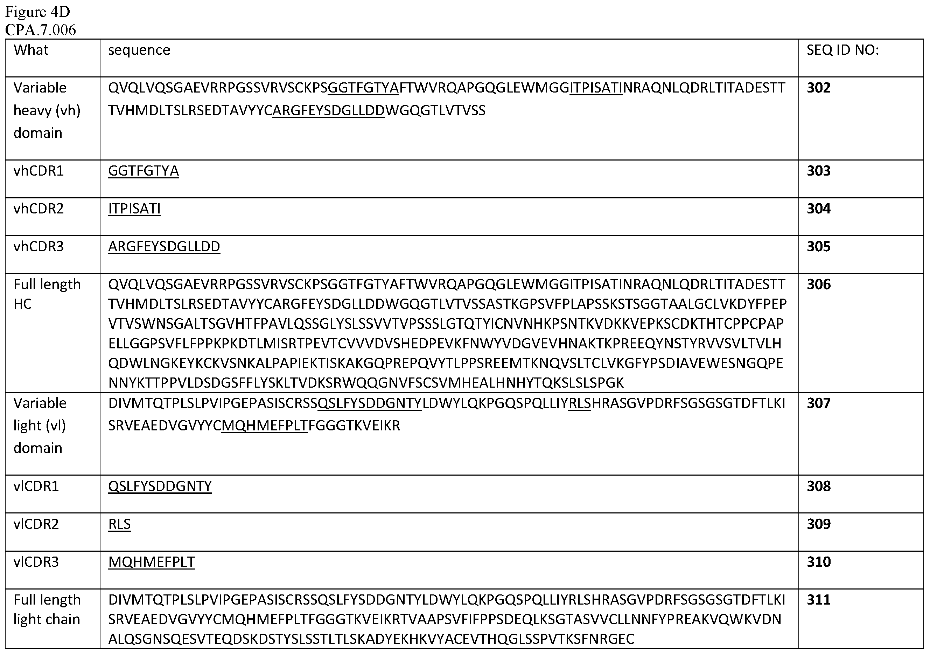

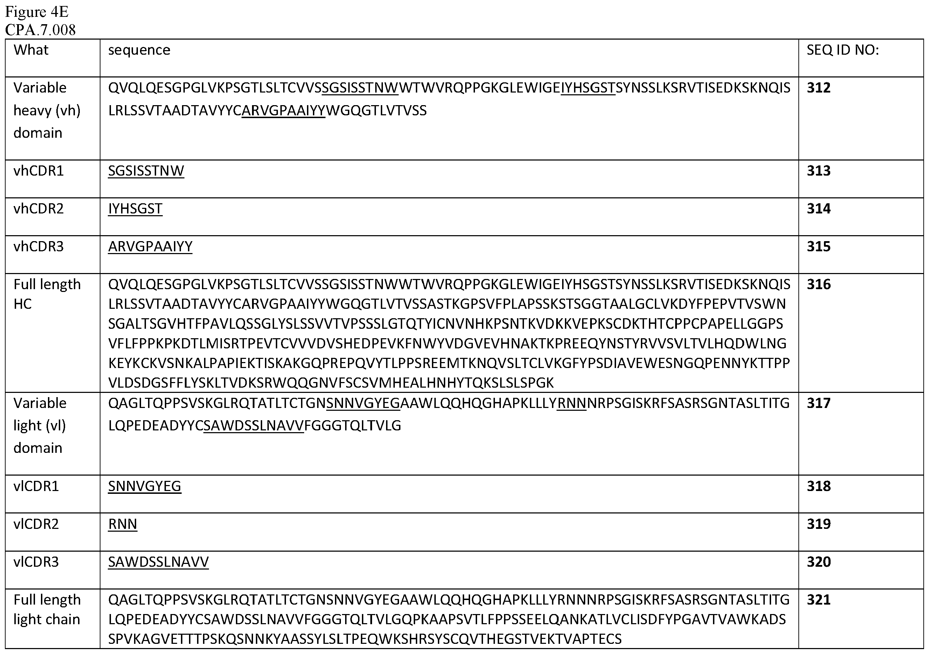

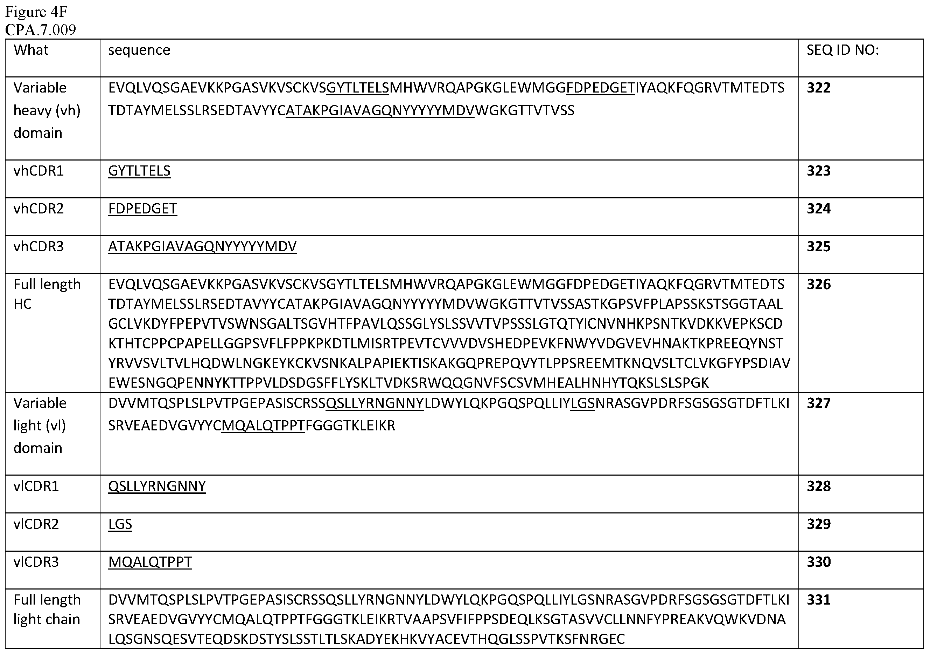

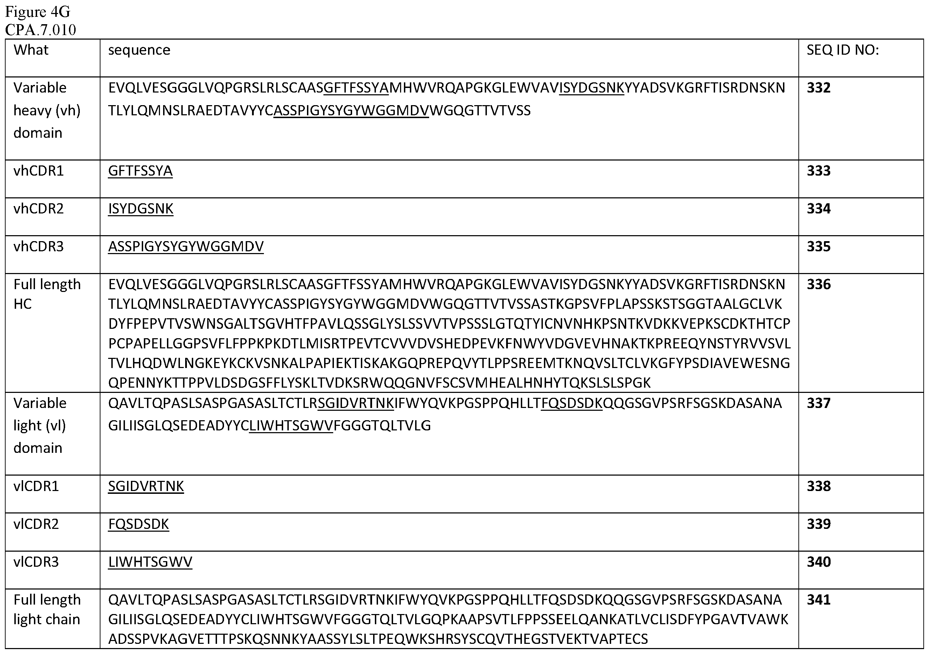

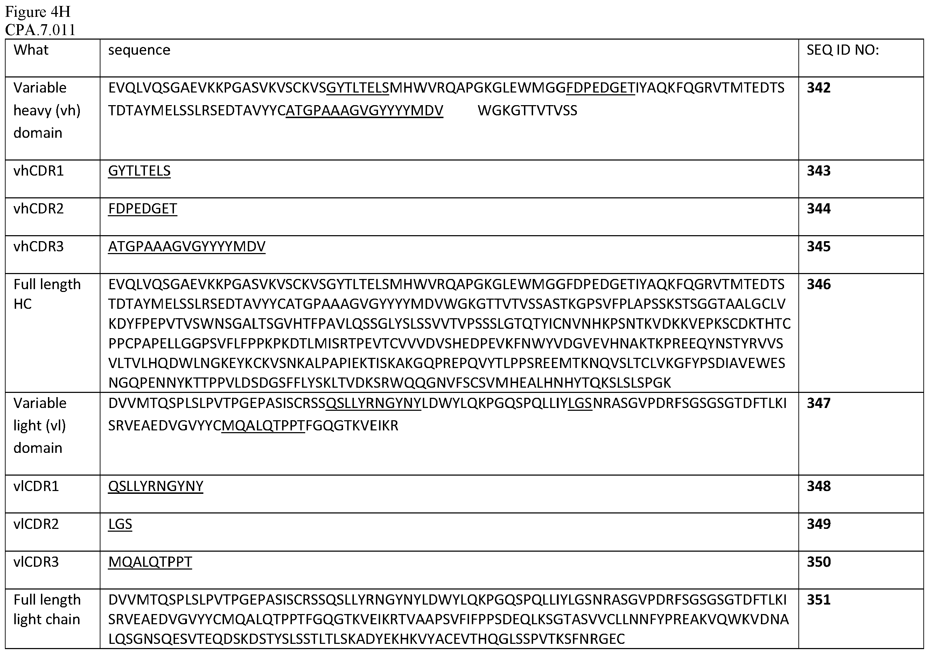

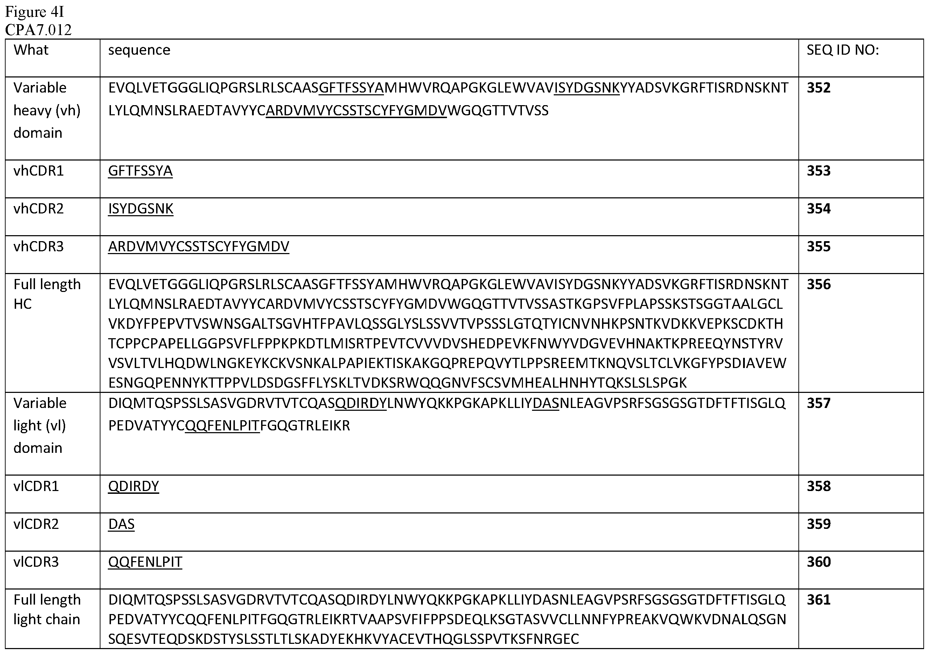

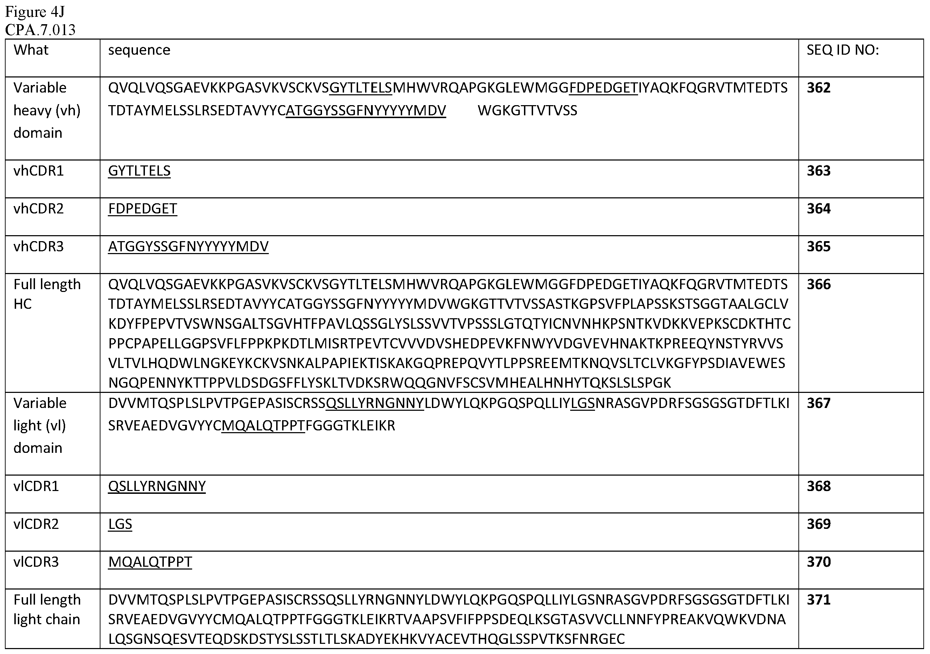

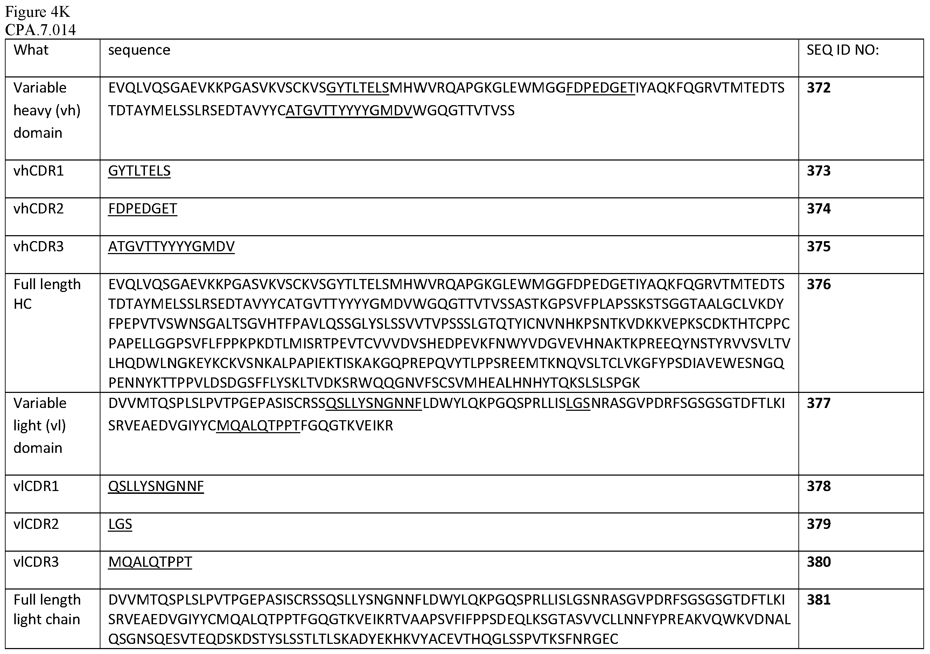

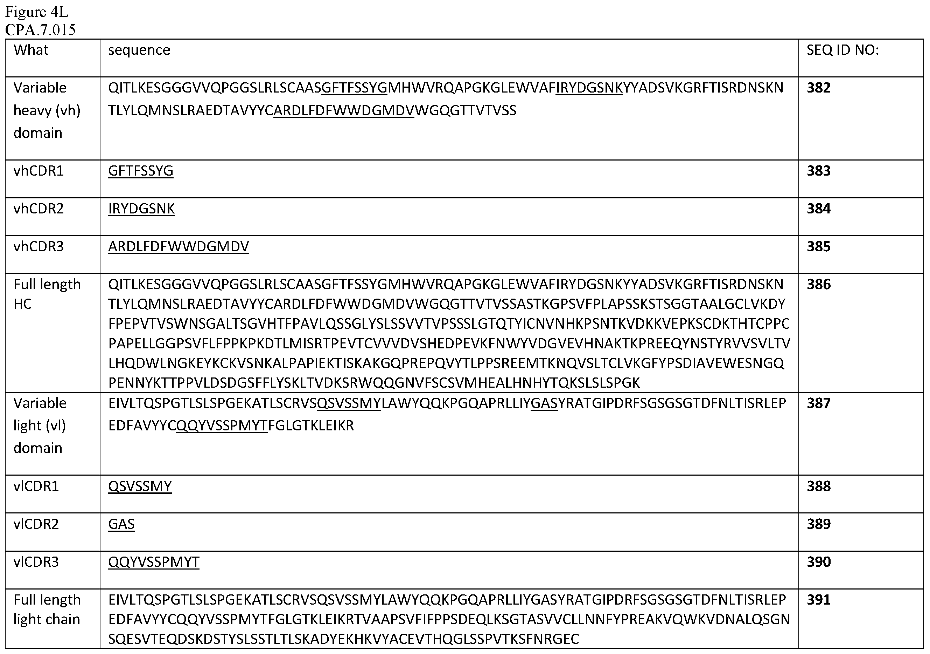

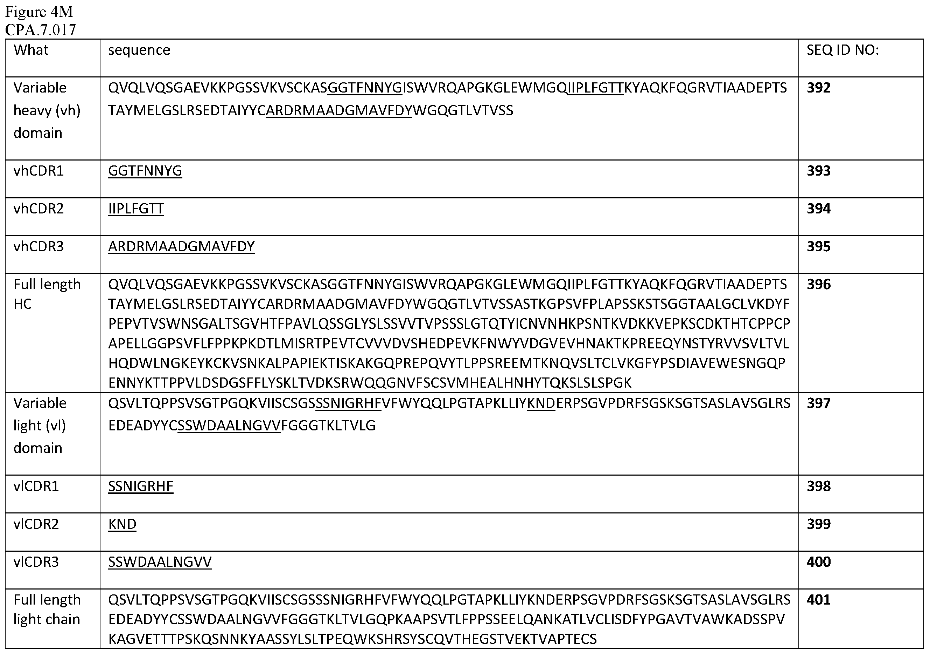

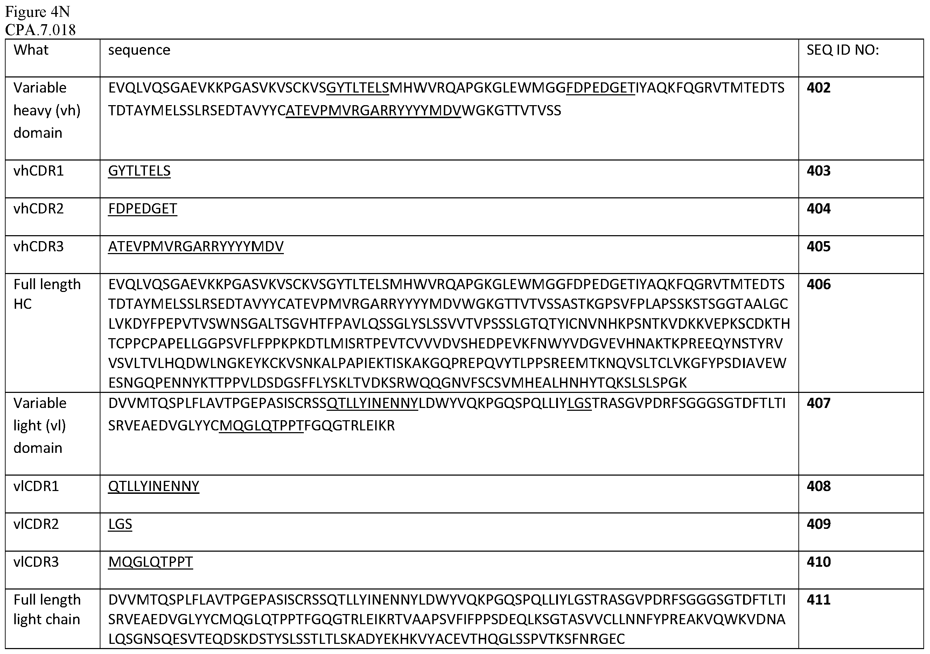

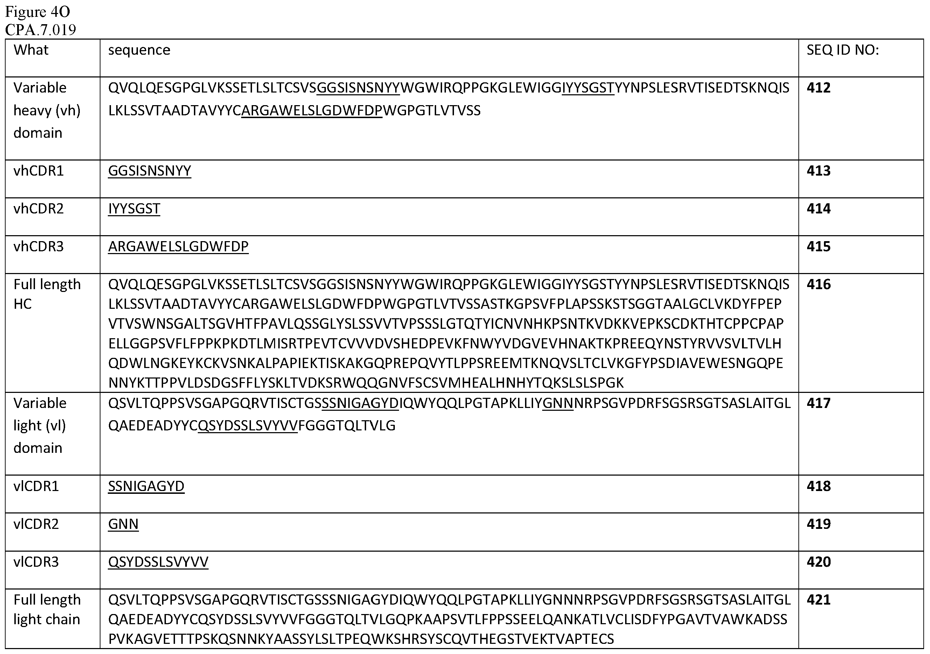

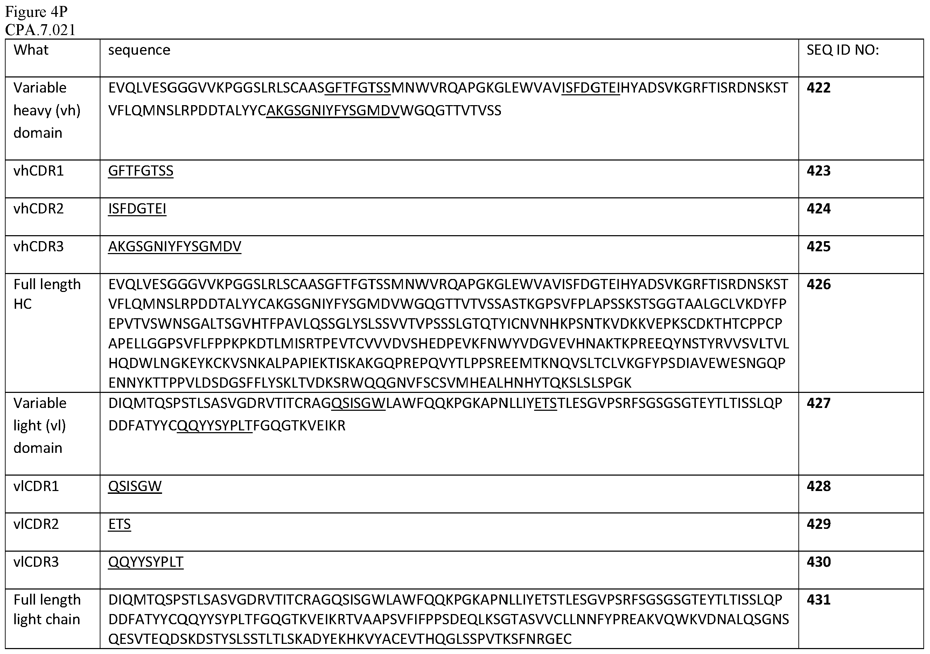

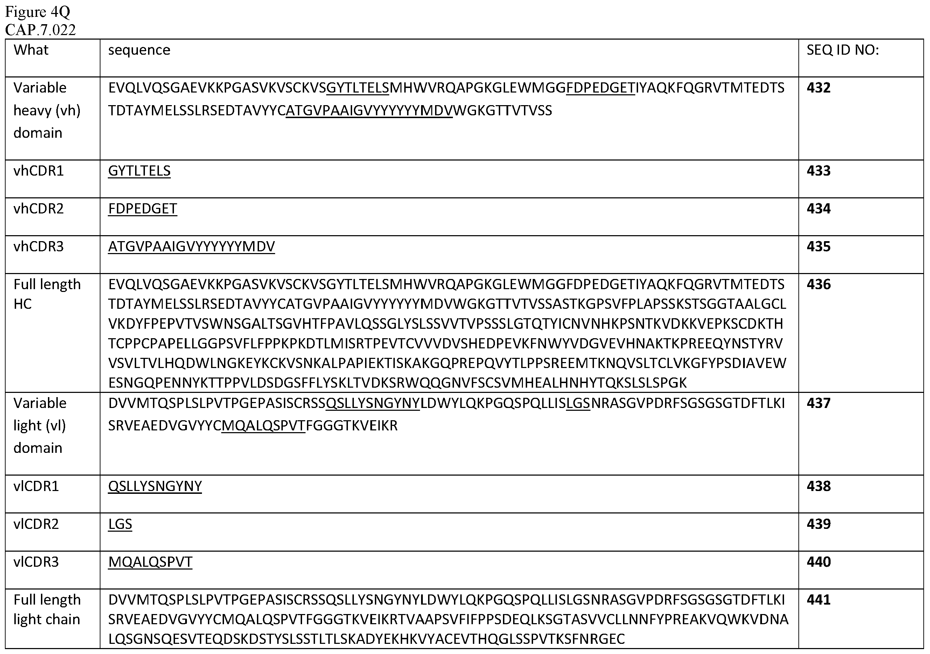

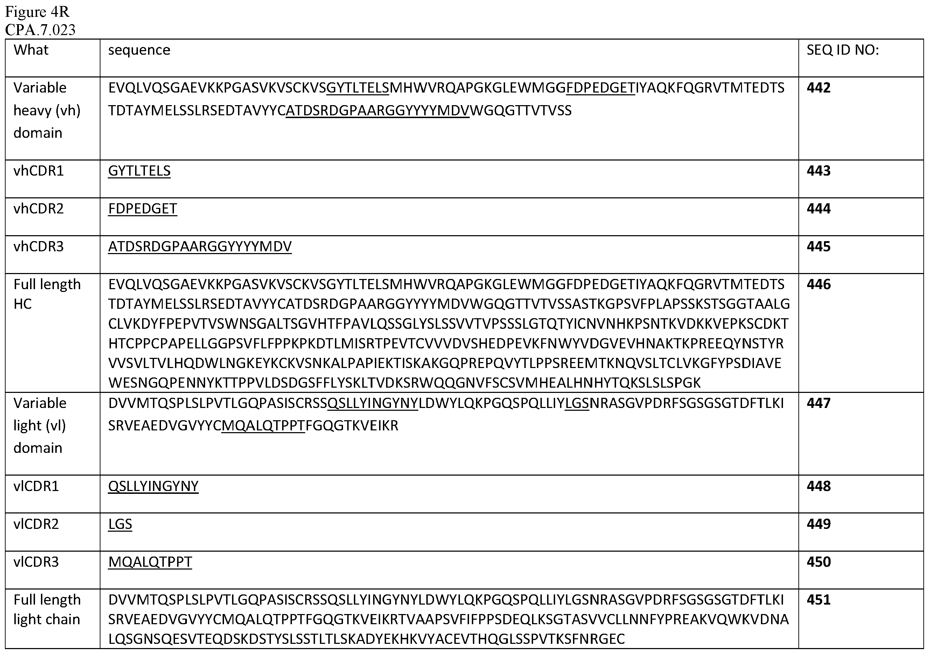

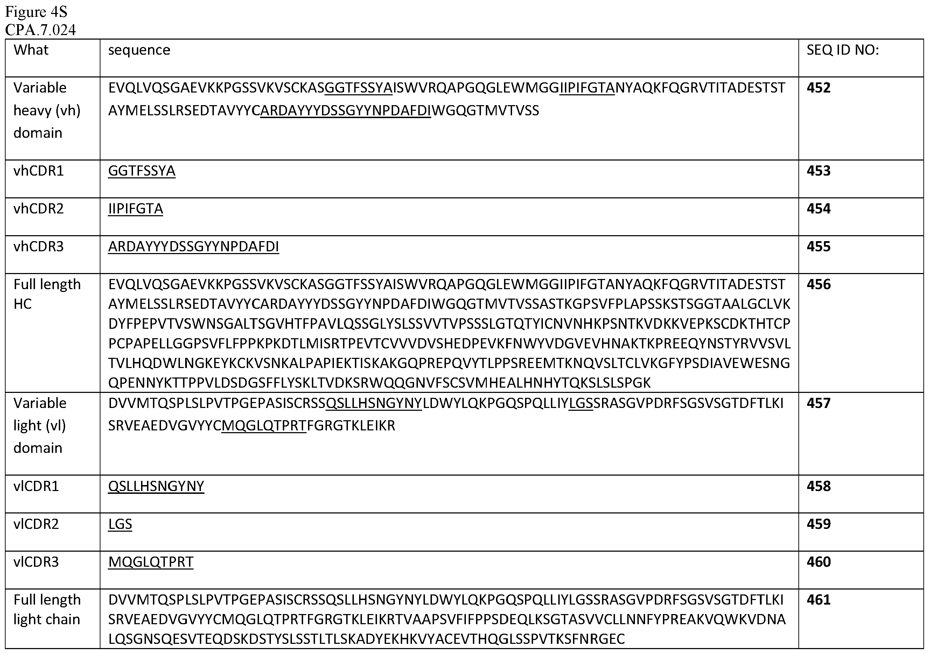

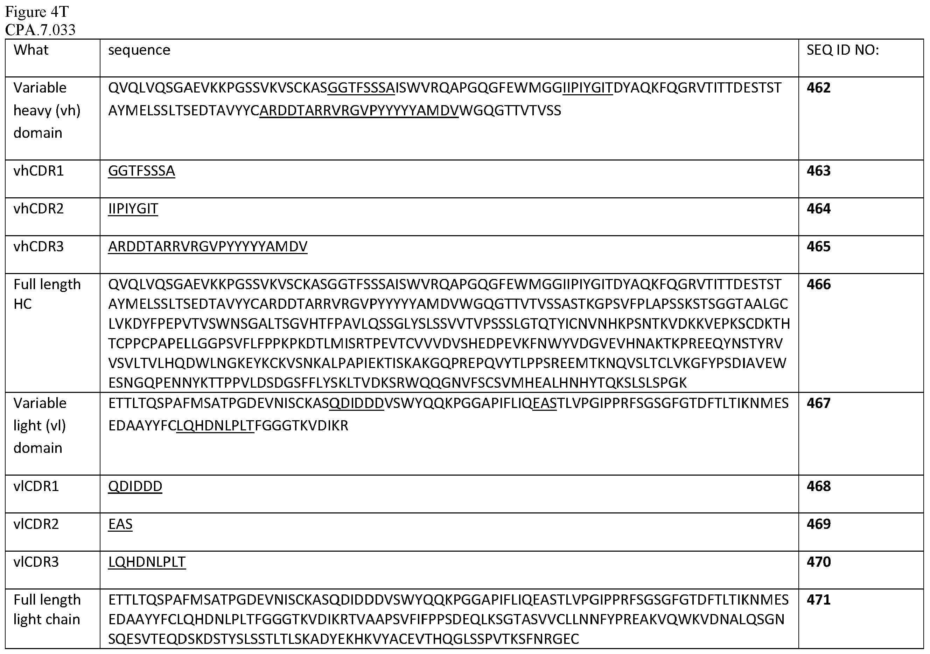

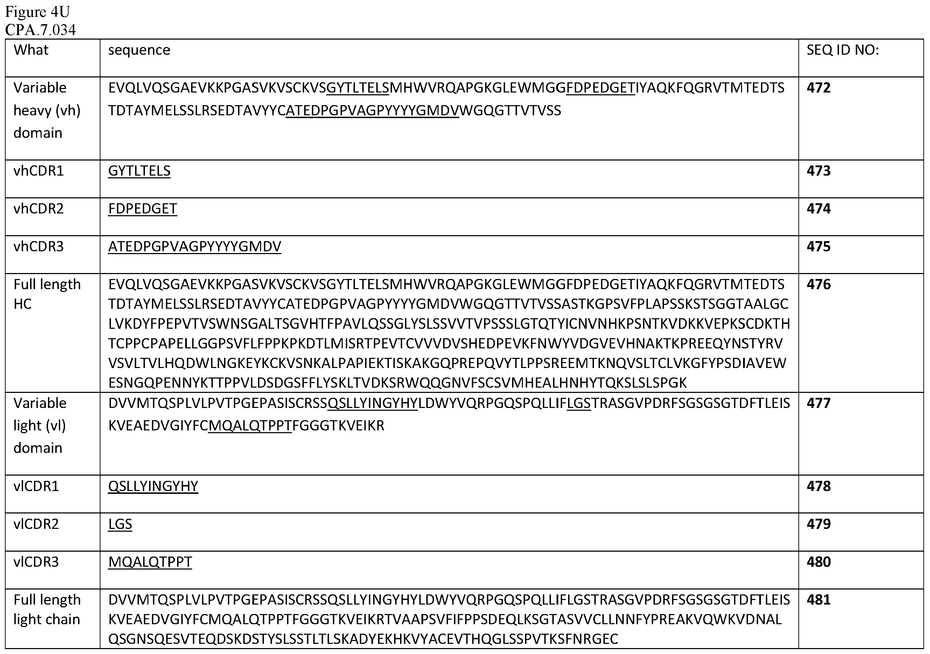

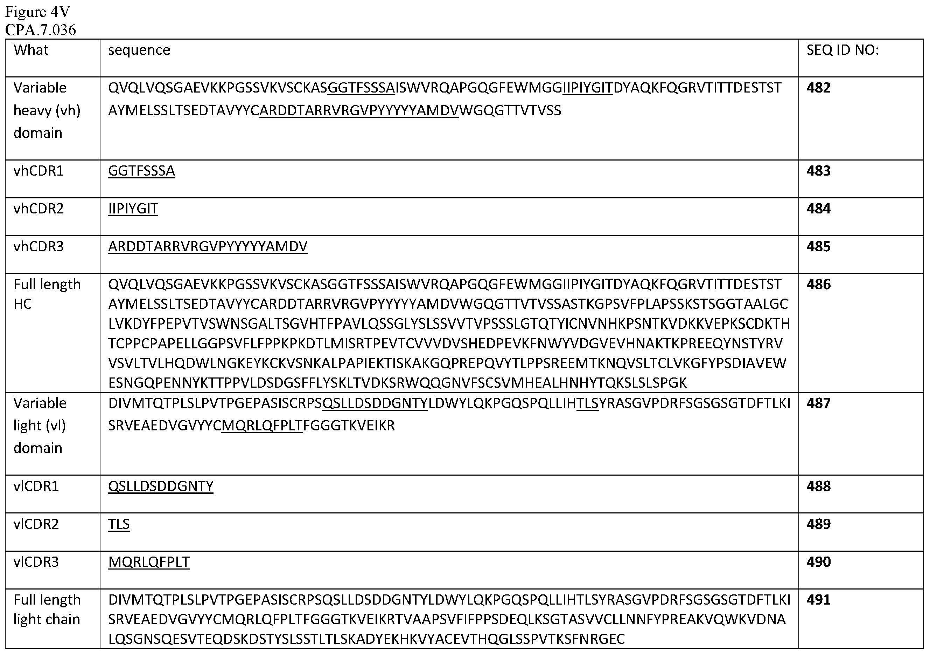

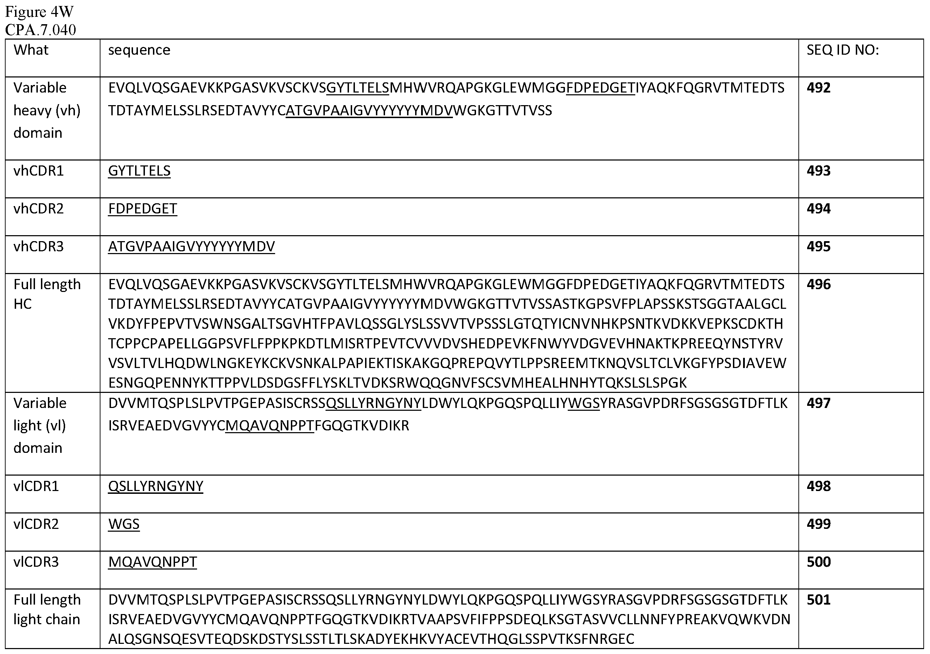

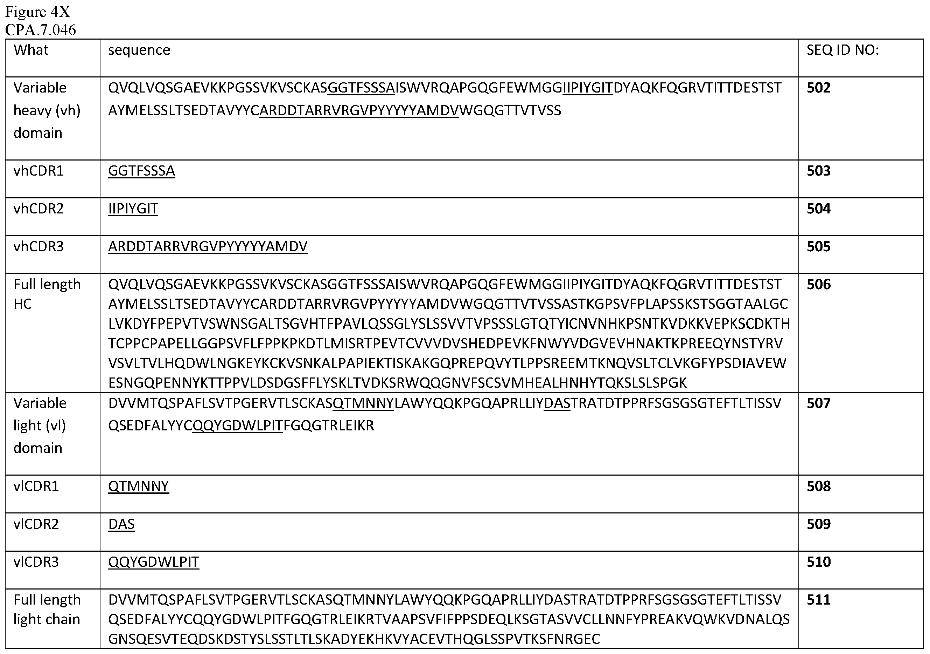

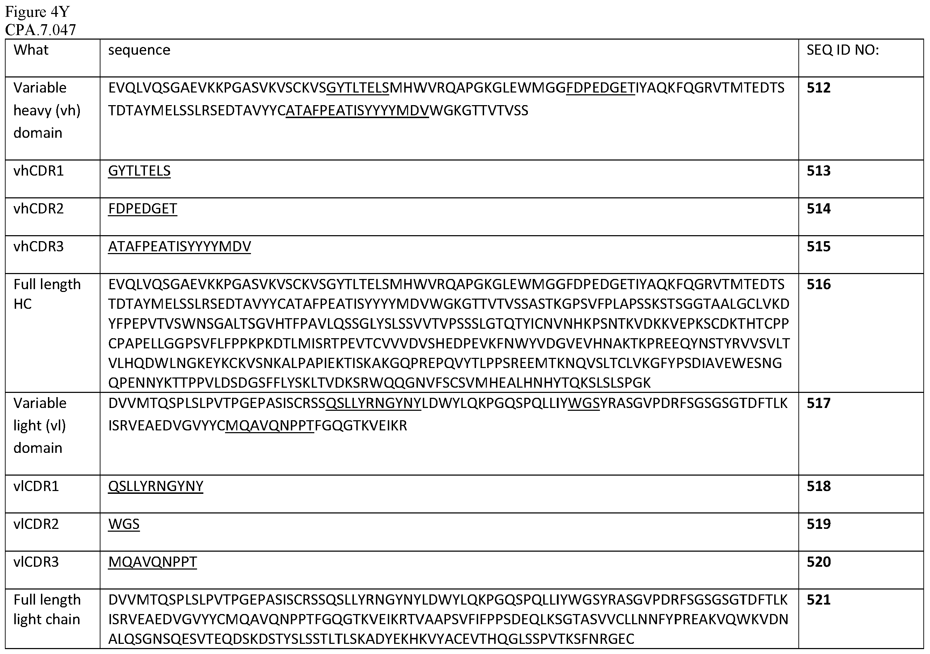

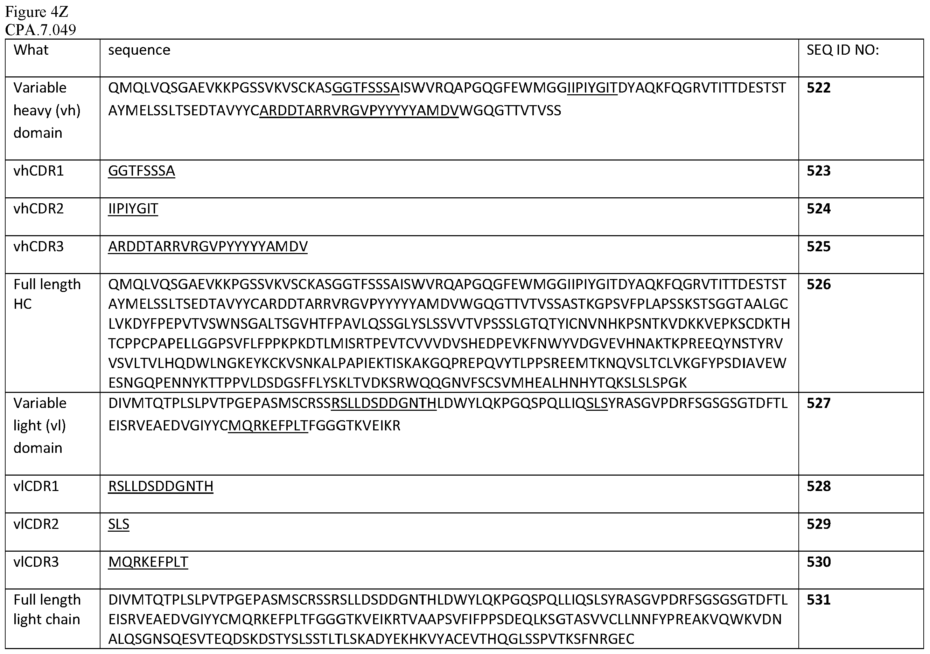

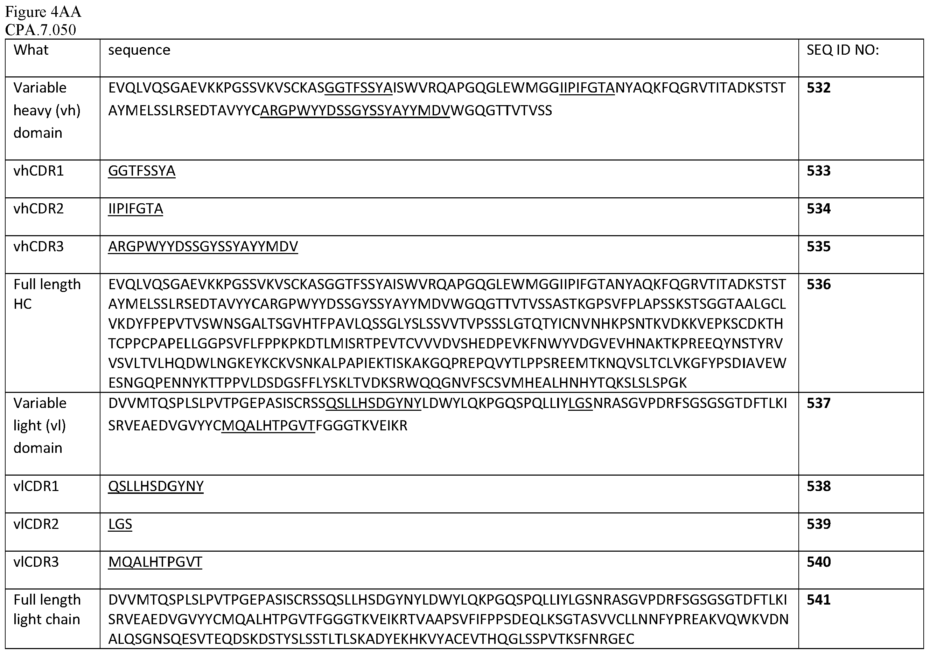

The antibodies described herein as labeled as follows. The antibodies have reference numbers, for example "CPA.7.013". This represents the combination of the variable heavy and variable light chains, as depicted in Figure 4A-4AA and Figure 5A-5H, for example. "CPA.7.013.VH" refers to the variable heavy portion of CPA.7.013, while "CPA.7.013.VL" is the variable light chain. "CPA.7.013.vhCDR1", "CPA.7.013.vhCDR2", "CPA.7.013.vhCDR3", "CPA.7.013.vlCDR1", "CPA.7.013.vlCDR2", and "CPA.7.013.vlCDR3", refers to the CDRs are indicated. "CPA.7.013.HC" refers to the entire heavy chain (e.g. variable and constant domain) of this molecule, and "CPA.7.013.LC" refers to the entire light light chain (e.g. variable and constant domain) of the same molecule. "CPA.7.013.H1" refers to a full length antibody comprising the variable heavy and light domains, including the constant domain of **H**uman IgG**1** (hence, the H1; IgG1, IgG2, IgG3 and IgG4 sequences are shown in Figure 7). Accordingly, "CPA.7.013.H2" would be the CPA.7.013 variable domains linked to a **H**uman IgG**2**. "CPA.7.013.H3" would be the CPA.7.013 variable domains linked to a **H**uman IgG**3**, and "CPA.7.013.H4" would be the CPA.7.013 variable domains linked to a **H**uman IgG**4**.

The invention further provides variable heavy and light domains as well as full length heavy and light chains.

In many embodiments, the antibodies of the invention are human (derived from phage) and block binding of PVRIG and PVLR2. As shown in Figure 4A-4AA, the CPA antibodies that both bind and block the receptor-ligand interaction are as below, with their components outlined as well:
CPA.7.001, CPA.7.001.VH, CPA.7.001.VL, CPA.7.001.HC, CPA.7.001.LC and CPA.7.001.H1, CPA.7.001.H2, CPA.7.001.H3, CPA.7.001.H4; CPA.7.001.vhCDR1, CPA.7.001.vhCDR2, CPA.7.001.vhCDR3, CPA.7.001.vlCDR1, CPA.7.001.vlCDR2, and CPA.7.001.vlCDR3;
CPA.7.003, CPA.7.003.VH, CPA.7.003.VL, CPA.7.003.HC, CPA.7.003.LC, CPA.7.003.H1, CPA.7.003.H2, CPA.7.003.H3, CPA.7.003.H4; CPA.7.003.vhCDR1, CPA.7.003.vhCDR2, CPA.7.003.vhCDR3, CPA.7.003.vlCDR1, CPA.7.003.vlCDR2, and CPA.7.003.vlCDR3;
CPA.7.004, CPA.7.004.VH, CPA.7.004.VL, CPA.7.004.HC, CPA.7.004.LC, CPA.7.004.H1, CPA.7.004.H2, CPA.7.004.H3 CPA.7.004.H4; CPA.7.004.vhCDR1, CPA.7.004.vhCDR2, CPA.7.004.vhCDR3, CPA.7.004.vlCDR1, CPA.7.004.vlCDR2, and CPA.7.004.vlCDR3;
CPA.7.006, CPA.7.006.VH, CPA.7.006.VL, CPA.7.006.HC, CPA.7.006.LC, CPA.7.006.H1, CPA.7.006.H2, CPA.7.006.H3 CPA.7.006.H4; CPA.7.006.vhCDR1, CPA.7.006.vhCDR2, CPA.7.006.vhCDR3, CPA.7.006.vlCDR1, CPA.7.006.vlCDR2, and CPA.7.006.vlCDR3;
CPA.7.008, CPA.7.008.VH, CPA.7.008.VL, CPA.7.008.HC, CPA.7.008.LC, CPA.7.008.H1, CPA.7.008.H2, CPA.7.008.H3 CPA.7.008.H4; CPA.7.008.vhCDR1, CPA.7.008.vhCDR2, CPA.7.008.vhCDR3, CPA.7.008.vlCDR1, CPA.7.008.vlCDR2, and CPA.7.008.vlCDR3;
CPA.7.009, CPA.7.009.VH, CPA.7.009.VL, CPA.7.009.HC, CPA.7.009.LC, CPA.7.009.H1, CPA.7.009.H2, CPA.7.009.H3 CPA.7.009.H4; CPA.7.009.vhCDR1, CPA.7.009.vhCDR2, CPA.7.009.vhCDR3, CPA.7.009.vlCDR1, CPA.7.009.vlCDR2, and CPA.7.009.vlCDR3;
CPA.7.010, CPA.7.010.VH, CPA.7.010.VL, CPA.7.010.HC, CPA.7.010.LC, CPA.7.010.H1, CPA.7.010.H2, CPA.7.010.H3 CPA.7.010.H4; CPA.7.010.vhCDR1, CPA.7.010.vhCDR2, CPA.7.010.vhCDR3, CPA.7.010.vlCDR1, CPA.7.010.vlCDR2, and CPA.7.010.vlCDR3;
CPA.7.011, CPA.7.011.VH, CPA.7.011.VL, CPA.7.011.HC, CPA.7.011.LC, CPA.7.011.H1, CPA.7.011.H2, CPA.7.011.H3 CPA.7.011.H4; CPA.7.011.vhCDR1, CPA.7.011.vhCDR2, CPA.7.011.vhCDR3, CPA.7.011.vlCDR1, CPA.7.011.vlCDR2, and CPA.7.011.vlCDR3;
CPA.7.012, CPA.7.012.VH, CPA.7.012.VL, CPA.7.012.HC, CPA.7.012.LC, CPA.7.012.H1, CPA.7.012.H2, CPA.7.012.H3 CPA.7.012.H4; CPA.7.012.vhCDR1, CPA.7.012.vhCDR2, CPA.7.012.vhCDR3, CPA.7.012.vlCDR1, CPA.7.012.vlCDR2, and CPA.7.012.vlCDR3;
CPA.7.013, CPA.7.013.VH, CPA.7.013.VL, CPA.7.013.HC, CPA.7.013.LC, CPA.7.013.H1, CPA.7.013.H2, CPA.7.013.H3 CPA.7.013.H4; CPA.7.013.vhCDR1, CPA.7.013.vhCDR2, CPA.7.013.vhCDR3, CPA.7.013.vlCDR1, CPA.7.013.vlCDR2, and CPA.7.013.vlCDR3;
CPA.7.014, CPA.7.014.VH, CPA.7.014.VL, CPA.7.014.HC, CPA.7.014.LC, CPA.7.014.H1, CPA.7.014.H2, CPA.7.014.H3 CPA.7.014.H4; CPA.7.014.vhCDR1, CPA.7.014.vhCDR2, CPA.7.014.vhCDR3, CPA.7.014.vlCDR1, CPA.7.014.vlCDR2, and CPA.7.014.vlCDR3;
CPA.7.015, CPA.7.015.VH, CPA.7.015.VL, CPA.7.015.HC, CPA.7.015.LC, CPA.7.015.H1, CPA.7.015.H2, CPA.7.015.H3 CPA.7.015.H4; CPA.7.015.vhCDR1, CPA.7.015.vhCDR2, CPA.7.015.vhCDR3, CPA.7.015.vlCDR1, CPA.7.015.vlCDR2, and CPA.7.015.vlCDR3;
CPA.7.017, CPA.7.017.VH, CPA.7.017.VL, CPA.7.017.HC, CPA.7.017.LC, CPA.7.017H1, CPA.7.017.H2, CPA.7.017.H3 CPA.7.017.H4; CPA.7.017.vhCDR1, CPA.7.000171.vhCDR2, CPA.7.017.vhCDR3, CPA.7.017.vlCDR1, CPA.7.017.vlCDR2, and CPA.7.017.vlCDR3;
CPA.7.018, CPA.7.018.VH, CPA.7.018.VL, CPA.7.018.HC, CPA.7.018.LC, CPA.7.018.H1, CPA.7.018.H2, CPA.7.018.H3 CPA.7.018.H4; CPA.7.017.vhCDR1, CPA.7.017.vhCDR2, CPA.7.017.vhCDR3, CPA.7.017.vlCDR1, CPA.7.017.vlCDR2, and CPA.7.017.vlCDR3;
CPA.7.019, CPA.7.019.VH, CPA.7.019.VL, CPA.7.019.HC, CPA.7.019.LC, CPA.7.019.H1, CPA.7.019.H2, CPA.7.019.H3 CPA.7.019.H4; CPA.7.019.vhCDR1, CPA.7.019.vhCDR2, CPA.7.019.vhCDR3, CPA.7.019.vlCDR1, CPA.7.019.vlCDR2, and CPA.7.019.vlCDR3;
CPA.7.021, CPA.7.021.VH, CPA.7.021.VL, CPA.7.021.HC, CPA.7.021.LC, CPA.7.021.H1, CPA.7.021.H2, CPA.7.021.H3 CPA.7.021.H4; CPA.7.021.vhCDR1, CPA.7.021.vhCDR2, CPA.7.021.vhCDR3, CPA.7.021.vlCDR1, CPA.7.021.vlCDR2, and CPA.7.021.vlCDR3;
CPA.7.022, CPA.7.022.VH, CPA.7.022.VL, CPA.7.022.HC, CPA.7.022.LC, CPA.7.022.H1, CPA.7.022.H2, CPA.7.022.H3 CPA.7.022.H4; CPA.7.022.vhCDR1, CPA.7.022.vhCDR2, CPA.7.002201.vhCDR3, CPA.7.022.vlCDR1, CPA.7.022.vlCDR2, and CPA.7.022.vlCDR3;
CPA.7.023, CPA.7.023.VH, CPA.7.023.VL, CPA.7.023.HC, CPA.7.023.LC, CPA.7.023.H1, CPA.7.023.H2, CPA.7.023 H3 CPA.7.023.H4; CPA.7.023.vhCDR1, CPA.7.023.vhCDR2, CPA.7.023.vhCDR3, CPA.7.023.vlCDR1, CPA.7.023.vlCDR2, and CPA.7.023.vlCDR3;
CPA.7.024, CPA.7.024.VH, CPA.7.024.VL, CPA.7.024.HC, CPA.7.024.LC, CPA.7.024.H1, CPA.7.024.H2, CPA.7.024.H3 CPA.7.024.H4; CPA.7.024.vhCDR1, CPA.7.024.vhCDR2, CPA.7.024.vhCDR3, CPA.7.024.vlCDR1, CPA.7.024.vlCDR2, and CPA.7.024.vlCDR3;
CPA.7.033, CPA.7.033.VH, CPA.7.033.VL, CPA.7.033.HC, CPA.7.033.LC, CPA.7.033.H1, CPA.7.033.H2, CPA.7.033.H3 CPA.7.033.H4; CPA.7.033.vhCDR1, CPA.7.033.vhCDR2, CPA.7.033.vhCDR3, CPA.7.033.vlCDR1, CPA.7.033.vlCDR2, and CPA.7.033.vlCDR3;
CPA.7.034, CPA.7.034.VH, CPA.7.034.VL, CPA.7.034.HC, CPA.7.034.LC, CPA.7.034.H1, CPA.7.034.H2, CPA.7.034.H3 CPA.7.034.H4; CPA.7.034.vhCDR1, CPA.7.034.vhCDR2, CPA.7.034.vhCDR3, CPA.7.034.vlCDR1, CPA.7.034.vlCDR2, and CPA.7.034.vlCDR3;
CPA.7.036, CPA.7.036.VH, CPA.7.036.VL, CPA.7.036.HC, CPA.7.036.LC, CPA.7.036.H1, CPA.7.036.H2, CPA.7.036.H3 CPA.7.036.H4; CPA.7.036.vhCDR1, CPA.7.036.vhCDR2, CPA.7.036.vhCDR3, CPA.7.036.vlCDR1, CPA.7.036.vlCDR2, and CPA.7.036.vlCDR3;
CPA.7.040, CPA.7.040.VH, CPA.7.040.VL, CPA.7.040.HC, CPA.7.040.LC, CPA.7.040.H1, CPA.7.040.H2, CPA.7.040.H3 and CPA.7.040.H4; CPA.7.040.vhCDR1, CPA.7.040.vhCDR2, CPA.7.040.vhCDR3, CPA.7.040.vlCDR1, CPA.7.040.vlCDR2, and CPA.7.040.vlCDR3;
CPA.7.046, CPA.7.046.VH, CPA.7.046.VL, CPA.7.046.HC, CPA.7.046.LC, CPA.7.046.H1, CPA.7.046.H2, CPA.7.046.H3 CPA.7.046.H4; CPA.7.046.vhCDR1, CPA.7.046.vhCDR2, CPA.7.046.vhCDR3, CPA.7.046.vlCDR1, CPA.7.046.vlCDR2, and CPA.7.046.vlCDR3;
CPA.7.047, CPA.7.047.VH, CPA.7.047.VL, CPA.7.047.HC, CPA.7.047.LC, CPA.7.047.H1, CPA.7.047.H2, CPA.7.047.H3 CPA.7.047.H4; CPA.7.047.vhCDR1, CPA.7.047.vhCDR2, CPA.7.047.vhCDR3, CPA.7.047.vlCDR1, CPA.7.004701.vlCDR2, and CPA.7.047.vlCDR3;
CPA.7.049, CPA.7.049.VH, CPA.7.049.VL, CPA.7.049.HC, CPA.7.049.LC, CPA.7.049.H1, CPA.7.049.H2, CPA.7.049.H3 CPA.7.049.H4; CPA.7.049.vhCDR1, CPA.7.049.vhCDR2, CPA.7.049.vhCDR3, CPA.7.049.vlCDR1, CPA.7.049.vlCDR2, and CPA.7.049.vlCDR3; and
CPA.7.050, CPA.7.050.VH, CPA.7.050.VL, CPA.7.050.HC, CPA.7.050.LC, CPA.7.050.H1, CPA.7.050.H2, CPA.7.050.H3 CPA.7.050.H4, CPA.7.050.vhCDR1, CPA.7.050.vhCDR2, CPA.7.050.vhCDR3, CPA.7.050.vlCDR1, CPA.7.050.vlCDR2, and CPA.7.050.vlCDR3.

In addition, there are a number of CPA antibodies generated herein that bound to PVRIG but did not block the interaction of PVRIG and PVLR2 as shown in Figure 5A-5H, the components of which are :
CPA.7.028, CPA.7.028.VH, CPA.7.028.VL, CPA.7.028.HC, CPA.7.028.LC, CPA.7.028.H1, CPA.7.028.H2, CPA.7.028.H3 and CPA.7.028.H4; CPA.7.028.vhCDR1, CPA.7.028.vhCDR2, CPA.7.028.vhCDR3, CPA.7.028.vlCDR1, CPA.7.028.vlCDR2, and CPA.7.028.vlCDR3.
CPA.7.030, CPA.7.030.VH, CPA.7.030.VL, CPA.7.030.HC, CPA.7.030.LC, CPA.7.030.H1, CPA.7.030.H2, CPA.7.030.H3 and CPA.7.030.H4; CPA.7.030.vhCDR1, CPA.7.030.vhCDR2, CPA.7.030.vhCDR3, CPA.7.030.vlCDR1, CPA.7.030.vlCDR2, and CPA.7.030.vlCDR3.
CPA.7.041, CPA.7.041.VH, CPA.7.041.VL, CPA.7.041.HC, CPA.7.041.LC, CPA.7.041.H1, CPA.7.041.H2, CPA.7.041.H3 and CPA.7.041.H4; CPA.7.041.vhCDR1, CPA.7.041.vhCDR2, CPA.7.041.vhCDR3, CPA.7.041.vlCDR1, CPA.7.041.vlCDR2, and CPA.7.041.vlCDR3.
CPA.7.016, CPA.7.016.VH, CPA.7.016.VL, CPA.7.016.HC, CPA.7.016.LC, CPA.7.016.H1, CPA.7.016.H2, CPA.7.016.H3 and CPA.7.016.H4; CPA.7.016.vhCDR1, CPA.7.016.vhCDR2, CPA.7.016.vhCDR3, CPA.7.016.vlCDR1, CPA.7.016.vlCDR2, and CPA.7.016.vlCDR3.
CPA.7.020, CPA.7.020.VH, CPA.7.020.VL, CPA.7.020.HC, CPA.7.020.LC, CPA.7.020.H1, CPA.7.020.H2, CPA.7.020.H3 and CPA.7.020.H4; CPA.7.020.vhCDR1, CPA.7.020.vhCDR2, CPA.7.020.vhCDR3, CPA.7.020.vlCDR1, CPA.7.020.vlCDR2, and CPA.7.020.vlCDR3.
CPA.7.038, CPA.7.038.VH, CPA.7.038.VL, CPA.7.038.HC, CPA.7.038.LC, CPA.7.038.H1, CPA.7.038.H2, CPA.7.038.H3 and CPA.7.038.H4; CPA.7.038.vhCDR1, CPA.7.038.vhCDR2, CPA.7.038.vhCDR3, CPA.7.038.vlCDR1, CPA.7.038.vlCDR2, and CPA.7.038.vlCDR3.
CPA.7.044, CPA.7.044.VH, CPA.7.044.VL, CPA.7.044.HC, CPA.7.044.LC, CPA.7.044.H1, CPA.7.044.H2, CPA.7.044.H3 and CPA.7.044.H4; CPA.7.044.vhCDR1, CPA.7.044.vhCDR2, CPA.7.044.vhCDR3, CPA.7.044.vlCDR1, CPA.7.044.vlCDR2, and CPA.7.044.vlCDR3.
CPA.7.045, CPA.7.045.VH, CPA.7.045.VL, CPA.7.045.HC, CPA.7.045.LC, CPA.7.045.H1, CPA.7.045.H2, CPA.7.045.H3 and CPA.7.045.H4; CPA.7.045.vhCDR1, CPA.7.045.vhCDR2, CPA.7.045.vhCDR3, CPA.7.045.vlCDR1, CPA.7.045.vlCDR2, and CPA.7.045.vlCDR3.

As discussed herein, the invention further provides variants of the above components, including variants in the CDRs, as outlined above. In addition, variable heavy chains can be 80%, 90%, 95%, 98% or 99% identical to the "VH" sequences herein, and/or contain from 1, 2, 3, 4, 5, 6, 7, 8, 9, 10 amino acid changes, or more, when Fc variants are used. Variable light chains are provided that can be 80%, 90%, 95%, 98% or 99% identical to the "VL" sequences herein, and/or contain from 1, 2, 3, 4, 5, 6, 7, 8, 9, 10 amino acid changes, or more, when Fc variants are used. Similarly, heavy and light chains are provided that are 80%, 90%, 95%, 98% or 99% identical to the "HC" and "LC" sequences herein, and/or contain from 1, 2, 3, 4, 5, 6, 7, 8, 9, 10 amino acid changes, or more, when Fc variants are used.

Furthermore, the present invention provides a number of CHA antibodies, which are murine antibodies generated from hybridomas. As is well known the art, the six CDRs are useful when put into either human framework variable heavy and variable light regions or when the variable heavy and light domains are humanized.

Accordingly, the present invention provides antibodies, usually full length or scFv domains, that comprise the following CHA sets of CDRs, the sequences of which are shown in Figure 3A-3AE:
CHA.7.502.vhCDR1, CHA.7.502.vhCDR2, CHA.7.502.vhCDR3, CHA.7.502.vlCDR1, CHA.7.502.vlCDR2, and CHA.7.502.vlCDR3.
CHA.7.503.vhCDR1, CHA.7.503.vhCDR2, CHA.7.503.vhCDR3, CHA.7.503.vlCDR1, CHA.7.503.vlCDR2, and CHA.7.503.vlCDR3.
CHA.7.506.vhCDR1, CHA.7.506.vhCDR2, CHA.7.506.vhCDR3, CHA.7.506.vlCDR1, CHA.7.506.vlCDR2, and CHA.7.506.vlCDR3.
CHA.7.508.vhCDR1, CHA.7.508.vhCDR2, CHA.7.508.vhCDR3, CHA.7.508.vlCDR1, CHA.7.508.vlCDR2, and CHA.7.508.vlCDR3.
CHA.7.510.vhCDR1, CHA.7.510.vhCDR2, CHA.7.510.vhCDR3, CHA.7.510.vlCDR1, CHA.7.510.vlCDR2, and CHA.7.510.vlCDR3.
CHA.7.512.vhCDR1, CHA.7.512.vhCDR2, CHA.7.512.vhCDR3, CHA.7.512.vlCDR1, CHA.7.512.vlCDR2, and CHA.7.512.vlCDR3.
CHA.7.514.vhCDR1, CHA.7.514.vhCDR2, CHA.7.514.vhCDR3, CHA.7.514.vlCDR1, CHA.7.514.vlCDR2, and CHA.7.514.vlCDR3.
CHA.7.516.vhCDR1, CHA.7.516.vhCDR2, CHA.7.516.vhCDR3, CHA.7.516.vlCDR1, CHA.7.516.vlCDR2, and CHA.7.516.vlCDR3.
CHA.7.518.vhCDR1, CHA.7.518.vhCDR2, CHA.7.518.vhCDR3, CHA.7.518.vlCDR1, CHA.7.518.vlCDR2, and CHA.7.518.vlCDR3.
CHA.7.520_1.vhCDR1, CHA.7.520_1.vhCDR2, CHA.7.520_1.vhCDR3, CHA.7.520_1.vlCDR1, CHA.7.520_1.vlCDR2, and CHA.7.520_1.vlCDR3.
CHA.7.520_2.vhCDR1, CHA.7.520_2.vhCDR2, CHA.7.520_2.vhCDR3, CHA.7.520_2.vlCDR1, CHA.7.520_2.vlCDR2, and CHA.7.520_2.vlCDR3.
CHA.7.522.vhCDR1, CHA.7.522.vhCDR2, CHA.7.522.vhCDR3, CHA.7.522.vlCDR1, CHA.7.522.vlCDR2, and CHA.7.522.vlCDR3.
CHA.7.524.vhCDR1, CHA.7.524.vhCDR2, CHA.7.524.vhCDR3, CHA.7.524.vlCDR1, CHA.7.524.vlCDR2, and CHA.7.524.vlCDR3.
CHA.7.526.vhCDR1, CHA.7.526.vhCDR2, CHA.7.526.vhCDR3, CHA.7.526.vlCDR1, CHA.7.526.vlCDR2, and CHA.7.526.vlCDR3.
CHA.7.527.vhCDR1, CHA.7.527.vhCDR2, CHA.7.527.vhCDR3, CHA.7.527.vlCDR1, CHA.7.527.vlCDR2, and CHA.7.527.vlCDR3.
CHA.7.528.vhCDR1, CHA.7.528.vhCDR2, CHA.7.528.vhCDR3, CHA.7.528.vlCDR1, CHA.7.528.vlCDR2, and CHA.7.528.vlCDR3.
CHA.7.530.vhCDR1, CHA.7.530.vhCDR2, CHA.7.530.vhCDR3, CHA.7.530.vlCDR1, CHA.7.530.vlCDR2, and CHA.7.530.vlCDR3.
CHA.7.534.vhCDR1, CHA.7.534.vhCDR2, CHA.7.534.vhCDR3, CHA.7.534.vlCDR1, CHA.7.534.vlCDR2, and CHA.7.534.vlCDR3.
CHA.7.535.vhCDR1, CHA.7.535.vhCDR2, CHA.7.535.vhCDR3, CHA.7.535.vlCDR1, CHA.7.535.vlCDR2, and CHA.7.535.vlCDR3.
CHA.7.537.vhCDR1, CHA.7.537.vhCDR2, CHA.7.537.vhCDR3, CHA.7.537.vlCDR1, CHA.7.537.vlCDR2, and CHA.7.537.vlCDR3.
CHA.7.538_1.vhCDR1, CHA.7.538_1.vhCDR2, CHA.7.538_1.vhCDR3, CHA.7.538_1.vlCDR1, CHA.7.538_1.vlCDR2, and CHA.7.538_1.vlCDR3.
CHA.7.538_2.vhCDR1, CHA.7.538_2.vhCDR2, CHA.7.538_2.vhCDR3, CHA.7.538_2.vlCDR1, CHA.7.538_2.vlCDR2, and CHA.7.538_2.vlCDR3.
CHA.7.543.vhCDR1, CHA.7.543.vhCDR2, CHA.7.543.vhCDR3, CHA.7.543.vlCDR1, CHA.7.543.vlCDR2, and CHA.7.543.vlCDR3.
CHA.7.544.vhCDR1, CHA.7.544.vhCDR2, CHA.7.544.vhCDR3, CHA.7.544.vlCDR1, CHA.7.544.vlCDR2, and CHA.7.544.vlCDR3.
CHA.7.545.vhCDR1, CHA.7.545.vhCDR2, CHA.7.545.vhCDR3, CHA.7.545.vlCDR1, CHA.7.545.vlCDR2, and CHA.7.545.vlCDR3.
CHA.7.546.vhCDR1, CHA.7.546.vhCDR2, CHA.7.546.vhCDR3, CHA.7.546.vlCDR1, CHA.7.546.vlCDR2, and CHA.7.546.vlCDR3.
CHA.7.547.vhCDR1, CHA.7.547.vhCDR2, CHA.7.547.vhCDR3, CHA.7.547.vlCDR1, CHA.7.547.vlCDR2, and CHA.7.547.vlCDR3.
CHA.7.548.vhCDR1, CHA.7.548.vhCDR2, CHA.7.548.vhCDR3, CHA.7.548.vlCDR1, CHA.7.548.vlCDR2, and CHA.7.548.vlCDR3.
CHA.7.549.vhCDR1, CHA.7.549.vhCDR2, CHA.7.549.vhCDR3, CHA.7.549.vlCDR1, CHA.7.549.vlCDR2, and CHA.7.549.vlCDR3.
CHA.7.550.vhCDR1, CHA.7.550.vhCDR2, CHA.7.550.vhCDR3, CHA.7.550.vlCDR1, CHA.7.550.vlCDR2, and CHA.7.550.vlCDR3.

As above, these sets of CDRs may also be amino acid variants as described above.

In addition, the framework regions of the variable heavy and variable light chains can be humanized as is known in the art (with occasional variants generated in the CDRs as needed), and thus humanized variants of the VH and VL chains of Figure 3A-3AE can be generated. Furthermore, the humanized variable heavy and light domains can then be fused with human constant regions, such as the constant regions from IgG1, IgG2, IgG3 and IgG4.

In particular, as is known in the art, murine VH and VL chains can be humanized as is known in the art, for example, using the IgBLAST program of the NCBI website, as outlined in Ye et al. Nucleic Acids Res. 41:W34-W40 (2013), herein incorporated by reference in its entirety for the humanization methods. IgBLAST takes a murine VH and/or VL sequence and compares it to a library of known human germline sequences. As shown herein, for the humanized sequences generated herein, the databases used were IMGT human VH genes (F+ORF, 273 germline sequences) and IMGT human VL kappa genes (F+ORF, 74 germline sequences). An exemplary five CHA sequences were chosen: CHA.7.518, CHA.7.530, CHA.7.538_1, CHA.7.538_2 and CHA.7.524 (see Figure 3A-3AE for the VH and VL sequences). For this embodiment of the humanization, human germline IGHV1-46(allele1) was chosen for all 5 as the acceptor sequence and the human heavy chain IGHJ4(allele1) joining region (J gene). For three of four (CHA.7.518, CHA.7.530, CHA.7.538_1 and CHA.7.538_2), human germline IGKV1-39(allele 1) was chosen as the acceptor sequence and human light chain IGKJ2(allele1) (J gene) was chosen. The J gene was chosen from human joining region sequences compiled at IMGT^{®} the international ImMunoGeneTics information system as www.imgt.org. CDRs were defined according to the AbM definition (see www.bioinfo.org.uk/abs/).

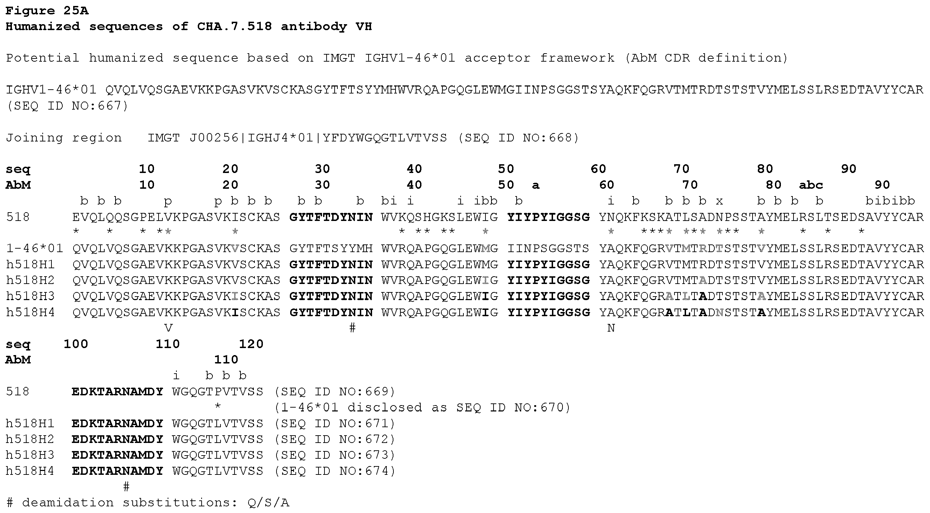

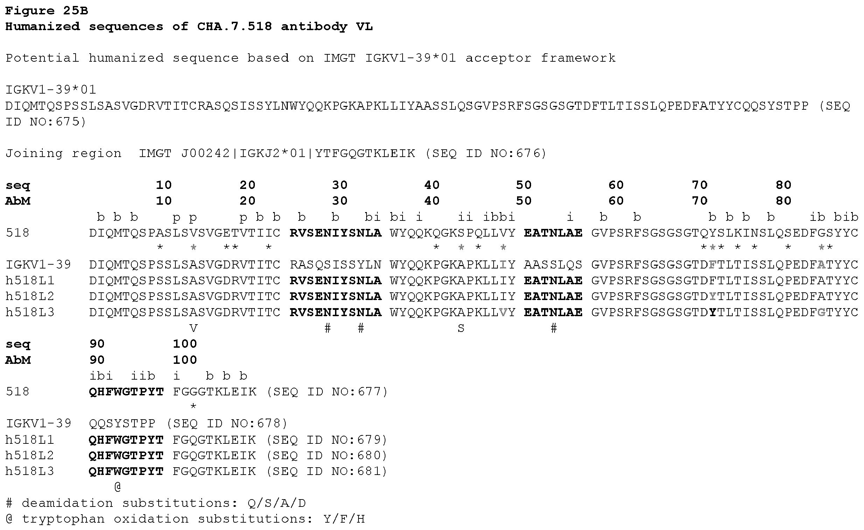

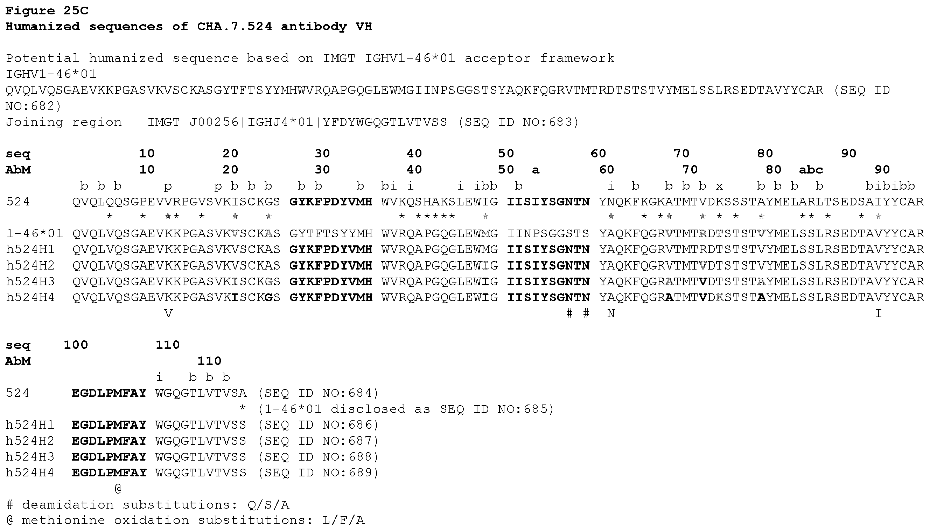

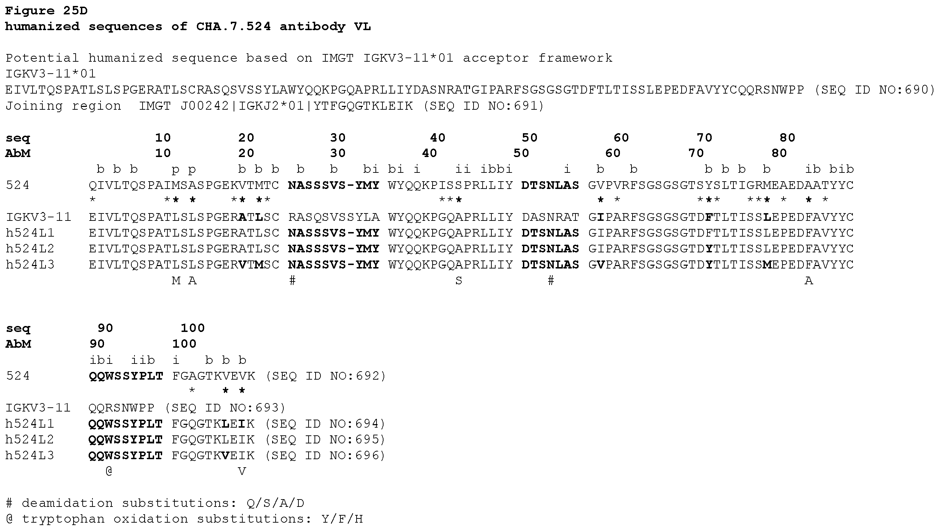

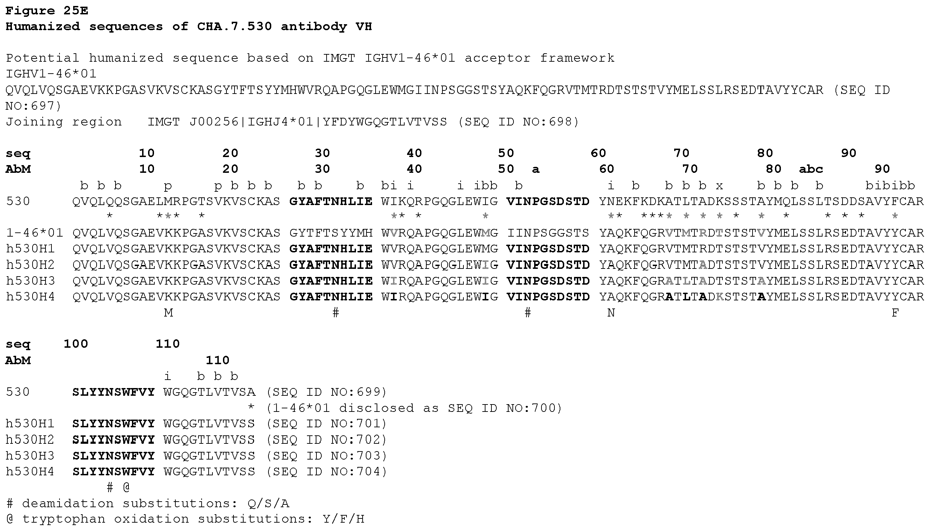

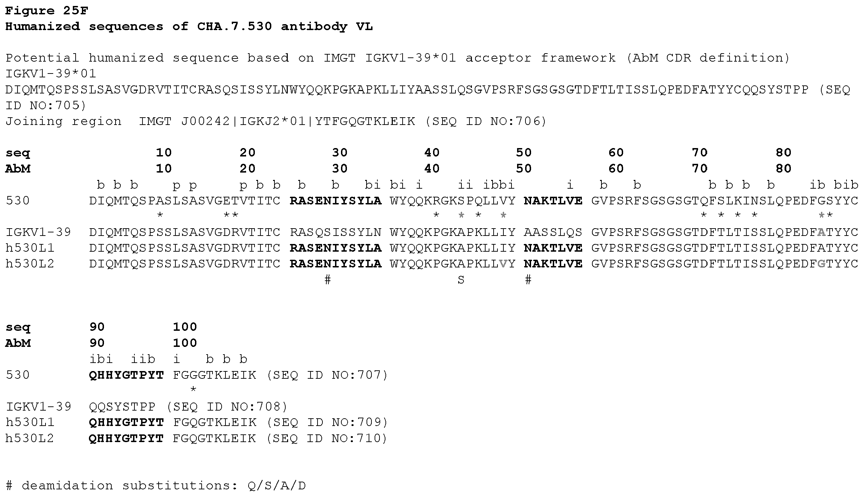

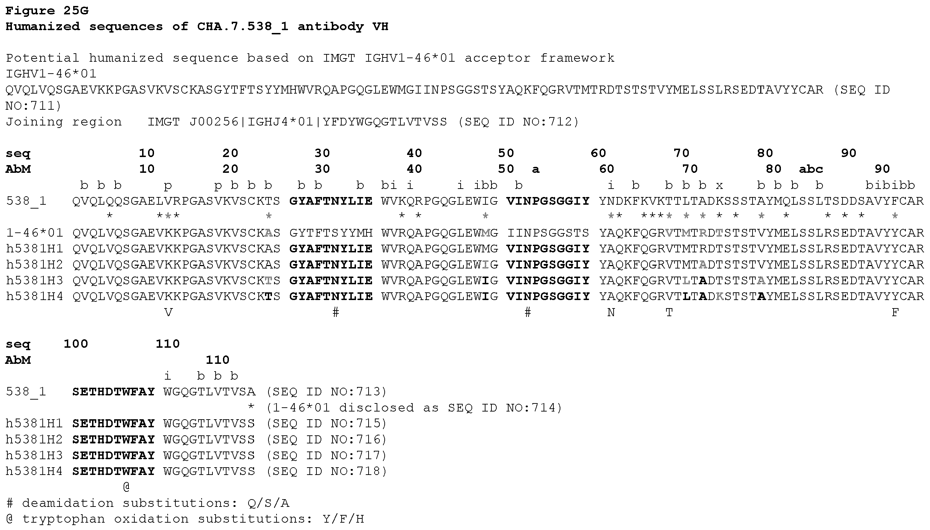

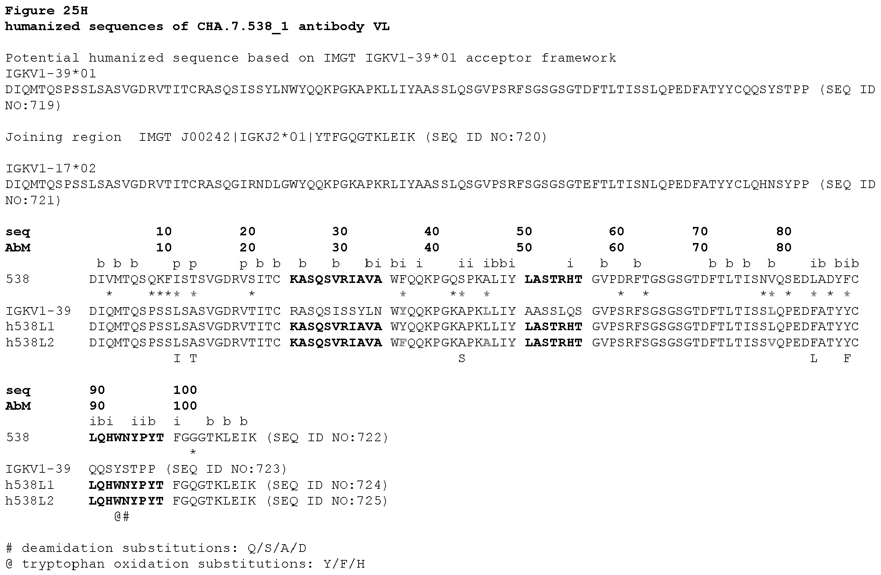

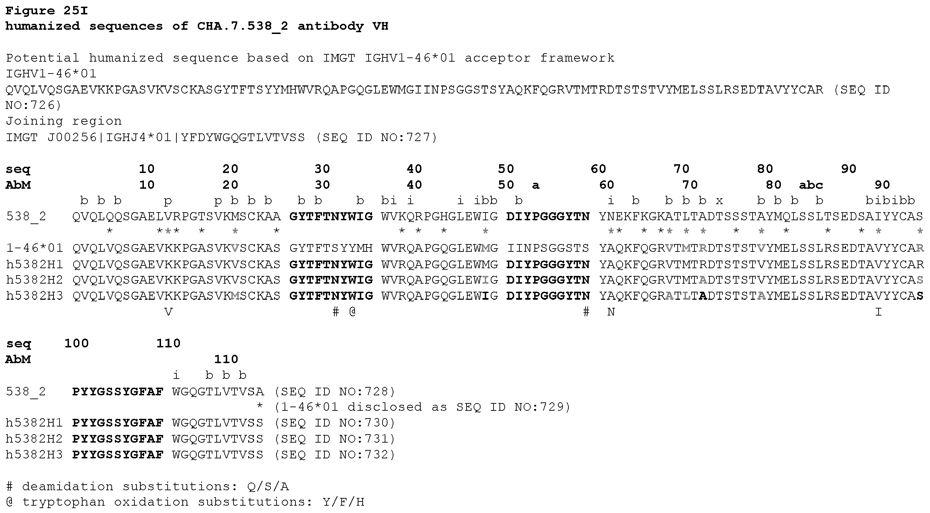

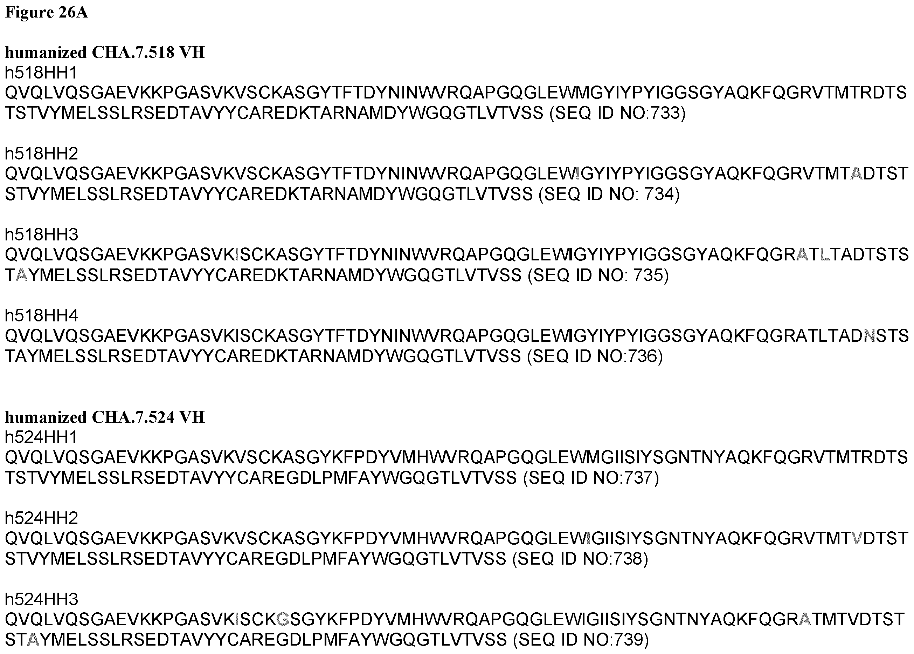

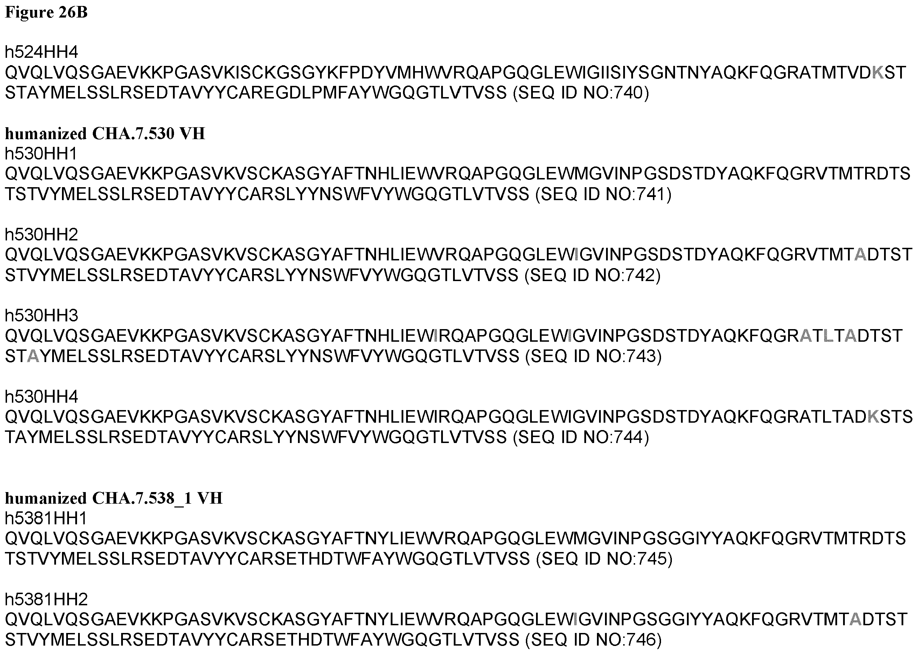

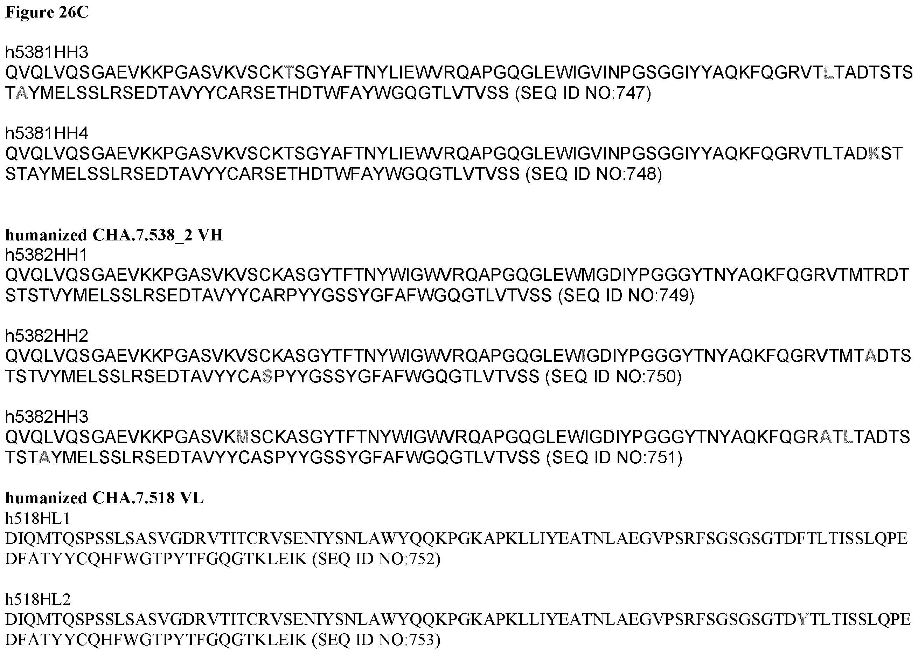

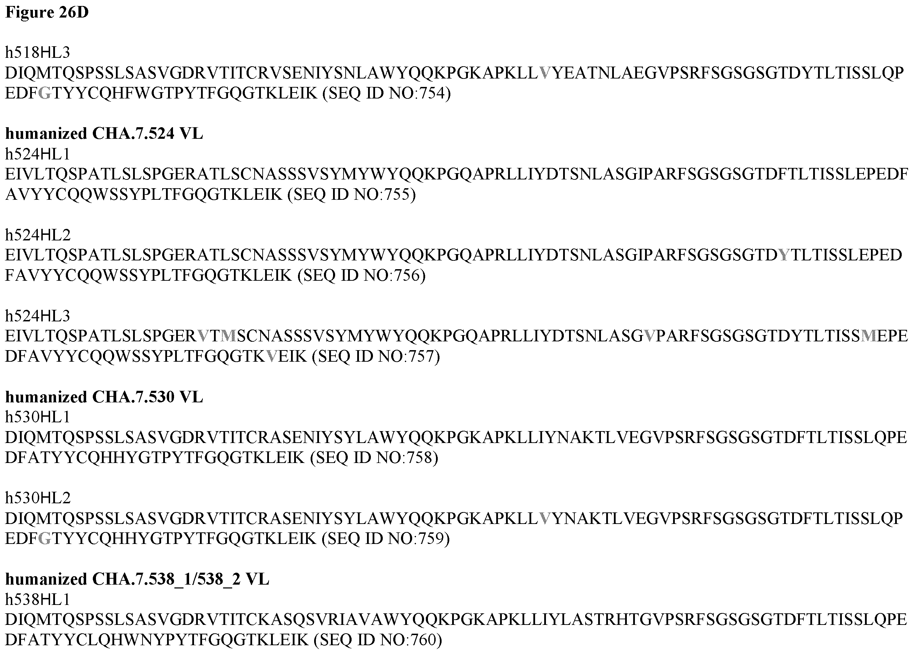

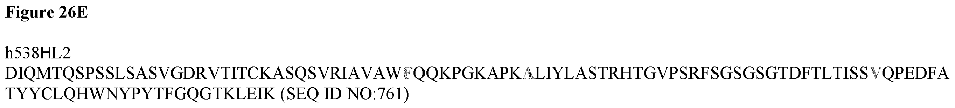

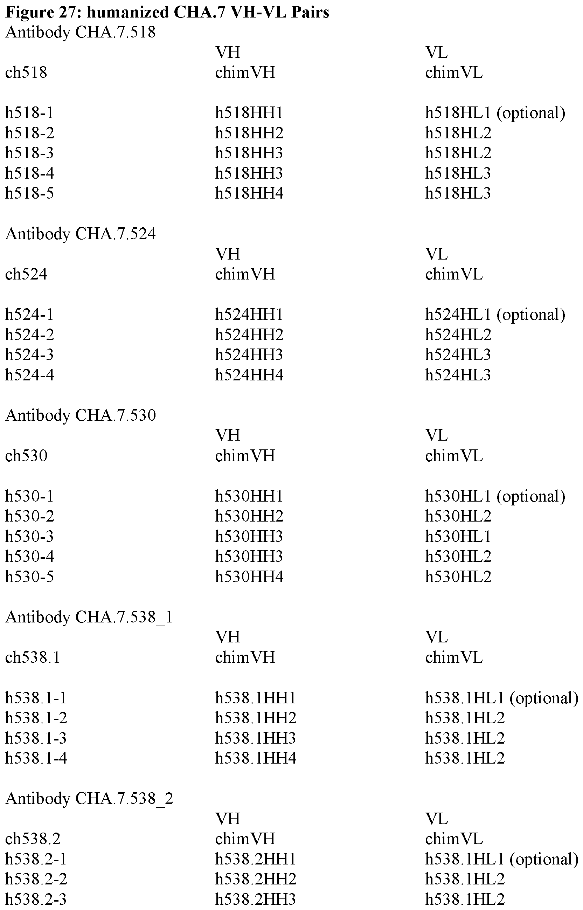

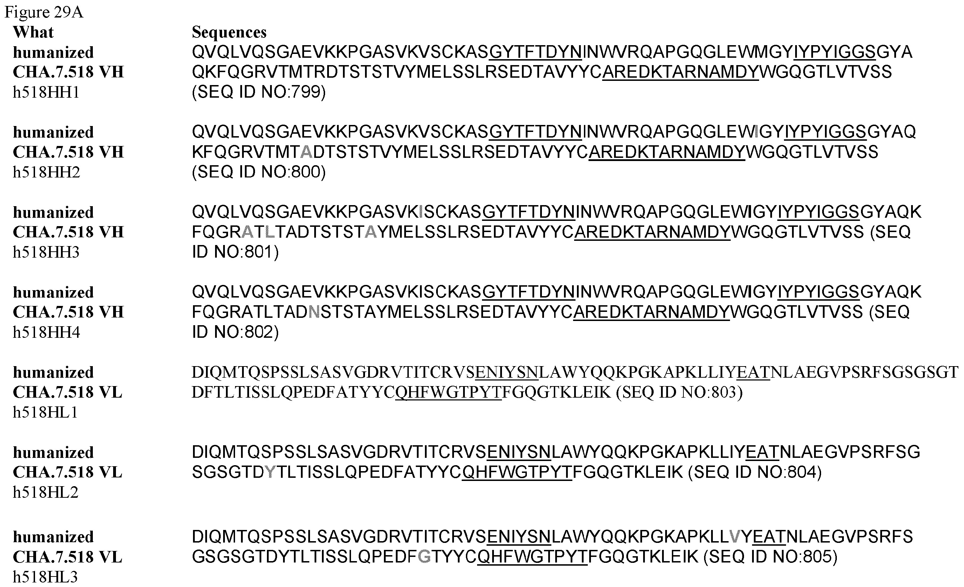

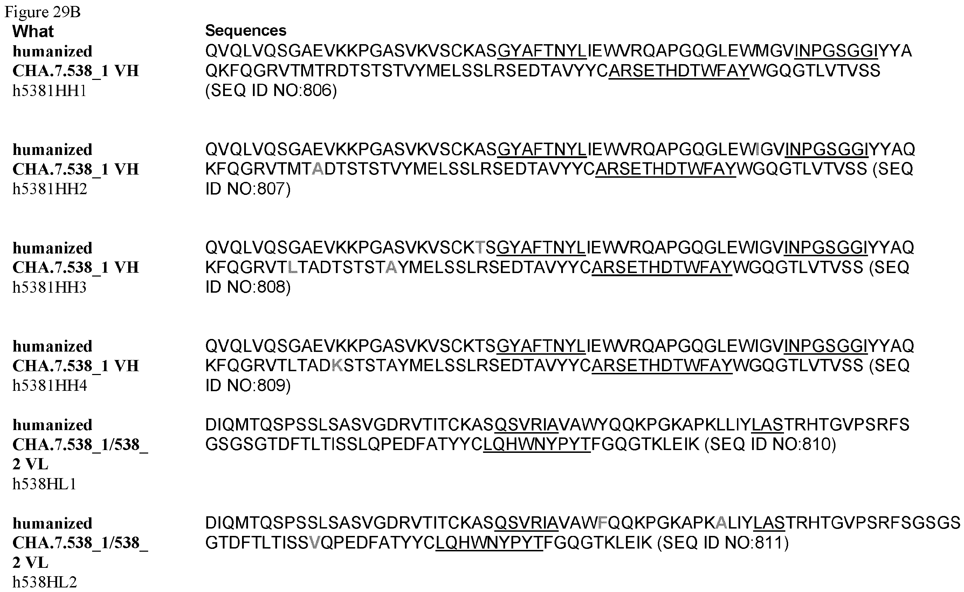

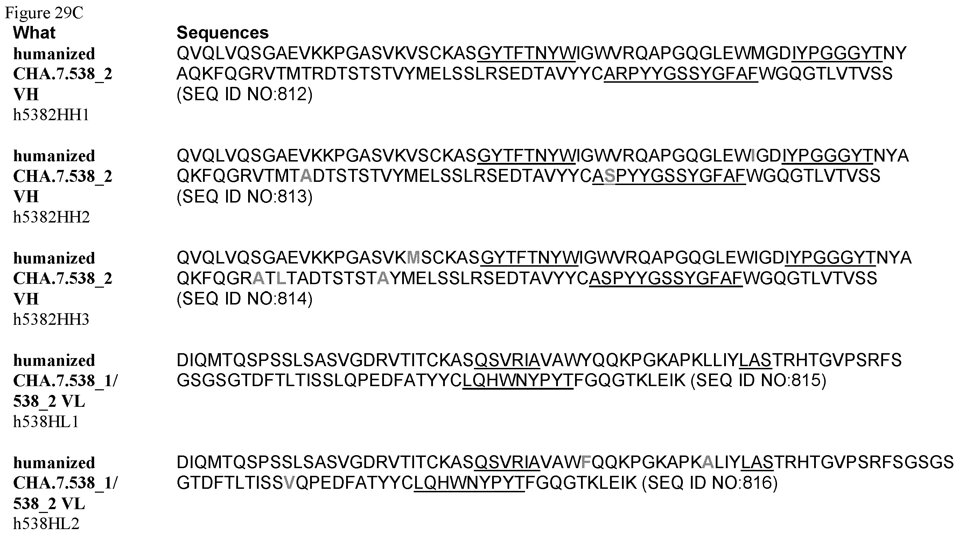

Specific humanized antibodies of CHA antibodies include those shown in Figures 25, 26, 27, 28, and 29. As will be appreciated by those in the art, each humanized variable heavy (**H**umanized **H**eavy; HH) and variable light (**H**umanized **L**ight, HL) sequence can be combined with the constant regions of human IgG1, IgG2, IgG3 and IgG4. That is, CHA.7.518.HH1 is the first humanized variable heavy chain, and CHA.7.518.HH1.**1** is the full length heavy chain, comprising the "HH1" humanized sequence with a IgG**1** constant region (CHA.7.518.HH1.**2** is CHA.7.518.HH1 with IgG**2**, etc,).

In some embodiments, the anti-PVRIG antibodies of the present invention include anti-PVRIG antibodies wherein the V_{H} and V_{L} sequences of different anti-PVRIG antibodies can be "mixed and matched" to create other anti-PVRIG antibodies. PVRIG binding of such "mixed and matched" antibodies can be tested using the binding assays described above. e.g., ELISAs). In some embodiments, when V_{H} and V_{L} chains are mixed and matched, a V_{H} sequence from a particular V_{H}/V_{L} pairing is replaced with a structurally similar V_{H} sequence. Likewise, in some embodiments, a V_{L} sequence from a particular V_{H}/V_{L} pairing is replaced with a structurally similar V_{L} sequence. For example, the V_{H} and V_{L} sequences of homologous antibodies are particularly amenable for mixing and matching.

Accordingly, the antibodies of the invention comprise CDR amino acid sequences selected from the group consisting of (a) sequences as listed herein; (b) sequences that differ from those CDR amino acid sequences specified in (a) by 1, 2, 3, 4, 5, 6, 7, 8, 9, 10 or more amino acid substitutions; (c) amino acid sequences having 90% or greater, 95% or greater, 98% or greater, or 99% or greater sequence identity to the sequences specified in (a) or (b); (d) a polypeptide having an amino acid sequence encoded by a polynucleotide having a nucleic acid sequence encoding the amino acids as listed herein.

Additionally included in the definition of PVRIG antibodies are antibodies that share identity to the PVRIG antibodies enumerated herein. That is, in certain embodiments, an anti-PVRIG antibody according to the invention comprises heavy and light chain variable regions comprising amino acid sequences that are homologous to isolated anti-PVRIG amino acid sequences of preferred anti-PVRIG immune molecules, respectively, wherein the antibodies retain the desired functional properties of the parent anti-PVRIG antibodies. The percent identity between the two sequences is a function of the number of identical positions shared by the sequences (i.e., % homology=# of identical positions/total # of positions X 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used.

In general, the percentage identity for comparison between PVRIG antibodies is at least 75%, at least 80%, at least 90%, with at least about 95, 96, 97, 98 or 99% percent identity being preferred. The percentage identity may be along the whole amino acid sequence, for example the entire heavy or light chain or along a portion of the chains. For example, included within the definition of the anti-PVRIG antibodies of the invention are those that share identity along the entire variable region (for example, where the identity is 95 or 98% identical along the variable regions), or along the entire constant region, or along just the Fc domain.

In addition, also included are sequences that may have the identical CDRs but changes in the variable domain (or entire heavy or light chain). For example, PVRIG antibodies include those with CDRs identical to those shown in described herein but whose identity along the variable region can be lower, for example 95 or 98% percent identical.

### 1. PVRIG Antibodies that Compete for binding with Enumerated Antibodies

The present invention provides not only the enumerated antibodies but additional antibodies that compete with the enumerated antibodies (the CPA and CHA numbers enumerated herein that specifically bind to PVRIG) to specifically bind to the PVRIG molecule. The PVRIG antibodies of the invention "bin" into different epitope bins. There are four separate bins outlined herein; 1) the epitope bin into which CPA.7.002, CPA.7.003, CPA.7.005, CPA.7.007, CPA.7.010, CPA.7.012, CPA.7.015, CPA.7.016, CPA.7.017, CPA.7.019, CPA.7.020, CPA.7.021, CPA.7.024, CPA.7.028, CPA.7.032, CPA.7.033, CPA.7.036, CPA.7.037, CPA.7.038, CPA.7.043, CPA.7.046 and CPA.7.041 all fall into; 2) the epitope bin into which CPA.7.004, CPA.7.009, CPA.7.011, CPA.7.014, CPA.7.018, CPA.7.022, CPA.7.023, CPA.7.034, CPA.7.040, CPA.7.045 and CPA.7.047 all fall; 3) CPA.7.039, which defines the distinction between bin 1 and bin 2, in that bin 1 blocks CPA.7.039 binding and bin 2 sandwiches the ligand with CPA.7.039, and bin 4) with CPA.7.050.

Thus, the invention provides anti-PVRIG antibodies that compete for binding with antibodies that are in bin 1, with antibodies that are in bin 2, with antibodies that are inbin 3 and/or with antibodies that are in bin 4.

Additional antibodies that compete with the enumerated antibodies are generated, as is known in the art and generally outlined below. Competitive binding studies can be done as is known in the art, generally using SPR/Biacore^{®} binding assays, as well as ELISA and cell-based assays.

### D. TIGIT Antibodies

The present invention provides anti-TIGIT antibodies. (For convenience, "anti-TIGIT antibodies" and "TIGIT antibodies" are used interchangeably). The anti- TIGIT antibodies of the invention specifically bind to human TIGIT, and preferably the ECD of human TIGIT. The invention further provides antigen binding domains, including full length antibodies, which contain a number of specific, enumerated sets of 6 CDRs that bind to TIGIT.

Specific binding for TIGIT or a TIGIT epitope can be exhibited, for example, by an antibody having a K_{D} of at least about 10⁻⁴ M, at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, at least about 10⁻¹³ M, at least about 10⁻¹⁴ M, at least about 10⁻¹⁵ M, or greater, where K_{D} refers to the equilibrium dissociation constant of a particular antibody-antigen interaction. Typically, an antibody that specifically binds an antigen will have a K_{D} that is 20-, 50-, 100-, 500-, 1000-, 5,000-, 10,000- or more times greater for a control molecule relative to the TIGIT antigen or epitope.

However, for optimal binding to TIGIT expressed on the surface of NK and T-cells, the antibodies preferably have a KD less 50 nM and most preferably less than 1 nM, with less than 0.1 nM and less than 1 pM finding use in the methods of the invention

Also, specific binding for a particular antigen or an epitope can be exhibited, for example, by an antibody having a ka (referring to the association rate constant) for a TIGIT antigen or epitope of at least 20-, 50-, 100-, 500-, 1000-, 5,000-, 10,000- or more times greater for the epitope relative to a control, where ka refers to the association rate constant of a particular antibody-antigen interaction.

In some embodiments, the anti-TIGIT antibodies of the invention bind to human TIGIT with a K_{D} of 100 nM or less, 50 nM or less, 10 nM or less, or 1 nM or less (that is, higher binding affinity), or 1pM or less, wherein K_{D} is determined by known methods, e.g. surface plasmon resonance (SPR, e.g. Biacore assays), ELISA, KINEXA, and most typically SPR at 25° or 37° C.

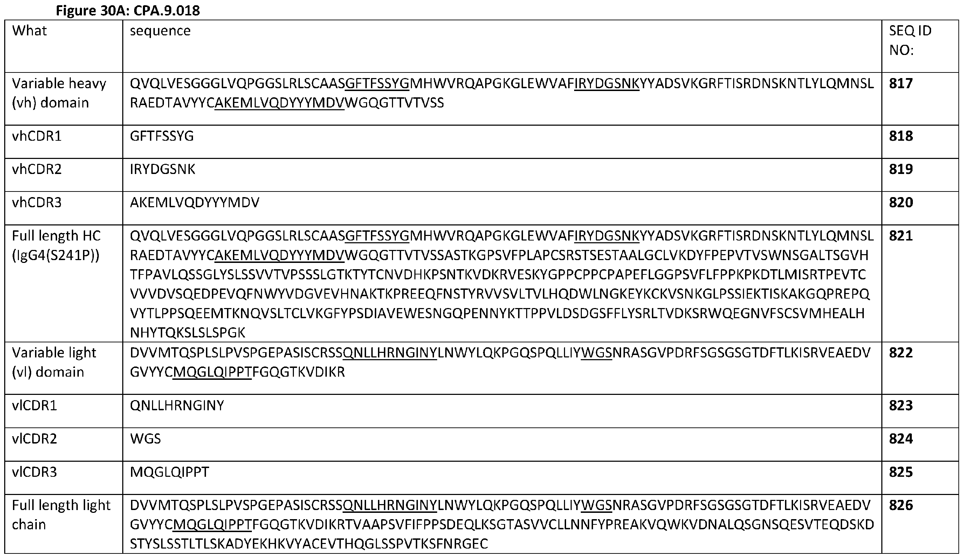

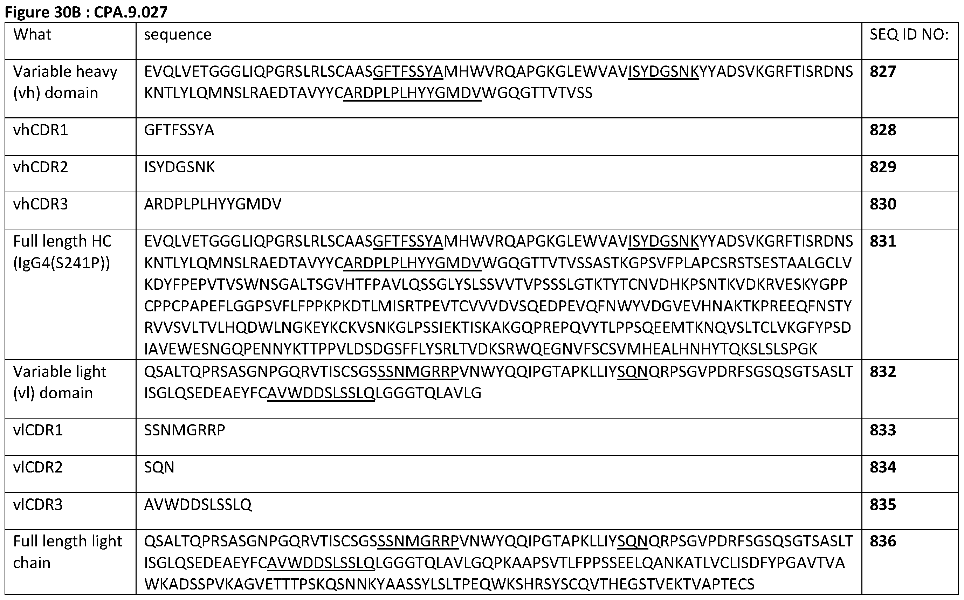

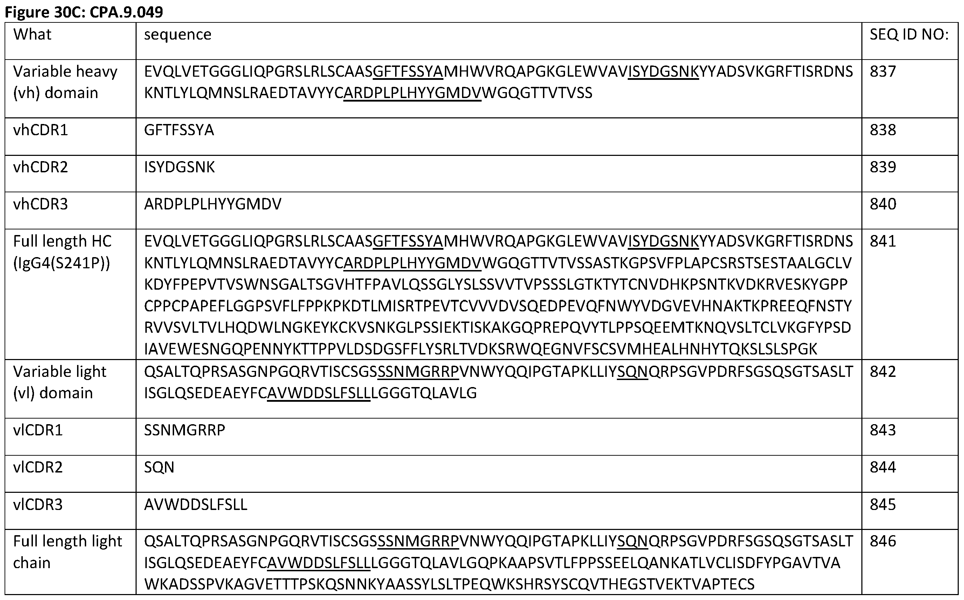

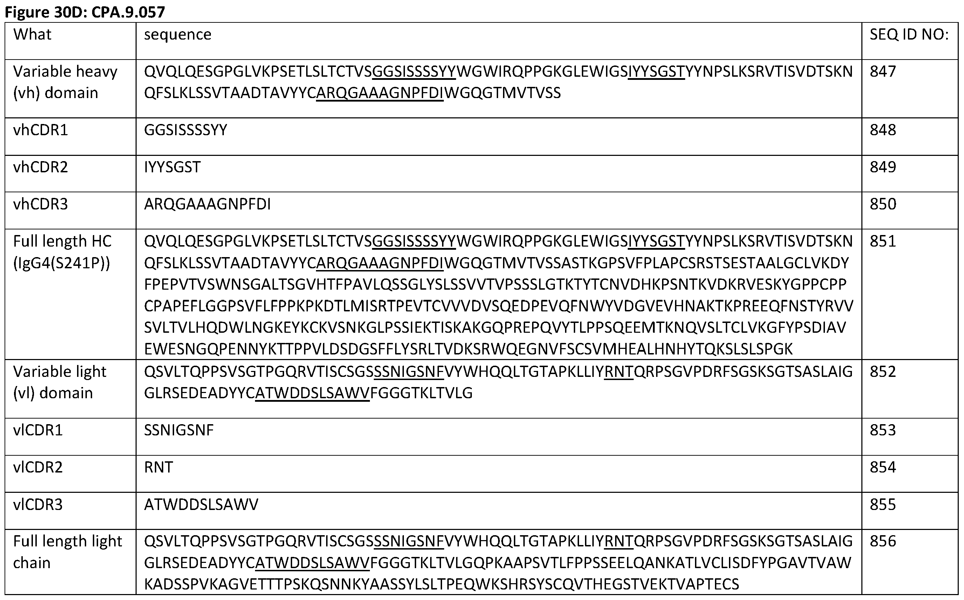

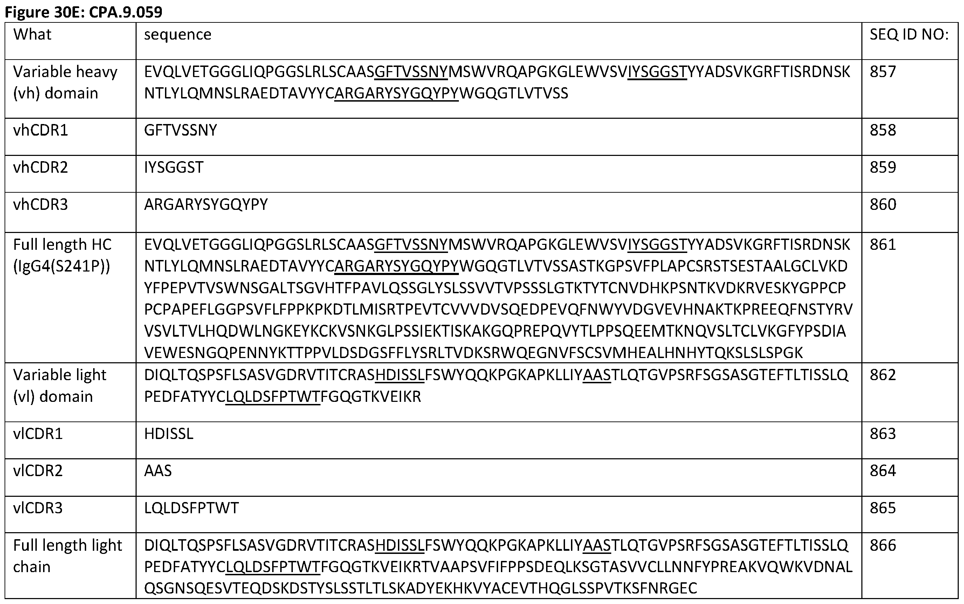

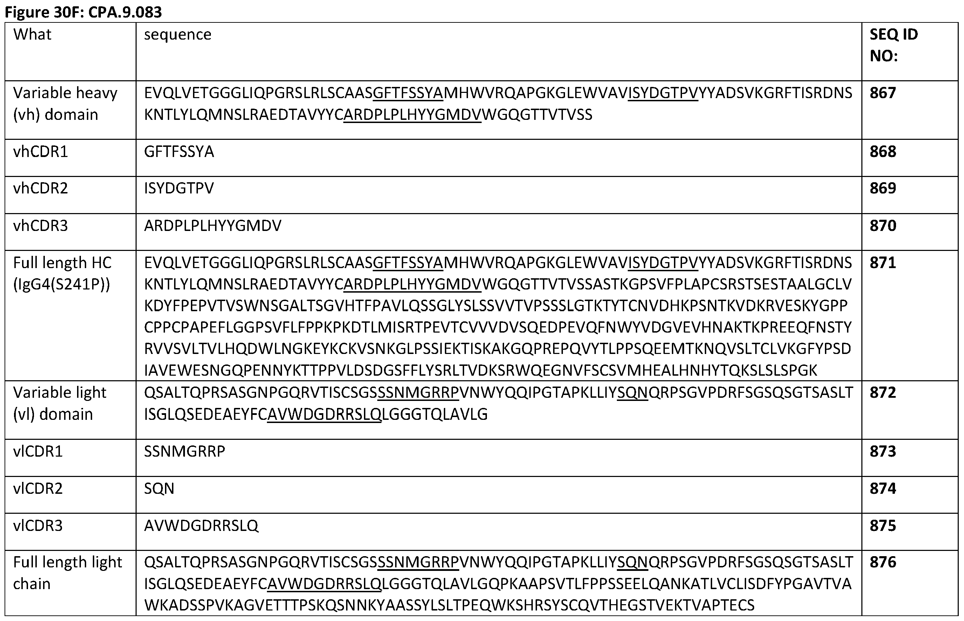

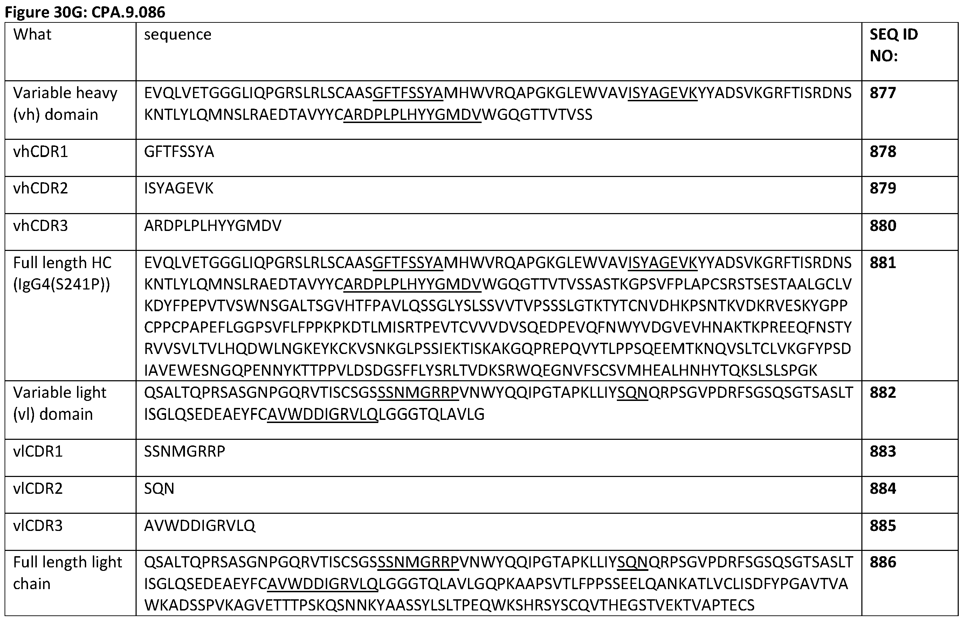

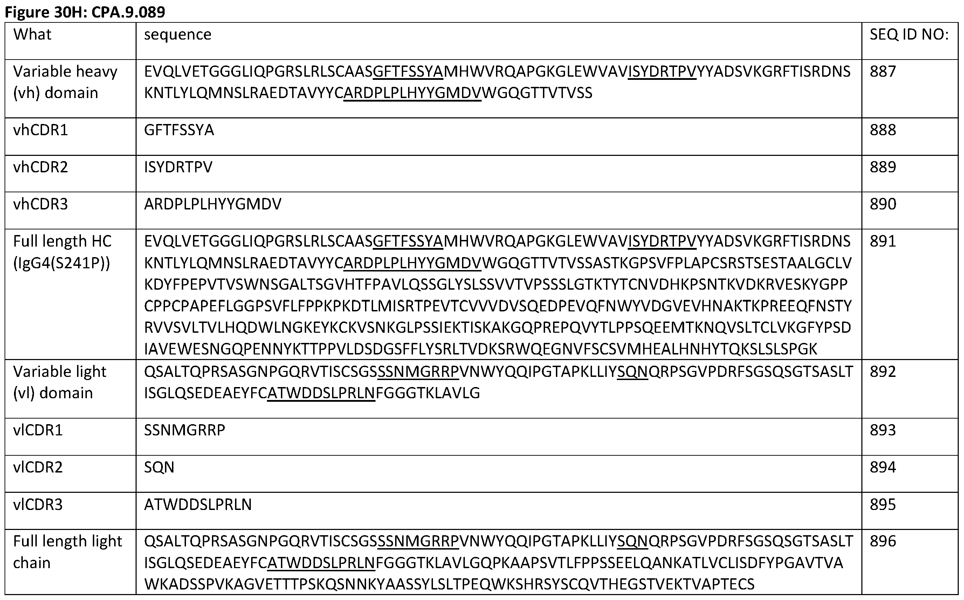

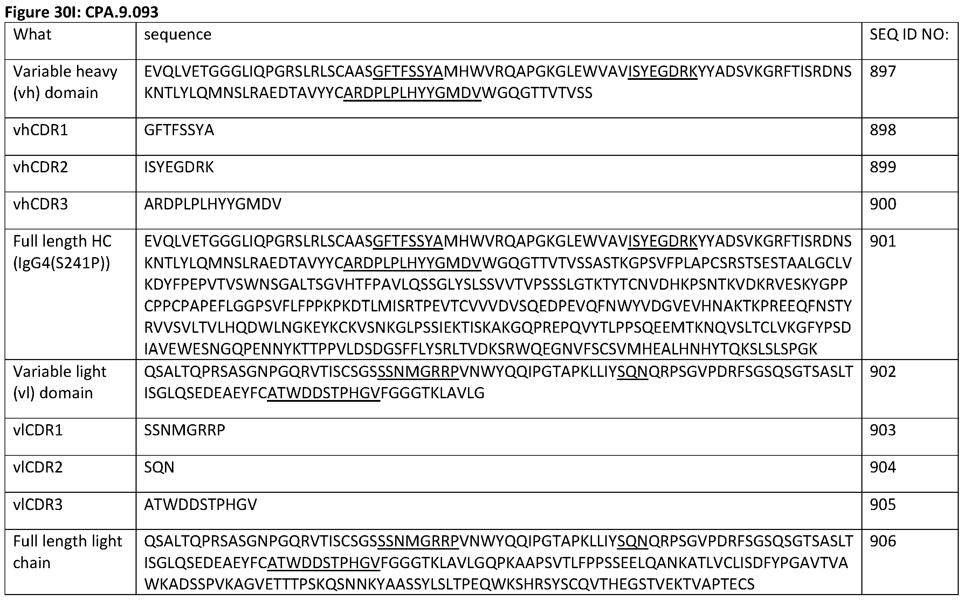

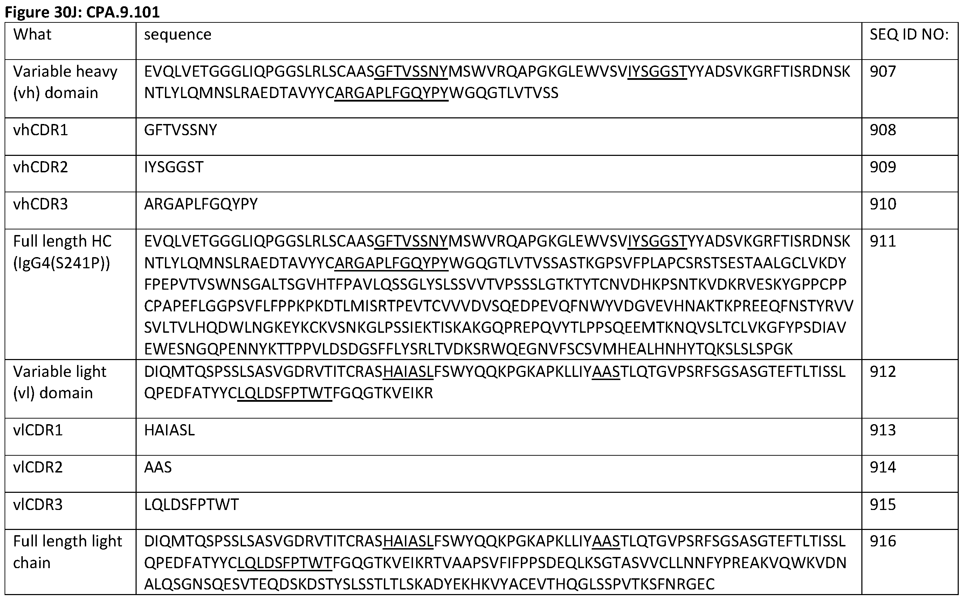

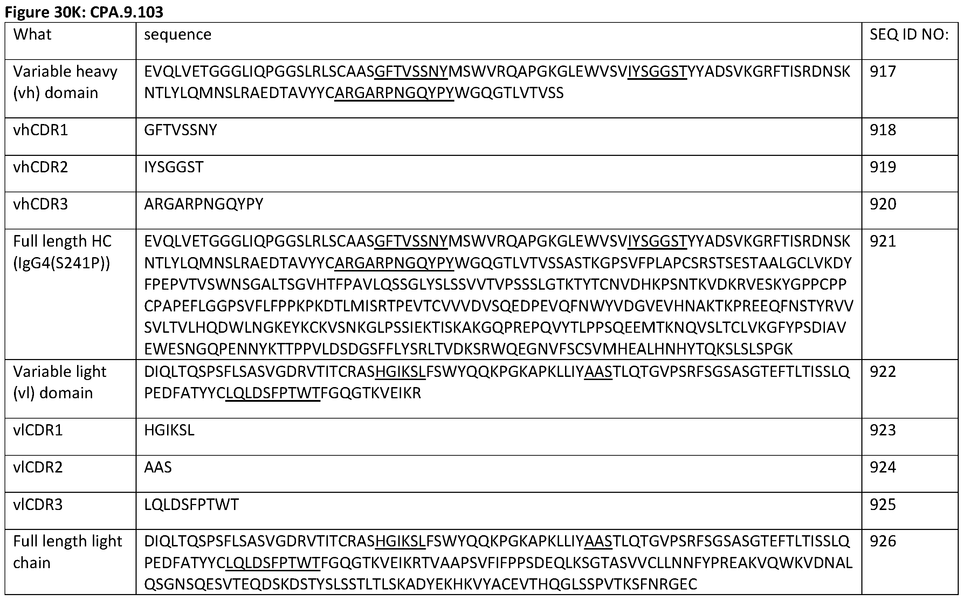

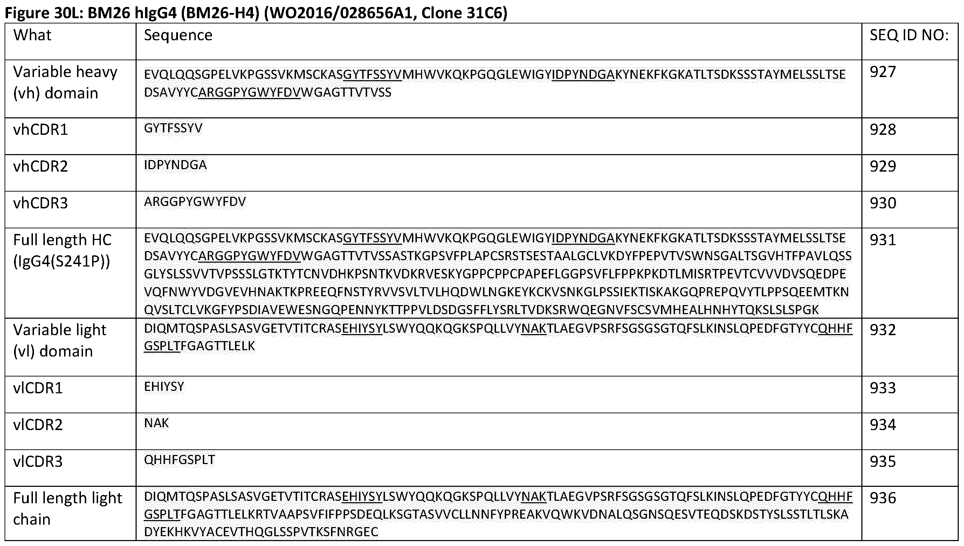

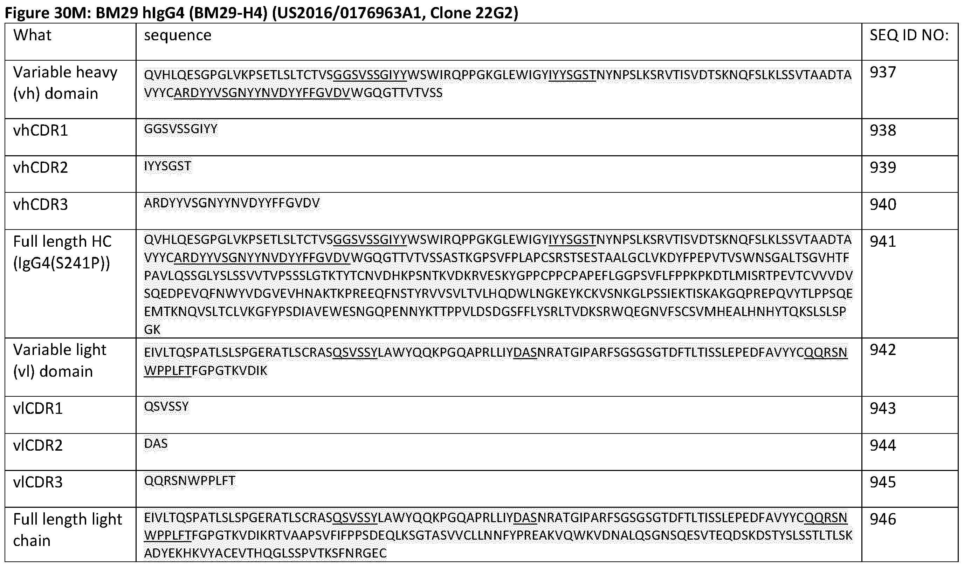

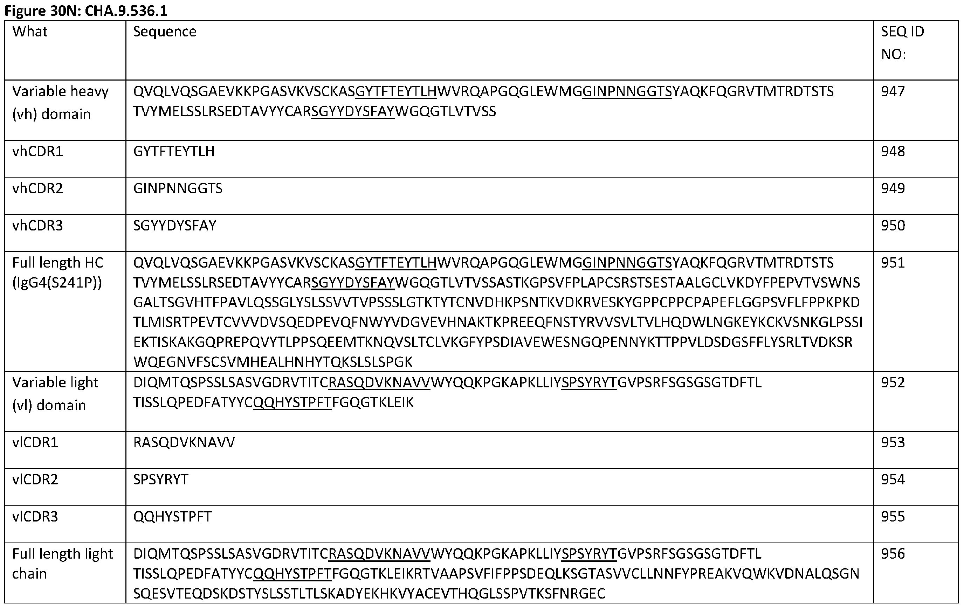

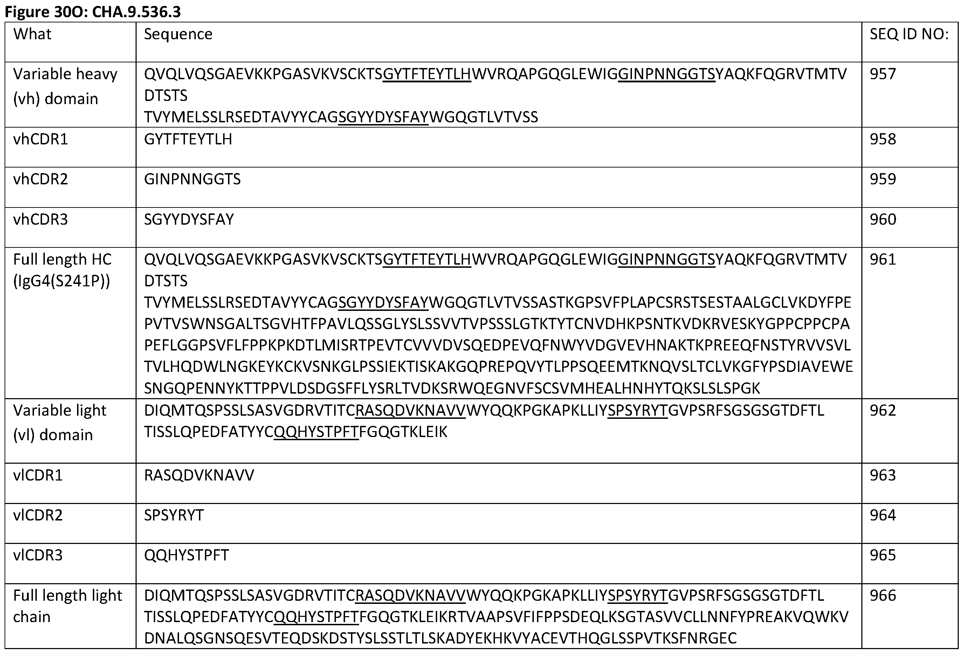

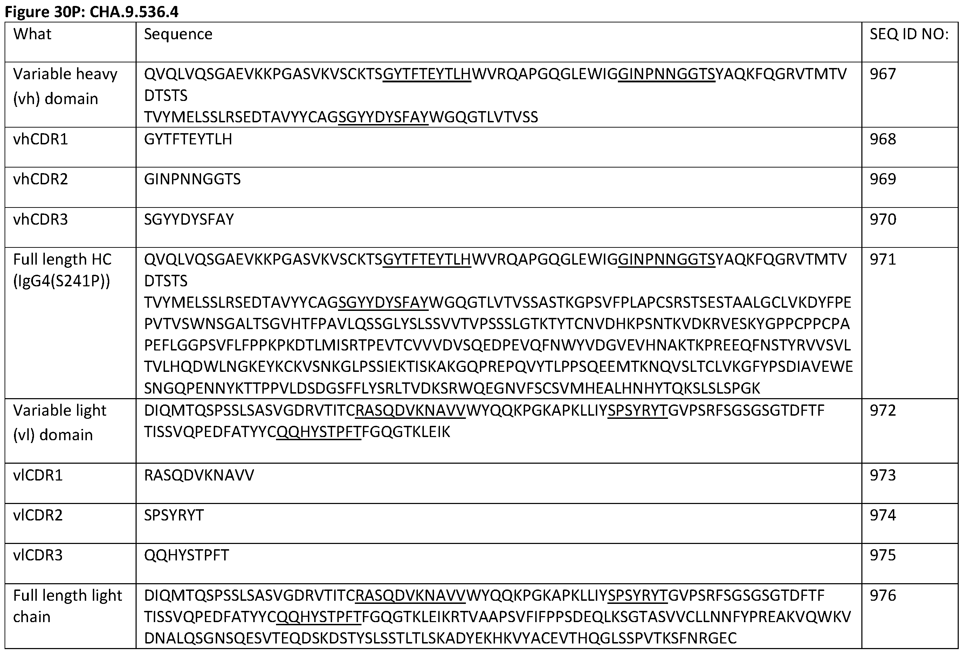

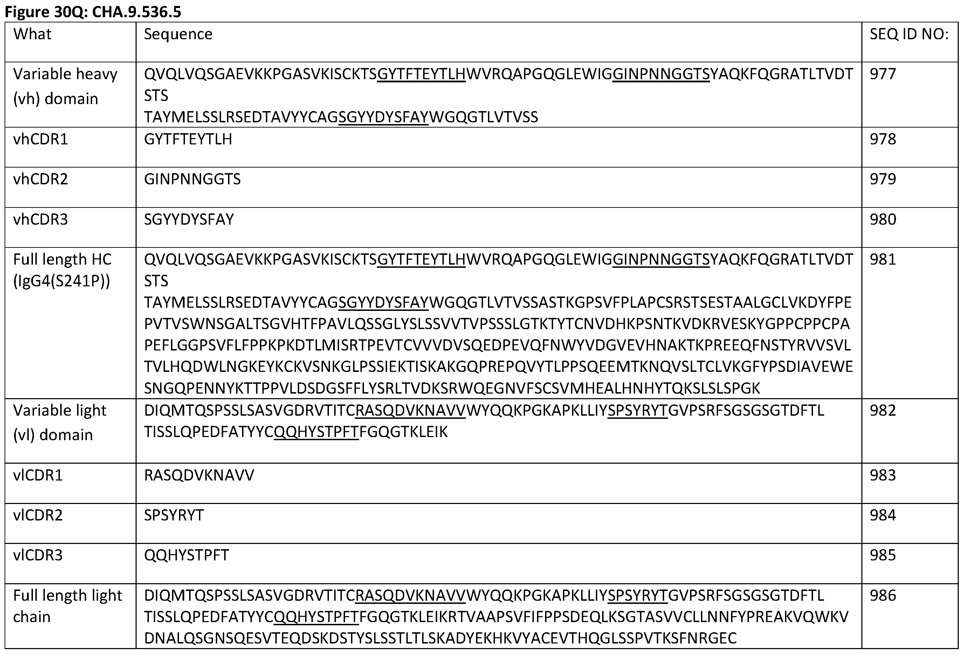

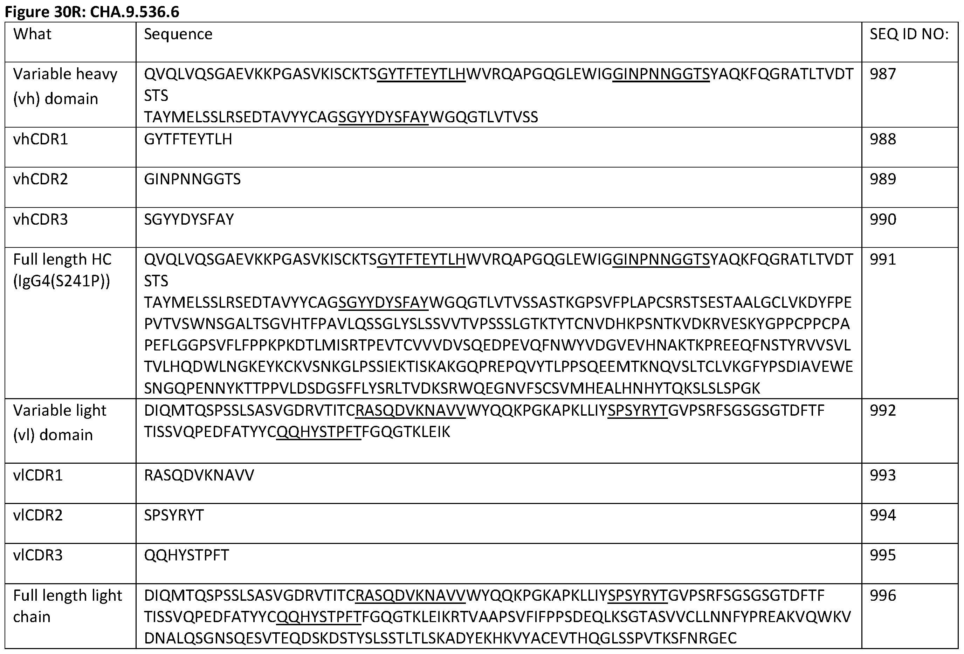

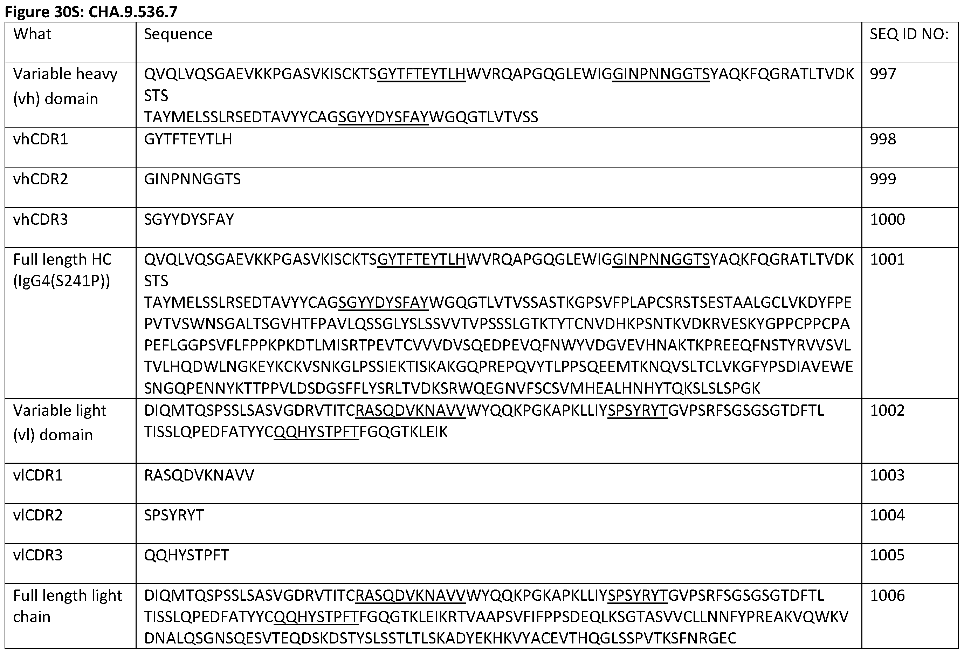

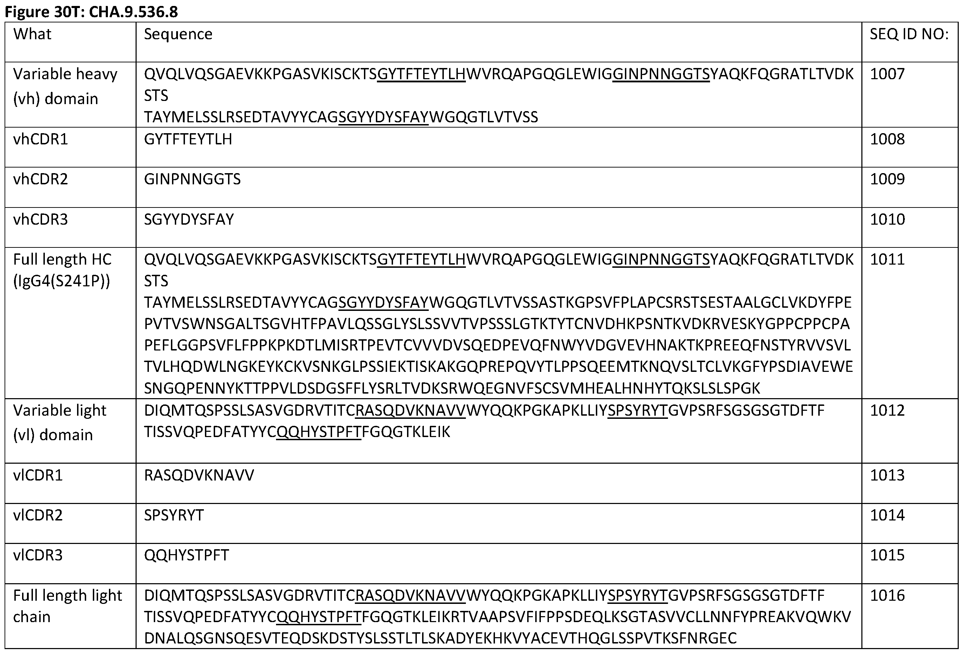

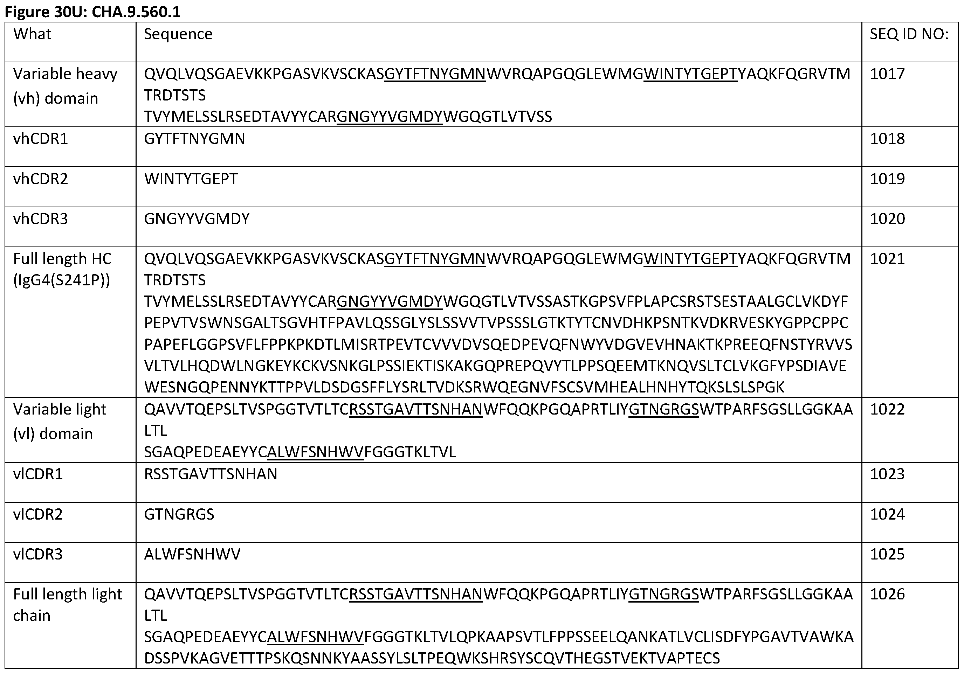

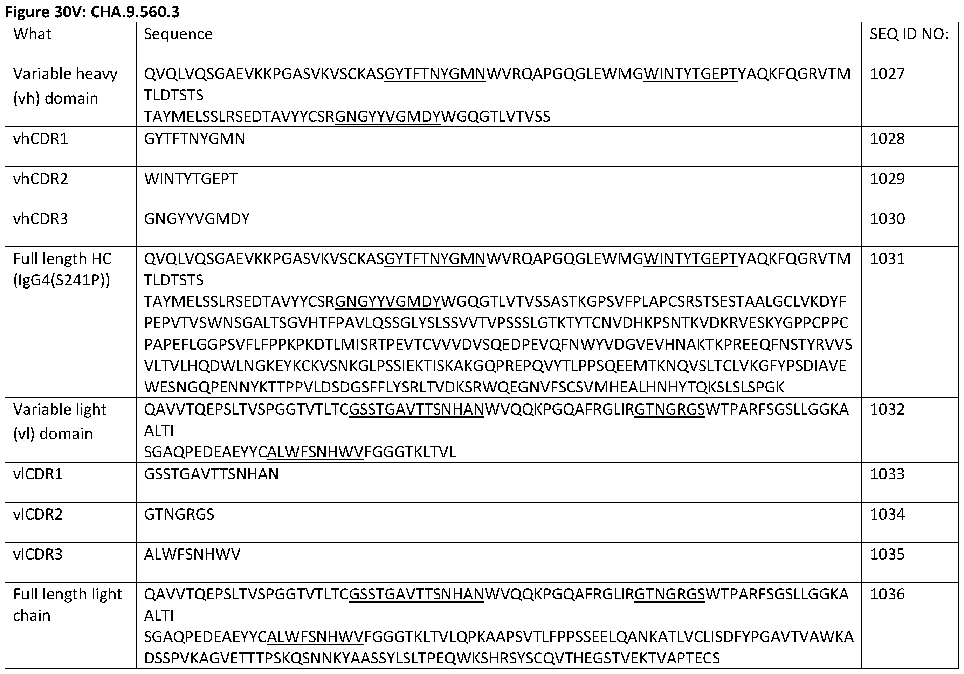

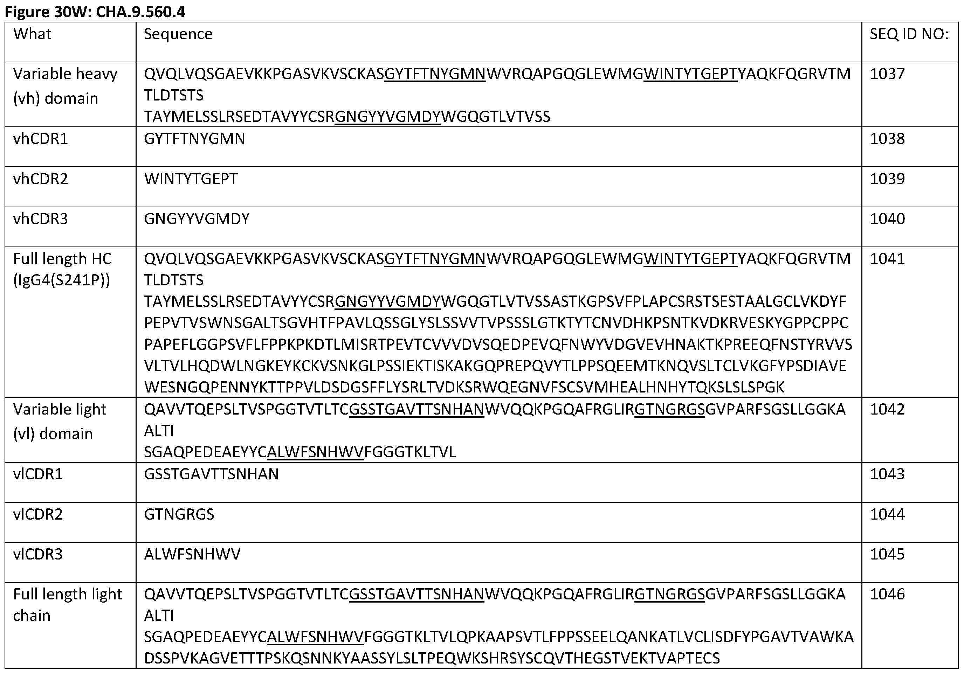

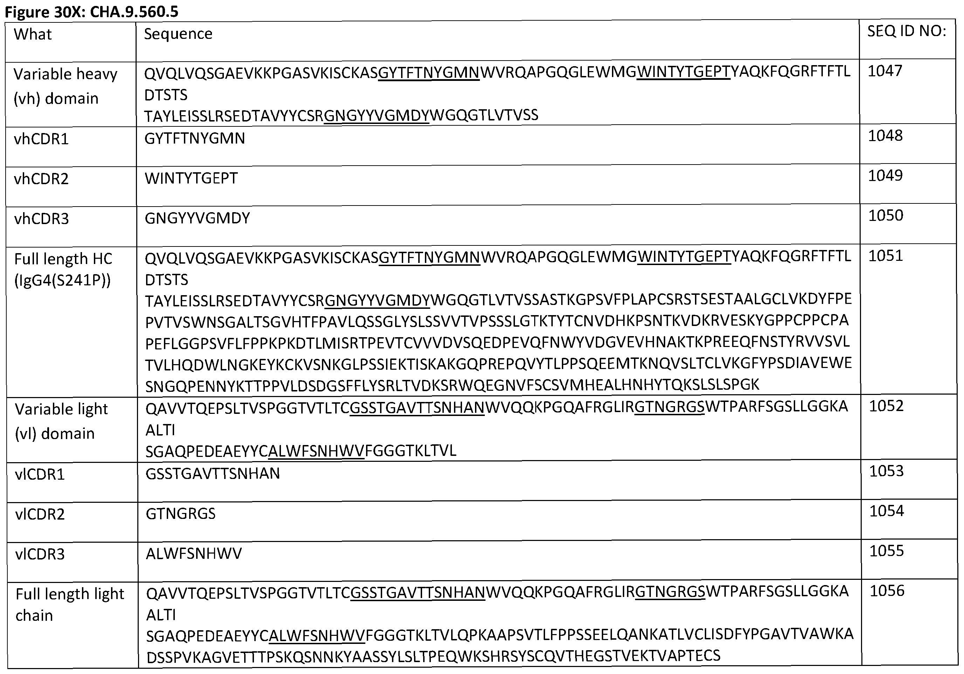

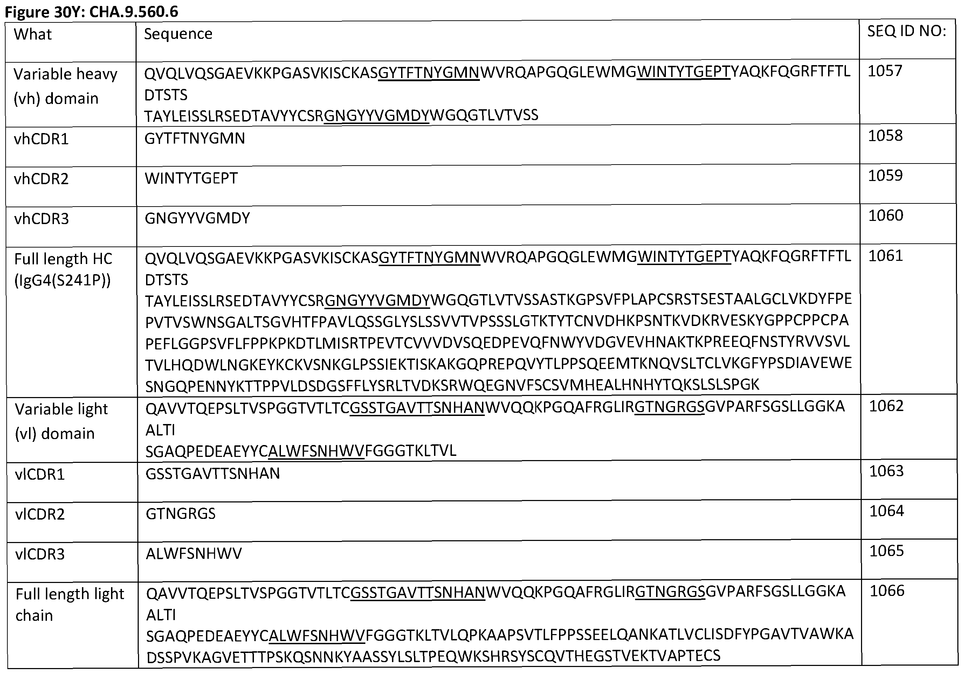

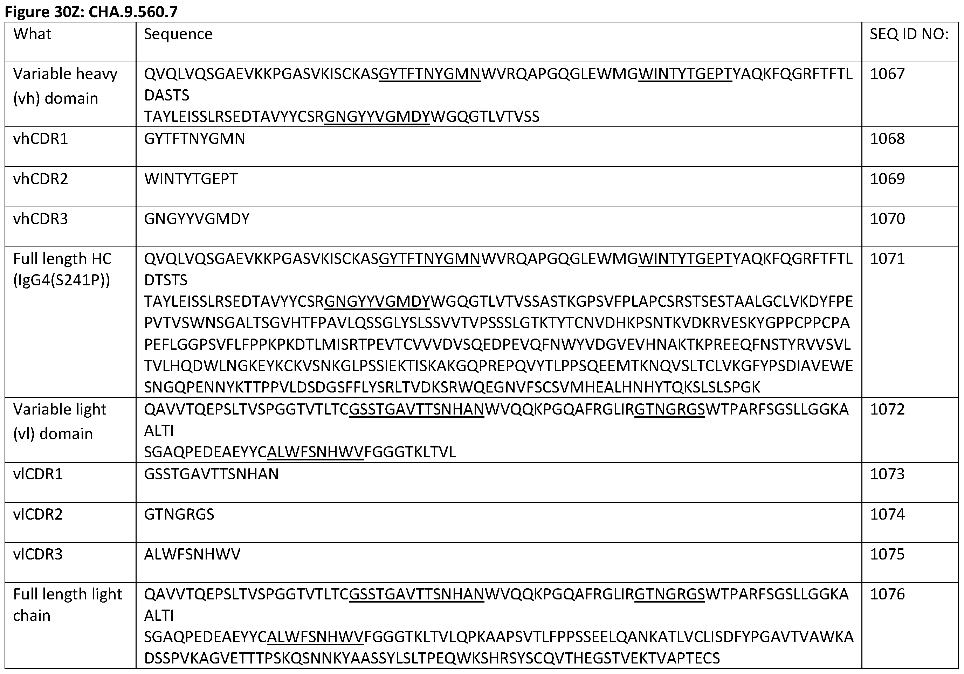

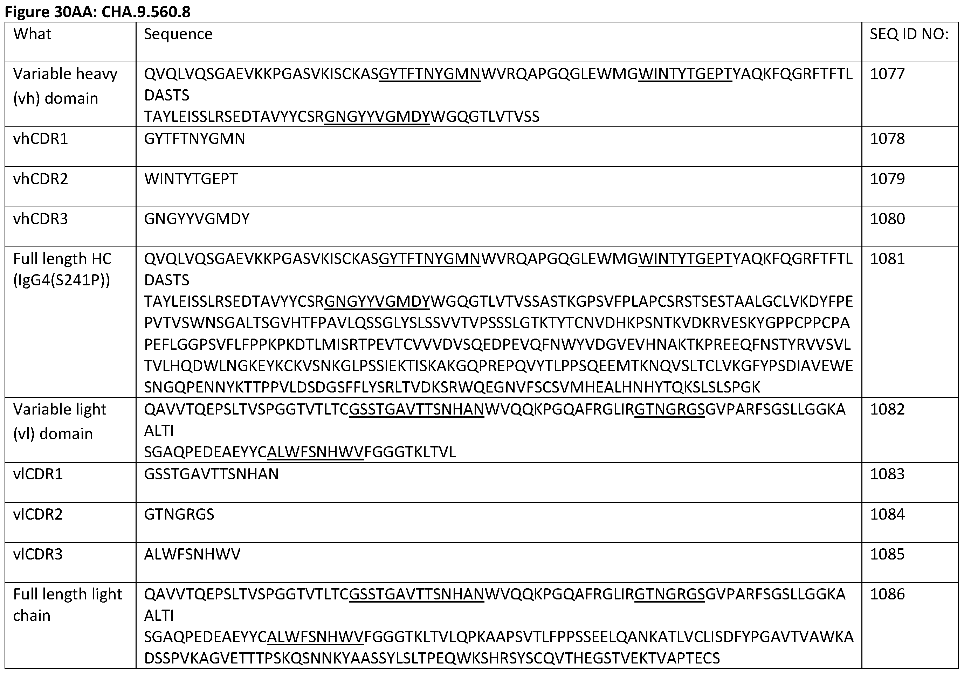

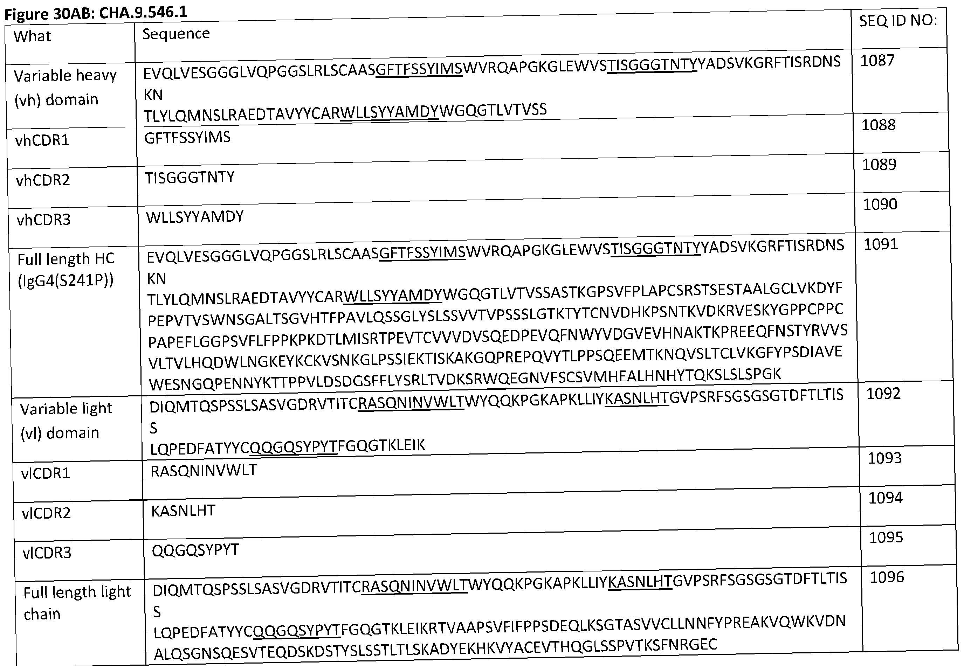

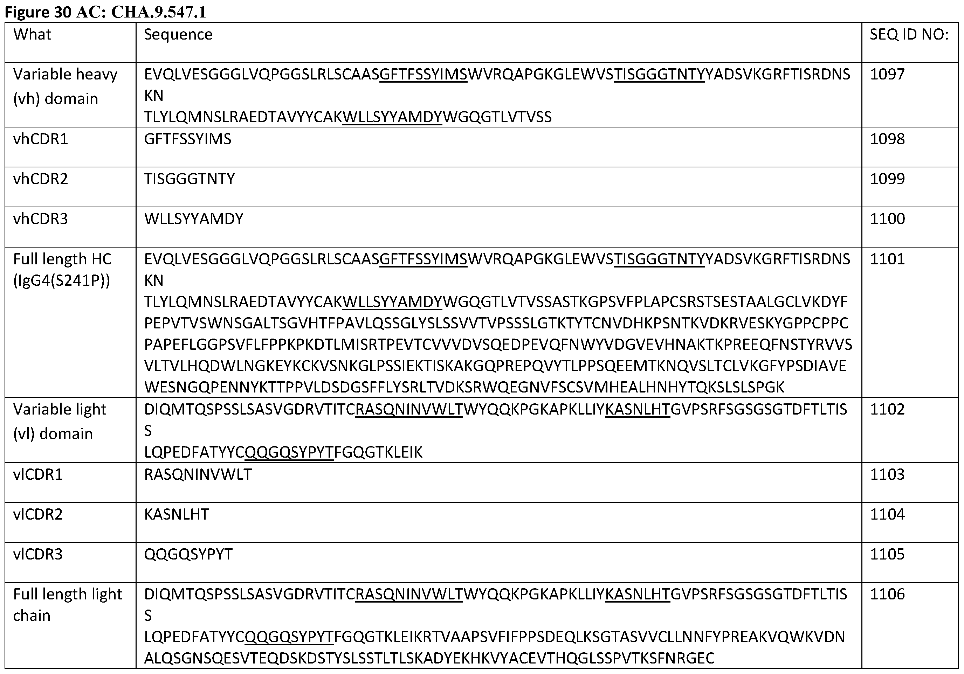

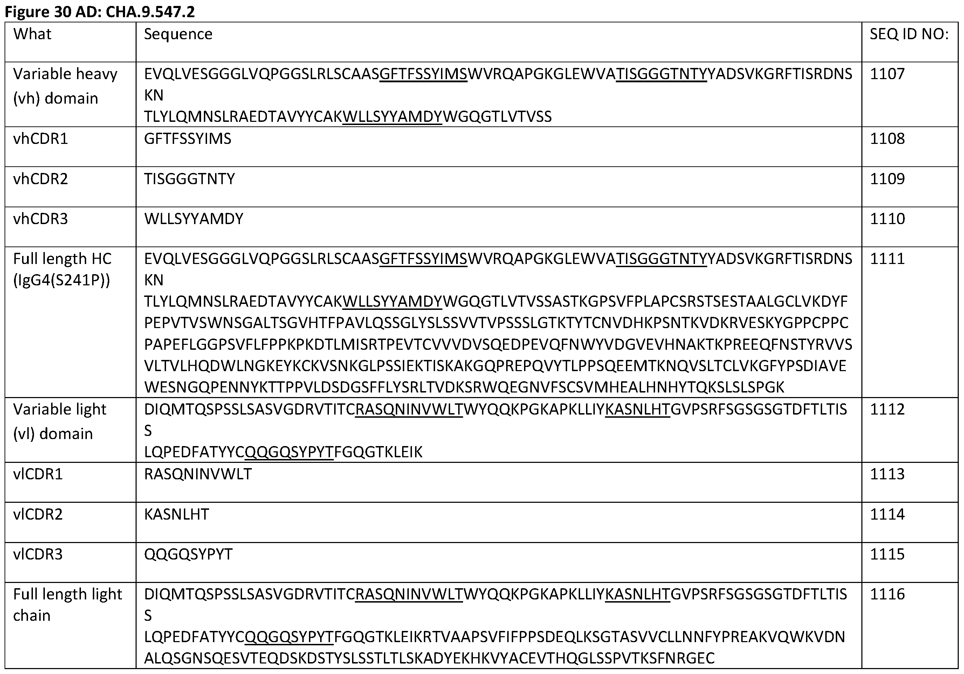

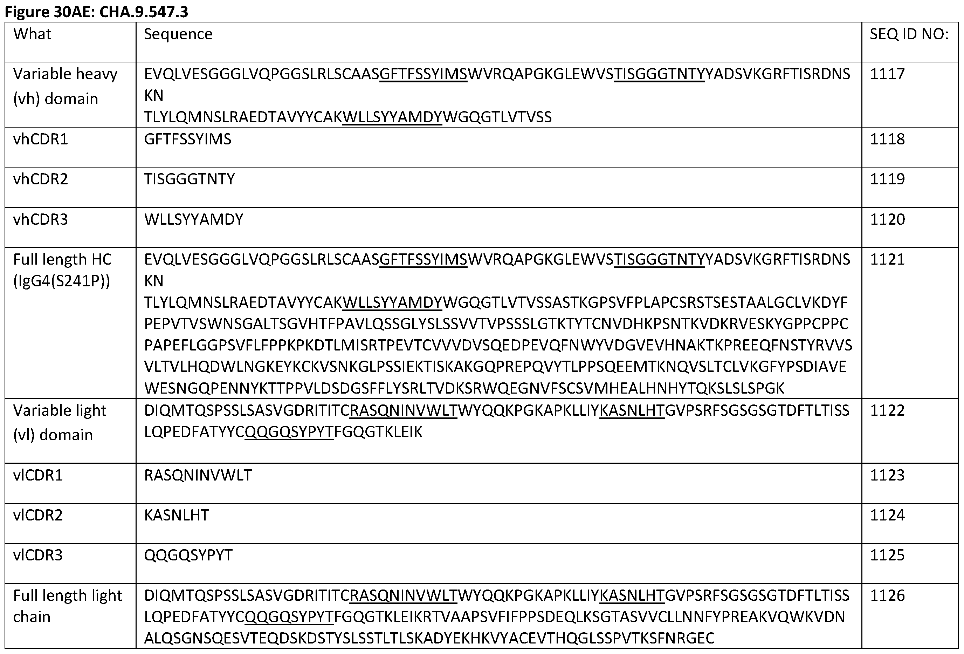

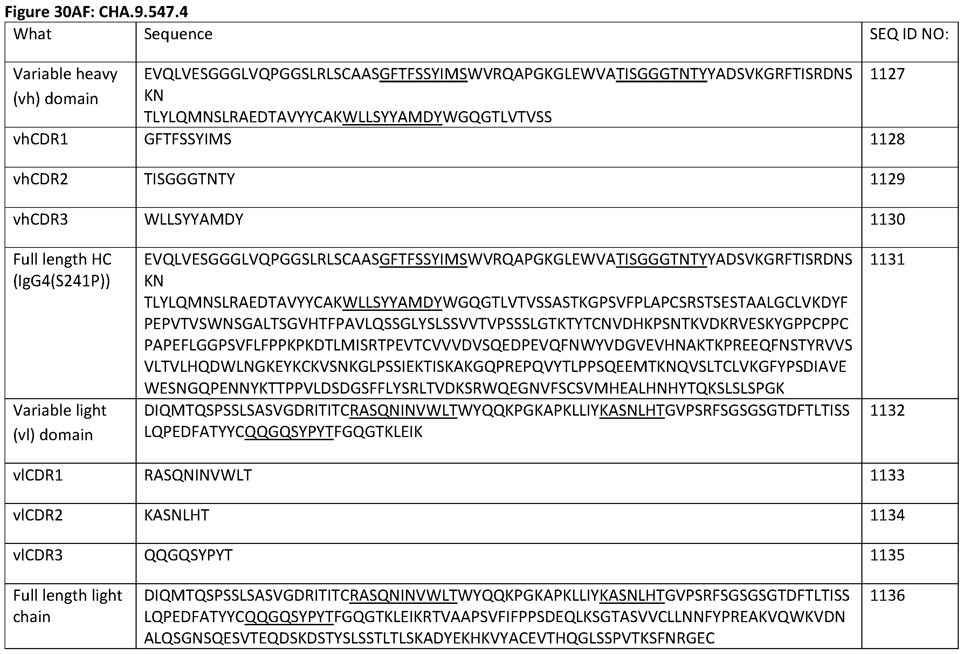

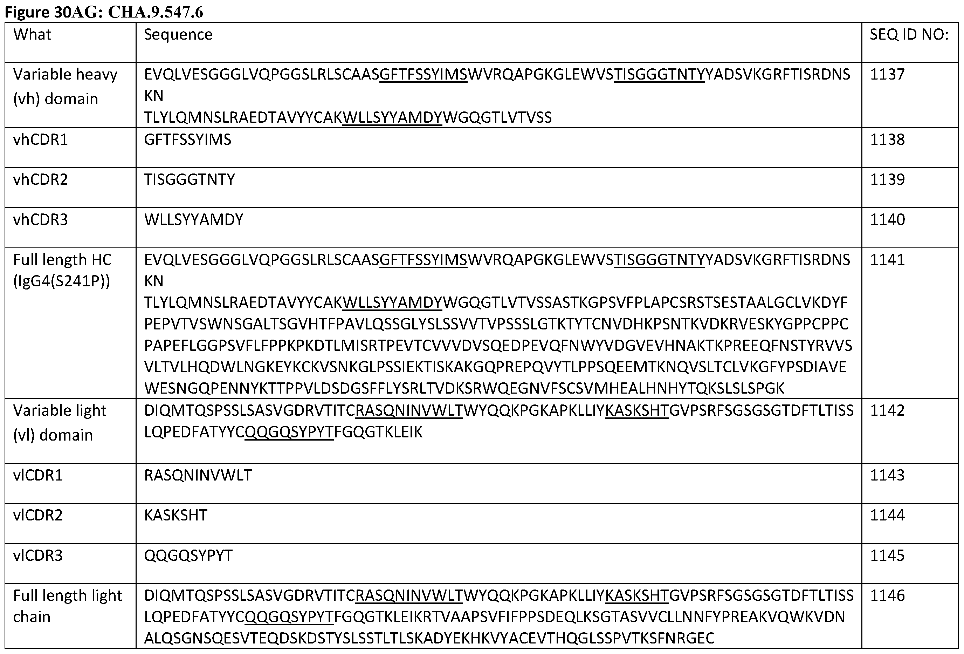

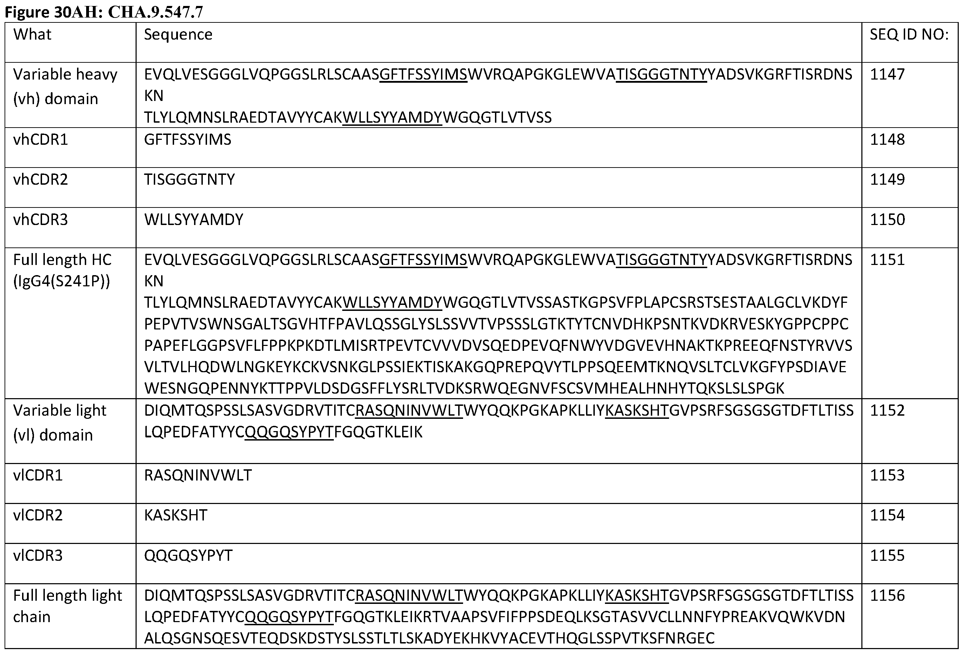

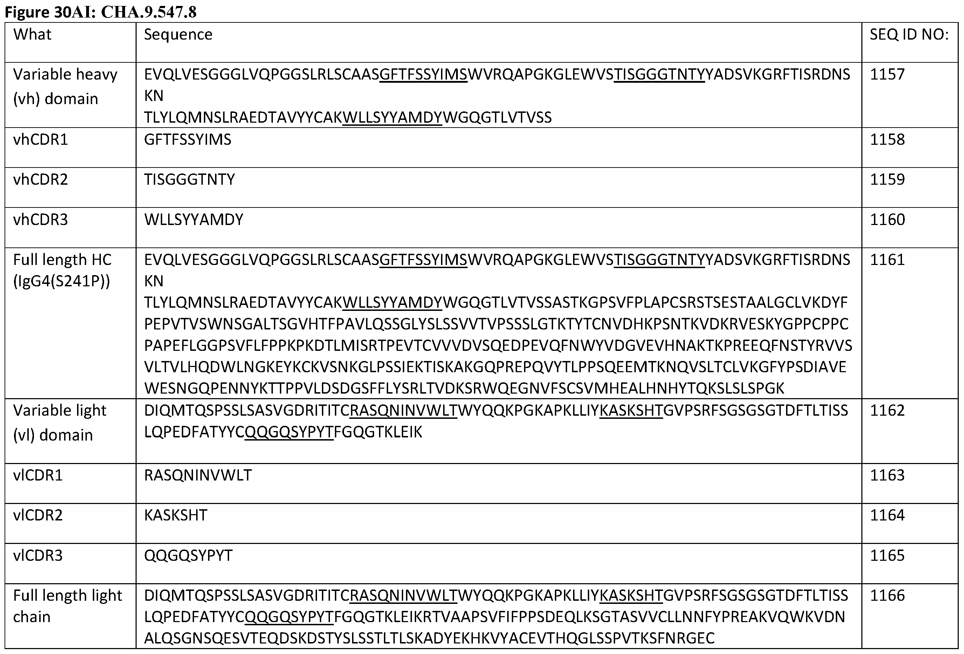

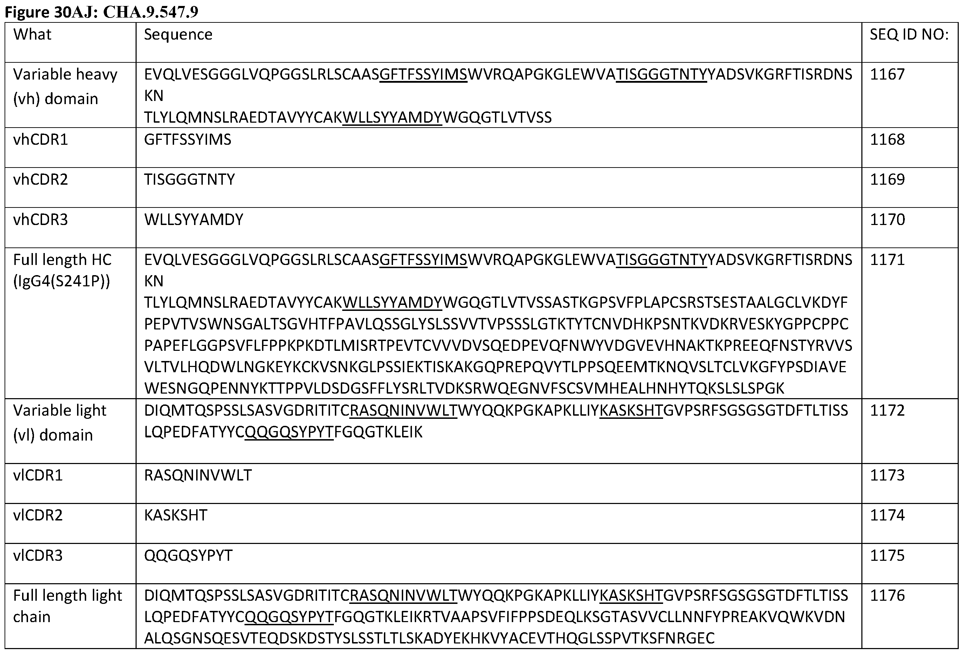

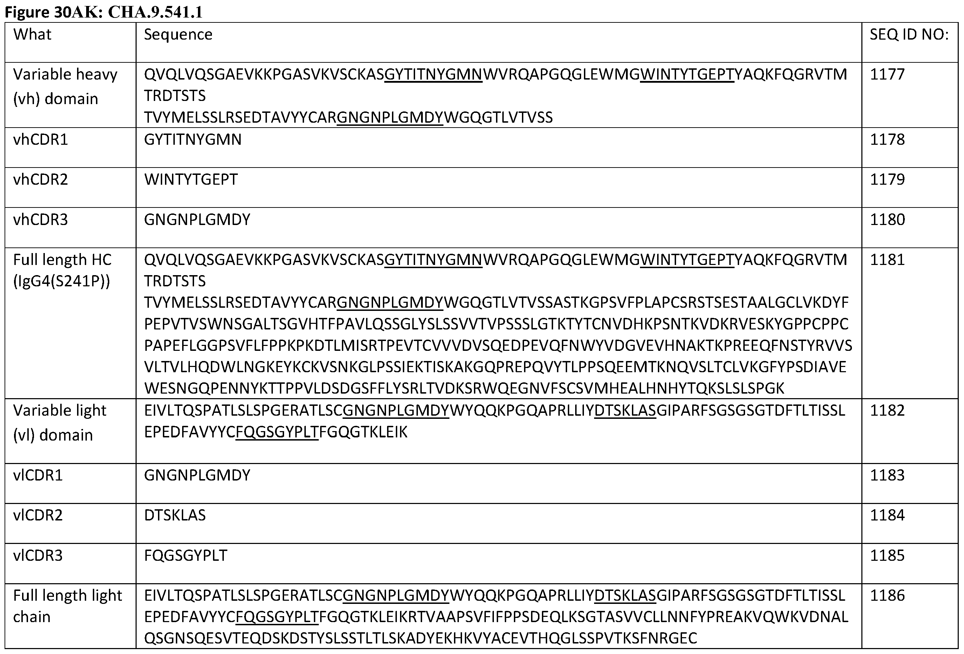

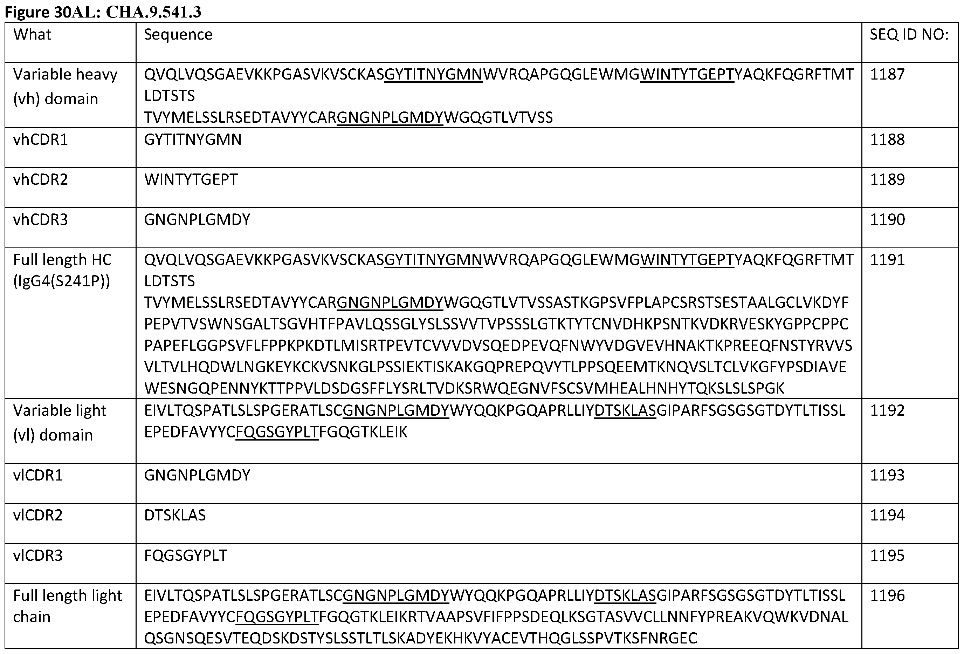

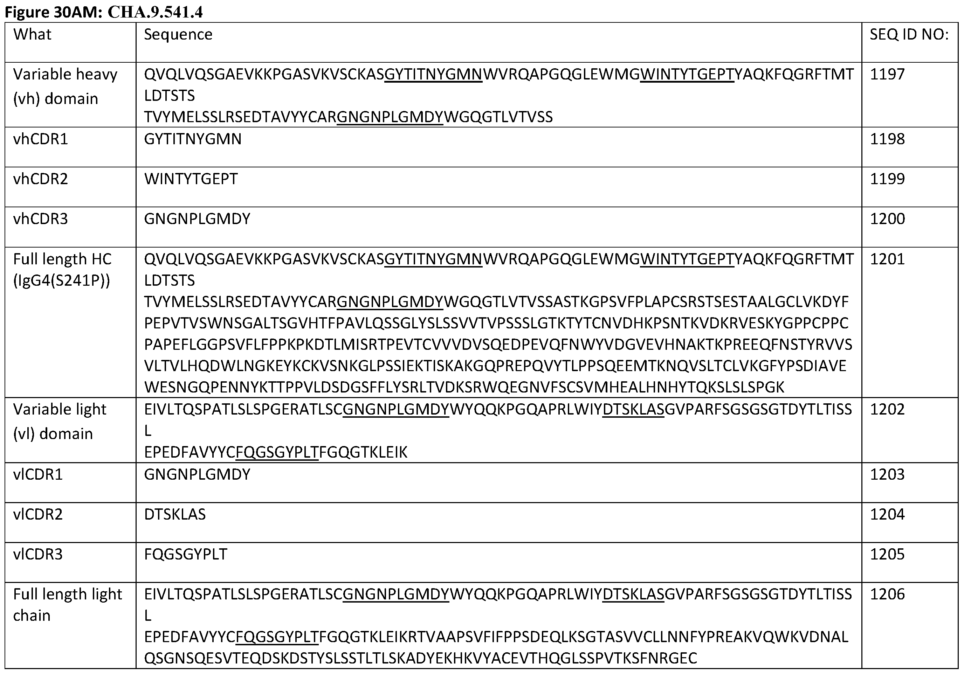

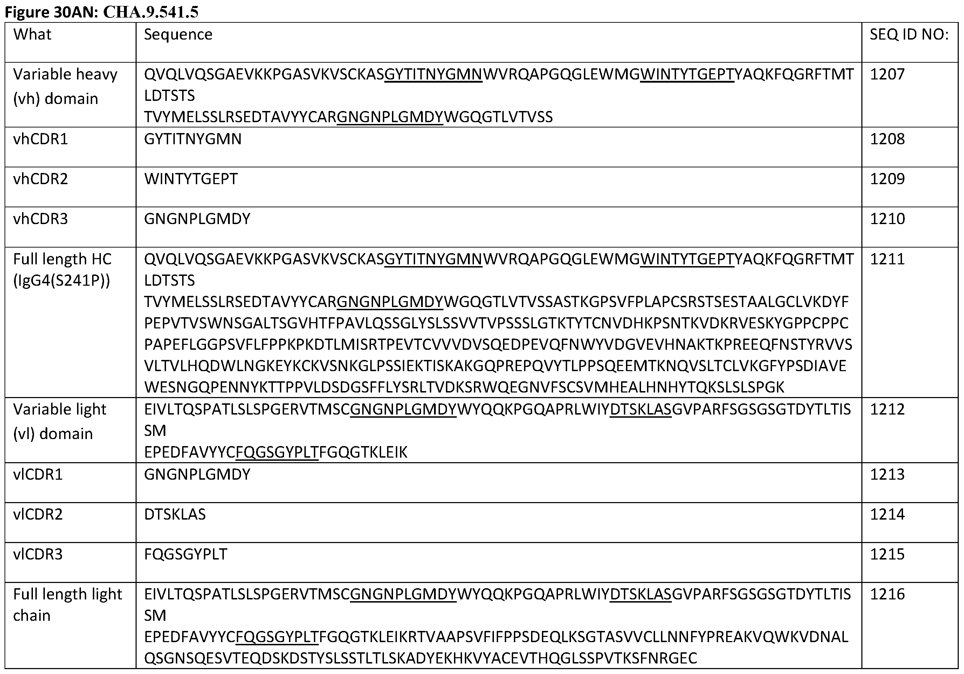

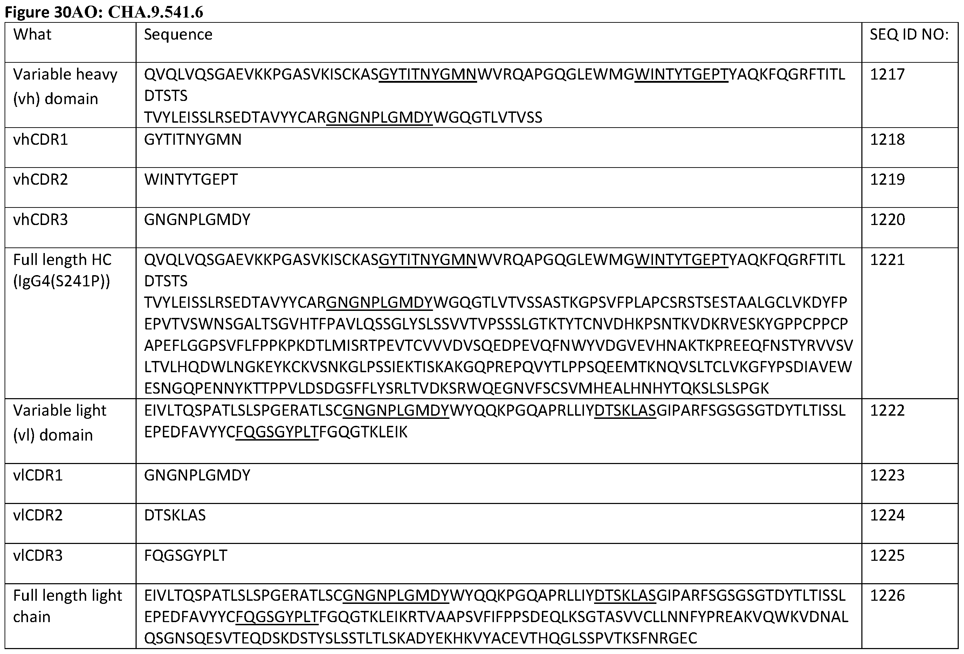

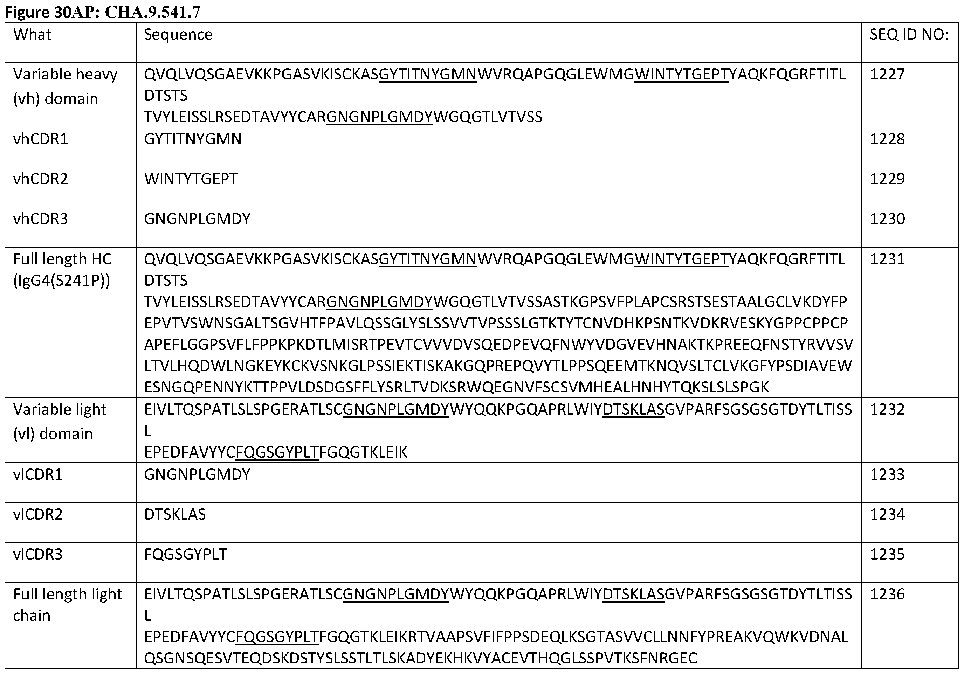

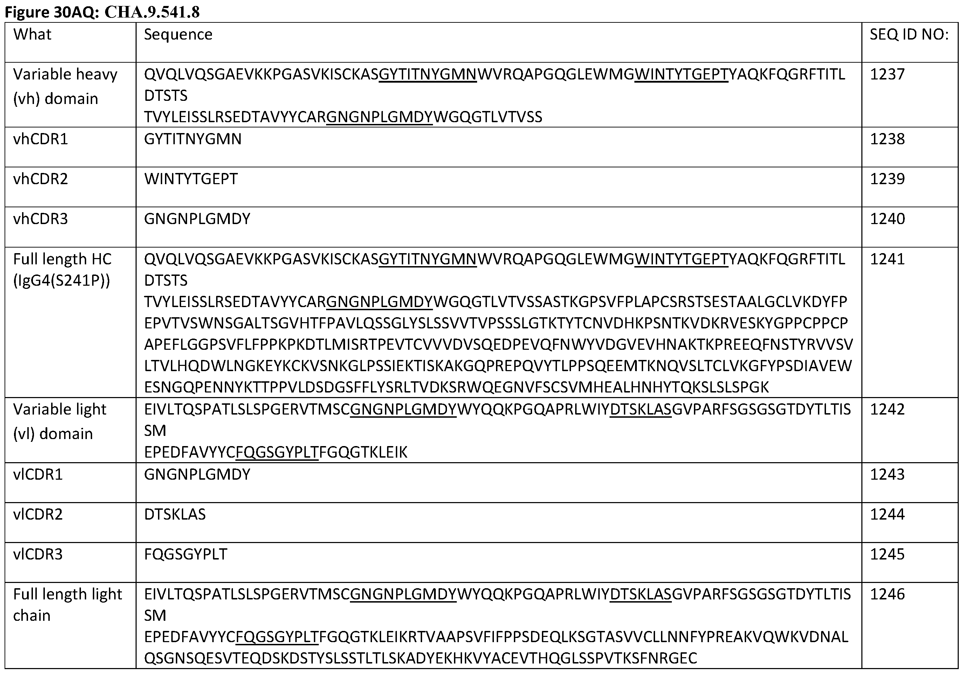

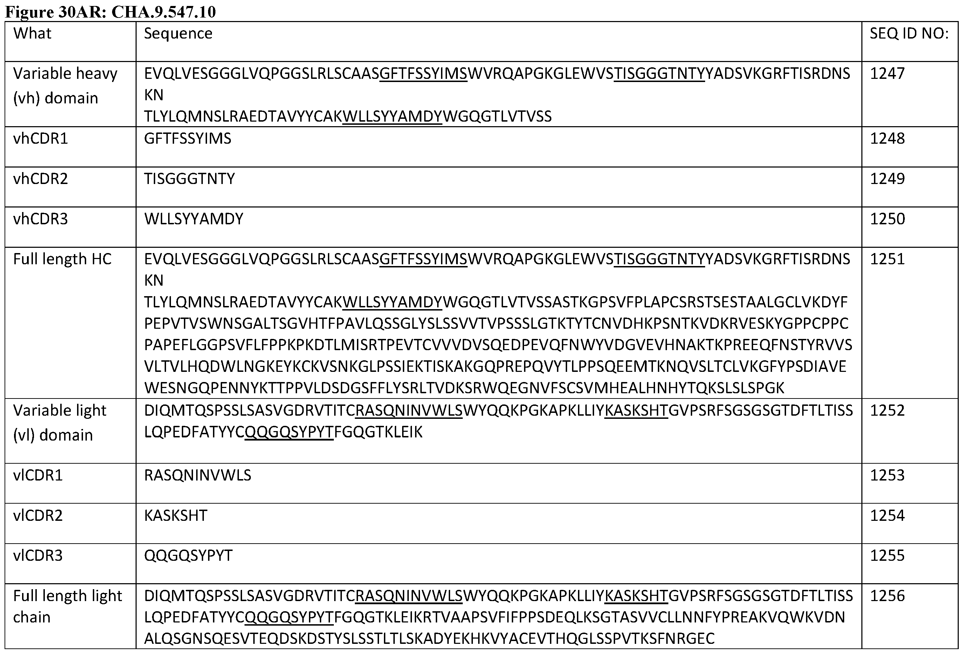

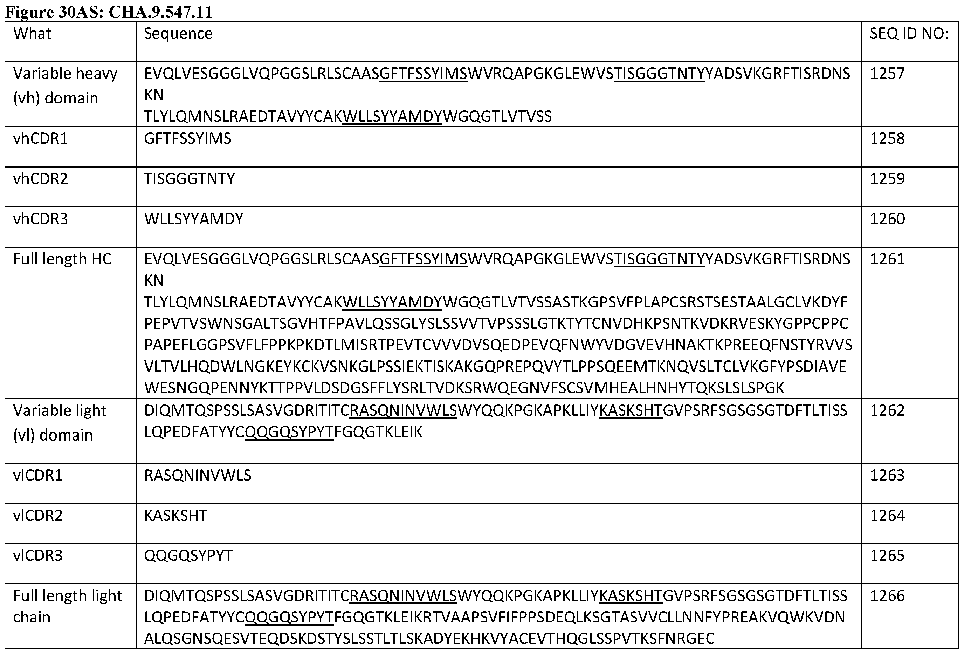

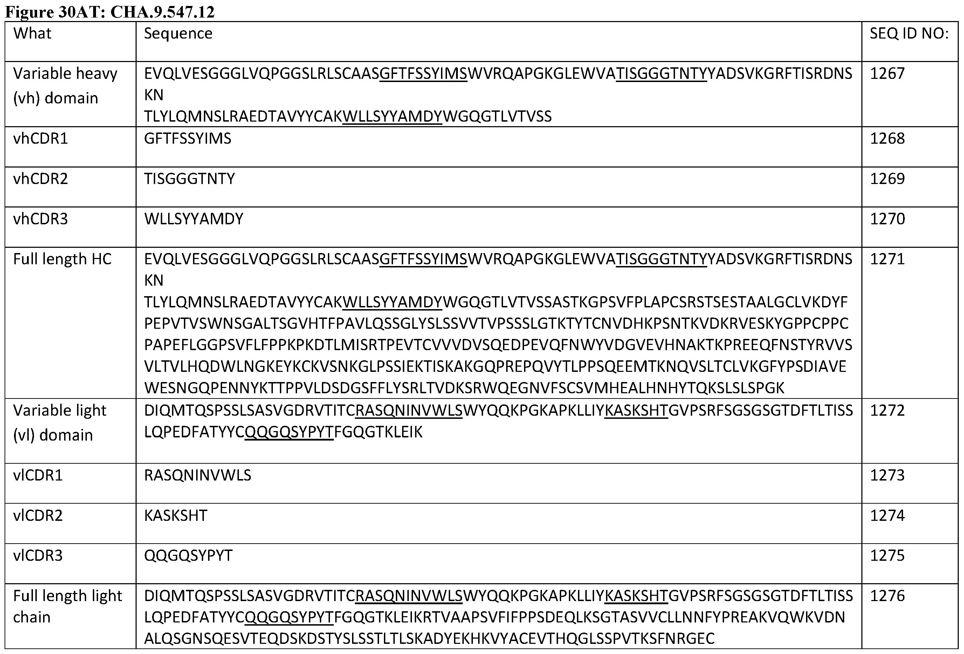

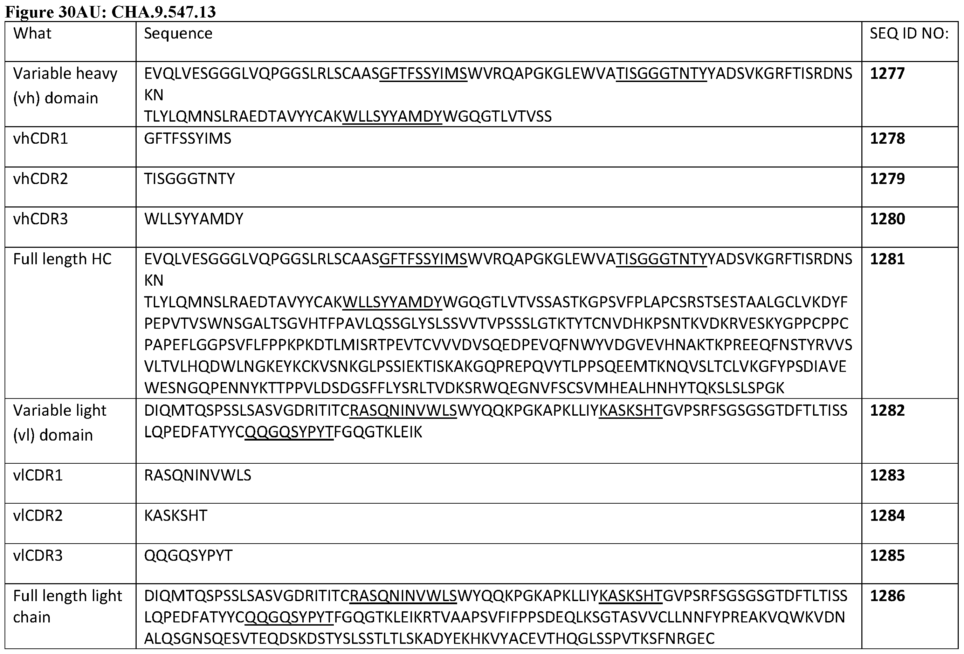

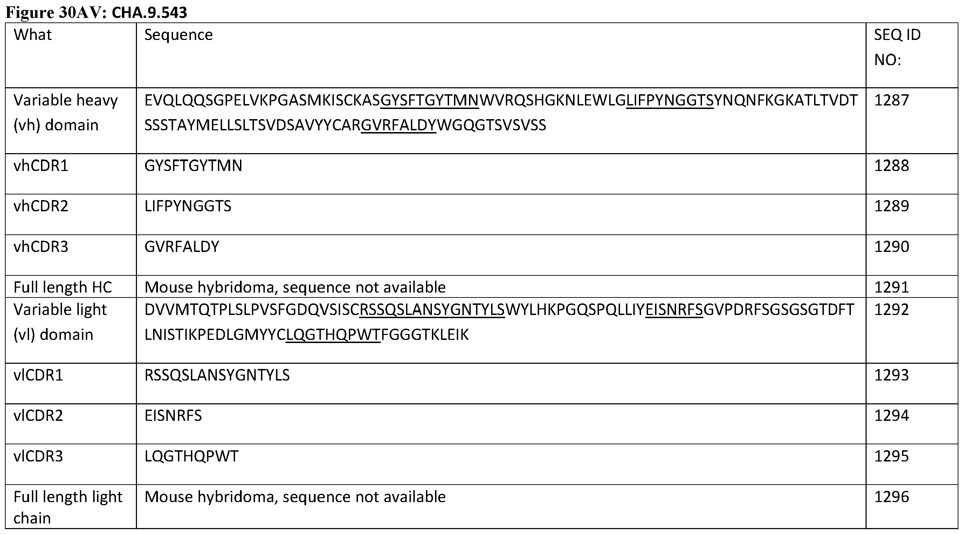

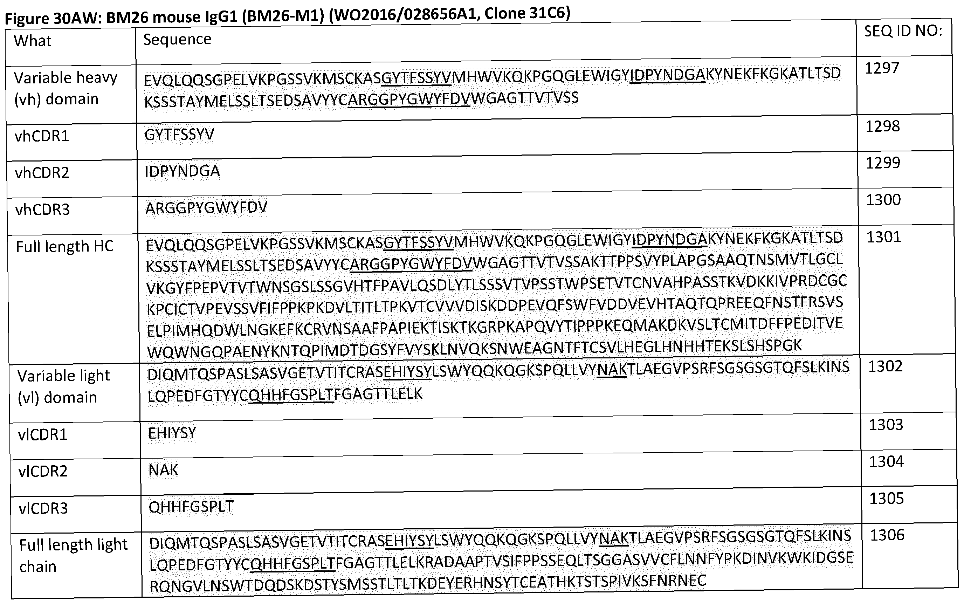

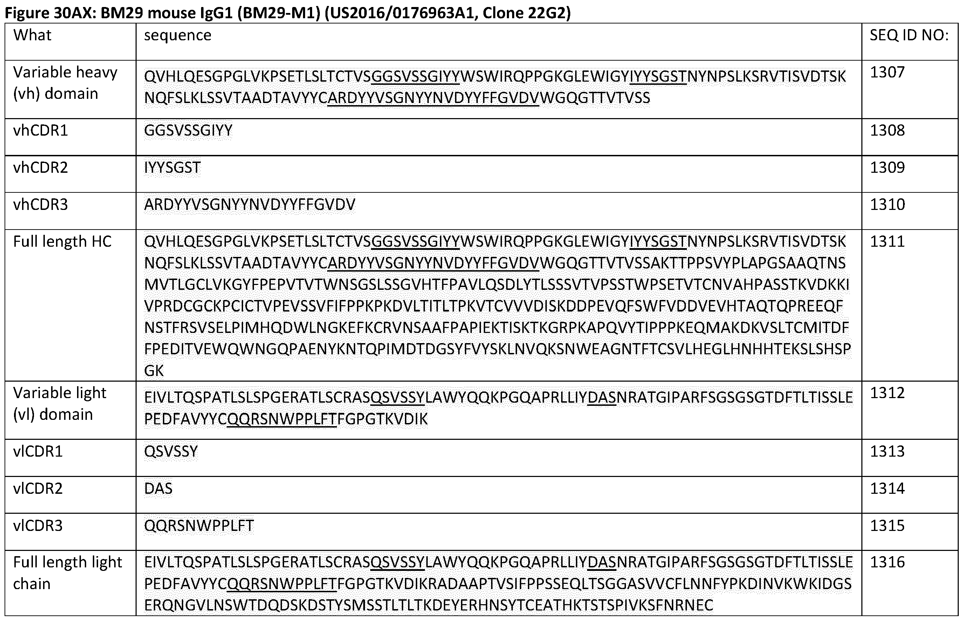

The TIGIT antibodies described herein are labeled as follows. The antibodies have reference numbers, for example "CPA.9.086". This represents the combination of the variable heavy and variable light chains, as depicted in Figure 30, for example, with the understanding that these antibodies include two heavy chains and two light chains. "CPA.9.086.VH" refers to the variable heavy portion of CPA. 9. 086, while "CPA. 9. 086.VL" is the variable light chain. "CPA. 9. 086.vhCDR1", "CPA. 9. 086.vhCDR2", "CPA. 9. 086.vhCDR3", "CPA. 9. 086.vlCDR1", "CPA. 9. 086.vlCDR2", and "CPA. 9. 086.vlCDR3", refers to the CDRs are indicated. "CPA. 9. 086.HC" refers to the entire heavy chain (e.g. variable and constant domain) of this molecule, and "CPA. 9. 086.LC" refers to the entire light chain (e.g. variable and constant domain) of the same molecule. In general, the human kappa light chain is used for the constant domain of each phage (or humanized hybridoma) antibody herein, although in some embodiments the lambda light constant domain is used. "CPA. 9. 086.H1" refers to a full length antibody comprising the variable heavy and light domains, including the constant domain of **H**uman IgG**1** (hence, the H1; IgG1, IgG2, IgG3 and IgG4 sequences are shown in Figure 7). Accordingly, "CPA. 9. 086.H2" would be the CPA. 9. 086 variable domains linked to a **H**uman IgG**2**. "CPA. 9. 086.H3" would be the CPA. 9. 086 variable domains linked to a **H**uman IgG**3**, and "CPA. 9. 086.H4" would be the CPA. 9. 086 variable domains linked to a **H**uman IgG**4**. Note that in some cases, the human IgGs may have additional mutations, such are described below, and this can be annotated. For example, in many embodiments, there may be a S241P mutation in the human IgG4, and this can be annotated as "CPA.9.086.H4(S241P)" for example. The human IgG4 sequence with this S241P hinge variant is shown in Figure 7. Other potential variants are IgG 1 (N297 A), (or other variants that ablate glycosylation at this site and thus many of the effector functions associated with FcγRIIIa binding), and IgG1(D265A), which reduces binding to FcyR receptors.

The invention further provides variable heavy and light domains as well as full length heavy and light chains.

In some embodiments, the invention provides scFvs that bind to TIGIT comprising a variable heavy domain and a variable light domain linked by an scFv linker as outlined above. The VL and VH domains can be in either orientation, e.g., from N- to C-terminus "VH-linker-VL" or "VL-linker-VH". These are named by their component parts; for example, "scFv-CPA. 9.086 VH-linker-VL" or "scFv-CPA.9.086.VL-linker-VH." Thus, "scFv-CPA.9.086" can be in either orientation.

In many embodiments, the antibodies of the invention are human (derived from phage) and block binding of TIGIT and PVR. As shown in Figure 30, the CPA antibodies that both bind and block the receptor-ligand interaction are as below, with their components outlined as well (as discussed in the "Sequence" section, the sequences of all but the scFv constructs are in the sequence listing):
CPA.9.018, CPA.9.018.VH, CPA.9.018.VL, CPA.9.018.HC, CPA.9.018.LC, CPA.9.018.H1, CPA.9.018.H2, CPA.9.018.H3, CPA.9.018.H4; CPA.9.018.H4(S241P); CPA.9.018.vhCDR1, CPA.9.018.vhCDR2, CPA.9.018.vhCDR3, CPA.9.018.vlCDR1, CPA.9.018.vlCDR2, CPA.9.018.vlCDR3 and scFv-CPA.9.018;
CPA.9.027, CPA.9.027.VH, CPA.9.027.VL, CPA.9.027.HC, CPA.9.027.LC, CPA.9.027.H1, CPA.9.027.H2, CPA.9.027.H3, CPA.9.027.H4; CPA.9.018.H4(S241P); CPA.9.027.vhCDR1, CPA.9.027.vhCDR2, CPA.9.027.vhCDR3, CPA.9.027.vlCDR1, CPA.9.027.vlCDR2, CPA.9.027.vlCDR3 and scFv-CPA.9.027;
CPA.9.049, CPA.9.049.VH, CPA.9.049.VL, CPA.9.049.HC, CPA.9.049.LC, CPA.9.049.H1, CPA.9.049.H2, CPA.9.049.H3; CPA.9.049.H4; CPA.9.049.H4(S241P); CPA.9.049.vhCDR1, CPA.9.049.vhCDR2, CPA.9.049.vhCDR3, CPA.9.049.vlCDR1, CPA.9.049.vlCDR2, CPA.9.049.vlCDR3 and scFv-CPA.9.049;
CPA.9.057, CPA.9.057.VH, CPA.9.057.VL, CPA.9.057.HC, CPA.9.057.LC, CPA.9.057.H1, CPA.9.057.H2, CPA.9.057.H3; CPA.9.057.H4; CPA.9.057.H4(S241P); CPA.9.057.vhCDR1, CPA.9.057.vhCDR2, CPA.9.057.vhCDR3, CPA.9.057.vlCDR1, CPA.9.057.vlCDR2, CPA.9.057.vlCDR3 and scFv-CPA.9.057;
CPA.9.059, CPA.9.059.VH, CPA.9.059.VL, CPA.9.059.HC, CPA.9.059.LC, CPA.9.059.H1, CPA.9.059.H2, CPA.9.059.H3; CPA.9.059.H4; CPA.9.059.H4(S241P); CPA.9.059.vhCDR1, CPA.9.059.vhCDR2, CPA.9.059.vhCDR3, CPA.9.059.vlCDR1, CPA.9.059.vlCDR2, CPA.9.059.vlCDR3 and scFv-CPA.9.059;
CPA.9.083, CPA.9.083.VH, CPA.9.083.VL, CPA.9.083.HC, CPA.9.083.LC, CPA.9.083.H1, CPA.9.083.H2, CPA.9.083.H3; CPA.9.083.H4; CPA.9.083.H4(S241P); CPA.9.083.vhCDR1, CPA.9.083.vhCDR2, CPA.9.083.vhCDR3, CPA.9.083.vlCDR1, CPA.9.083.vlCDR2, CPA.9.083.vlCDR3 and scFv-CPA.9.083;
CPA.9.086, CPA.9.086.VH, CPA.9.086.VL, CPA.9.086.HC, CPA.9.086.LC, CPA.9.086.H1, CPA.9.086.H2, CPA.9.086.H3; CPA.9.086.H4; CPA.9.086.H4(S241P); CPA.9.086.vhCDR1, CPA.9.086.vhCDR2, CPA.9.086.vhCDR3, CPA.9.086.vlCDR1, CPA.9.086.vlCDR2, CPA.9.086.vlCDR3 and scFv-CPA.9.086;
CPA.9.089, CPA.9.089.VH, CPA.9.089.VL, CPA.9.089.HC, CPA.9.089.LC, CPA.9.089.H1, CPA.9.089.H2, CPA.9.089.H3; CPA.9.089.H4; CPA.9.089.H4(S241P); CPA.9.089.vhCDR1, CPA.9.089.vhCDR2, CPA.9.089.vhCDR3, CPA.9.089.vlCDR1, CPA.9.089.vlCDR2, CPA.9.089.vlCDR3 and scFv-CPA.9.089;
CPA.9.093, CPA.9.093.VH, CPA.9.093.VL, CPA.9.093.HC, CPA.9.093.LC, CPA.9.093.H1, CPA.9.093.H2, CPA.9.093.H3; CPA.9.093.H4; CPA.9.093.H4(S241P); CPA.9.093.vhCDR1, CPA.9.093.vhCDR2, CPA.9.093.vhCDR3, CPA.9.093.vlCDR1, CPA.9.093.vlCDR2, CPA.9.093.vlCDR3 and scFv-CPA.9.093;
CPA.9.101, CPA.9.101.VH, CPA.9.101.VL, CPA.9.101.HC, CPA.9.101.LC, CPA.9.101.H1, CPA.9.101.H2, CPA.9.101.H3; CPA.9.101.H4; CPA.9.101.H4(S241P); CPA.9.101.vhCDR1, CPA.9.101.vhCDR2, CPA.9.101.vhCDR3, CPA.9.101.vlCDR1, CPA.9.101.vlCDR2, CPA.9.101.vlCDR3 and scFv-CPA.9.101; and
CPA.9.103, CPA.9.103.VH, CPA.9.103.VL, CPA.9.103.HC, CPA.9.103.LC, CPA.9.103.H1, CPA.9.103.H2, CPA.9.103.H3; CPA.9.103.H4; CPA.9.103.H4(S241P); CPA.9.103.vhCDR1, CPA.9.103.vhCDR2, CPA.9.103.vhCDR3, CPA.9.103.vlCDR1, CPA.9.103.vlCDR2, CPA.9.103.vlCDR3 and scFv-CPA.9.103.

Furthermore, the present invention provides a number of CHA antibodies, which are murine antibodies generated from hybridomas. As is well known the art, the six CDRs are useful when put into either human framework variable heavy and variable light regions or when the variable heavy and light domains are humanized.

Accordingly, the present invention provides antibodies, usually full length or scFv domains, that comprise the following sets of CDRs, the sequences of which are shown in Figure 30 and/or the sequence listing:
CHA.9.536.1, CHA.9.536.1.VH, CHA.9.536.1.VL, CHA.9.536.1.HC, CHA.9.536.1.LC, CHA.9.536.1.H1, CHA.9.536.1.H2, CHA.9.536.1.H3; CHA.9.536.1.H4, CHA.9.536.1.H4(S241P), CHA.9.536.1.vhCDR1, CHA.9.536.1.vhCDR2, CHA.9.536.1.vhCDR3, CHA.9.536.1.vlCDR1, CHA.9.536.1.vlCDR2 and CHA.9.536.1.vhCDR3;
CHA.9.536.3, CHA.9.536.3.VH, CHA.9.536.3.VL, CHA.9.536.3.HC, CHA.9.536.3.LC, CHA.9.536.3.H1, CHA.9.536.3.H2, CHA.9.536.3.H3; CHA.9.536.3.H4, CHA.9.536.3.H4(S241P); CHA.9.536.3.vhCDR1, CHA.9.536.3.vhCDR2, CHA.9.536.3.vhCDR3, CHA.9.536.3.vlCDR1, CHA.9.536.3.vlCDR2 and CHA.9.536.3.vhCDR3;
CHA.9.536.4, CHA.9.536.4.VH, CHA.9.536.4.VL, CHA.9.536.4.HC, CHA.9.536.4.LC, CHA.9.536.4.H1, CHA.9.536.4.H2, CHA.9.536.4.H3; CHA.9.536.4.H4, CHA.9.536.4.H4(S241P), CHA.9.536.4.vhCDR1, CHA.9.536.4.vhCDR2, CHA.9.536.4.vhCDR3, CHA.9.536.4.vlCDR1, CHA.9.536.4.vlCDR2 and CHA.9.536.4.vhCDR3;
CHA.9.536.5, CHA.9.536.5.VH, CHA.9.536.5.VL, CHA.9.536.5.HC, CHA.9.536.5.LC, CHA.9.536.5.H1, CHA.9.536.5.H2, CHA.9.536.5.H3; CHA.9.536.5.H4, CHA.9.536.5.H4(S241P), CHA.9.536.5.vhCDR1, CHA.9.536.5.vhCDR2, CHA.9.536.5.vhCDR3, CHA.9.536.5.vlCDR1, CHA.9.536.5.vlCDR2 and CHA.9.536.5.vhCDR3;
CHA.9.536.6, CHA.9.536.6.VH, CHA.9.536.6.VL, CHA.9.536.6.HC, CHA.9.536.6.LC, CHA.9.536.6.H1, CHA.9.536.6.H2, CHA.9.536.6.H3; CHA.9.536.6.H4, CHA.9.536.6.vhCDR1, CHA.9.536.6.vhCDR2, CHA.9.536.6.vhCDR3, CHA.9.536.6.vlCDR1, CHA.9.536.6.vlCDR2 and CHA.9.536.6.vhCDR3;
CHA.9.536.7, CHA.9.536.7.VH, CHA.9.536.7.VL, CHA.9.536.7.HC, CHA.9.536.7.LC, CHA.9.536.7.H1, CHA.9.536.7.H2, CHA.9.536.7.H3; CHA.9.536.7.H4, CHA.9.536.5.H4(S241P); CHA.9.536.7.vhCDR1, CHA.9.536.7.vhCDR2, CHA.9.536.7.vhCDR3, CHA.9.536.7.vlCDR1, CHA.9.536.7.vlCDR2 and CHA.9.536.7.vhCDR3;
CHA.9.536.8, CHA.9.536.8.VH, CHA.9.536.8.VL, CHA.9.536.8.HC, CHA.9.536.8.LC, CHA.9.536.8.H1, CHA.9.536.8.H2, CHA.9.536.8.H3; CHA.9.536.8.H4, CHA.9.536.8.H4(S241P), CHA.9.536.8.vhCDR1, CHA.9.536.8.vhCDR2, CHA.9.536.8.vhCDR3, CHA.9.536.8.vlCDR1, CHA.9.536.8.vlCDR2 and CHA.9.536.8.vhCDR3;
CHA.9.560.1, CHA. 9.560.1VH, CHA. 9.560.1.VL, CHA. 9.560.1.HC, CHA. 9.560.1. LC, CHA. 9.560.1.H1, CHA. 9.560.1.H2, CHA. 9.560.1.H3; CHA. 9.560.1.H4, CHA. 9.560.1.H4(S241P), CHA. 9.560.1.vhCDR1, CHA. 9.560.1.vhCDR2, CHA. 9.560.1. vhCDR3, CHA. 9.560.1.vlCDR1, CHA. 9.560.1.vlCDR2 and CHA. 9.560.1. vhCDR3;
CHA.9.560.3, CHA. 9.560. 3VH, CHA. 9.560. 3.VL, CHA. 9.560. 3.HC, CHA. 9.560. 3.LC, CHA. 9.560. 3.H1, CHA. 9.560. 3.H2, CHA. 9.560. 3.H3; CHA.9.560.3.H4, CHA.9.560.3.H4(S241P); CHA. 9.560. 3.vhCDR1, CHA. 9.560. 3.vhCDR2, CHA. 9.560. 3.vhCDR3, CHA. 9.560. 3.vlCDR1, CHA. 9.560. 3.vlCDR2 and CHA. 9.560. 3.vhCDR3;
CHA.9.560.4, CHA. 9.560. 4VH, CHA. 9.560. 4.VL, CHA. 9.560. 4.HC, CHA. 9.560. 4.LC, CHA. 9.560. 4.H1, CHA. 9.560. 4.H2, CHA. 9.560. 4.H3; CHA.9.560.4.H4, CHA.9.560.4.H4(S241P), CHA. 9.560. 4.vhCDR1, CHA. 9.560. 4.vhCDR2, CHA. 9.560. 4.vhCDR3, CHA. 9.560. 4.vlCDR1, CHA. 9.560. 4.vlCDR2 and CHA. 9.560. 4.vhCDR3;
CHA.9.560.5, CHA. 9.560. 5VH, CHA. 9.560. 5.VL, CHA. 9.560. 5.HC, CHA. 9.560. 5.LC, CHA. 9.560. 5.H1, CHA. 9.560. 5.H2, CHA. 9.560. 5.H3; CHA. 9.560. 5.H4, CHA. 9.560. 5.vhCDR1, CHA. 9.560. 5.vhCDR2, CHA. 9.560. 5.vhCDR3, CHA. 9.560. 5.vlCDR1, CHA. 9.560. 5.vlCDR2 and CHA. 9.560. 5.vhCDR3;
CHA.9.560.6, CHA. 9.560. 6VH, CHA. 9.560. 6.VL, CHA. 9.560. 6.HC, CHA. 9.560. 6.LC, CHA. 9.560. 6.H1, CHA. 9.560. 6.H2, CHA. 9.560. 6.H3; CHA.9.560.6.H4, CHA.9.560.6.H4(S241P), CHA. 9.560. 6.vhCDR1, CHA. 9.560. 6.vhCDR2, CHA. 9.560. 6.vhCDR3, CHA. 9.560. 6.vlCDR1, CHA. 9.560. 6.vlCDR2 and CHA. 9.560. 6.vhCDR3;
CHA.9.560.7, CHA. 9.560. 7VH, CHA. 9.560. 7.VL, CHA. 9.560. 7.HC, CHA. 9.560. 7.LC, CHA. 9.560. 7.H1, CHA. 9.560. 7.H2, CHA. 9.560. 7.H3; CHA.9.560.7.H4; CHA.9.560.7.H4(S241P); CHA. 9.560. 7.vhCDR1, CHA. 9.560. 7.vhCDR2, CHA. 9.560. 7.vhCDR3, CHA. 9.560. 7.vlCDR1, CHA. 9.560. 7.vlCDR2 and CHA. 9.560. 7.vhCDR3;
CHA.9.560.8, CHA. 9.560. 8VH, CHA. 9.560. 8.VL, CHA. 9.560. 8.HC, CHA. 9.560. 8.LC, CHA. 9.560. 8.H1, CHA. 9.560. 8.H2, CHA. 9.560. 8.H3; CHA.9.560.8.H4, CHA.9.560.8.H4(S241P); CHA. 9.560. 8.vhCDR1, CHA. 9.560. 8.vhCDR2, CHA. 9.560. 8.vhCDR3, CHA. 9.560. 8.vlCDR1, CHA. 9.560. 8.vlCDR2 and CHA. 9.560. 8.vhCDR3;
CHA.9.546.1, CHA. 9. 546.1VH, CHA. 9. 546.1.VL, CHA. 9. 546.1.HC, CHA. 9. 546.1.LC, CHA. 9. 546.1.H1, CHA. 9. 546.1.H2, CHA. 9. 546.1.H3; CHA.9.546.1.H4, CHA.9.546.1.H4(S241P), CHA. 9. 546.1.vhCDR1, CHA. 9. 546.1.vhCDR2, CHA. 9. 546.1.vhCDR3, CHA. 9. 546.1.vlCDR1, CHA. 9. 546.1.vlCDR2 and CHA. 9. 546.1.vhCDR3;
CHA.9.547.1, CHA. 9. 547.1VH, CHA. 9. 547.1.VL, CHA. 9. 547.1.HC, CHA. 9. 547.1.LC, CHA. 9. 547.1.H1, CHA. 9. 547.1.H2, CHA. 9. 547.1.H3; CHA.9.547.1.H4, CHA.9.547.1.H4(S241P), CHA. 9. 547.1.vhCDR1, CHA. 9. 547.1.vhCDR2, CHA. 9. 547.1.vhCDR3, CHA. 9. 547.1.vlCDR1, CHA. 9. 547.1.vlCDR2 and CHA. 9. 547.1.vhCDR3;
CHA.9.547.2, CHA. 9. 547. 2VH, CHA. 9. 547. 2.VL, CHA. 9. 547. 2.HC, CHA. 9. 547. 2.LC, CHA. 9. 547. 2.H1, CHA. 9. 547. 2.H2, CHA. 9. 547. 2.H3; CHA.9.547.2.H4, CHA.9.547.2.H4(S241P), CHA. 9. 547. 2.vhCDR1, CHA. 9. 547. 2.vhCDR2, CHA. 9. 547. 2.vhCDR3, CHA. 9. 547. 2.vlCDR1, CHA. 9. 547. 2.vlCDR2 and CHA. 9. 547. 2.vhCDR3;
CHA.9.547.3, CHA. 9. 547. 3VH, CHA. 9. 547. 3.VL, CHA. 9. 547. 3.HC, CHA. 9. 547. 3.LC, CHA. 9. 547. 3.H1, CHA. 9. 547. 3.H2, CHA. 9. 547. 3.H3; CHA.9.547.3.H4, CHA.9.547.3.H4(S241P), CHA. 9. 547. 3.vhCDR1, CHA. 9.547. 3.vhCDR2, CHA. 9. 547. 3.vhCDR3, CHA. 9. 547. 3.vlCDR1, CHA. 9. 547. 3.vlCDR2 and CHA. 9. 547. 3.vhCDR3;
CHA.9.547.4, CHA. 9. 547. 4VH, CHA. 9. 547. 4.VL, CHA. 9. 547. 4.HC, CHA. 9.547. 4.LC, CHA. 9. 547. 4.H1, CHA. 9. 547. 4.H2, CHA. 9. 547. 4.H3; CHA.9.547.4.H4, CHA.9.547.4.H4(S241P), CHA. 9. 547. 4.vhCDR1, CHA. 9. 547. 4.vhCDR2, CHA. 9. 547. 4.vhCDR3, CHA. 9. 547. 4.vlCDR1, CHA. 9. 547. 4.vlCDR2 and CHA. 9. 547. 4.vhCDR3;
CHA.9.547.6, CHA. 9. 547. 6 VH, CHA. 9. 547. 6.VL, CHA. 9. 547. 6.HC, CHA. 9. 547. 6.LC, CHA. 9. 547. 6.H1, CHA. 9. 547. 6.H2, CHA. 9. 547. 6.H3; CHA.9.547.6.H4, CHA.9.547.6.H4(S241P), CHA. 9. 547. 6.vhCDR1, CHA. 9. 547. 6.vhCDR2, CHA. 9. 547. 6.vhCDR3, CHA. 9. 547. 6.vlCDR1, CHA. 9. 547. 6.vlCDR2 and CHA. 9. 547. 6.vhCDR3;
CHA.9.547.7, CHA. 9. 547. 7VH, CHA. 9. 547. 7.VL, CHA. 9. 547. 7.HC, CHA. 9. 547. 7.LC, CHA. 9. 547. 7.H1, CHA. 9. 547. 7.H2, CHA. 9. 547. 7.H3; CHA.9.547.7.H4, CHA.9.547.7.H4(S241P), CHA. 9. 547. 7.vhCDR1, CHA. 9. 547. 7.vhCDR2, CHA. 9. 547. 7.vhCDR3, CHA. 9. 547. 7.vlCDR1, CHA. 9. 547. 7.vlCDR2 and CHA. 9. 547. 7.vhCDR3;
CHA.9.547.8, CHA. 9. 547. 8VH, CHA. 9. 547. 8.VL, CHA. 9. 547. 8.HC, CHA.9.547.8.LC, CHA. 9. 547. 8.H1, CHA. 9. 547. 8.H2, CHA. 9. 547. 8.H3; CHA.9.547.8.H4, CHA.9.547.8.H4(S241P), CHA. 9. 547. 8.vhCDR1, CHA. 9. 547. 8.vhCDR2, CHA. 9. 547. 8.vhCDR3, CHA. 9. 547. 8.vlCDR1, CHA. 9. 547. 8.vlCDR2 and CHA. 9. 547. 8.vhCDR3;
CHA.9.547.9, CHA.9.547.9, CHA.9.547.9VH, CHA.9.547.9.VL, CHA.9. 547.9.HC, CHA.9.547.9.LC, CHA.9.547.9.H1, CHA.9.547.9.H2, CHA.9.547.9.H3; CHA.9.547.9.H4, CHA.9.547.9.H4, CHA.9.547.9.H4(S241P), CHA.9.547.9.H4(S241P), CHA.9.547.9.vhCDR1, CHA.9.547.9.vhCDR2, CHA.9.547.9.vhCDR3, CHA.9.547.9.vlCDR1, CHA.9.547.9.vlCDR2 and CHA.9.547.9.vhCDR3;
CHA.9.547.13, CHA.9.547.13, CHA.9.547. 13VH, CHA.9. 547.13.VL, CHA.9. 547.13.HC, CHA. 9.547.13.LC, CHA. 9.547.13.H1, CHA.9.547.13.H2, CHA.9. 547.13.H3; CHA.9.547.13.H4, CHA.9.547.13.H4, CHA.9.547.13.H4(S241P), CHA.9.547.13.H4(S241P), CHA. 9. 547.13.vhCDR1, CHA.9.547.13.vhCDR2, CHA.9.547. 13.vhCDR3, CHA. 9. 547.13.vlCDR1, CHA. 9. 547.13.vlCDR2 and CHA. 9. 547. 13.vhCDR3;
CHA.9.541.1, CHA. 9. 541.1.VH, CHA. 9. 541.1.VL, CHA. 9. 541.1.HC, CHA. 9. 541.1.LC, CHA. 9. 541.1.H1, CHA. 9. 541.1.H2, CHA. 9. 541.1.H3; CHA.9.541.1.H4, CHA.9.541.1.H4(S241P), CHA. 9. 541.1.vhCDR1, CHA. 9. 541.1.vhCDR2, CHA. 9. 541.1.vhCDR3, CHA. 9. 541.1.vlCDR1, CHA. 9. 541.1.vlCDR2 and CHA. 9.541.1.vhCDR3;
CHA.9.541.3, CHA. 9. 541. 3.VH, CHA. 9. 541. 3.VL, CHA. 9. 541. 3.HC, CHA. 9. 541. 3.LC, CHA. 9. 541. 3.H1, CHA. 9. 541. 3.H2, CHA. 9. 541. 3.H3; CHA.9.541.3.H4, CHA.9.541.3.H4(S241P), CHA. 9. 541. 3.vhCDR1, CHA. 9. 541. 3.vhCDR2, CHA. 9. 541. 3.vhCDR3, CHA. 9. 541. 3.vlCDR1, CHA. 9. 541. 3.vlCDR2 and CHA. 9.541. 3.vhCDR3;
CHA.9.541.4, CHA. 9. 541.4.VH, CHA. 9. 541. 4.VL, CHA. 9. 541. 4.HC, CHA. 9. 541. 4.LC, CHA. 9. 541. 4.H1, CHA. 9. 541. 4.H2, CHA. 9. 541. 4.H3; CHA.9.541.4.H4, CHA.9.541.4.H4(S241P), CHA. 9. 541. 4.vhCDR1, CHA. 9. 541. 4.vhCDR2, CHA. 9. 541. 4.vhCDR3, CHA. 9. 541. 4.vlCDR1, CHA. 9. 541. 4.vlCDR2 and CHA. 9.541. 4.vhCDR3;
CHA.9.541.5, CHA. 9. 541. 5.VH, CHA. 9. 541. 5.VL, CHA. 9. 541. 5.HC, CHA. 9. 541. 5.LC, CHA. 9. 541. S.H1, CHA. 9. 541. 5.H2, CHA. 9. 541. 5.H3; CHA.9.541.5.H4, CHA.9.541.5.H4(S241P), CHA. 9. 541. 5.vhCDR1, CHA. 9. 541. 5.vhCDR2, CHA. 9. 541. 5.vhCDR3, CHA. 9. 541. 5.vlCDR1, CHA. 9. 541. 5.vlCDR2 and CHA. 9.541. 5.vhCDR3;
CHA.9.541.6, CHA. 9. 541. 6.VH, CHA. 9. 541. 6.VL, CHA. 9. 541. 6.HC, CHA. 9. 541. 6.LC, CHA. 9. 541. 6.H1, CHA. 9. 541. 6.H2, CHA. 9. 541.6.H3; CHA.9.541.6.H4, CHA.9.541.6.H4(S241P), CHA. 9. 541. 6.vhCDR1, CHA. 9. 541. 6.vhCDR2, CHA. 9. 541. 6.vhCDR3, CHA. 9. 541. 6.vlCDR1, CHA. 9. 541. 6.vlCDR2 and CHA. 9.541. 6.vhCDR3;
CHA.9.541.7, CHA. 9. 541. 7.VH, CHA. 9. 541. 7.VL, CHA. 9. 541. 7.HC, CHA. 9. 541. 7.LC, CHA. 9. 541. 7.H1, CHA. 9. 541. 7.H2, CHA. 9. 541. 7.H3; CHA.9.541.7.H4, CHA.9.541.7.H4(S241P), CHA. 9. 541. 7.vhCDR1, CHA. 9. 541. 7.vhCDR2, CHA. 9. 541. 7.vhCDR3, CHA. 9. 541. 7.vlCDR1, CHA. 9. 541. 7.vlCDR2 and CHA. 9.541. 7.vhCDR3; and
CHA.9.541.8, CHA. 9. 541. 8.VH, CHA. 9. 541. 8.VL, CHA. 9. 541. 8.HC, CHA. 9. 541. 8.LC, CHA. 9. 541. 8.H1, CHA. 9. 541. 8.H2, CHA. 9. 541. 8.H3; CHA.9.541.8.H4, CHA.9.541.8.H4(S241P); CHA. 9. 541. 8vhCDR1, CHA. 9. 541. 8.vhCDR2, CHA. 9. 541. 8.vhCDR3, CHA. 9. 541. 8.vlCDR1, CHA. 9. 541. 8.vlCDR2 and CHA. 9.541. 8.vhCDR3.

In the case of scFvs comprising the CDRs of the antibodies above, these are labeled as scFvs that include a scFv comprising a variable heavy domain with the vhCDRs, a linker and a variable light domain with the vlCDRs, again as above in either orientation. Thus the invention includes scFv-CHA.9.536.3.1, scFv-CHA.9.536.3, scFv-CHA.9.536.4, scFv-CHA.9.536.5, scFv-CHA.9.536.7, scFv-CHA.9.536.8, scFv-CHA.9.560.1, scFv-CHA.9.560.3, scFv-CHA.9.560.4, scFv-CHA.9.560.5, scFv-CHA.9.560.6, scFv-CHA.9.560.7, scFv-CHA.9.560.8, scFv-CHA.9.546.1, scFv-CHA.9.547.1, scFv-CHA.9.547.2, scFv-CHA.9.547.3, scFv-CHA.9.547.4, scFv-CHA.9.547.6, scFv-CHA.9.547.7, scFv-CHA.9.547.8, scFv-CHA.9.547.9, scFv-CHA.9.547.13, scFv-CHA.9.541.1, scFv-CHA.9.541.3, scFv-CHA.9.541.4, scFv-CHA.9.541.5, scFv-CHA.9.541.6, scFv-CHA.9.541.7 and scFv-CHA.9.541.8.

In addition, CHA.9.543 binds to TIGIT but does not block the TIGIT-PVR interaction.

As discussed herein, the invention further provides variants of the above components (CPA and CHA), including variants in the CDRs, as outlined above. Thus, the invention provides antibodies comprising a set of 6 CDRs as outlined herein that can contain one, two or three amino acid differences in the set of CDRs, as long as the antibody still binds to TIGIT. Suitable assays for testing whether an anti-TIGIT antibody that contains mutations as compared to the CDR sequences outlined herein are known in the art, such as Biacore assays.

In addition, the invention further provides variants of the above variable heavy and light chains. In this case, the variable heavy chains can be 80%, 90%, 95%, 98% or 99% identical to the "VH" sequences herein, and/or contain from 1, 2, 3, 4, 5, 6, 7, 8, 9, 10 amino acid changes, or more, when Fc variants are used. Variable light chains are provided that can be 80%, 90%, 95%, 98% or 99% identical to the "VL" sequences herein (and in particular CPA.9.086), and/or contain from 1, 2, 3, 4, 5, 6, 7, 8, 9, 10 amino acid changes, or more, when Fc variants are used. In these embodiments, the invention includes these variants as long as the antibody still binds to TIGIT. Suitable assays for testing whether an anti-TIGIT antibody that contains mutations as compared to the CDR sequences outlined herein are known in the art, such as Biacore assays.

Similarly, heavy and light chains are provided that are 80%, 90%, 95%, 98% or 99% identical to the full length "HC" and "LC" sequences herein (and in particular CPA.9.086), and/or contain from 1, 2, 3, 4, 5, 6, 7, 8, 9, 10 amino acid changes, or more, when Fc variants are used. In these embodiments, the invention includes these variants as long as the antibody still binds to TIGIT. Suitable assays for testing whether an anti-TIGIT antibody that contains mutations as compared to the CDR sequences outlined herein are known in the art, such as Biacore assays.

In addition, the framework regions of the variable heavy and variable light chains of either the CPA or CHA antibodies herein can be humanized (or, in the case of the CHA antibodies, "rehumanized", to the extent that alternative humanization methods can be done) as is known in the art (with occasional variants generated in the CDRs as needed), and thus humanized variants of the VH and VL chains of Figure 30 can be generated (and in particular CPA.9.086). Furthermore, the humanized variable heavy and light domains can then be fused with human constant regions, such as the constant regions from IgG1, IgG2, IgG3 and IgG4 (including IgG4(S241P)).

In particular, as is known in the art, murine VH and VL chains can be humanized as is known in the art, for example, using the IgBLAST program of the NCBI website, as outlined in Ye et al. Nucleic Acids Res. 41:W34-W40 (2013), herein incorporated by reference in its entirety for the humanization methods. IgBLAST takes a murine VH and/or VL sequence and compares it to a library of known human germline sequences. As shown herein, for the humanized sequences generated herein, the databases used were IMGT human VH genes (F+ORF, 273 germline sequences) and IMGT human VL kappa genes (F+ORF, 74 germline sequences). An exemplary five CHA sequences were chosen: CHA.9.536, CHA9.560, CHA.9.546, CHA.9.547 and CHA.9.541 (see Figure 30). For this embodiment of the humanization, human germline IGHV1-46(allele1) was chosen for all 5 as the acceptor sequence and the human heavy chain IGHJ4(allele1) joining region (J gene). For three of four (CHA.7.518, CHA.7.530, CHA.7.538_1 and CHA.7.538_2), human germline IGKV1-39(allele 1) was chosen as the acceptor sequence and human light chain IGKJ2(allele1) (J gene) was chosen. The J gene was chosen from human joining region sequences compiled at IMGT^{®} the international ImMunoGeneTics information system as www.imgt.org. CDRs were defined according to the AbM definition (see www.bioinfo.org.uk/abs/).

In some embodiments, the anti-TIGIT antibodies of the present invention include anti-TIGIT antibodies wherein the V_{H} and V_{L} sequences of different anti-TIGIT antibodies can be "mixed and matched" to create other anti-TIGIT antibodies. TIGIT binding of such "mixed and matched" antibodies can be tested using the binding assays described above. e.g., ELISAs or Biacore assays). In some embodiments, when V_{H} and V_{L} chains are mixed and matched, a V_{H} sequence from a particular V_{H}/V_{L} pairing is replaced with a structurally similar V_{H} sequence. Likewise, in some embodiments, a V_{L} sequence from a particular V_{H}/V_{L} pairing is replaced with a structurally similar V_{L} sequence. For example, the V_{H} and V_{L} sequences of homologous antibodies are particularly amenable for mixing and matching.

Accordingly, the TIGIT antibodies of the invention comprise CDR amino acid sequences selected from the group consisting of (a) sequences as listed herein; (b) sequences that differ from those CDR amino acid sequences specified in (a) by 1, 2, 3, 4, 5, 6, 7, 8, 9, 10 or more amino acid substitutions; (c) amino acid sequences having 90% or greater, 95% or greater, 98% or greater, or 99% or greater sequence identity to the sequences specified in (a) or (b); (d) a polypeptide having an amino acid sequence encoded by a polynucleotide having a nucleic acid sequence encoding the amino acids as listed herein. In particular, the CPA.9.086 antibody can have sequences selected from (a), (b), (c) or (d).

Additionally included in the definition of TIGIT antibodies are antibodies that share identity to the TIGIT antibodies enumerated herein. That is, in certain embodiments, an anti-TIGIT antibody according to the invention comprises heavy and light chain variable regions comprising amino acid sequences that are identical to all or part of the anti-TIGIT amino acid sequences of preferred anti-TIGIT antibodies, respectively, wherein the antibodies retain the desired functional properties of the parent anti-TIGIT antibodies. The percent identity between the two sequences is a function of the number of identical positions shared by the sequences (i.e., % homology=# of identical positions/total # of positions X 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used.

In general, the percentage identity for comparison between TIGIT antibodies is at least 75%, at least 80%, at least 90%, with at least about 95, 96, 97, 98 or 99% percent identity being preferred. The percentage identity may be along the whole amino acid sequence, for example the entire heavy or light chain or along a portion of the chains. For example, included within the definition of the anti-TIGIT antibodies of the invention are those that share identity along the entire variable region (for example, where the identity is 95 or 98% identical along the variable regions), or along the entire constant region, or along just the Fc domain. In particular, the invention provides TIGIT antibodies that have at least 75%, at least 80%, at least 90%, with at least about 95, 96, 97, 98 or 99% percent identity being preferred, with the CPA.9.086 antibody.

In addition, also included are sequences that may have the identical CDRs but changes in the framework portions of the variable domain (or entire heavy or light chain). For example, TIGIT antibodies include those with CDRs identical to those shown in Figure 30 but whose identity along the variable region can be lower, for example 95 or 98% percent identical. In particular, the invention provides TIGIT antibodies that have identical CDRs to CPA.9.086 but with framework regions that are 95 or 98% identical to CPA.9.086.

### 1. TIGIT Antibodies That Compete For Binding

The present invention provides not only the enumerated antibodies but additional antibodies that compete with the enumerated antibodies (the CPA numbers enumerated herein that specifically bind to TIGIT) to specifically bind to the TIGIT molecule. As is shown in Example 16, the TIGIT antibodies of the invention "bin" into different epitope bins. Among the 44 TIGIT antibodies in the epitope binning study, there are four communities, each having related pairwise blocking patterns, which separate into 12 total discrete bins outlined herein and shown in Figures 67 and 68. There are twelve discrete bins outlined herein; 1) BM9-H4, CHA.9.525, CPA.9.081-H4, CHA.9.538, CHA.9.553, CPA.9.069-H4, CHA.9.543, CHA.9.556, CPA.9.077-H4 and CHA.9.561; 2) CHA.9.560 andCHA.9.528; 3) CHA.9.552, CHA.9.521, CHA.9.541, CHA.9.529, CHA.9.519, CHA.9.527 and CHA.9.549;4) CPA.9.057-H4 and CHA.9.554; 5) CHA.9.546, CPA.9.012-H4, CHA.9.547, CPA.9.013-H4, CPA.9.018-H4, MBSA43-M1, Sino PVR-Fc(ligand), CHA.9.555, PVR-Fc M2A(ligand), BM29-H4, CPA.9.027-H4, CPA.9.049-H4 and CPA.9.053-H4; 6) CPA.9.064-H4; 7) BM26-H4; 8) CPA.9.059-H4; 9) CHA.9.535 and CPA.9.009-H4; 10) CHA.9.536, CHA.9.522 and CPA.9.015-H4; 11) CPA.9.011-H4 and BM8-H4 and 12) CPA.9.071-H4.

Thus, the invention provides anti-TIGIT antibodies that compete for binding with antibodies that are in discrete epitope bins 1 to 12. In a particular embodiment, the invention provides anti-TIGIT antibodies that compete for binding with CPA.9.086 and are at least 95, 96, 97, 98, or 99% identical to CPA.9.086.

Additional antibodies that compete with the enumerated antibodies are generated, as is known in the art and generally outlined below. Competitive binding studies can be done as is known in the art, generally using SPR/Biacore^{®} binding assays, as well as ELISA and cell-based assays.

### E. Optional Antibody Engineering

The anti-PVRIG antibodies (*e*.*g*., anti-PVRIG antibodies including those with CDRs identical to those shown in Figure 3) of the invention can be modified, or engineered, to alter the amino acid sequences by amino acid substitutions.

By "amino acid substitution" or "substitution" herein is meant the replacement of an amino acid at a particular position in a parent polypeptide sequence with a different amino acid. In particular, in some embodiments, the substitution is to an amino acid that is not naturally occurring at the particular position, either not naturally occurring within the organism or in any organism. For example, the substitution E272Y refers to a variant polypeptide, in this case an Fc variant, in which the glutamic acid at position 272 is replaced with tyrosine. For clarity, a protein which has been engineered to change the nucleic acid coding sequence but not change the starting amino acid (for example exchanging CGG (encoding arginine) to CGA (still encoding arginine) to increase host organism expression levels) is not an "amino acid substitution"; that is, despite the creation of a new gene encoding the same protein, if the protein has the same amino acid at the particular position that it started with, it is not an amino acid substitution.

As discussed herein, amino acid substitutions can be made to alter the affinity of the CDRs for the PVRIG protein (including both increasing and decreasing binding, as is more fully outlined below), as well as to alter additional functional properties of the antibodies. For example, the antibodies may be engineered to include modifications within the Fc region, typically to alter one or more functional properties of the antibody, such as serum half-life, complement fixation, Fc receptor binding, and/or antigen-dependent cellular cytotoxicity. Furthermore, an antibody according to at least some embodiments of the invention may be chemically modified (*e*.*g*., one or more chemical moieties can be attached to the antibody) or be modified to alter its glycosylation, again to alter one or more functional properties of the antibody. Such embodiments are described further below. The numbering of residues in the Fc region is that of the EU index of Kabat.

In one embodiment, the hinge region of C_{H1} is modified such that the number of cysteine residues in the hinge region is altered, e.g., increased or decreased. This approach is described further in U.S. Pat. No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired *Staphylococcyl* protein A (SpA) binding relative to native Fc-hinge domain SpA binding. This approach is described in further detail in U.S. Pat. No. 6,165,745 by Ward et al.

In some embodiments, amino acid substitutions can be made in the Fc region, in general for altering binding to FcyR receptors. By "Fc gamma receptor", "FcyR" or "FcgammaR" as used herein is meant any member of the family of proteins that bind the IgG antibody Fc region and is encoded by an FcyR gene. In humans this family includes but is not limited to FcyRI (CD64), including isoforms FcyRIa, FcyRIb, and FcyRIc; FcyRII (CD32), including isoforms FcyRIIa (including allotypes H131 and R131), FcyRIIb (including FcyRIIb-1 and FcyRIIb-2), and FcyRIIc; and FcyRIII (CD16), including isoforms FcγRIIIa (including allotypes V158 and F158) and FcyRIIIb (including allotypes FcγRIIIb-NA1 and FcyRIIIb-NA2) (Jefferis et al., 2002, Immunol Lett 82:57-65, entirely incorporated by reference), as well as any undiscovered human FcyRs or FcyR isoforms or allotypes. An FcyR may be from any organism, including but not limited to humans, mice, rats, rabbits, and monkeys. Mouse FcyRs include but are not limited to FcyRI (CD64), FcyRII (CD32), FcyRIII-1 (CD16), and FcyRIII-2 (CD16-2), as well as any undiscovered mouse FcyRs or FcyR isoforms or allotypes.

There are a number of useful Fc substitutions that can be made to alter binding to one or more of the FcyR receptors. Substitutions that result in increased binding as well as decreased binding can be useful. For example, it is known that increased binding to FcγRIIIa generally results in increased ADCC (antibody dependent cell-mediated cytotoxicity; the cell-mediated reaction wherein nonspecific cytotoxic cells that express FcyRs recognize bound antibody on a target cell and subsequently cause lysis of the target cell. Similarly, decreased binding to FcyRIIb (an inhibitory receptor) can be beneficial as well in some circumstances. Amino acid substitutions that find use in the present invention include those listed in U.S. Ser. Nos. 11/124,620 (particularly FIG. 41) and U.S. Patent No. 6,737,056, both of which are expressly incorporated herein by reference in their entirety and specifically for the variants disclosed therein. Particular variants that find use include, but are not limited to, 236A, 239D, 239E, 332E, 332D, 239D/332E, 267D, 267E, 328F, 267E/328F, 236A/332E, 239D/332E/330Y, 239D, 332E/330L, 299T and 297N.

In addition, the antibodies of the invention are modified to increase its biological half-life. Various approaches are possible. For example, one or more of the following mutations can be introduced: T252L, T254S, T256F, as described in U.S. Pat. No. 6,277,375 to Ward. Alternatively, to increase the biological half-life, the antibody can be altered within the C_{H1} or C_{L} region to contain a salvage receptor binding epitope taken from two loops of a CH2 domain of an Fc region of an IgG, as described in U.S. Pat. Nos. 5,869,046 and 6,121,022 by Presta et al. Additional mutations to increase serum half-life are disclosed in U.S. Patent Nos. 8,883,973, 6,737,056 and 7,371,826, and include 428L, 434A, 434S, and 428L/434S.

In yet other embodiments, the Fc region is altered by replacing at least one amino acid residue with a different amino acid residue to alter the effector functions of the antibody. For example, one or more amino acids selected from amino acid residues 234, 235, 236, 237, 297, 318, 320 and 322 can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Pat. Nos. 5,624,821 and 5,648,260, both by Winter et al.

In another example, one or more amino acids selected from amino acid residues 329, 331 and 322 can be replaced with a different amino acid residue such that the antibody has altered C1q binding and/or reduced or abolished complement dependent cytotoxicity (CDC). This approach is described in further detail in U.S. Pat. Nos. 6,194,551 by Idusogie et al.

In another example, one or more amino acid residues within amino acid positions 231 and 239 are altered to thereby alter the ability of the antibody to fix complement. This approach is described further in PCT Publication WO 94/29351 by Bodmer et al.

In yet another example, the Fc region is modified to increase the ability of the antibody to mediate antibody dependent cellular cytotoxicity (ADCC) and/or to increase the affinity of the antibody for an Fcy receptor by modifying one or more amino acids at the following positions: 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439. This approach is described further in PCT Publication WO 00/42072 by Presta. Moreover, the binding sites on human IgG1 for FcyRI, FcyRII, FcyRIII and FcRn have been mapped and variants with improved binding have been described (see Shields, R. L. et al. (2001) J. Biol. Chem. 276:6591-6604). Specific mutations at positions 256, 290, 298, 333, 334 and 339 are shown to improve binding to FcyRIII. Additionally, the following combination mutants are shown to improve FcγRIII binding: T256A/S298A, S298A/E333A, S298A/K224A and S298A/E333A/K334A. Furthermore, mutations such as M252Y/S254T/T256E or M428L/N434S improve binding to FcRn and increase antibody circulation half-life (see Chan CA and Carter PJ (2010) Nature Rev Immunol 10:301-316).

In still another embodiment, the antibody can be modified to abrogate *in vivo* Fab arm exchange. Specifically, this process involves the exchange of IgG4 half-molecules (one heavy chain plus one light chain) between other IgG4 antibodies that effectively results in bispecific antibodies which are functionally monovalent. Mutations to the hinge region and constant domains of the heavy chain can abrogate this exchange (see, Aalberse, RC, Schuurman J., 2002, Immunology 105:9-19).

In still another embodiment, the glycosylation of an antibody is modified. For example, an aglycosylated antibody can be made (*i.e.*, the antibody lacks glycosylation). Glycosylation can be altered to, for example, increase the affinity of the antibody for antigen or reduce effector function such as ADCC. Such carbohydrate modifications can be accomplished by, for example, altering one or more sites of glycosylation within the antibody sequence, for example N297. For example, one or more amino acid substitutions can be made that result in elimination of one or more variable region framework glycosylation sites to thereby eliminate glycosylation at that site.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies according to at least some embodiments of the invention to thereby produce an antibody with altered glycosylation. For example, the cell lines Ms704, Ms705, and Ms709 lack the fucosyltransferase gene, FUT8 (α (1,6) fucosyltransferase), such that antibodies expressed in the Ms704, Ms705, and Ms709 cell lines lack fucose on their carbohydrates. The Ms704, Ms705, and Ms709 FUT8 cell lines are created by the targeted disruption of the FUT8 gene in CHO/DG44 cells using two replacement vectors (see U.S. Patent Publication No. 20040110704 by Yamane et al. and Yamane-Ohnuki et al. (2004) Biotechnol Bioeng 87:614-22). As another example, EP 1,176,195 by Hanai et al. describes a cell line with a functionally disrupted FUT8 gene, which encodes a fucosyl transferase, such that antibodies expressed in such a cell line exhibit hypofucosylation by reducing or eliminating the α 1,6 bond-related enzyme. Hanai et al. also describe cell lines which have a low enzyme activity for adding fucose to the N-acetylglucosamine that binds to the Fc region of the antibody or does not have the enzyme activity, for example the rat myeloma cell line YB2/0 (ATCC CRL 1662). PCT Publication WO 03/035835 by Presta describes a variant CHO cell line, Lec13 cells, with reduced ability to attach fucose to Asn(297)-linked carbohydrates, also resulting in hypofucosylation of antibodies expressed in that host cell (see also Shields, R. L. et al. (2002) J. Biol. Chem. 277:26733-26740). PCT Publication WO 99/54342 by Umana et al. describes cell lines engineered to express glycoprotein-modifying glycosyl transferases (*e*.*g*., β(1,4)-N-acetylglucosaminyltransferase III (GnTIII)) such that antibodies expressed in the engineered cell lines exhibit increased bisecting GlcNac structures which results in increased ADCC activity of the antibodies (see also Umana et al. (1999) Nat. Biotech. 17:176-180). Alternatively, the fucose residues of the antibody may be cleaved off using a fucosidase enzyme. For example, the fucosidase α-L-fucosidase removes fucosyl residues from antibodies (Tarentino, A. L. et al. (1975) Biochem. 14:5516-23).

Another modification of the antibodies herein that is contemplated by the invention is pegylation or the addition of other water soluble moieties, typically polymers, *e*.*g*., in order to enhance half-life. An antibody can be pegylated to, for example, increase the biological (*e*.*g*., serum) half-life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C₁-C₁₀) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies according to at least some embodiments of the invention. See for example, EP 0 154 316 by Nishimura et al. and EP 0 401 384 by Ishikawa et al.

In addition to substitutions made to alter binding affinity to FcyRs and/or FcRn and/or increase in vivo serum half-life, additional antibody modifications can be made, as described in further detail below.

In some cases, affinity maturation is done. Amino acid modifications in the CDRs are sometimes referred to as "affinity maturation". An "affinity matured" antibody is one having one or more alteration(s) in one or more CDRs which results in an improvement in the affinity of the antibody for antigen, compared to a parent antibody which does not possess those alteration(s). In some cases, although rare, it may be desirable to decrease the affinity of an antibody to its antigen, but this is generally not preferred.

In some embodiments, one or more amino acid modifications are made in one or more of the CDRs of the PVRIG antibodies of the invention. In general, only 1 or 2 or 3-amino acids are substituted in any single CDR, and generally no more than from 1, 2, 3. 4, 5, 6, 7, 8 9 or 10 changes are made within a set of CDRs. However, it should be appreciated that any combination of no substitutions, 1, 2 or 3 substitutions in any CDR can be independently and optionally combined with any other substitution.

Affinity maturation can be done to increase the binding affinity of the antibody for the PVRIG antigen by at least about 10% to 50-100-150% or more, or from 1 to 5 fold as compared to the "parent" antibody. Preferred affinity matured antibodies will have nanomolar or even picomolar affinities for the PVRIG antigen. Affinity matured antibodies are produced by known procedures. See, for example, Marks et al., 1992, Biotechnology 10:779-783 that describes affinity maturation by variable heavy chain (VH) and variable light chain (VL) domain shuffling. Random mutagenesis of CDR and/or framework residues is described in: Barbas, et al. 1994, Proc. Nat. Acad. Sci, USA 91:3809-3813; Shier et al., 1995, Gene 169:147-155; Yelton et al., 1995, J. Immunol. 155:1994-2004; Jackson et al., 1995, J. Immunol. 154(7):3310-9; and Hawkins et al, 1992, J. Mol. Biol. 226:889-896, for example.

Alternatively, amino acid modifications can be made in one or more of the CDRs of the antibodies of the invention that are "silent", *e.g.*, that do not significantly alter the affinity of the antibody for the antigen. These can be made for a number of reasons, including optimizing expression (as can be done for the nucleic acids encoding the antibodies of the invention).

Thus, included within the definition of the CDRs and antibodies of the invention are variant CDRs and antibodies; that is, the antibodies of the invention can include amino acid modifications in one or more of the CDRs of the enumerated antibodies of the invention. In addition, as outlined below, amino acid modifications can also independently and optionally be made in any region outside the CDRs, including framework and constant regions.

### V. PVRIG TREATMENT ANTIBODIES

The present invention provides anti-PVRIG antibodies for treatments purposes and uses. (For convenience, "anti-PVRIG antibodies" and "PVRIG antibodies" are used interchangeably). The anti-PVRIG antibodies of the invention specifically bind to human PVRIG, and preferably the ECD of human PVRIG, as depicted in Figure 3, including, *e*.*g*., anti-PVRIG antibodies including those with CDRs identical to those shown in Figure 3.

Specific binding for PVRIG or a PVRIG epitope can be exhibited, for example, by an antibody having a KD of at least about 10⁻⁴ M, at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, or greater, where KD refers to a dissociation rate of a particular antibody-antigen interaction. Typically, an antibody that specifically binds an antigen will have a KD that is 20-, 50-, 100-, 500-, 1000-, 5,000-, 10,000- or more times greater for a control molecule relative to the PVRIG antigen or epitope.

However, as shown in the Examples, for optimal binding to PVRIG expressed on the surface of NK and T-cells, the antibodies preferably have a KD less 50 nM and most preferably less than 1 nM, with less than 0.1 nM and less than 1 pM and 0.1 pM finding use in the methods of the invention.

Also, specific binding for a particular antigen or an epitope can be exhibited, for example, by an antibody having a KA or Ka for a PVRIG antigen or epitope of at least 20-, 50-, 100-, 500-, 1000-, 5,000-, 10,000- or more times greater for the epitope relative to a control, where KA or Ka refers to an association rate of a particular antibody-antigen interaction. s

In some embodiments, the anti-PVRIG antibodies of the invention bind to human PVRIG with a K_{D} of 100 nM or less, 50 nM or less, 10 nM or less, or 1 nM or less (that is, higher binding affinity), or 1pM or less, wherein K_{D} is determined by known methods, *e*.*g*., surface plasmon resonance (SPR, *e*.*g*., Biacore assays), ELISA, KINEXA, and most typically SPR at 25° or 37° C.

The invention provides antigen binding domains, including full length antibodies, which contain a number of specific, enumerated sets of 6 CDRs, as provided in Figure 3. The invention provides antigen binding domains, including full length antibodies, which contain a number of specific, enumerated sets of 6 CDRs, as provided in Figure 3.

The invention further provides variable heavy and light domains as well as full length heavy and light chains.

As discussed herein, the invention further provides variants of the above components, including variants in the CDRs, as outlined above. In addition, variable heavy chains can be at least 80%, at least 90%, at least 95%, at least 98% or at least 99% identical to the "VH" sequences herein, and/or contain from 1, 2, 3, 4, 5, 6, 7, 8, 9, 10 amino acid changes, or more, when Fc variants are used. Variable light chains are provided that can be at least 80%, at least 90%, at least 95%, at least 98% or at least 99% identical to the "VL" sequences herein, and/or contain from 1, 2, 3, 4, 5, 6, 7, 8, 9, 10 amino acid changes, or more, when Fc variants are used. Similarly, heavy and light chains are provided that are at least 80%, at least 90%, at least 95%, at least 98% or at least 99% identical to the "HC" and "LC" sequences herein, and/or contain from 1, 2, 3, 4, 5, 6, 7, 8, 9, 10 amino acid changes, or more, when Fc variants are used.

Accordingly, the present invention provides antibodies, usually full length or scFv domains, that comprise the following CHA sets of CDRs, the sequences of which are shown in Figure 3:
CHA.7.518.1.H4(S241P)vhCDR1, CHA.7.518.1.H4(S241P)vhCDR2,
CHA.7.518.1.H4(S241P)vhCDR3, CHA.7.518.1.H4(S241P)vlCDR1,
CHA.7.518.1.H4(S241P)vlCDR2, and CHA.7.518.1.H4(S241P)vlCDR3.

In addition, the framework regions of the variable heavy and variable light chains can be humanized as is known in the art (with occasional variants generated in the CDRs as needed), and thus humanized variants of the VH and VL chains of Figure 3 can be generated. Furthermore, the humanized variable heavy and light domains can then be fused with human constant regions, such as the constant regions from IgG1, IgG2, IgG3 and IgG4.

In addition, also included are sequences that may have the identical CDRs but changes in the variable domain (or entire heavy or light chain). For example, PVRIG antibodies include those with CDRs identical to those shown in Figure 3 or Figures 5A-5D but whose identity along the variable region can be lower, for example 95 or 98% percent identical. For example, PVRIG antibodies include those with CDRs identical to those shown in Figure 3 but whose identity along the variable region can be lower, for example 95 or 98% percent identical, and in some embodiments at least 95% or at least 98%.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (*e*.*g*., XBLAST and NBLAST) can be used.

In general, the percentage identity for comparison between PVRIG antibodies is at least 75%, at least 80%, at least 90%, with at least about 95, 96, 97, 98 or 99% percent identity being preferred. The percentage identity may be along the whole amino acid sequence, for example the entire heavy or light chain or along a portion of the chains. For example, included within the definition of the anti-PVRIG antibodies of the invention are those that share identity along the entire variable region (for example, where the identity is 95 or 98% identical along the variable regions, and in some embodiments at least 95% or at least 98%), or along the entire constant region, or along just the Fc domain.

### VI. BIOMARKERS AND/OR COMPANION DIAGNOSTIC

### A. PVRL2

In some embodiments, the level of PVRL2 expression can be employed as a biomarker or companion diagnostic for use in determining treatment regimens as well as predicting or determining treatment efficacy. In some embodiments, the level of PVRL2 expression can be employed as a biomarker or companion diagnostic for use in the determining treatment regimens as well as predicting or determining treatment efficacy for treatment with an anti-PVRIG antibody. In some embodiments, the PVRL2 status is defined based on the percentage staining for all tumor infiltrating immune cells. In some embodiments, the expression is categorized based on expression staining criteria below, in Table 1.

**Table 1: PVRL2 Staining Criteria**

| PVRL2 status | Staining criteria | Predicted response |
|---|---|---|
| negative | 0% membrane | No to minimal |
| Tumor Positivity (TS) | >20% tumor membrane staining at +1 IHC score | likely |
| Immune Positivity (IS) | >20% immune infiltrating cells at any intensity membrane or cytoplasmatic | likely |
| Endothelial Positivity (ES) | >20% endothelial cells at any intensity membrane or cytoplasmatic | likely |
| Total Positive (ToP = TS+IS+ES) | Any combination of two Positivity scores | Very likely |

In some embodiments, the level of PVRL2 expression is determined by measuring the level of PVRL2 using immunohistochemistry. In some embodiments, the level of PVRL2 expression, including the level of PVRL2 expression, is scored by a board-certified pathologist.

In some embodiments, the PVRL2 expression level is determined using immunohistochemistry. In some embodiments, the PVRIG expression level is determined using immunohistochemistry.

In some embodiments, the PVRL2 antibody used for the immunohistochemistry is CST PVRL2 Ab (Nectin-2/CD112 (D8D3F) XP^{®} Rabbit mAb). (See, the World Wide Web at www.cellsignal.com/products/primary-antibodies/nectin-2-cd112-d8d3f-xp-rabbit-mab/95333.)

In some embodiments, the PVRL2 expression level is categorized as strong, moderate, weak, or negative.

In some embodiments, the PVRL2 expression is categorized as strong or moderate. In some embodiments, when PVRL2 expression is categorized as strong or moderate, there is predicted to be a response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, the PVRL2 expression level is categorized as 0-no signal, 1-low, 2-medium, or 3- high.

In some embodiments, the PVRL2 expression is categorized as 2-medium or 3-high. In some embodiments, when PVRL2 expression is categorized as 2-medium or 3- high, there is predicted to be a response to an anti-PVRIG antibody. In some embodiments, when PVRL2 expression is categorized as 3- high, there is predicted to be an increased likelihood of response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, the PVRL2 expression is 0% membrane staining there is predicted to be no response or a minimal response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, the PVRL2 expression is >20% tumor membrane staining at +1 IHC score there is predicted to be a response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, the PVRL2 expression is >20% tumor membrane staining at +1 IHC score there is predicted to be a response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, the PVRL2 expression is >20% immune infiltrating cells at any intensity membrane or cytoplasmatic staining there is predicted to be a response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, there is an increased prediction of a response to an anti-PVRIG antibody when the PVRL2 expression is characterized by at least two of the following:
- >20% tumor membrane staining at +1 IHC score there is predicted to be no response or a minimal response to an anti-PVRIG antibody,
- >20% tumor membrane staining at +1 IHC score there is predicted to be a response to an anti-PVRIG antibody, and/or
- >20% immune infiltrating cells at any intensity membrane or cytoplasmatic staining.

In some embodiments, there is an increased prediction of a response to the anti-PVRIG antibody comprising the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3, when the PVRL2 expression is characterized by at least two of the following:
- >20% tumor membrane staining at +1 IHC score there is predicted to be no response or a minimal response to an anti-PVRIG antibody,
- >20% tumor membrane staining at +1 IHC score there is predicted to be a response to an anti-PVRIG antibody, and/or
- >20% immune infiltrating cells at any intensity membrane or cytoplasmatic staining.

In some embodiments, cancer patients having dendritic cells in the tumor, tumor microenvironment, and/or peripheral blood with at least 10%, 20%, 30%, 40%, 50%, 60%, 70%, 80%, or 90% PVRL2 expression had a higher probability to respond an anti-PVRIG antibody treatment as compared to cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having activated dendritic cells in the tumor, tumor microenvironment, and/or peripheral blood, with at least 10%, 20%, 30%, 40%, 50%, 60%, 70%, 80%, or 90% PVRL2 expression had a higher probability to respond an anti-PVRIG antibody treatment as compared to cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having DC1 in the tumor, tumor microenvironment, and/or peripheral blood, with at least 5%, 10% 20%, 30%, 40%, 50%, 60%, 70%, 80%, or 90% PVRL2 expression had a higher probability to respond an anti-PVRIG antibody treatment as compared to cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having immune cells in the tumor, tumor microenvironment, and/or peripheral blood with at least 10%, 20%, 30%, 40%, 50%, 60%, 70%, 80%, or 90% PVRL2 expression had a higher probability to respond to an anti-PVRIG antibody treatment as compared to a cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having NK cells, NKT cells, gamma-delta T cells, T cells, CD4 positive T cells, and/or CD8 positive T cells, in the tumor, tumor microenvironment, and/or peripheral blood with at least 10%, 20%, 30%, 40%, 50%, 60%, 70%, 80%, or 90% PVRL2 expression had a higher probability to respond to an anti-PVRIG antibody treatment as compared to a cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having CD4 positive T cells and/or CD8 positive T cells, in the tumor, tumor microenvironment, and/or peripheral blood with at least 10%, 20%, 30%, 40%, 50%, 60%, 70%, 80%, or 90% PVRL2 expression had a higher probability to respond to an anti-PVRIG antibody treatment as compared to cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients having TSCM, TRM, naive, exhausted, cycling, memory, and/or effector CD8 and/or CD4 positive T cells in the tumor, tumor microenvironment, and/or peripheral blood with at least 10%, 20%, 30%, 40%, 50%, 60%, 70%, 80%, or 90% PVRL2 expression had a higher probability to respond to an anti-PVRIG antibody treatment as compared to a cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having dendritic cells in the tumor, tumor microenvironment, and/or peripheral blood with at least 10%, 20%, 30%, 40%, 50%, 60%, 70%, 80%, or 90% PVRL2 expression had a higher probability to respond to an anti-PVRIG antibody treatment as compared to a cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having activated dendritic cells in the tumor, tumor microenvironment, and/or peripheral blood, with at least 10%, 20%, 30%, 40%, 50%, 60%, 70%, 80%, or 90% PVRL2 expression had a higher probability to respond to an anti-PVRIG antibody treatment as compared to a cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having DC1 in the tumor, tumor microenvironment, and/or peripheral blood, with at least 5%, 10% 20%, 30%, 40%, 50%, 60%, 70%, 80%, or 90% PVRL2 expression had a higher probability to respond to an anti-PVRIG antibody treatment as compared to a cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having DC2 in the tumor, tumor microenvironment, and/or peripheral blood, with at least 10%, 20%, 30%, 40%, 50%, 60%, 70%, 80%, or 90% PVRL2 expression, had a higher probability to respond an anti-PVRIG antibody treatment as compared to cancer patient with lower PVRL2 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

Cancer patients that have the presence of any of: Activated DC cells, DC1, and DC2, expressing PVRL2, at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in a biological sample obtained from a tumor, tumor microenvironment, and/or peripheral blood, have a higher probability to respond the anti-PVRIG treatment.

In some embodiments, the DC cells disclosed herein further express CXCL10. In some embodiments, the DC cells expressing CXCL10 are activated DC cells. In some embodiments, the expression level of CXCL10 by activated DC cells is correlated with the expression level of one or more biomarkers of T cells such as early memory CD8 T cells that are selected from the group consisting of GZMK, GZMA, GZMM, NKG7, TNFSF9, SH2D1A, EOMES, DTHD1, SLAMF7, FCRL3, CD28, CMC1, CCL4, CTS7, ITM2C, KLRG1, CRTAM, PECAM1, TCF7, CXCR3, LYAR, and HLA-DRB5. In some embodiments, the expression of CXCL10 is correlated with the expression of CXCR3. In some embodiments, the correlated expressions of CXCL10 and CXCR3 can be employed as a biomarker or companion diagnostic for use in the determining treatment regimens as well as predicting or determining treatment efficacy with an anti-PVRIG antibody. In some embodiments, the co-presence of CXCL10+ activated DC cells and CXCR3 T cells can be employed as a biomarker or companion diagnostic for use in the determining treatment regimens as well as predicting or determining treatment efficacy for treatment with an anti-PVRIG antibody.

### B. PVRIG

In some embodiments, the level of PVRIG expression can be employed as a biomarker or companion diagnostic for use in the determining treatment regimens as well as predicting or determining treatment efficacy. In some embodiments, the level of PVRIG expression can be employed as a biomarker or companion diagnostic for use in the determining treatment regimens as well as predicting or determining treatment efficacy for treatment with an anti-PVRIG antibody. In some embodiments, the PVRIG status is defined based on the percentage staining for all tumor infiltrating immune cells. In some embodiments, the expression is categorized based on expression staining criteria below, in Table 2.

**Table 2: PVRIG Staining Criteria**

| PVRIG status | Staining criteria | Predicted response |
|---|---|---|
| negative | 0% membrane | No to minimal |
| Low positive | >5% immune infiltrating cells at any intensity membrane or cytoplasmatic | likely |
| Low positive | Any positive immune infiltrating cells in TLS or lymphoid aggregates at any intensity membrane or cytoplasmatic | likely |
| Strong positive | Combination of the two criteria above | Very likely |

In some embodiments, the level of PVRIG expression is determined by measuring the level of PVRIG using immunohistochemistry. In some embodiments, the level of PVRIG expression, including the level of PVRIG expression, is scored by a board-certified pathologist.

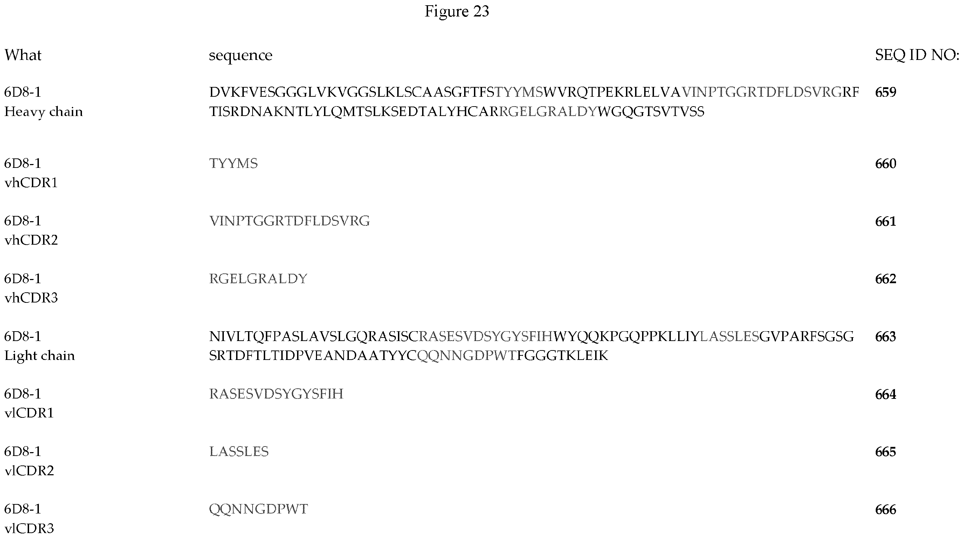

In some embodiments, the PVRIG expression level is determined using immunohistochemistry. In some embodiments, the PVRIG antibody used for the immunohistochemistry is AB-635 PVRIG Ab (6D8-1 clone). The amino acids sequence of the variable light chain, variable heavy chain and the corresponding CDRs of 6D8-1 Ab is presented in Figure 23. In some embodiments, the PVRIG antibody used for the immunohistochemistry is 6D8-1. In some embodiments, the PVRIG antibody used for the immunohistochemistry is 6D8-1 (heavy chain SEQ ID NO:659 and light chain SEQ ID NO:663). In some embodiments, the PVRIG antibody used for the immunohistochemistry comprises:
i) a heavy chain variable domain comprising the vhCDR1, vhCDR2, and vhCDR3 from the heavy chain of 6D8-1 (SEQ ID NO:659), and
ii) a light chain variable domain comprising the vlCDR1, vlCDR2, and vlCDR3 from the light chain of 6D8-1 (SEQ ID NO:663).

In some embodiments, the PVRIG expression level is categorized as strong positive, low positive, or negative. In some embodiments, the PVRIG expression level is categorized as strong positive, low positive with >5% immune infiltrating cells at any intensity membrane or cytoplasmatic, low positive with any positive immune infiltrating cells in TLS or lymphoid aggregates at any intensity membrane or cytoplasmatic, or negative.

In some embodiments, the PVRIG expression is categorized as strong positive or low positive. In some embodiments, the PVRIG expression level is categorized as strong positive, low positive with >5% immune infiltrating cells at any intensity membrane or cytoplasmatic, or low positive with any positive immune infiltrating cells in TLS or lymphoid aggregates at any intensity membrane or cytoplasmatic. In some embodiments, when PVRIG expression is categorized as strong positive or low positive, there is predicted to be a response to an anti-PVRIG antibody. In some embodiments, when PVRIG expression is categorized as strong positive, there is predicted to be an increased likelihood of response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, the PVRIG expression is 0% membrane staining there is predicted to be no response or a minimal response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, the PVRIG expression is >5% immune infiltrating cells at any intensity membrane or cytoplasmatic is predicted to be a response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, the PVRIG expression is any positive immune infiltrating cells in TLS or lymphoid aggregates at any intensity membrane or cytoplasmatic there is predicted to be a response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, there is an increased prediction of a response to an anti-PVRIG antibody when the PVRIG expression is characterized by the following two expression levels:
- >5% immune infiltrating cells staining at any intensity membrane or cytoplasmatic, and
- any positive immune infiltrating cells in TLS or lymphoid aggregates staining at any intensity membrane or cytoplasmatic.

In some embodiments, there is an increased prediction of a response to the anti-PVRIG antibody comprising the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3, when the PVRL2 expression is characterized by at least two of the following:
- >5% immune infiltrating cells staining at any intensity membrane or cytoplasmatic, and
- any positive immune infiltrating cells in TLS or lymphoid aggregates staining at any intensity membrane or cytoplasmatic.

In some embodiments, cancer patients having at least 10%, 20%, 30%, 40%, 50%, 60%, 70%, 80%, 90%, or 100% of immune cells in the tumor, tumor microenvironment, and/or peripheral blood expressing PVRIG, had a higher probability to respond to the anti-PVRIG antibody treatment. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients having at least 10%, 20%, 30%, 40%, 50%, 60%, 70%, 80%, 90%, or 100% of TSCM, TRM, naive, exhausted, cycling, memory, and/or effector CD8 and/or CD4 positive T cells in the tumor, tumor microenvironment, and/or peripheral blood expressing PVRIG, had a higher probability to respond to the anti-PVRIG antibody treatment. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients having at least 10%, 20%, 30%, 40%, 50%, 60%, 70%, 80%, 90%, or 100% of NK cells, NKT cells, gamma-delta T cells, T cells, CD4 positive T cells, and/or CD8 positive T cells in the tumor, tumor microenvironment, and/or peripheral blood expressing PVRIG, had a higher probability to respond to the anti-PVRIG antibody treatment. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients having at least 10%, 20%, 30%, 40%, 50%, 60%, 70%, 80%, 90%, or 100% of CD4 positive T cells and/or CD8 positive T cells in the tumor, tumor microenvironment, and/or peripheral blood expressing PVRIG, had a higher probability to respond to the anti-PVRIG antibody treatment. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients, having at least 10%%, 20%, 30%, 40%, 50%, 60%, 70%, 80%, 90%, or 100% of CD8 positive T cells in the tumor, tumor microenvironment, and/or peripheral blood expressing PVRIG, had a higher probability to respond to the anti-PVRIG antibody treatment. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients, having at least 5%, 10%, 20%, 30%, 40%, 50%, 60%, 70%, 80%, 90%, or 100% of memory CD4 positive T cells in the tumor, tumor microenvironment, and/or peripheral blood expressing PVRIG, had a higher probability to respond to the anti-PVRIG antibody treatment as compared to a cancer patient with lower PVRIG expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients, having at least 10%%, 20%, 30%, 40%, 50%, 60%, 70%, 80%, 90%, or 100% of TSCM cells in the tumor, tumor microenvironment, and/or peripheral blood expressing PVRIG, had a higher probability to respond to the anti-PVRIG antibody treatment as compared to cancer patient with lower PVRIG expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients, having at least 10%, 20%, 30%, 40%, 50%, 60%, 70%, 80%, 90%, or 100% of Naïve CD8 cells in the tumor, tumor microenvironment, and/or peripheral blood expressing PVRIG, had a higher probability to respond to the anti-PVRIG antibody treatment as compared to cancer patient with lower PVRIG expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients, having at least 10%, 20%, 30%, 40%, 50%, 60%, 70%, 80%, 90%, or 100% of exhausted CD8 cells in the tumor, tumor microenvironment, and/or peripheral blood expressing PVRIG, had a higher probability to respond to the anti-PVRIG antibody treatment as compared to cancer patient with lower PVRIG expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients, having at least 10%, 20%, 30%, 40%, 50%, 60%, 70%, 80%, 90%, or 100% of Naïve CD4 positive T cells in the tumor, tumor microenvironment, and/or peripheral blood expressing PVRIG, had a higher probability to respond to the anti-PVRIG antibody treatment as compared to a cancer patient with lower PVRIG expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients, having at least 10%, 20%, 30%, 40%, 50%, 60%, 70%, 80%, 90%, or 100% of exhausted CD4 positive T cells in the tumor, tumor microenvironment, and/or peripheral blood expressing PVRIG, had a higher probability to respond to the anti-PVRIG antibody treatment as compared to a cancer patient with lower PVRIG expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients, having at least 5%, 10%, 20%, 30%, 40%, 50%, 60%, 70%, 80%, 90%, or 100% of TRM cells in the tumor, tumor microenvironment, and/or peripheral blood expressing PVRIG, exhibit a higher probability to respond to the anti-PVRIG antibody treatment as compared to cancer patient with lower PVRIG expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients, having at least 5%, 10%, 20%, 30%, 40%, 50%, 60%, 70%, 80%, 90%, or 100% of Effector cells in the tumor, tumor microenvironment, and/or peripheral blood expressing PVRIG, had a higher probability to respond to the anti-PVRIG antibody treatment as compared to cancer patient with lower PVRIG expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients, the invention provides a method for determining a cancer patient population for treatment with an anti-PVRIG antibody, the method comprising:
(a) detecting the presence in a biological sample from the cancer patient one or more cellular components selected from the group consisting of:
   (i) TSCM, TRM, naive, exhausted, cycling, and effector CD8 positive T cells, that express PVRIG; and/or
   (ii) activated DC cells, DC1, and/or DC2 that express PVRL2,
(b) quantitating the measurement of the level of components selected from the group consisting of:
   (i) TSCM, TRM, naive, exhausted, cycling, and effector CD8 positive T cells, that express PVRIG; and/or
   (ii) activated DC cells, DC1, and/or DC2 that express PVRL2; and
(c) treating the cancer patient with an anti-PVRIG antibody when one or more cellular components in (a) are present at an increased level as compared to a control or a patient that does not have detectable levels of the cells.

In some embodiments, cancer patients, the invention provides a method for predicting or determining the efficacy of treatment with an anti-PVRIG treatment antibody, the method comprising:
(a) measuring the level of one or more cellular components selected from the group consisting of:
   (i) TSCM, TRM, naive, exhausted, cycling, and effector CD8 positive T cells, that express PVRIG; and/or
   (ii) activated DC cells, DC1, and DC2 that express PVRL2;
(b) quantitating the measurement of the level of one or more cellular components selected from the group consisting of:
   (i) TSCM, TRM, naive, exhausted, cycling, and effector CD8 positive T cells" expressing PVRIG; and/or
   (ii) activated DC cells, DC1, and DC2 that express PVRL2; and
(c) correlating the level of the cellular components with the efficacy of treatment, wherein any of the cellular components being present and/or being present at an increased level as compared to a control or a patient that does not have detectable levels of the cells, is indicative of treatment efficacy for treatment with an anti-PVRIG treatment antibody; and
(d) optionally treating the cancer patient with an anti-PVRIG antibody when any of the cellular components in (a) are present at an increased level as compared to a control or a patient that does not have detectable levels of the cells. In some embodiments, provided is a method for predicting or determining the efficacy of treatment or determining a population for treatment with an anti-PVRIG treatment antibody when any of TSCM, TRM, naive, exhausted, cycling, and effector CD8 positive T cells, that express PVRIG comprise at least 0.5%, at least 1%, at least 1.5%, at least 2%, at least 2.5%, at least 3%, at least 3.5%, at least 4%, at least 4.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, of the total CD8 cells in the biological sample is indicative of treatment efficacy and/or indicative for treatment with an anti-PVRIG antibody. In some embodiments, cancer patients where any of TSCM, TRM, naive, exhausted, cycling, and effector CD8 positive T cells, that express PVRIG comprise at least 0.5%, at least 1%, at least 1.5%, at least 2%, at least 2.5%, at least 3%, at least 3.5%, at least 4%, at least 4.5%, or at least 5% at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, of the total CD8 cells in the biological sample exhibit a higher probability to respond to the anti-PVRIG antibody treatment.

In some embodiments, provided is a method for predicting or determining the efficacy of treatment or determining a population for treatment with an anti-PVRIG treatment antibody when any of activated DC cells, DC1, and DC2 that express the PVRL2 comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in the biological sample.

In some embodiments, cancer patients where any of activated DC cells, DC1, and DC2 that express the PVRL2 comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in the biological sample exhibit a higher probability to respond to the anti-PVRIG antibody treatment.

In some embodiments, the presence of and/or an increased level as compared to an untreated control and/or to a control prior to treatment, of one more cellular components is indicative of anti-PVRIG treatment efficacy:
(i) TSCM, TRM, naive, exhausted, cycling, and effector CD8 positive T cells, that express PVRIG comprise at least 0.5%, at least 1%, at least 1.5%, at least 2%, at least 2.5%, at least 3%, at least 3.5%, at least 4%, at least 4.5%, or at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, out of total CD8 cells in the biological sample; and/or
(ii) DC cells, DC1, and DC2 that express PVRL2 comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in the biological sample.

In some embodiments, the biological sample is obtained from a tumor, tumor microenvironment, and/or peripheral blood from the cancer patient.

In some embodiments, the invention provides a method for determining a cancer patient population for treatment with an anti-PVRIG antibody, the method comprising:
(a) detecting the presence of DNAM-1 and/or PVRIG in a biological sample from the cancer patient;
(b) quantitating the measurement of the level of DNAM-1 and/or PVRIG; and
(c) treating the cancer patient with an anti-PVRIG antibody when any of DNAM-1 and/or PVRIG in (a) as quantitated in step (b) are present and/or present at an increased level as compared to a control or a patient that does not have detectable levels of the cells.

In some embodiments, present and/or present at an increased level is as compared to absent.

In some embodiments, the biological sample comprises immune cells.

In some embodiments, the invention provides a method for predicting or determining the efficacy of treatment for a cancer patient with an anti-PVRIG treatment antibody, the method comprising:
(a) measuring the level of DNAM-1 and/or PVRIG;
(b) quantitating the measurement of the level of DNAM-1 and/or PVRIG;
(c) correlating the level of the DNAM-1 and/or PVRIG with the efficacy of treatment, wherein any of DNAM-1 and/or PVRIG being present and/or being present at an increased level as compared to a control or a patient that does not have detectable levels of the cells, is indicative of treatment efficacy for treatment with an anti-PVRIG treatment antibody; and
(d) optionally treating the cancer patient with an anti-PVRIG antibody when any DNAM-1 and/or PVRIG in (a) are present at an increased level as compared to a control or a patient that does not have detectable levels of the cells.

In some embodiments, present and/or being present at an increased level is as compared to the absence of expression or the absence of detectable levels.

In some embodiments, provided is a method for predicting or determining the efficacy of treatment or determining a population for treatment with an anti-PVRIG treatment antibody when any of TSCM, TRM, naive, memory, exhausted, cycling, and effector CD8 and/or CD4 T cells that express PVRIG comprise at least 0.5%, at least 1%, at least 1.5%, at least 2%, at least 2.5%, at least 3%, at least 3.5%, at least 4%, at least 4.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, of the total CD8 and/or CD4 cells in the biological sample is indicative of treatment efficacy and/or indicative for treatment with an anti-PVRIG antibody. In some embodiments, cancer patients where any of TSCM, TRM, naive, exhausted, cycling, memory, effector CD8 positive and effector CD4 positive T cells, that express PVRIG comprise at least 0.5%, at least 1%, at least 1.5%, at least 2%, at least 2.5%, at least 3%, at least 3.5%, at least 4%, at least 4.5%, or at least 5% at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, of the total CD8 and/or CD4 cells in the biological sample exhibit a higher probability to respond to the anti-PVRIG antibody treatment.

In some embodiments, provided is a method for predicting or determining the efficacy of treatment or determining a population for treatment with an anti-PVRIG treatment antibody when any of activated DC cells, DC1, and DC2 that express the DNAM-1 comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in the biological sample.

In some embodiments, cancer patients where any of activated DC cells, DC1, and DC2 that express the DNAM-1 comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in the biological sample exhibit a higher probability to respond to the anti-PVRIG antibody treatment.

In some embodiments, the presence of and/or an increased level as compared to an untreated control and/or to a control prior to treatment, of one or more cellular components is indicative of anti-PVRIG treatment efficacy:
(i) TSCM, TRM, naive, exhausted, cycling, memory, and effector CD4 positive T cells, that express PVRIG comprise at least 0.5%, at least 1%, at least 1.5%, at least 2%, at least 2.5%, at least 3%, at least 3.5%, at least 4%, at least 4.5%, or at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, out of total CD4 cells in the biological sample; and/or
(ii) DC cells, DC1, and DC2 that express DNAM-1 comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in the biological sample.

In some embodiments, the biological sample is obtained from a tumor, tumor microenvironment, and/or peripheral blood from the cancer patient.

### C. DNAM-1

In some embodiments, the level of DNAM-1 (also referred to as CD226) expression can be employed as a biomarker or companion diagnostic for use in determining treatment regimens as well as predicting or determining treatment efficacy. In some embodiments, the level of DNAM-1 expression can be employed as a biomarker or companion diagnostic for use in determining treatment regimens as well as predicting or determining treatment efficacy for treatment with an anti-PVRIG antibody. In some embodiments, the DNAM-1 status is defined based on the percentage staining for all tumor infiltrating immune cells. In some embodiments, the expression is categorized based on expression staining criteria below, in Table 3.

**Table 3: DNAM-1 Staining Criteria**

| DNAM-1 status | Staining criteria | Predicted response |
|---|---|---|
| negative | 0% | No to minimal |
| Immune Positivity (IS) | >3% immune infiltrating cells at any intensity | likely |

In some embodiments, the level of DNAM-1 expression is determined by measuring the level of DNAM-1 using immunohistochemistry. In some embodiments, the level of DNAM-1 expression is scored by a board-certified pathologist.

In some embodiments, the DNAM-1 expression level is determined using immunohistochemistry. In some embodiments, the PVRIG expression level is determined using immunohistochemistry.

In some embodiments, the DNAM-1 antibody used for the immunohistochemistry is selected from the group consisting of DNAM-1/CD226 (E8L9G) XP^{®} Rabbit mAb #66631 from Cell Signaling Technology, Mouse Anti-CD226 Recombinant Antibody (clone MM0248-1X20) from Creative Biolabs, Cluster of Differentiation 226 (CD226) Antibody abx171784 from Abbexa, and Recombinant Anti-CD226 antibody [EPR20710] (ab212077) from Abcam.

In some embodiments, the DNAM-1 expression level is categorized as strong, moderate, weak, or negative.

In some embodiments, the DNAM-1 expression is categorized as strong or moderate. In some embodiments, when DNAM-1 expression is categorized as strong or moderate, there is predicted to be a response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, the DNAM-1 expression level is categorized as 0-no signal, 1-low, 2-medium, or 3- high.

In some embodiments, the DNAM-1 expression is categorized as 2-medium or 3-high. In some embodiments, when DNAM-1 expression is categorized as 2-medium or 3-high, there is predicted to be a response to an anti-PVRIG antibody. In some embodiments, when DNAM-1 expression is categorized as 3- high, there is predicted to be an increased likelihood of response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, when the DNAM-1 expression is 0% immune cells staining, there is predicted to be no response or a minimal response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, when the DNAM-1 expression is >3% immune cellsstaining at +1 IHC score, there is predicted to be a response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, when the DNAM-1 expression is >3% immune cellsstaining at +1 IHC score, there is predicted to be a response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, when the DNAM-1 expression is >3% immune infiltrating cells at any intensity staining, there is predicted to be a response to an anti-PVRIG antibody. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients having immune cells in the tumor, tumor microenvironment, and/or peripheral blood with at least 3%, 10%, 20%, 30%, 40%, 50%, 60%, 70%, 80%, or 90% DNAM-1 expression had a higher probability to respond to an anti-PVRIG antibody treatment as compared to a cancer patient with lower DNAM-1 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having NK cells, NKT cells, gamma-delta T cells, T cells, CD8 and /or CD4 positive T cells in the tumor, tumor microenvironment, and/or peripheral blood with at least 3%, 10%, 20%, 30%, 40%, 50%, 60%, 70%, 80%, or 90% DNAM-1 expression had a higher probability to respond to an anti-PVRIG antibody treatment as compared to a cancer patient with lower DNAM-1 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3.

In some embodiments, cancer patients having CD8 and/or CD4 positive T cells in the tumor, tumor microenvironment, and/or peripheral blood with at least 3%, 10%, 20%, 30%, 40%, 50%, 60%, 70%, 80%, or 90% DNAM-1 expression had a higher probability to respond to an anti-PVRIG antibody treatment as compared to cancer patient with lower DNAM-1 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients having TSCM, TRM, naive, exhausted, cycling, memory, and/or effector CD8 and/or CD4 positive T cells in the tumor, tumor microenvironment, and/or peripheral blood with at least 3%, 10%, 20%, 30%, 40%, 50%, 60%, 70%, 80%, or 90% DNAM-1 expression had a higher probability to respond to an anti-PVRIG antibody treatment as compared to a cancer patient with lower DNAM-1 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients having dendritic cells in the tumor, tumor microenvironment, and/or peripheral blood with at least 3%, 5%, 10%, 20%, 30%, 40%, 50%, 60%, 70%, 80%, or 90% DNAM-1 expression had a higher probability to respond to an anti-PVRIG antibody treatment as compared to a cancer patient with lower DNAM-1 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients having activated dendritic cells in the tumor, tumor microenvironment, and/or peripheral blood, with at least 3%, 5%, 10%, 20%, 30%, 40%, 50%, 60%, 70%, 80%, or 90% DNAM-1 expression had a higher probability to respond to an anti-PVRIG antibody treatment as compared to a cancer patient with lower DNAM-1 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients having DC1 in the tumor, tumor microenvironment, and/or peripheral blood, with at least 3%, 5%, 10% 20%, 30%, 40%, 50%, 60%, 70%, 80%, or 90% DNAM-1 expression had a higher probability to respond to an anti-PVRIG antibody treatment as compared to a cancer patient with lower DNAM-1 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

In some embodiments, cancer patients having DC2 in the tumor, tumor microenvironment, and/or peripheral blood, with at least 3%, 5%, 10%, 20%, 30%, 40%, 50%, 60%, 70%, 80%, or 90% DNAM-1 expression, had a higher probability to respond to an anti-PVRIG antibody treatment as compared to a cancer patient with lower DNAM-1 expression. In some embodiments, the anti-PVRIG antibody comprises the antibody sequence in Figure 3, including for example, CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3 .

Cancer patients that have the presence of any of: Activated DC cells, DC1, and DC2, expressing DNAM-1, at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in a biological sample obtained from a tumor, tumor microenvironment, and/or peripheral blood, have a higher probability to respond the anti-PVRIG treatment.

### a. Early Memory CD8 T cells

There are many various names refering to the "early memory T cells" in the literature, which include but are not limited to Transitional-memory T cells, TEM (T effector memory), Pre-dysfunctional, Stem-like memory, Circulating pre-cursors, Tpex- T progenitor exhausted. *See,* for example, van der Leun, et al., Nat Rev Cancer. 20(4):218-232 (2020; found on the World Wide Web at www.ncbi.nlm.nih.gov/pmc/articles/PMC7115982/; J. Exp. Med. 215(10):2520-2535 (2018); and Gueguen et al., Sci. Immunol. 6, eabd5778 (2021); all if which are incorporated herein by reference.

In some embodiments, the early memory CD8 T cells can be employed as a biomarker or companion diagnostic for use in the determining treatment regimens as well as predicting or determining treatment efficacy. In some embodiments, the presence of early memory CD8 T cells can be employed as a biomarker or companion diagnostic for use in the determining treatment regimens as well as predicting or determining treatment efficacy for treatment with an anti-PVRIG antibody. In some embodiments, the percentage of early memory CD8 T cells can be employed as a biomarker or companion diagnostic for use in the determining treatment regimens as well as predicting or determining treatment efficacy for treatment with an anti-PVRIG antibody. In some embodiments, the presence of at least 1% of early memory CD8 T cells out of total CD8 T cells in TME is indicative for predicting or determining treatment efficacy for treatment with an anti-PVRIG antibody. In some embodiments, the early memory CD8 T cell is a CD8 T cell expressing one or more of the markers selected from the group consisting of GZMK, GZMA, GZMM, NKG7, TNFSF9, SH2D1A, EOMES, DTHD1, SLAMF7, FCRL3, CD28, CMC1, CCL4, CTS7, ITM2C, KLRG1, CRTAM, PECAM1, TCF7, CXCR3, LYAR, and HLA-DRB5. In some embodiments, the early memory CD8 T cell is a CD8 T cell expressing two, three, four, five or more of the markers selected from the group consisting of GZMK, GZMA, GZMM, NKG7, TNFSF9, SH2D1A, EOMES, DTHD1, SLAMF7, FCRL3, CD28, CMC1, CCL4, CTS7, ITM2C, KLRG1, CRTAM, PECAM1, TCF7, CXCR3, LYAR, and HLA-DRB5. In some embodiments, the early memory CD8 T cell is a CD8 T cell expressing CXCR3. In some embodiments, the CXCR3 is localized in the vicinity of activated DC cells expressing such as CXCL10 expressing activated DC cells.

### D. Cancer Types

In some embodiments, the PVRL2, DNAM-1, and/or PVRIG biomarkers can be used for determining, modifying, altering, and/or predicting the outcome for treatment of vascularized tumors. In some embodiments, the PVRL2 biomarker can be used for determining, modifying, altering, and/or predicting the outcome for treatment of vascularized tumors. In some embodiments, the DNAM-1 biomarker can be used for determining, modifying, altering, and/or predicting the outcome for treatment of vascularized tumors. In some embodiments, the PVRL2, DNAM-1, and/or PVRIG biomarkers can be used for determining, modifying, altering, and/or predicting the outcome for treatment of cancer including carcinoma, lymphoma, sarcoma, and/or leukemia. In some embodiments, the PVRL2 biomarker can be used for determining, modifying, altering, and/or predicting the outcome for treatment of cancer including carcinoma, lymphoma, sarcoma, and/or leukemia. In some embodiments, the DNAM-1 biomarker can be used for determining, modifying, altering, and/or predicting the outcome for treatment of cancer including carcinoma, lymphoma, sarcoma, and/or leukemia. In some embodiments, the PVRL2, DNAM-1, and/or PVRIG biomarkers can be used for determining, modifying, altering, and/or predicting the outcome for cancer including melanoma, non-melanoma skin cancer (squamous and basal cell carcinoma), mesothelioma, squamous cell cancer, lung cancer (including small-cell lung cancer, non-small cell lung cancer, soft-tissue sarcoma, Kaposi's sarcoma, adenocarcinoma of the lung, squamous carcinoma of the lung, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC; including refractory MSS colorectal; MSS = microsatellite stable status), primary peritoneal cancer, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia), Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma, post-transplant lymphoproliferative disorder (PTLD), abnormal vascular proliferation associated with phakomatoses, Meigs' syndrome, Merkel Cells cancer, MSI-high cancer, KRAS mutant tumors, adult T-cell leukemia/lymphoma, adenoid cystic cancer (including adenoid cystic carcinoma), malignant melanoma, pancreatic cancer, pancreatic adenocarcinoma, ovarian cancer (including ovarian carcinoma), pleural mesothelioma, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, cervical squamous cell carcinoma (cervical SCC), anal squamous cell carcinoma (anal SCC), neuroendocrine lung carcinoma, carcinoma of unknown primary, gallbladder cancer, malignant melanoma, pleural mesothelioma, Myelodysplastic syndromes (MDS), HNSCC, PD1 refractory or relapsing cancer, gastroesophageal junction cancer, gastric cancer, and/or fallopian tube cancer.

In some embodiments, the PVRL2, DNAM-1, and/or PVRIG biomarkers can be used for determining, modifying, altering, and/or predicting the outcome for a cancer selected from the group consisting of prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, and/or fallopian tube cancer.

In some embodiments, the PVRL2 biomarker can be used for determining, modifying, altering, and/or predicting the outcome for a cancer selected from the group consisting of prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, and/or fallopian tube cancer.

In some embodiments, the PVRIG biomarker can be used for determining, modifying, altering, and/or predicting the outcome for a cancer selected from the group consisting of prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, and/or fallopian tube cancer), HNSCC, PD1 refractory or relapsing cancer, gastroesophageal junction cancer, gastric cancer, and/or fallopian tube cancer.

In some embodiments, the DNAM-1 biomarker can be used for determining, modifying, altering, and/or predicting the outcome for a cancer selected from the group consisting of prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, and/or fallopian tube cancer.

### VII. FORMULATIONS OF ANTI-PVRIG ANTIBODIES

The anti-PVRIG antibodies and/or antigen binding portions thereof compositions (e.g., anti-PVRIG antibodies including those with CDRs identical to those shown in Figure 3) can be formulated into pharmaceutical compositions comprising a carrier suitable for the desired delivery method. Suitable carriers include any material that when combined with the therapeutic composition retains the anti-tumor function of the therapeutic composition and is generally non-reactive with the patient's immune system. Examples include, but are not limited to, any of a number of standard pharmaceutical carriers such as sterile phosphate buffered saline solutions, bacteriostatic water, and the like (see, generally, Remington's Pharmaceutical Sciences 16th Edition, A. Osal., Ed., 1980). Acceptable carriers, excipients, or stabilizers are nontoxic to recipients at the dosages and concentrations employed, and include buffers such as phosphate, citrate, acetate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl orbenzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; sweeteners and other flavoring agents; fillers such as microcrystalline cellulose, lactose, corn and other starches; binding agents; additives; coloring agents; salt-forming counter-ions such as sodium; metal complexes (e.g., Zn-protein complexes); and/or non-ionic surfactants such as TWEEN^{™}, PLURONICS^{™}, or polyethylene glycol (PEG).

In a preferred embodiment, the pharmaceutical composition that comprises anti-PVRIG antibodies including those with CDRs identical to those shown in Figure 3) of the invention may be in a water-soluble form, such as being present as pharmaceutically acceptable salts, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic nontoxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine. The formulations to be used for in vivo administration are preferrably sterile. This is readily accomplished by filtration through sterile filtration membranes or other methods.

As used herein, the term "activity" refers to a functional activity or activities of anti-PVRIG antibodies and/or antigen binding portions thereof. Functional activities include, but are not limited to, biological activity and/or binding affinity.

As used herein, the term "stability" is used in a structural context, e.g., relating to the structural integrity of an anti-PVRIG antibody and/or antigen binding portion thereof, or in a functional context, *e*.*g*., relating to a an anti-PVRIG antibody and/or antigen binding portion thereof's ability to retain its function and/or activity over time (*e*.*g*., including anti-PVRIG antibody and/or antigen binding portion thereof stability or anti-PVRIG antibody and/or antigen binding portion thereof formulation stability, wherein the anti-PVRIG antibody includes those with CDRs identical to those shown in Figure 3). As will be appreciated, the an anti-PVRIG antibody and/or antigen binding portion thereof under discussion may be contained within a formulation in accordance with the methods and compositions described herein, and the stability of that protein refers to its stability in that formulation. In some embodiments, the stability of an anti-PVRIG antibody and/or antigen binding portion thereof composition is determined by measuring the binding activity of the composition, including for example, using the assays described in the application and figures provided herewith, as well as other applicable assays known in the art. In some embodiments, the stability of an anti-PVRIG antibody and/or antigen binding portion thereof composition is formulated with sugar, sugar alcohol, and/or non-ionic surfactant, as described herein, is compared to an anti-PVRIG antibody and/or antigen binding portion thereof composition formulated without the at least one amino acid, salt, and/or non-ionic surfactant and/or with a different combination of components. In some embodiments, the formulation does not comprise a sugar and/or sugar alcohol.

As used herein, a "storage stable" aqueous an anti-PVRIG antibody and/or antigen binding portion thereof composition refers to a an anti-PVRIG antibody and/or antigen binding portion thereof comprising solution that has been formulated to increase the stability of the protein in solution, for example by at least 10%, over a given storage time. In the context of the present disclosure, an anti-PVRIG antibody and/or antigen binding portion thereof can be made "storage stable" by the addition of at least one amino acid, salt, or non-ionic surfactant as a stabilizing agent. In some embodiments, the stability of the an anti-PVRIG antibody and/or antigen binding portion thereof in any given formulation can be measured, for example, by monitoring the formation of aggregates, loss of bulk binding activity, or formation of degradation products, over a period of time. The absolute stability of a formulation, and the stabilizing effects of the sugar, sugar alcohol, or non-ionic surfactant, will vary dependent upon the particular composition being stabilized. In one embodiment, the stability of an anti-PVRIG antibody and/or antigen binding portion thereof composition is determined by measuring the anti-PVRIG antibody and/or antigen binding portion thereof binding activity of the composition. For example, by using an ELISA or other binding activity assay. In one embodiment, the stability of an anti-PVRIG antibody and/or antigen binding portion thereof composition formulated with sugar, sugar alcohol, and/or non-ionic surfactant, as described herein, is compared to an anti-PVRIG antibody and/or antigen binding portion thereof composition formulated without the a least one amino acid, salt, and/or non-ionic surfactant and/or with a different combination of components. In some embodiments, the formulation does not comprise a sugar and/or sugar alcohol.

As used herein, "shelf-life" refers to the period of time a formulation maintains a predetermined level of stability at a predetermined temperature. In particular embodiments, the predetermined temperature refers to frozen *(e.g.,* -80°C, -25°C, 0°C), refrigerated (*e*.*g*., 0° to 10°C), or room temperature (*e*.*g*., 18°C to 32° C) storage.

As used herein, the term "time of stability" refers to the length of time a formulation is considered stable. For example, the time of stability for a formulation may refer to the length of time for which the level of protein aggregation and/or degradation in the formulation remains below a certain threshold (*e*.*g*., 1 %, 2%, 3%, 4%, 5%, 6%, 7%, 8%, 9%, 10%, 1 1 %, 12%, 13%, 14%, 15%, 16%, 17%, 18%, 19%, 20%, etc.), and/or the length of time a formulation maintains biological activity above a certain threshold (*e*.*g*., 100%, 95%, 90%, 85%, 80%, 75%, 70%, 65%, 60%, 55%, 50%, etc.) of the amount of activity (including, for example, binding activity) present in the formulation at the start of the storage period.

In the context of the present disclosure, a storage stable aqueous composition of a an anti-PVRIG antibody and/or antigen binding portion thereof formulated with a sugar, sugar alcohol, and/or non-ionic surfactant will have a longer time of stability than a composition of the same an anti-PVRIG antibody and/or antigen binding portion thereof formulated without the at least one amino acid, salt, and/or non-ionic surfactant. In some embodiments, a storage stable aqueous composition of an anti-PVRIG antibody and/or antigen binding portion thereof, will have a time of stability that is, for example, at least 10% greater than the time of stability for the an anti-PVRIG antibody and/or antigen binding portion thereof composition formulated in the absence of the at least one amino acid, salt, and/or non-ionic surfactant, or at least 15%, 20%, 25%, 30%, 35%, 40%, 45%, 50%, 55%, 60%, 65%, 70%, 75%, 80%, 85%, 90%, 95%, 100%, 1 10%, 120%, 130%, 140%, 150%, 160%, 170%, 180%, 190% greater, or at least 2 times greater, or at least 2.5 times, 3.0 times, 3.5 times, 4.0 times, 4.5 times, 5.0 times, 5.5 times, 6.0 times, 6.5 times, 7.0 times, 7.5 times, 8.0 times, 8.5 times, 9.0 times, 9.5 times, 10 times, or more times greater than the time of stability for the an anti-PVRIG antibody and/or antigen binding portion thereof composition formulated in the absence of the at least amino acid, salt, and/or non-ionic surfactant.

As used herein, "BDS" refers to "Bulk Drug Substance."

### A. Stabilized Liquid Formulations

In some embodiments, the present disclosure provides stabilized aqueous formulations of an anti-PVRIG antibody and/or antigen binding portion thereof *(e.g.,* anti-PVRIG antibodies including those with CDRs identical to those shown in Figure 3). The following embodiments are based in part on the discovery that inclusion of at least one amino acid, salt, and/or non-ionic surfactant stabilizes the liquid anti-PVRIG antibody and/or antigen binding portion thereof compositions, as compared to compositions lacking the at least one amino acid, salt, and/or non-ionic surfactant. In some embodiments, the formulation does not comprise a sugar and/or sugar alcohol.

As will be recognized by one of skill in the art, an anti-PVRIG antibody and/or antigen binding portion thereof formulated according to the embodiments provided herein may contain, in addition to the components explicitly disclosed, counter ions contributed by the inclusion of solution components or pH modifying agents, for example, sodium or potassium contributed from an acetate salt, sodium hydroxide, or potassium hydroxide or chloride contributed by calcium chloride or hydrochloric acid. In the context of the present disclosure, a storage stable an anti-PVRIG antibody and/or antigen binding portion thereof composition consisting of or consisting essentially of a given formulation may further comprise one or more counter ion, as necessitated by the formulation process at a particular pH.

In one embodiment, a storage stable anti-PVRIG antibody and/or antigen binding portion provided herein will be stabilized at refrigerated temperature (i.e., between 2°C and 10°C) for a period of time. For example, in one embodiment, a stable liquid pharmaceutical formulations comprising an anti-PVRIG antibody or antigen binding fragment thereof will be stable when stored at refrigerated temperature for at least 4 days. In other embodiments, the anti-PVRIG antibody and/or antigen binding portion composition will be stable at refrigerated temperature for at least 1, 2, 3, 4, 5, 6, 7, 8, 9, 10, 11, 12, 13, 14, 21, 28, or more days. In some embodiments, the anti-PVRIG antibody and/or antigen binding portion composition will be stable for at least 1 week, 2 weeks, 3 weeks, 4 weeks, 5 weeks, 6 weeks, 7 weeks, 8 weeks, 9 weeks, 10 weeks, or more. In some embodiments, the anti-PVRIG antibody and/or antigen binding portion composition will be stable for at least 1 month. In some embodiments, the composition will be stable for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or more months. In some embodiments, the anti-PVRIG antibody and/or antigen binding portion composition will be stable for an extended period of time when stored at a temperature between 2°C and 8°C.

In one embodiment, a stable liquid pharmaceutical formulations comprising an anti-PVRIG antibody or antigen binding fragment thereof provided herein will be stabilized at room temperature (*i*.*e*., between 18°C and 32°C) for a period of time. For example, in one embodiment, a stable liquid pharmaceutical formulations comprising an anti-PVRIG antibody or antigen binding fragment thereof will be stable when stored at room temperature for at least 4 days. In some embodiments, the anti-PVRIG antibody and/or antigen binding portion composition will be stable at room temperature for at least 1, 2, 3, 4, 5, 6, 7, 8, 9, 10, 11, 12, 13, 14, 21, 28, or more days. In some embodiments, the anti-PVRIG antibody and/or antigen binding portion composition will be stable for at least 1 week, 2 weeks, 3 weeks, 4 weeks, 5 weeks, 6 weeks, or more. In some embodiments, the anti-PVRIG antibody and/or antigen binding portion composition will be stable for at least 1 month. In yet other embodiments, the composition will be stable for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or more months. In some embodiments, room temperature refers to between 20°C and 30°C, between 21°C and 29°C, between 22°C and 28°C, between 23°C and 27°C, between 24°C and 26°C, or about 25°C. In some embodiments, the anti-PVRIG antibody and/or antigen binding portion composition will be stable for an extended period of time when stored at a temperature between 20°C and 25°C. In some embodiments, the anti-PVRIG antibody and/or antigen binding portion composition will be stable for an extended period of time when stored at a temperature of about 25°C.

In one embodiment, a storage stable anti-PVRIG antibody and/or antigen binding portion provided herein will be stabilized at elevated temperature (*i*.*e*., between 32°C and 42°C) for a period of time. For example, in one embodiment, a stable liquid pharmaceutical formulations comprising an anti-PVRIG antibody or antigen binding fragment thereof will be stable when stored at elevated temperature for at least 4 days. In some embodiments, the anti-PVRIG antibody and/or antigen binding portion composition will be stable at elevated temperature for at least 1, 2, 3, 4, 5, 6, 7, 8, 9, 10, 11, 12, 13, 14, 21, 28, or more days. In some embodiments, the anti-PVRIG antibody and/or antigen binding portion composition will be stable for at least 1 week, 2 weeks, 3 weeks, 4 weeks, 5 weeks, or more. In some embodiments, the anti-PVRIG antibody and/or antigen binding portion composition will be stable for at least 1 month. In yet other embodiments, the anti-PVRIG antibody and/or antigen binding portion composition will be stable for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, or more months. In some embodiments, the anti-PVRIG antibody and/or antigen binding portion composition will be stable for an extended period of time when stored at a temperature between 35°C and 40°C.

In one embodiment, a stored anti-PVRIG antibody and/or antigen binding portion composition is considered storage stable as long as the composition maintains at least 40% of the antibody binding activity present at the start of the storage period (*e*.*g*., at time = 0). In another embodiment, a stored composition is considered stable as long as the composition maintains at least 45%, 50%, 55%, 60%, 65%, 70%, 75%, 80%, 85%, 90%, 95% or more of the antibody binding activity present at the start of the storage period (*e*.*g*., at time = 0). In one embodiment, antibody binding activity is measure using any assay known in the art.

In some embodiments, an anti-PVRIG antibody and/or antigen binding portion composition is considered to have been stabilized by the addition of a stabilizing agent (*e*.*g*., at least one amino acid, salt, and/or non-ionic surfactant) when the anti-PVRIG antibody and/or antigen binding portion composition contains at least 10% more antibody binding activity after storage for a period of time, as compared to an anti-PVRIG antibody and/or antigen binding portion composition not containing the stabilizing agent or containing a lower amount of the stabilizing agent. In other embodiments, an anti-PVRIG antibody and/or antigen binding portion composition is considered to have been stabilized by the addition of a stabilizing agent (*e*.*g*., at least one amino acid, salt, and/or non-ionic surfactant) when the composition contains at least 15%, 20%, 25%, 30%, 35%, 40%, 45%, 50%, 55%, 60%, 65%, 70%, 75%, 80%, 85%, 90%, 95%, 100%, or a greater percentage more anti-PVRIG antibody and/or antigen binding portion activity after storage for a period of time, as compared to an anti-PVRIG antibody and/or antigen binding portion composition not containing the stabilizing agent or containing a lower amount of the stabilizing agent.

In one embodiment, a stored anti-PVRIG antibody and/or antigen binding portion composition is considered stable as long as the percentage of anti-PVRIG antibody and/or antigen binding portion present in an aggregated state remains no more than 50%. In some embodiments, a stored anti-PVRIG antibody and/or antigen binding portion thereof composition is considered stable as long as the percentage of the anti-PVRIG antibody and/or antigen binding portion thereof present in an aggregated state remains no more than 45%, 40%, 35%, 30%, 25%, 24%, 23%, 22%, 21 %, 20%, 19%, 18%, 17%, 16%, 15%, 14%, 13%, 12%, 1 1%, 10%, 9%, 8%, 7%, 6%, 5%, 4%, 3%, 2%, 1 %, or less.

In some embodiments, an anti-PVRIG antibody and/or antigen binding portion composition is considered to have been stabilized by the addition of a stabilizing agent (anti-PVRIG antibody and/or antigen binding portion composition, at least one amino acid, salt, and/or non-ionic surfactant) when the composition contains at least 10% less anti-PVRIG antibody and/or antigen binding portion present in an aggregated state after storage for a period of time, as compared to an anti-PVRIG antibody and/or antigen binding portion composition not containing the stabilizing agent or containing a lower amount of the stabilizing agent. In other embodiments, an anti-PVRIG antibody and/or antigen binding portion composition is considered to have been stabilized by the addition of a stabilizing agent (*e*.*g*., at least one amino acid, salt, and/or non-ionic surfactant) when the composition contains at least 15%, 20%, 25%, 30%, 35%, 40%, 45%, 50%, 55%, 60%, 65%, 70%, 75%, 80%, 85%, 90%, 95%, 100%, or a greater percentage less anti-PVRIG antibody and/or antigen binding portion present in an aggregated state after storage for a period of time, as compared to an anti-PVRIG antibody and/or antigen binding portion composition not containing the stabilizing agent or containing a lower amount of the stabilizing agent

In some embodiments, a stored anti-PVRIG antibody and/or antigen binding portion composition is considered stable as long as the composition maintains at least 40% of the starting binding activity (*e*.*g*., at time = 0) after being subjected to mechanical stress. In another embodiment, a stored composition is considered stable as long as the composition maintains 45%, 50%, 55%, 60%, 65%, 70%, 75%, 80%, 85%, 90%, 95% or more of the starting binding activity (*e*.*g*., at time = 0) after being subjected to mechanical stress. In some embodiments, the mechanical stress is agitation (*e*.*g*., shaking).

In some embodiments, an anti-PVRIG antibody and/or antigen binding portion composition is considered to have been stabilized by the addition of a stabilizing agent (*e*.*g*., at least one amino acid, salt, or non-ionic surfactant) when the anti-PVRIG antibody and/or antigen binding portion composition contains at least 10% more binding activity after being subjected to mechanical stress, as compared to an anti-PVRIG antibody and/or antigen binding portion composition not containing the stabilizing agent or containing a lower amount of the stabilizing agent. In other embodiments, an anti-PVRIG antibody and/or antigen binding portion composition is considered to have been stabilized by the addition of a stabilizing agent (*e*.*g*., a sugar, sugar alcohol, or non-ionic surfactant) when the composition contains at least 15%, 20%, 25%, 30%, 35%, 40%, 45%, 50%, 55%, 60%, 65%, 70%, 75%, 80%, 85%, 90%, 95%, 100%, or a greater percentage more antibody activity after being subjected to mechanical stress, as compared to an anti-PVRIG antibody and/or antigen binding portion composition not containing the stabilizing agent or containing a lower amount of the stabilizing agent. In a specific embodiment, the mechanical stress is agitation (*e*.*g*., shaking).

In some embodiments, a stored anti-PVRIG antibody and/or antigen binding portion composition is considered stable as long as the percentage of anti-PVRIG antibody and/or antigen binding portion present in an aggregated state remains no more than 50% after being subjected to mechanical stress. In other embodiments, a stored anti-PVRIG antibody and/or antigen binding portion composition is considered stable as long as the percentage of anti-PVRIG antibody and/or antigen binding portion present in an aggregated state remains no more than 45%, 40%, 35%, 30%, 25%, 24%, 23%, 22%, 21 %, 20%, 19%, 18%, 17%, 16%, 15%, 14%, 13%, 12%, 11%, 10%, 9%, 8%, 7%, 6%, 5%, 4%, 3%, 2%, 1%, or less after being subjected to mechanical stress. In some embodiments, the mechanical stress is agitation (*e*.*g*., shaking).

In some embodiments, an anti-PVRIG antibody and/or antigen binding portion composition is considered to have been stabilized by the addition of a stabilizing agent (*e*.*g*., at least one amino acid, salt, or non-ionic surfactant) when the composition contains at least 10% less anti-PVRIG antibody and/or antigen binding portion present in an aggregated state after being subjected to mechanical stress, as compared to an anti-PVRIG antibody and/or antigen binding portion composition not containing the stabilizing agent or containing a lower amount of the stabilizing agent. In some embodiments, an anti-PVRIG antibody and/or antigen binding portion composition is considered to have been stabilized by the addition of a stabilizing agent (*e*.*g*., at least one amino acid, salt, or non-ionic surfactant) when the composition contains at least 15%, 20%, 25%, 30%, 35%, 40%, 45%, 50%, 55%, 60%, 65%, 70%, 75%, 80%, 85%, 90%, 95%, 100%, or a greater percentage less anti-PVRIG antibody and/or antigen binding portion present in an aggregated state after being subjected to mechanical stress, as compared to an anti-PVRIG antibody and/or antigen binding portion composition not containing the stabilizing agent or containing a lower amount of the stabilizing agent. In a specific embodiment, the mechanical stress is agitation (*e*.*g*., shaking).

In some embodiments, the highly stabilized formulations of the invention have a shelf life of at least 6 months. As will be appreciated, this shelf life may be at frozen temperatures (*i.e.*, -80°C, -25°C, 0°C), refrigerated (0°C to 10°C), or room temperature (20°C to 32°C) in liquid or lyophilized form. I n further aspects, the highly stabilized formulations of the invention have a shelf life of at least 12, 18, 24, 30, 36, 42, 48, 54, or 60 months.

In some embodiments, shelf life is determined by a percent activity remaining after storage at any of the above temperatures for any of the above periods of time. In some embodiments, shelf life means that the formulation retains at least 10%, 15%, 20%, 25%, 30%, 35%, 40%, 45%, 50%, 55%, 60%, 65%, 70%, 75%, 80%, 85%, 90%, 95%, 96%, 97%, 98%, 99%, 100% of antibody activity as measured by any of the assays described herein or known in the art as compared to activity prior to storage for any of the above amounts of time at any of the above temperatures.

In some embodiments, the present invention provides a stable liquid pharmaceutical formulation of an anti-PVRIG antibody comprising:
(a) an anti-PVRIG antibody, wherein the anti-PVRIG antibody comprises an antibody with CDRs identical to those shown in Figure 3;
(b) 25 mM histidine;
(c) 60 mM NaCl;
(d) 100 mM L-Arginine, and
(e) 0.01% w/v polysorbate 80,
wherein the composition has a pH from 5.5 to 7.0.

In some embodiments, the anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL. In some embodiments, the anti-PVRIG antibody is at a concentration of from 10 mg/mL to 40 mg/mL. In some embodiments, the anti-PVRIG antibody is at a concentration of from 15 mg/mL to 40 mg/mL. In some embodiments, the anti-PVRIG antibody is at a concentration of from 15 mg/mL to 30 mg/mL. In some embodiments, the anti-PVRIG antibody is at a concentration of from 10 mg/mL to 25 mg/mL. In some embodiments, the anti-PVRIG antibody is at a concentration of from 15 mg/mL to 25 mg/mL. In some embodiments, the anti-PVRIG antibody is at a concentration of from 10 mg/mL to 25 mg/mL. In some embodiments, the anti-PVRIG antibody is at a concentration of from 15 mg/mL to 25 mg/mL. In some embodiments, the anti-PVRIG antibody is at a concentration of from 20 mg/mL to 25 mg/mL. In some embodiments, the anti-PVRIG antibody is at a concentration of about 20 mg/mL.

In some embodiments, the present invention provides a stable liquid pharmaceutical formulation of an anti-PVRIG antibody comprising:
(a) an anti-PVRIG antibody, wherein the anti-PVRIG antibody comprises an antibody with CDRs identical to those shown in Figure 3;
(b) from 10 mM to 100 mM histidine;
(c) from 30 mM to 100 mM NaCl;
(d) from 20 mM to 150 mM L-Arginine, and
(e) from 0.005% to 0.1% w/v polysorbate 80
wherein the composition has a pH from 5.5 to 7.0.

### B. Amino acids

In some embodiments, the present invention provides a stable liquid pharmaceutical formulation of an anti-PVRIG antibody or antigen binding fragment thereof (*e*.*g*., anti-PVRIG antibodies including those with CDRs identical to those shown in Figure 3) comprising at least one amino acid. In some embodiments, the at least one amino acid is histidine. In some embodiments, the at least one amino acid is arginine. In some embodiments, the present invention provides a stable liquid pharmaceutical formulation of an anti-PVRIG antibody or antigen binding fragment thereof comprising at least two amino acids. In some embodiments, the at least two amino acids are histidine and arginine.

In some embodiments, the pharmaceutical formulation comprises from 10 mM to 80 mM histidine, from 15 mM to 70 mM histidine, from 20 mM to 60 mM histidine, from 20 mM to 50 mM histidine, or from 20 mM to 30 mM histidine. In some embodiments, the pharmaceutical formulation comprises from 10 mM to 80 mM histidine. In some embodiments, the pharmaceutical formulation comprises from 15 mM to 70 mM histidine. In some embodiments, the pharmaceutical formulation comprises from 20 mM to 60 mM histidine. In some embodiments, the pharmaceutical formulation comprises from 20 mM to 50 mM histidine. In some embodiments, the pharmaceutical formulation comprises from 20 mM to 30 mM histidine. In some embodiments, the pharmaceutical formulation comprises about 25 mM histidine.

In some embodiments, the pharmaceutical formulation comprises from 10 mM to 80 mM histidine. In some embodiments, the pharmaceutical formulation comprises from 15 mM to 70 mM histidine. In some embodiments, the pharmaceutical formulation comprises from 20 mM to 60 mM histidine. In some embodiments, the pharmaceutical formulation comprises from 20 mM to 50 mM histidine. In some embodiments, the pharmaceutical formulation comprises from 20 mM to 30 mM histidine. In some embodiments, the pharmaceutical formulation comprises about 25 mM histidine.

In some embodiments, the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine. In some embodiments, the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine.

In some embodiments, the pharmaceutical formulation comprises from 20 mM to 140 mM L-arginine. In some embodiments, the pharmaceutical formulation comprises from 30 mM to 140 mM L-arginine. In some embodiments, the pharmaceutical formulation comprises from 40 mM to 130 mM L-arginine. In some embodiments, the pharmaceutical formulation comprises from 50 mM to 120 mM L-arginine. In some embodiments, the pharmaceutical formulation comprises from 60 mM to 110 mM L-arginine. In some embodiments, the pharmaceutical formulation comprises from 70 mM to 110 mM L-arginine. In some embodiments, the pharmaceutical formulation comprises from 80 mM to 110 mM L-arginine. In some embodiments, the pharmaceutical formulation comprises from 90 mM to 110 mM L-arginine. In some embodiments, the pharmaceutical formulation comprises about 100 mM L-arginine.

### C. Sugar and/or sugar alcohol

In some embodiments, the present invention provides a stable liquid pharmaceutical formulation of an anti-PVRIG antibody or antigen binding fragment thereof (*e*.*g*., anti-PVRIG antibodies including those with CDRs identical to those shown in Figure 3) comprising no sugar and/or sugar alcohol. In some embodiments, the present invention provides a stable liquid pharmaceutical formulation of an anti-PVRIG antibody or antigen binding fragment thereof (*e*.*g*., anti-PVRIG antibodies including those with CDRs identical to those shown in Figure 3) comprising no sugar. In some embodiments, the present invention provides a stable liquid pharmaceutical formulation of an anti-PVRIG antibody or antigen binding fragment thereof (*e*.*g*., anti-PVRIG antibodies including those with CDRs identical to those shown in Figure 3) comprising no sugar alcohol.

In some embodiments, the present invention provides a stable liquid pharmaceutical formulation of an anti-PVRIG antibody or antigen binding fragment thereof comprising a sugar and/or sugar alcohol. In some embodiments, the sugar is trehalose or sucrose. In some embodiments, the sugar is trehalose. In some embodiments, the sugar is sucrose. In some embodiments, the sugar is only one of trehalose or sucrose but not both.

In some embodiments, the sugar is in an amount of from about 0.5% to 10%, 1 % to 9.5%, 1.5% to 9%, 2.0% to 8.5%, 2.5% to 8%, 3.0% to 7.5%, 3.5% to 7%, 4.0% to 6.5%, 4.5% to 6%, and/or 4.5% to 5.5%. In some embodiments, the sugar is in an amount of from about 0.5% to 10%. In some embodiments, the sugar is in an amount of from about 1 % to 9.5%. In some embodiments, the sugar is in an amount of from about 1.5% to 9%. In some embodiments, the sugar is in an amount of from about 2.0% to 8.5%. In some embodiments, the sugar is in an amount of from about 2.5% to 8%. In some embodiments, the sugar is in an amount of from about 3.0% to 7.5%. In some embodiments, the sugar is in an amount of from about 3.5% to 7%. In some embodiments, the sugar is in an amount of from about 4.0% to 6.5%. In some embodiments, the sugar is in an amount of from about 4.5% to 6%. In some embodiments, the sugar is in an amount of from about 4.5% to 5.5%. In some embodiments, the sugar is in an amount of about 5%

### D. Non-Ionic Surfactants

In some embodiments, the present invention provides a stable liquid pharmaceutical formulation of an anti-PVRIG antibody or antigen binding fragment thereof (*e*.*g*., anti-PVRIG antibodies including those with CDRs identical to those shown in Figure 3) comprising a non-ionic surfactant. In some embodiments, the storage stable compositions of an anti-PVRIG antibody or antigen binding fragment comprise a non-ionic surfactant selected from a non-ionic water soluble monoglyceride, a non-ionic water soluble diglyceride, a non-ionic water soluble triglyceride, a non-ionic water soluble monofatty acid esters of polyethyelene glycol, a non-ionic water soluble difatty acid esters of polyethyelene glycol, a non-ionic water soluble sorbitan fatty acid ester, a non-ionic polyglycolyzed glyceride, a non-ionic water soluble triblock copolymer, and a combination thereof. In some embodiments, the non-ionic surfactant is polysorbate 80 (polyoxyethylene (20) sorbitan monooleate).

In some embodiments, the stable liquid pharmaceutical formulation comprises from 0.006% to 0.1% w/v polysorbate 80, from 0.007% to 0.09% w/v polysorbate 80, from 0.008% to 0.08% w/v polysorbate 80, from 0.009% to 0.09% w/v polysorbate 80, from 0.01% to 0.08% w/v polysorbate 80, from 0.01% to 0.07% w/v polysorbate 80, from 0.01% to 0.07% w/v polysorbate 80, or from 0.01% to 0.06% w/v polysorbate 80, or from 0.009% to 0.05% w/v polysorbate 80. In some embodiments, the stable liquid pharmaceutical formulation comprises from 0.006% to 0.1% w/v polysorbate 80. In some embodiments, the stable liquid pharmaceutical formulation comprises from 0.007% to 0.09% w/v polysorbate 80. In some embodiments, the stable liquid pharmaceutical formulation comprises from 0.008% to 0.08% w/v polysorbate 80. In some embodiments, the stable liquid pharmaceutical formulation comprises from 0.009% to 0.09% w/v polysorbate 80. In some embodiments, the stable liquid pharmaceutical formulation comprises from 0.01% to 0.08% w/v polysorbate 80. In some embodiments, the stable liquid pharmaceutical formulation comprises from 0.01% to 0.07% w/v polysorbate 80. In some embodiments, the stable liquid pharmaceutical formulation comprises from 0.01% to 0.07% w/v polysorbate 80. In some embodiments, the stable liquid pharmaceutical formulation comprises from 0.01% to 0.06% w/v polysorbate 80. In some embodiments, the stable liquid pharmaceutical formulation comprises from 0.009% to 0.05% w/v polysorbate 80. In some embodiments, the stable liquid pharmaceutical formulation comprises about 0.01% polysorbate 80.

### E. Pharmaceutically Acceptable Salts

In some embodiments, the present invention provides a stable liquid pharmaceutical formulation of an anti-PVRIG antibody or antigen binding fragment thereof (*e*.*g*., anti-PVRIG antibodies including those with CDRs identical to those shown in Figure 3) comprising a salt, for example, a pharmaceutically acceptable salt.

In some embodiments, the stable liquid pharmaceutical formulation comprising an anti-PVRIG antibody or antigen binding fragment thereof provided herein include a pharmaceutically acceptable salt at a concentration tolerated by the an anti-PVRIG antibody or antigen binding fragment thereof during storage. In some embodiments, the pharmaceutically acceptable salt is a chloride salt. In some embodiments, the pharmaceutically acceptable salt is a monovalent chloride salt. In a more specific embodiment, the pharmaceutically acceptable salt is sodium chloride, potassium chloride, or a combination thereof.

In some embodiments, the stable liquid pharmaceutical formulation comprises from 30 mM to 100 mM NaCl, from 30 mM to 90 mM NaCl, from 40 mM to 80 mM NaCl, from 30 mM to 70 mM histidine, or from 45 mM to 70 mM NaCl.

In some embodiments, the stable liquid pharmaceutical formulation comprises from 30 mM to 100 mM NaCl. In some embodiments, the stable liquid pharmaceutical formulation comprises from 30 mM to 90 mM NaCl. In some embodiments, the stable liquid pharmaceutical formulation comprises from 40 mM to 80 mM NaCl. In some embodiments, the stable liquid pharmaceutical formulation comprises from 30 mM to 70 mM histidine. In some embodiments, the stable liquid pharmaceutical formulation comprises or from 45 mM to 70 mM NaCl. In some embodiments, pharmaceutical formulation comprises about 60 mM NaCl.

### F. Buffering Agents

In some embodiments, the present invention provides a stable liquid pharmaceutical formulation of an anti-PVRIG antibody or antigen binding fragment thereof (*e*.*g*., anti-PVRIG antibodies including those with CDRs identical to those shown in Figure 3) that is buffered at a physiologically acceptable pH. In some embodiments, the physiologically acceptable pH is from about 6.0 to about 7.0.

In some embodiments, stable liquid pharmaceutical formulation of an anti-PVRIG antibody or antigen binding fragment thereof has a pH of from 6 to 7.0. In some embodiments, stable liquid pharmaceutical formulation of an anti-PVRIG antibody or antigen binding fragment thereof has a pH of 6, 6.1, 6.2, 6.3, 6.4, 6.5, 6.6, 6.7, 6.8, 6.9, or 7.0. In some embodiments, the pH is from 6.1 to 6.9. In some embodiments, the pH is from 6.2 to 6.9. In some embodiments, the pH is from 6.3 to 6.8. In some embodiments, the pH is from 6.3 to 6.7. In some embodiments, the pH is from 6.4 to 6.8. In some embodiments, the pH is from 6.5 to 6.8. In some embodiments, the pH is from 6.6 to 6.8. In some embodiments, the pH is 6.3, 6.4, 6.5, 6.6, or 6.7. In some embodiments, the pH is 6.5 +/- 0.2.

### G. Methods for Diluting Aqueous Compositions

[In some embodiments, the method includes adding a dilution buffer, to form a diluted stable liquid pharmaceutical formulation comprising an anti-PVRIG antibody or antigen binding fragment thereof (*e*.*g*., anti-PVRIG antibodies including those with CDRs identical to those shown in Figure 3). In some embodiments, the dilution buffer is added at a ratio of from 1:1 (dilution buffer:formulation) to 1000:1 (dilution buffer:formulation). In another embodiment, the dilution buffer is added at a ratio of from 1:1 dilution buffer:formulation) to 500:1 (dilution buffer:formulation). In another embodiment, the dilution buffer is added at a ratio of from 1:1 (dilution buffer:formulation) to 250:1 (dilution buffer:formulation). In another embodiment, the dilution buffer is added at a ratio of from 1:1 (dilution buffer:formulation) to 200:1 (dilution buffer:formulation). In another embodiment, the dilution buffer is added at a ratio of from 1:1 (dilution buffer:formulation) to 100:1 (dilution buffer:formulation). In another embodiment, the dilution buffer is added at a ratio of from 1:1 (dilution buffer:formulation) to 50:1 (dilution buffer:formulation).

In some embodiments, the stable liquid pharmaceutical formulation comprising an anti-PVRIG antibody or antigen binding fragment thereof is diluted from 1-fold to 1000-fold, from 1-fold to 500-fold, from 1-fold to 250-fold, from 1-fold to 200-fold, from 1-fold to 100-fold, from 1-fold to 50-fold, from 1-fold to 10-fold, from 10-fold to 1000-fold, from 10-fold to 500-fold, from 10-fold to 250-fold, from 10-fold to 200-fold, from 10-fold to 100-fold, from 10-fold to 50-fold, from 50-fold to 1000-fold, from 50-fold to 500-fold, from 50-fold to 250-fold, from 50-fold to 200-fold, from 50-fold to 100-fold, from 100-fold to 1000-fold, from 100-fold to 500-fold, from 100-fold to 250-fold, from 100-fold to 200-fold, from 200-fold to 1,000-fold, from 200-fold to 500-fold, or from 200-fold to 250-fold.

### H. Stability Assays

As discussed herein, the stable liquid pharmaceutical formulations comprising an anti-PVRIG antibody or antigen binding fragment thereof (*e*.*g*., anti-PVRIG antibodies including those with CDRs identical to those shown in Figure 3) show improved stability as compared to control formulations. In one embodiment, improved stability includes retention of a higher percentage of binding activity and/or no reduction in binding activity as compared to control formulations in various stability assays. Such assays can be used to determine if a formulation is a highly stabilized formulation. In some embodiments, the highly stabilized formulation has at least 5%, 10%, 20%, 30%, 40%, 50%, 55%, 60%, 65%, 70%, 75%, 80%, 85%, 90%, 95%, 96%, 97%, 98%, 99% or greater activity than a control formulation when assessed by any of the stability assays discussed herein or known in the art.

In some embodiments, the liquid pharmaceutical formulations comprising an anti-PVRIG antibody or antigen binding fragment thereof are tested under stressor conditions, such as storage at high temperature, agitation, freeze/thaw cycles, or some combination thereof. After such stressors, the formulations are assayed using any of the methods described herein or known in the art to determine the stability under these conditions.

### A280 and Appearance Analysis

In some embodiments, an A280 by SoloVPE assay can be employed to examine the appearance of the stable liquid pharmaceutical formulations comprising an anti-PVRIG antibody or antigen binding fragment thereof.

In some embodiments, the SoloVPE assay can be employed to examine concentrations for the stable liquid pharmaceutical formulations comprising an anti-PVRIG antibody or antigen binding fragment thereof.

A280: Amino acids containing aromatic side chains exhibit strong UV-light absorption at the wavelength of 280nm. Once an absorptivity coefficient has been established for a given protein, the protein's concentration in solution can be calculated from its absorbance. The method is designed to determine the protein concentration by measuring its absorbance at 280nm using the SoloVPE instrument without dilution

### (https://www.ctechnologiesinc.com/products/solovpe)

Appearance: Sample appearance determination is assessed by holding the sample within a controlled light source and observe the appearance of the material. Gently agitate the solution and determine if the appearance changes when viewed against a black and white background. Use adjectives such as "clear", "turbid", or "slightly turbid" to assess clarity. Be specific with regards to the color of the material. If the material is colorless then state that as a result (*i.e.*, clear, colorless solution). specify the physical state of the sample (*i.e.*, liquid or frozen liquid)

### Binding Assay Analysis

In some embodiments, a binding assay can be performed to examine the activity of the stable liquid pharmaceutical formulations comprising an anti-PVRIG antibody or antigen binding fragment thereof.

### LabChip Analysis

In some embodiments, a LabChip analysis is employed to examine purity, including for example, IgG purity as well as HC + LC percentages for the stable liquid pharmaceutical formulations comprising an anti-PVRIG antibody or antigen binding fragment thereof. In some embodiments, the stable liquid pharmaceutical formulations comprising an anti-PVRIG antibody or antigen binding fragment thereof exhibit IgG purity percentages greater than 94%, greater than 95%, greater than 96%, greater than 97%, or greater than 98%. In some embodiments, the stable liquid pharmaceutical formulations comprising an anti-PVRIG antibody or antigen binding fragment thereof exhibit IgG purity percentages were from about 95% to 98%. In some embodiments, the stable liquid pharmaceutical formulations comprising an anti-PVRIG antibody or antigen binding fragment thereof exhibit IgG purity percentages from about 96% to 97%. In some embodiments, the stable liquid pharmaceutical formulations comprising an anti-PVRIG antibody or antigen binding fragment thereof exhibit HC+LC percentages from about 96% to 100%. In some embodiments, the stable liquid pharmaceutical formulations comprising an anti-PVRIG antibody or antigen binding fragment thereof exhibit HC+LC percentages from about 97% to 100%. In some embodiments, the stable liquid pharmaceutical formulations comprising an anti-PVRIG antibody or antigen binding fragment thereof exhibit HC+LC percentages from about 98% to 100%.

### cIEF Analysis

In some embodiments, a capillary isoelectric focusing (cIEF) can be employed to analyze the stable liquid pharmaceutical formulations comprising an anti-PVRIG antibody or antigen binding fragment thereof for the presence of additional species, including for example, minor acidic species.

### MFI Analysis

Antibodies can form sub-visible particles in response to stressed conditions, such as heat, freeze/thaw cycles, and agitation. In some embodiments, a microflow imaging (MFI) analysis can be employed to analyze the stable liquid pharmaceutical formulations comprising an anti-PVRIG antibody or antigen binding fragment thereof for the formation of particles in response to stressed conditions. In some embodiments, the stable liquid pharmaceutical formulations of the anti-PVRIG antibody or antigen binding fragment thereof provide for a formulation capable of stabilizing the anti-PVRIG antibody or antigen binding fragment thereof against these stressed conditions and protecting against the formation of particles. MFI can be used to evaluate particle counts at different size ranges (< 2 µm, < 5 µm, < 10 µm, and < 25 µm) in different formulations under stressed conditions. Typically, MFI data can be evaluated to choose an appropriate formulation based on generation of the lowest amount of particles/mL for all sizes of particles across all time points, conditions, and formulations.

### SEC Analysis

In some embodiments, size exclusion chromatography (SEC) can be employed to analyze the stable liquid pharmaceutical formulations comprising an anti-PVRIG antibody or antigen binding fragment thereof. The SEC data showed HMW throughout all time points and conditions; however, it remained stable at about 1%. LMW was present in accelerated conditions and 2-8 °C 8 week time point. Within the 40 °C condition, the LMW did increase from about 1% to 3% from Week 1 to Week 2.

### I. Selected Formulation Embodiments

In some embodiments, the present invention provides a stable liquid pharmaceutical formulation of an anti-PVRIG antibody comprising:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides a stable liquid pharmaceutical formulation of an anti-PVRIG antibody comprising:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).

(a) an anti-PVRIG antibody;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides a stable liquid pharmaceutical formulation comprising:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides a stable liquid pharmaceutical formulation comprising:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides a stable liquid pharmaceutical formulation comprising:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides a stable liquid pharmaceutical formulation comprising:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides a stable liquid pharmaceutical formulation comprising:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides a stable liquid pharmaceutical formulation comprising:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

### VIII. ADMINISTRATION OF FORMULATIONS OF ANTI-PVRIG ANTIBODIES

Administration of the pharmaceutical composition comprising anti-PVRIG antibodies of the present invention (*e*.*g*., anti-PVRIG antibodies including those with CDRs identical to those shown in Figure 3), preferably in the form of a sterile aqueous solution, may be done in a variety of ways, As is known in the art, protein therapeutics are often delivered by IV infusion. The antibodies of the present invention may also be delivered using such methods. For example, administration may venious be by intravenous infusion with 0.9% sodium chloride as an infusion vehicle. Such techniques are disclosed in Remington's Pharmaceutical Sciences 16th edition, Osol, A. Ed., 1980.

The dosing amounts and frequencies of administration are, in some embodiments, selected to be therapeutically or prophylactically effective. As is known in the art, adjustments for protein degradation, systemic versus localized delivery, and rate of new protease synthesis, as well as the age, body weight, general health, sex, diet, time of administration, drug interaction and the severity of the condition may be necessary, and will be ascertainable with routine experimentation by those skilled in the art. In order to treat a patient, a therapeutically effective dose of the Fc variant of the present invention may be administered. By "therapeutically effective dose" herein is meant a dose that produces the effects for which it is administered.

### IX. DOSING

In some embodiments, the anti-PVRIG antibody and/or antigen binding portion thereof formulations of the present invention can be formulated for administration, including as a unit dosage formulation. In some embodiments, the anti-PVRIG antibody and/or antigen binding portion thereof formulations are administered at a dosage of 0.01 mg/kg of the anti-PVRIG antibody and/or antigen binding portion thereof. In some embodiments, the anti-PVRIG antibody and/or antigen binding portion thereof formulations are administered at a dosage of 0.02 mg/kg of the anti-PVRIG antibody and/or antigen binding portion thereof. In some embodiments, the anti-PVRIG antibody and/or antigen binding portion thereof formulations are administered at a dosage of 0.03 mg/kg of the anti-PVRIG antibody and/or antigen binding portion thereof. In some embodiments, the anti-PVRIG antibody and/or antigen binding portion thereof formulations are administered at a dosage of 0.04 mg/kg of the anti-PVRIG antibody and/or antigen binding portion thereof. In some embodiments, the anti-PVRIG antibody and/or antigen binding portion thereof formulations are administered at a dosage of 0.05 mg/kg of the anti-PVRIG antibody and/or antigen binding portion thereof. In some embodiments, the anti-PVRIG antibody and/or antigen binding portion thereof formulations are administered at a dosage of 0.06 mg/kg of the anti-PVRIG antibody and/or antigen binding portion thereof. In some embodiments, the anti-PVRIG antibody and/or antigen binding portion thereof formulations are administered at a dosage of 0.07 mg/kg of the anti-PVRIG antibody and/or antigen binding portion thereof. In some embodiments, the anti-PVRIG antibody and/or antigen binding portion thereof formulations are administered at a dosage of 0.08 mg/kg of the anti-PVRIG antibody and/or antigen binding portion thereof. In some embodiments, the anti-PVRIG antibody and/or antigen binding portion thereof formulations are administered at a dosage of 0.09 mg/kg of the anti-PVRIG antibody and/or antigen binding portion thereof. In some embodiments, the anti-PVRIG antibody and/or antigen binding portion thereof formulations are administered at a dosage of 0.1 mg/kg of the anti-PVRIG antibody and/or antigen binding portion thereof. In some embodiments, the anti-PVRIG antibody and/or antigen binding portion thereof formulations are administered at a dosage of 0.2 mg/kg of the anti-PVRIG antibody and/or antigen binding portion thereof. In some embodiments, the anti-PVRIG antibody and/or antigen binding portion thereof formulations are administered at a dosage of 0.3 mg/kg of the anti-PVRIG antibody and/or antigen binding portion thereof. In some embodiments, the anti-PVRIG antibody and/or antigen binding portion thereof formulations are administered at a dosage of 0.5 mg/kg of the anti-PVRIG antibody and/or antigen binding portion thereof. In some embodiments, the anti-PVRIG antibody and/or antigen binding portion thereof formulations are administered at a dosage of 0.8 mg/kg of the anti-PVRIG antibody and/or antigen binding portion thereof. In some embodiments, the anti-PVRIG antibody and/or antigen binding portion thereof formulations are administered at a dosage of 1 mg/kg of the anti-PVRIG antibody and/or antigen binding portion thereof. In some embodiments, the anti-PVRIG antibody and/or antigen binding portion thereof formulations are administered at a dosage of 2 mg/kg of the anti-PVRIG antibody and/or antigen binding portion thereof. In some embodiments, the anti-PVRIG antibody and/or antigen binding portion thereof formulations are administered at a dosage of 3 mg/kg of the anti-PVRIG antibody and/or antigen binding portion thereof. In some embodiments, the anti-PVRIG antibody and/or antigen binding portion thereof formulations are administered at a dosage of 4 mg/kg of the anti-PVRIG antibody and/or antigen binding portion thereof. In some embodiments, the anti-PVRIG antibody and/or antigen binding portion thereof formulations are administered at a dosage of 5 mg/kg of the anti-PVRIG antibody and/or antigen binding portion thereof. In some embodiments, the anti-PVRIG antibody and/or antigen binding portion thereof formulations are administered at a dosage of 6 mg/kg of the anti-PVRIG antibody and/or antigen binding portion thereof. In some embodiments, the anti-PVRIG antibody and/or antigen binding portion thereof formulations are administered at a dosage of 7 mg/kg of the anti-PVRIG antibody and/or antigen binding portion thereof. In some embodiments, the anti-PVRIG antibody and/or antigen binding portion thereof formulations are administered at a dosage of 8 mg/kg of the anti-PVRIG antibody and/or antigen binding portion thereof. In some embodiments, the anti-PVRIG antibody and/or antigen binding portion thereof formulations are administered at a dosage of 9 mg/kg of the anti-PVRIG antibody and/or antigen binding portion thereof. In some embodiments, the anti-PVRIG antibody and/or antigen binding portion thereof formulations are administered at a dosage of 10 mg/kg of the anti-PVRIG antibody and/or antigen binding portion thereof. In some embodiments, the anti-PVRIG antibody and/or antigen binding portion thereof formulations are administered at a dosage of 20 mg/kg of the anti-PVRIG antibody and/or antigen binding portion thereof.

In some embodiments, the anti-PVRIG antibody and/or antigen binding portion thereof formulations is administered at a dosage of about 0.01 mg/kg to about 20 mg/kg of the anti-PVRIG antibody. In some embodiments, the anti-PVRIG antibody and/or antigen binding portion thereof formulations is administered at a dosage of about 0.01 mg/kg to about 10 mg/kg of the anti-PVRIG antibody. In some embodiments, the anti-PVRIG antibody and/or antigen binding portion thereof formulations is administered at a dosage of about 20mg/kg. In some embodiments, the anti-PVRIG antibody and/or antigen binding portion thereof formulations is administered at a dosage of about 20mg/kg each 4 weeks. In some embodiments, the anti-PVRIG antibody and/or antigen binding portion thereof formulations is administered at a dosage of about 20mg/kg IV each 4 weeks. In some embodiments, formulation is administered at a dosage of about 0.1 mg/kg to about 10 mg/kg of the anti-PVRIG antibody. In some embodiments, formulation is administered at a dosage of about 1 mg/kg to about 10 mg/kg of the anti-PVRIG antibody. In some embodiments, formulation is administered at a dosage of about 2 mg/kg to about 10 mg/kg of the anti-PVRIG antibody. In some embodiments, formulation is administered at a dosage of about 3 mg/kg to about 10 mg/kg of the anti-PVRIG antibody. In some embodiments, formulation is administered at a dosage of about 4 mg/kg to about 10 mg/kg of the anti-PVRIG antibody. In some embodiments, formulation is administered at a dosage of about 5 mg/kg to about 10 mg/kg of the anti-PVRIG antibody. In some embodiments, formulation is administered at a dosage of about 5 mg/kg to about 10 mg/kg of the anti-PVRIG antibody. In some embodiments, formulation is administered at a dosage of about 7 mg/kg to about 10 mg/kg of the anti-PVRIG antibody. In some embodiments, formulation is administered at a dosage of about 8 mg/kg to about 10 mg/kg of the anti-PVRIG antibody. In some embodiments, formulation is administered at a dosage of about 9 mg/kg to about 10 mg/kg of the anti-PVRIG antibody. In some embodiments, formulation is administered at a dosage of about 20 mg/kg of the anti-PVRIG antibody. In some embodiments, formulation is administered at a dosage of about 0.01 mg/kg, 0.03 mg/kg, 0.1 mg/kg, 0.3 mg/kg, 1 mg/kg, 3 mg/kg, 10 mg/kg or 20 mg/kg of the anti-PVRIG antibody.

### A. Selected Dosing with Formulation Embodiments

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).

(a) an anti-PVRIG antibody;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments of the stable liquid pharmaceutical formulation, the formulation is administered with an anti-PD-1 antibody.

In some embodiments of the stable liquid pharmaceutical formulation, the anti-PD-1 antibody is an antibody selected from the group consisting of pembrolizumab and nivolumab.

In some embodiments of the stable liquid pharmaceutical formulation, the anti-PD-1 antibody is nivolumab. In some embodiments of the stable liquid pharmaceutical formulation, the anti-PD-1 antibody is nivolumab is administered at a dosage of about 360 mg or 480 mg. In some embodiments of the stable liquid pharmaceutical formulation, the anti-PD-1 antibody is nivolumab is administered at a dosage of about 360 mg. In some embodiments of the stable liquid pharmaceutical formulation, the anti-PD-1 antibody is nivolumab is administered at a dosage of about 480 mg.

In some embodiments of the stable liquid pharmaceutical formulation, the anti-PD-1 antibody is pembrolizumab.

### X. METHODS OF USING THE ANTI-PVRIG ANTIBODY FORMULATIONS

### A. Therapeutic Uses

The anti-PVRIG antibodies (e.g., anti-PVRIG antibodies including those with CDRs identical to those shown in Figure 3) find use in treating patients, such as human subjects, generally with a condition associated with PVRIG. The term "treatment" as used herein, refers to both therapeutic treatment and prophylactic or preventative measures, which in this example relates to treatment of cancer; Those in need of treatment include those already with cancer as well as those in which the cancer is to be prevented. Hence, the mammal to be treated herein may have been diagnosed as having the cancer or may be predisposed or susceptible to the cancer. As used herein the term "treating" refers to preventing, delaying the onset of, curing, reversing, attenuating, alleviating, minimizing, suppressing, halting the deleterious effects or stabilizing of discernible symptoms of the above-described cancerous diseases, disorders or conditions. It also includes managing the cancer as described above. By "manage" it is meant reducing the severity of the disease, reducing the frequency of episodes of the disease, reducing the duration of such episodes, reducing the severity of such episodes, slowing/reducing cancer cell growth or proliferation, slowing progression of at least one symptom, amelioration of at least one measurable physical parameter and the like. For example, immunostimulatory anti-PVRIG immune molecules should promote T cell or NK or cytokine immunity against target cells, e.g., cancer, infected or pathogen cells and thereby treat cancer by depleting the cells involved in the disease condition.

The PVRIG antibodies of the invention are provided in therapeutically effective dosages. A "therapeutically effective dosage" of an anti-PVRIG immune molecule according to at least some embodiments of the present invention preferably results in a decrease in severity of disease symptoms, an increase in frequency and duration of disease symptom-free periods, an increase in lifespan, disease remission, or a prevention or reduction of impairment or disability due to the disease affliction. For example, for the treatment of PVRIG positive tumors, a "therapeutically effective dosage" preferably inhibits cell growth or tumor growth by at least about 20%, more preferably by at least about 40%, even more preferably by at least about 60%, and still more preferably by at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subj ect.

One of ordinary skill in the art would be able to determine a therapeutically effective amount based on such factors as the subject's size, the severity of the subject's symptoms, and the particular composition or route of administration selected.

### 1. Cancer Treatment

The PVRIG antibody formulations of the invention find particular use in the treatment of cancer. In general, the antibodies of the invention are immunomodulatory, in that rather than directly attack cancerous cells, the anti-PVRIG antibodies of the invention stimulate the immune system, generally by inhibiting the action of PVRIG. Thus, unlike tumor-targeted therapies, which are aimed at inhibiting molecular pathways that are crucial for tumor growth and development, and/or depleting tumor cells, cancer immunotherapy is aimed to stimulate the patient's own immune system to eliminate cancer cells, providing long-lived tumor destruction. Various approaches can be used in cancer immunotherapy, among them are therapeutic cancer vaccines to induce tumor-specific T cell responses, and immunostimulatory antibodies (*i.e.* antagonists of inhibitory receptors = immune checkpoints) to remove immunosuppressive pathways.

Clinical responses with targeted therapy or conventional anti-cancer therapies tend to be transient as cancer cells develop resistance, and tumor recurrence takes place. However, the clinical use of cancer immunotherapy in the past few years has shown that this type of therapy can have durable clinical responses, showing dramatic impact on long term survival. However, although responses are long term, only a small number of patients respond (as opposed to conventional or targeted therapy, where a large number of patients respond, but responses are transient).

By the time a tumor is detected clinically, it has already evaded the immune-defense system by acquiring immunoresistant and immunosuppressive properties and creating an immunosuppressive tumor microenvironment through various mechanisms and a variety of immune cells.

Accordingly, the anti-PVRIG antibodies of the invention are useful in treating cancer. Due to the nature of an immuno-oncology mechanism of action, PVRIG does not necessarily need to be overexpressed on or correlated with a particular cancer type; that is, the goal is to have the anti-PVRIG antibodies de-suppress T cell and NK cell activation, such that the immune system will go after the cancers.

"Cancer," as used herein, refers broadly to any neoplastic disease (whether invasive or metastatic) characterized by abnormal and uncontrolled cell division causing malignant growth or tumor (*e*.*g*., unregulated cell growth). The term "cancer" or "cancerous" as used herein should be understood to encompass any neoplastic disease (whether invasive, non-invasive or metastatic) which is characterized by abnormal and uncontrolled cell division causing malignant growth or tumor, non-limiting examples of which are described herein. This includes any physiological condition in mammals that is typically characterized by unregulated cell growth.

In some embodiments, the anti-PVRIG formulations of the present invention can be used in the treatment of solid tumors (including, for example, cancers of the lung, liver, breast, brain, GI tract) and blood cancers (including for example, leukemia and preleukemic disorders, lymphoma, plasma cell disorders) carcinoma, lymphoma, blastoma, sarcoma, and leukemia or lymphoid malignancies. In some embodiments, the cancer is early. In some embodiments, the cancer is advanced (including metastatic). In some embodiments, the cancers amenable for treatment of the invention include cancers that express or do not express PVRIG and further include non-metastatic or non-invasive, as well as invasive or metastatic cancers, including cancers where PVRIG expression by immune, stromal, or diseased cells suppresses antitumor responses and anti-invasive immune responses. In some embodiments, the anti-PVRIG formulations can be used for the treatment of vascularized tumors. In some embodiments, the cancer for treatment using the anti-PVRIG formulations of the present invention includes carcinoma, lymphoma, sarcoma, and/or leukemia. In some embodiments, the cancer for treatment using the anti-PVRIG formulations of the present invention includes melanoma, non-melanoma skin cancer (squamous and basal cell carcinoma), mesothelioma, squamous cell cancer, lung cancer (including small-cell lung cancer, non-small cell lung cancer, soft-tissue sarcoma, Kaposi's sarcoma, adenocarcinoma of the lung, squamous carcinoma of the lung, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC; including refractory MSS colorectal; MSS = microsatellite stable status), primary peritoneal cancer, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia), Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma, post-transplant lymphoproliferative disorder (PTLD), abnormal vascular proliferation associated with phakomatoses, Meigs' syndrome, Merkel Cells cancer, MSI-high cancer, KRAS mutant tumors, adult T-cell leukemia/lymphoma, adenoid cystic cancer (including adenoid cystic carcinoma), malignant melanoma, pancreatic cancer, pancreatic adenocarcinoma, ovarian cancer (including ovarian carcinoma), pleural mesothelioma, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, cervical squamous cell carcinoma (cervical SCC), anal squamous cell carcinoma (anal SCC), neuroendocrine lung carcinoma, carcinoma of unknown primary, gallbladder cancer, malignant melanoma, pleural mesothelioma, Myelodysplastic syndromes (MDS), HNSCC, PD1 refractory or relapsing cancer, gastroesophageal junction cancer, gastric cancer, and/or fallopian tube cancer.

In some embodiments, the cancer for treatment using the anti-PVRIG formulations of the present invention includes a cancer selected from the group consisting of prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, and/or fallopian tube cancer.

"Cancer therapy" herein refers to any method that prevents or treats cancer or ameliorates one or more of the symptoms of cancer. Typically such therapies will comprises administration of immunostimulatory anti-PVRIG antibodies (including antigen-binding fragments) either alone or in combination with chemotherapy or radiotherapy or other biologics and for enhancing the activity thereof, *i.e.*, in individuals wherein expression of PVRIG suppresses antitumor responses and the efficacy of chemotherapy or radiotherapy or biologic efficacy.

In some embodiments, anti-PVRIG antibodies are used in combination with antagonistic antibodies targeting PD-1 (*e.g.*, anti-PD-1 antibodies), including for example but not limited to nivolumab and/or pembrolizumab. In some embodiments, the anti-PD-1 antibody is an antibody selected from the group consisting of nivolumab and pembrolizumab. In some embodiments, the anti-PD-1 antibody is nivolumab. In some embodiments, the anti-PD-1 antibody is pembrolizumab. In some embodiments, the anti-PD-1 antibody is nivolumab is administered at 360mg. In some embodiments, the anti-PD-1 antibody is nivolumab is administered at 360mg IV. In some embodiments, the anti-PD-1 antibody is nivolumab is administered at 360mg IV 3 weeks (*e*.*g*., 360mg IV Q3 weeks). In some embodiments, the anti-PD-1 antibody is nivolumab is administered at 480mg. In some embodiments, the anti-PD-1 antibody is nivolumab is administered at 480mg IV. In some embodiments, the anti-PD-1 antibody is nivolumab is administered at 480mg IV 3 weeks *(e.g.,* 480mg IV Q3 weeks). In some embodiments, the subject administered the anti-PVRIG antibody in combination with the anti-PD-1 antibody has exhausted all available standard therapy, including for example, but not limited to ECOG 0-1, prior anti-PD-1, prior anti-PD-L1, prior anti-CTLA-4, prior OX-40, and/or prior CD137 therapies.

### 2. Selected Monotherapy Treatment with Formulation Embodiments

In some embodiments, the present invention provides for treatment of cancer in a subject in need thereof by administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).

(a) an anti-PVRIG antibody;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

### 3. Selected Combination Treatment with Formulation Embodiments

In some embodiments, the present invention provides for treatment of cancer in a subject in need thereof by administration of an anti-TIGIT and/or and an anti-PD1 and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the anti-PD1 is selected from the group consisting of nivolumab, pembrolizumab, BMS-936558/MDX-1106/ONO-4538, AMP224, CT-011, MK-3475

In some embodiments, the invention provides the specific combinations of: CHA.7.518.1.H4(S241P) with pembrolizumab; CHA.7.518.1.H4(S241P) with nivolumab; CHA.7.538.1.2.H4(S241P) with pembrolizumab and CHA.7.538.1.2.H4(S241P) with nivolumab.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).

(a) an anti-PVRIG antibody;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 360 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain variable domain comprising the vhCDR1, vhCDR2, and vhCDR3 from the heavy chain of CHA.7.518.1.H4(S241P) (SEQ ID NO:4), and
   ii) a light chain variable domain comprising the vlCDR1, vlCDR2, and vlCDR3 from the light chain of CHA.7.518.1.H4(S241P) (SEQ ID NO:9).
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) heavy chain variable domain is from the heavy chain of CHA.7.518.1.H4(S241P) (SEQ ID NO:4), and
   ii) a light chain variable domain is from the light chain of CHA.7.518.1.H4(S241P) (SEQ ID NO:9);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising:
      a) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   ii) a light chain comprising:
      a) a VL-CL, wherein the VL from CHA.7.518.1.H4(S241P) (SEQ ID NO:9) and wherein the CL region is from human kappa 2 light chain;
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO:13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

In some embodiments, the present invention provides for treatment of cancer in a subject in need thereof by administration of 480 mg nivolumab and a stable liquid pharmaceutical formulation of an anti-PVRIG antibody, wherein the anti-PVRIG antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg, and wherein the stable liquid formulation of the anti-PVRIG antibody comprises:
(a) an anti-PVRIG antibody comprising:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13);
(b) about 25 mM histidine;
(c) about 60 mM NaCl;
(d) about 100 mM L-Arginine; and
(e) about 0.01% % w/v polysorbate 80,
wherein the composition has a pH from 6.5 +/- 0.2.

### LIST OF EMBODIMENTS:

In one aspect, the present invention disclosed herein provides for the following:
1. A method for determining a cancer patient population for treatment with an anti-PVRIG antibody, the method comprising:
   (a) detecting the presence in a biological sample from the cancer patient one or more cellular components selected from the group consisting of:
      (i) TSCM, TRM, naive, exhausted, cycling, and effector CD8 positive T cells, that express PVRIG; and/or
      (ii) activated DC cells, DC1, and/or DC2 that express PVRL2,
   (b) quantitating the measurement of the level of components selected from the group consisting of:
      (i) TSCM, TRM, naive, exhausted, cycling, and effector CD8 positive T cells, that express PVRIG; and/or
      (ii) activated DC cells, DC1, and/or DC2 that express PVRL2; and
   (c) treating the cancer patient with an anti-PVRIG antibody when one or more cellular components in (a) as quantitated in step (b) are present at an increased level as compared to a control or a patient that does not have detectable levels of the cells.
2. A method for predicting or determining the efficacy of treatment with an anti-PVRIG treatment antibody, the method comprising:
   (a) measuring the level of one or more cellular components selected from the group consisting of:
      (i) TSCM, TRM, naive, exhausted, cycling, and effector CD8 positive T cells that express PVRIG; and/or
      (ii) activated DC cells, DC1, and DC2 that express PVRL2;
   (b) quantitating the measurement of the level of one or more cellular components selected from the group consisting of:
      (i) TSCM, TRM, naive, exhausted, cycling, and effector CD8 positive T cells, expressing PVRIG; and/or
      (ii) activated DC cells, DC1, and DC2 that express PVRL2; and
   (c) correlating the level of the cellular components with the efficacy of treatment, wherein any of the cellular components being present and/or being present at an increased level as compared to a control or a patient that does not have detectable levels of the cells, is indicative of treatment efficacy for treatment with an anti-PVRIG treatment antibody.
3. The method according to item 2, wherein the method further comprises:
   (d) treating the cancer patient with an anti-PVRIG antibody when any of the cellular components in (a) are present at an increased level as compared to a control or a patient that does not have detectable levels of the cells.
4. A method for predicting or determining the efficacy of treatment or determining a population for treatment with an anti-PVRIG treatment antibody when any of TSCM, TRM, naive, exhausted, cycling, and effector CD8 positive T cells, that express PVRIG comprise at least 0.5%, at least 1%, at least 1.5%, at least 2%, at least 2.5%, at least 3%, at least 3.5%, at least 4%, at least 4.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, of the total CD8 cells in the biological sample is indicative of treatment efficacy and/or indicative for treatment with an anti-PVRIG antibody.
5. A method for predicting or determining the efficacy of treatment or determining a population for treatment with an anti-PVRIG treatment antibody when any of activated DC cells, DC1, and DC2 that express the PVRL2 comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in the biological sample.
6. The method according to any one of items 4 to 5, wherein the presence of and/or an increased level as compared to an untreated control and/or to a control prior to treatment, of one more cellular components, is indicative of anti-PVRIG treatment efficacy:(i) TSCM, TRM, naive, exhausted, cycling, and effector CD8 positive T cells, that express PVRIG comprise at least 0.5%, at least 1%, at least 1.5%, at least 2%, at least 2.5%, at least 3%, at least 3.5%, at least 4%, at least 4.5%, or at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, out of total CD8 cells in the biological sample; and/or
   (ii) DC cells, DC1, and DC2 that express PVRL2 comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in the biological sample.
7. The method according to any one of items 1 to 6, wherein the biological sample is obtained from a tumor, tumor microenvironment, and/or peripheral blood from the cancer patient.
8. A method for predicting or determining the efficacy of treatment with an anti-PVRIG treatment antibody, the method comprising: (a) measuring the level of early memory CD8 T cells; (b) quantitating the measurement of the level of early memory CD8 T cells; (c) correlating the level of the early memory CD8 T cells with the efficacy of treatment.
9. The method of item 8, wherein the early memory CD8 T cells include CD8 T cells expressing one or more of the markers, selected from the group consisting of GZMK, GZMA, GZMM, NKG7, TNFSF9, SH2D1A, EOMES, DTHD1, SLAMF7, FCRL3, CD28, CMC1, CCL4, CTS7, ITM2C, KLRG1, CRTAM, PECAM1, TCF7, CXCR3, LYAR, and HLA-DRB5.
10. A method for determining a cancer patient population for treatment with an anti-PVRIG treatment antibody, the method comprising:
   (a) measuring the level of PVRIG and/or PVRL2 expression in a biological sample obtained from a cancer and/or tumor microenvironment and/or peripheral blood in the cancer patient,
   (b) quantitating the measurement of the level of PVRIG and/or PVRL2 expression in the biological sample, and
   (c) treating the cancer patient with an anti-PVRIG antibody wherein PVRIG and/or PVRL2 is expressed and/or expressed at an increased level as compared to a control or a patient that does not have detectable levels of the PVRIG and/or PVRL2 in the biological sample.
11. A method for predicting or determining the efficacy of treatment with an anti-PVRIG treatment antibody, the method comprising:
   (a) measuring the level of PVRIG and/or PVRL2 expression in a biological sample obtained from a cancer and/or tumor microenvironment and/or peripheral blood in the cancer patient,
   (b) quantitating the measurement of the level of PVRIG and/or PVRL2 expression in the biological sample, and
   (c) correlating the level of PVRIG and/or PVRL2 with the efficacy of treatment, wherein PVRIG and/or PVRL2 is expressed and/or expressed at an increased level as compared to a control or a patient that does not have detectable levels of the PVRIG and/or PVRL2, is indicative of treatment efficacy for treatment with an anti-PVRIG treatment antibody.
12. A method for determining a cancer patient population for treatment with an anti-PVRIG antibody, the method comprising:
   (a) detecting the presence in a biological sample from the cancer patient early memory CD8 T cells;
   (b) quantitating the measurement of the level of early memory CD8 T cells;
   (c) treating the cancer patient with an anti-PVRIG antibody when the level of early memory CD8 T cells are present at an increased level as compared to a control or a patient that does not have detectable levels of the cells.
13. A method for altering the regimen of treatment for a patient with cancer, the method comprising:
   (a) measuring the level of PVRIG and/or PVRL2 expression in a biological sample obtained from a cancer and/or tumor microenvironment and/or peripheral blood in the cancer patient,
   (b) quantitating the measurement of the level of PVRIG and/or PVRL2 expression in the biological sample, wherein PVRIG and/or PVRL2 being expressed and/or being expressed at an increased level as compared to a control or a patient that does not have detectable levels of the PVRIG and/or PVRL2 is indicative of treatment efficacy,
   (c) correlating the level of PVRIG and/or PVRL2 expression with the efficacy of treatment, wherein a high level of PVRIG and/or PVRL2 expression is indicative of altering the treatment regimen for treatment with anti-PVRIG treatment antibody, and
   (d) altering the treatment regimen to include an anti-PVRIG antibody when a moderate or high level of PVRIG and/or PVRL2 expression is measured.
14. The method according to any one of items 1 to 12, wherein the anti-PVRIG antibody comprises the vhCDR1, vhCDR2, vhCDR3, vlCDR1, vlCDR2, and vlCDR3 sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.520.1, CHA.7.520.2, CHA.7.522, CHA.7.524, CHA.7.526, CHA.7.527, CHA.7.528, CHA.7.530, CHA.7.534, CHA.7.535, CHA.7.537, CHA.7.538.1.2.H4(S241P), CHA.7.538.1, CHA.7.538.2, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.
15. The method according to any one of items 1 to 12, wherein the anti-PVRIG antibody comprises the variable heavy domain and the variable light domain sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.518.1, CHA.7.518.2, CHA.7.518.3, CHA.7.518.4, CHA.7.518.5, CHA.7.520.1, CHA.7.520.2, CHA.7.522, CHA.7.524, CHA.7.524.1, CHA.7.524.2, CHA.7.524.3, CHA.7.524.4, CHA.7.526, CHA.7.527, CHA.7.528, CHA.7.530, CHA.7.530.1, CHA.7.530.2, CHA.7.530.3, CHA.7.530.4, CHA.7.530.5, CHA.7.534, CHA.7.535, CHA.7.537, CHA.7.538.1.2.H4(S241P), CHA.7.538.1, CHA.7.538.1.1, CHA.7.538.1.2, CHA.7.538.1.3, CHA.7.538.1.4, CHA.7.538.2, CHA.7.538.2.1, CHA.7.538.2.2, CHA.7.538.2.3, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.
16. The method according to any one of items 1 to 12, wherein the anti-PVRIG treatment antibody comprises a heavy chain variable domain from the heavy chain of CHA.7.518.1.H4(S241P) (SEQ ID NO:8) and a light chain variable domain from the light chain of CHA.7.518.1.H4(S241P) (SEQ ID NO:13).
17. The method according to any one of items 1 to 12, wherein the anti-PVRIG treatment antibody comprises a heavy chain variable domain from the heavy chain of CHA.7.538.1.2.H4(S241P) (SEQ ID NO:266) and a light chain variable domain from the light chain of CHA.7.538.1.2.H4(S241P) (SEQ ID NO:271).
18. The method according to any one of items 1 to 17, wherein the PVRIG and/or PVRL2 expression is determined using single-cell resolution analysis.
19. The method according to item 18, wherein the single-cell resolution analysis includes RNAseq, immunohistochemistry (IHC), multiplex immunohistochemistry (mIHC) and/or immunofluorescence (IF), flow cytometry (e.g., FACS) and mass cytometry (e.g., CyTOF), as well as combinations thereof.
20. The method according to any one of items 1 to 17, wherein the PVRIG and/or PVRL2 expression is determined using immunohistochemistry.
21. The method according to any one of items 18 to 20, wherein the PVRIG antibody used for the single-cell resolution analysis and/or immunohistochemistry is AB-635 PVRIG Ab (6D8-1 clone).
22. The method according to any one of items 18 to 20, wherein the PVRIG antibody used for the single-cell resolution analysis and/or immunohistochemistry is 6D8-1 (heavy chain SEQ ID NO:659 and light chain SEQ ID NO:663).
23. The method according to any one of items 18 to 20, wherein the PVRIG antibody used for the single-cell resolution analysis and/or immunohistochemistry comprises:
   i) a heavy chain variable domain comprising the vhCDR1, vhCDR2, and vhCDR3 from the heavy chain of 6D8-1 (SEQ ID NO:659), and
   ii) a light chain variable domain comprising the vlCDR1, vlCDR2, and vlCDR3 from the light chain of 6D8-1 (SEQ ID NO:663).
24. The method according to any one of items 18 to 20, wherein the PVRL2 antibody used for the immunohistochemistry and/or single-cell resolution analysis is CST PVRL2 Ab (Nectin-2/CD112 (D8D3F) XP^{®} Rabbit mAb).
25. The method according to any one of items 1 to 24, wherein the PVRL2 expression level is categorized as strong, moderate, weak, or negative.
26. The method according to any one of items 1 to 25, wherein the PVRL2 expression is categorized as strong or moderate.
27. The method according to any one of items 1 to 26, wherein the PVRL2 expression level is categorized as 0-no signal, 1-low, 2-medium, 3- high.
28. The method according to any one of items 1 to 27, wherein the PVRL2 expression is categorized as 2-medium or 3- high.
29. The method according to any one of items 1 to 28, wherein the PVRL2 expression is 0% membrane staining there is predicted to be no response or a minimal response to an anti-PVRIG antibody.
30. The method according to any one of items 1 to 28, wherein the PVRL2 expression is under 20% tumor membrane staining at +1 staining score there is predicted to be no response or a minimal response to an anti-PVRIG antibody.
31. The method according to any one of items 1 to 28, wherein the PVRL2 expression is >20% tumor membrane staining at +1 staining score there is predicted to be a response to an anti-PVRIG antibody.
32. The method according to any one of items 1 to 28, wherein the PVRL2 expression is >20% immune infiltrating cells at any intensity membrane or cytoplasmatic staining there is predicted to be a response to an anti-PVRIG antibody.
33. The method according to any one of items 1 to 28, wherein the PVRL2 expression is characterized by at least two of the following
   i. >20% tumor membrane staining at +1 staining score there is predicted to be no response or a minimal response to an anti-PVRIG antibody,
   ii. >20% tumor membrane staining at +1 staining score there is predicted to be a response to an anti-PVRIG antibody, or
   iii. >20% immune infiltrating cells at any intensity membrane or cytoplasmatic staining,
   there is an increased prediction of a response to an anti-PVRIG antibody.
34. The method according to any one of items 30 to 33, wherein the staining score is an IHC score.
35. The method according to any one of items 30 to 33, wherein the staining score is an mIHC/IF score.
36. The method according to any one of items 1 to 35, wherein a cancer patient having dendritic cells in the tumor, tumor microenvironment, and/or peripheral blood having at least 10%, 20%, 30%, 40%, 50%, 60%, 70%, 80%, 90%, or 100% PVRL2 expression exhibits a higher probability to respond the anti-PVRIG antibody treatment as compared to cancer patient with lower PVRL2 expression.
37. The method according to any one of items 1 to 36, wherein a cancer patient having activated dendritic cells in the tumor, tumor microenvironment, and/or peripheral blood having at least 10%, 20%, 30%, 40%, 50%, 60%, 70%, 80%, 90%, or 100% PVRL2 expression exhibits a higher probability to respond the anti-PVRIG antibody treatment as compared to cancer patient with lower PVRL2 expression.
38. The method according to any one of items 1 to 37, wherein a cancer patient having DC1 in the tumor, tumor microenvironment, and/or peripheral blood having with at least 5%, 10%, 20%, 30%, 40%, 50%, 60%, 70%, 80%, 90%, or 100% PVRL2 expression exhibits a higher probability to respond the anti-PVRIG antibody treatment as compared to cancer patient with lower PVRL2 expression.
39. The method according to any one of items 1 to 38, wherein a cancer patient having DC2 in the tumor, tumor microenvironment, and/or peripheral blood having at least 10%, 20%, 30%, 40%, 50%, 60%, 70%, 80%, 90%, or 100% PVRL2 expression exhibits a higher probability to respond the anti-PVRIG antibody treatment as compared to cancer patient with lower PVRL2 expression.
40. The method according to any one of items 1 to 39, wherein a cancer patient having at least 10%, 20%, 30%, 40%, 50%, 60%, 70%, 80%, 90%, or 100% of CD8 cells in the tumor, tumor microenvironment, and/or peripheral blood expressing PVRIG exhibits a higher probability to respond to the anti-PVRIG antibody treatment as compared to cancer patient with lower PVRIG expression.
41. The method according to any one of items 1 to 40, wherein a cancer patient having at least 10%, 20%, 30%, 40%, 50%, 60%, 70%, 80%, 90%, or 100% of TSCM cells in the tumor, tumor microenvironment, and/or peripheral blood expressing PVRIG exhibits a higher probability to respond to the anti-PVRIG antibody treatment as compared to cancer patient with lower PVRIG expression.
42. The method according to any one of items 1 to 41, wherein a cancer patient having at least 10%, 20%, 30%, 40%, 50%, 60%, 70%, 80%, 90%, or 100% of Naive CD8 cells in the tumor, tumor microenvironment, and/or peripheral blood expressing PVRIG exhibits a higher probability to respond to the anti-PVRIG antibody treatment as compared to cancer patient with lower PVRIG expression.
43. The method according to any one of items 1 to 42, wherein a cancer patient having at least 10%, 20%, 30%, 40%, 50%, 60%, 70%, 80%, 90%, or 100% of exhausted CD8 cells in the tumor, tumor microenvironment, and/or peripheral blood expressing PVRIG exhibits a higher probability to respond to the anti-PVRIG antibody treatment as compared to cancer patient with lower PVRIG expression.
44. The method according to any one of items 1 to 43, wherein a cancer patient having at least 5%, 10%, 20%, 30%, 40%, 50%, 60%, 70%, 80%, 90%, or 100% of TRM cells in the tumor, tumor microenvironment, and/or peripheral blood expressing PVRIG exhibits a higher probability to respond to the anti-PVRIG antibody treatment as compared to cancer patient with lower PVRIG expression.
45. The method according to any one of items 1 to 44, wherein a cancer patient having at least 5%, 10%, 20%, 30%, 40%, 50%, 60%, 70%, 80%, 90%, or 100% of Effector cells in the tumor, tumor microenvironment, and/or peripheral blood expressing PVRIG exhibits a higher probability to respond to the anti-PVRIG antibody treatment as compared to cancer patient with lower PVRIG expression.
46. The method according to any one of items 1 to 45, wherein a cancer patient having more than 2 copies of PVRL2 in cells obtained from the tumor, tumor microenvironment, and/or peripheral blood exhibits a higher probability to respond to the anti-PVRIG antibody treatment as compared to cancer patient with only 2 copies of PVRL2 in cells obtained from the tumor, tumor microenvironment, and/or peripheral blood.
47. The method according to any one of items 1 to 46, wherein the anti-PVRIG treatment antibody is administered as a stable liquid pharmaceutical formulation of the anti-PVRIG antibody comprising:
   (a) an anti-PVRIG antibody, wherein the anti-PVRIG antibody comprises:
      i) a heavy chain variable domain comprising the vhCDR1, vhCDR2, and vhCDR3 from the heavy chain of CHA.7.518.1.H4(S241P) (SEQ ID NO:8), and
      ii) a light chain variable domain comprising the vlCDR1, vlCDR2, and vlCDR3 from the light chain of CHA.7.518.1.H4(S241P) (SEQ ID NO:13);
   (b) from 10 mM to 100 mM histidine;
   (c) from 30 mM to 100 mM NaCl;
   (d) from 20 mM to 150 mM L-Arginine; and
   (e) from 0.005% to 0.1% w/v polysorbate 80,
   wherein the composition has a pH from 5.5 to 7.0.
48. The method according to any one of items 1 to 47, wherein the anti-PVRIG treatment antibody comprises a CH1-hinge-CH2-CH3 sequence of IgG4 (SEQ ID NO:657 or SEQ ID NO:658), wherein the hinge region optionally comprises mutations.
49. The method according to any one of items 1 to 48, wherein the anti-PVRIG antibody comprises the CH1-hinge-CH2-CH3 region from IgG1, IgG2, IgG3, or IgG4, wherein the hinge region optionally comprises mutations.
50. The method according to any one of items 1 to 49, wherein the heavy chain variable domain is from the heavy chain of CHA.7.518.1.H4(S241P) (SEQ ID NO:8) and the light chain variable domain is from the light chain of CHA.7.518.1.H4(S241P) (SEQ ID NO:13).
51. The method according to any one of items 1 to 50, wherein the anti-PVRIG antibody comprises a CL region of human kappa 2 light chain.
52. The method according to any one of items 47 to 51, wherein the stable liquid formulation comprises from 10 mM to 80 mM histidine, from 15 mM to 70 mM histidine, from 20 mM to 60 mM histidine, from 20 mM to 50 mM histidine, or from 20 mM to 30 mM histidine.
53. The method according to any one of items 47 to 52, wherein the pharmaceutical formulation comprises about 25 mM histidine.
54. The method according to any one of items 47 to 53, wherein the pharmaceutical formulation comprises from 30 mM to 100 mM NaCl, from 30 mM to 90 mM NaCl, from 40 mM to 80 mM NaCl, from 30 mM to 70 mM histidine, or from 45 mM to 70 mM NaCl.
55. The method according to any one of items 47 to 54, wherein the pharmaceutical formulation comprises about 60 mM NaCl.
56. The method according to any one of items 47 to 55, wherein the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine.
57. The method according to any one of items 47 to 56, wherein the pharmaceutical formulation comprises about 100 mM L-arginine.
58. The method according to any one of items 47 to 57, wherein the pharmaceutical formulation comprises from 0.006% to 0.1% w/v polysorbate 80, from 0.007% to 0.09% w/v polysorbate 80, from 0.008% to 0.08% w/v polysorbate 80, from 0.009% to 0.09% w/v polysorbate 80, from 0.01% to 0.08% w/v polysorbate 80, from 0.01% to 0.07% w/v polysorbate 80, from 0.01% to 0.07% w/v polysorbate 80, or from 0.01% to 0.06% w/v polysorbate 80, or from 0.009% to 0.05% w/v polysorbate 80.
59. The method according to any one of items 47 to 58, wherein the pharmaceutical formulation comprises about 0.01% polysorbate 80.
60. The method according to any one of items 47 to 59, wherein the pH is from 6 to 7.0.
61. The method according to any one of items 47 to 60, wherein the pH is from 6.3 to 6.8.
62. The method according to any one of items 47 to 61, wherein the pH is 6.5 +/- 0.2.
63. The method according to any one of items 47 to 62, wherein the anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL.
64. The method according to any one of items 47 to 63, wherein the anti-PVRIG antibody is at a concentration of about 20 mg/mL.
65. The method according to any one of items 47 to 64, wherein the anti-PVRIG antibody formulation comprises:
   a) a heavy chain comprising:
      i) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   b) a light chain comprising:
      i) a VL-CL, wherein the VL from CHA.7.518.1.H4(S241P) (SEQ ID NO:9) and wherein the CL region is from human kappa 2 light chain.
66. The method according to item 65, wherein the hinge region optionally comprises mutations.
67. The method according to item 66, wherein the hinge region optionally comprises mutations.
68. The method according to any one of items 1 to 67, wherein the anti-PVRIG antibody formulation comprises:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13).
69. The method according to any one of items 1 to 67, the anti-PVRIG antibody formulation comprising:
   (a) an anti-PVRIG antibody, wherein the anti-PVRIG antibody comprises:
      i) a heavy chain variable domain comprising the vhCDR1, vhCDR2, and vhCDR3 from the heavy chain of CHA.7.518.1.H4(S241P) (SEQ ID NO:8), and
      ii) a light chain variable domain comprising the vlCDR1, vlCDR2, and vlCDR3 from the light chain of CHA.7.518.1.H4(S241P) (SEQ ID NO:13);
   (b) about 25 mM histidine;
   (c) about 60 mM NaCl;
   (d) about 100 mM L-Arginine; and
   (e) about 0.01% % w/v polysorbate 80,
   wherein the composition has a pH from 6.5 +/- 0.2.
70. The method according to any one of items 1 to 67, the anti-PVRIG antibody formulation comprising:
   (a) an anti-PVRIG antibody, wherein the anti-PVRIG antibody comprises:
      i) a heavy chain comprising the heavy chain from CHA.7.518.1.H4(S241P) (SEQ ID NO:8); and
      ii) a light chain comprising the light chain from CHA.7.518.1.H4(S241P) (SEQ ID NO: 13);
   (b) about 25 mM histidine;
   (c) about 60 mM NaCl;
   (d) about 100 mM L-Arginine; and
   (e) about 0.01% % w/v polysorbate 80,
   wherein the composition has a pH from 6.5 +/- 0.2.
71. The method according to any one of items 1 to 67, wherein the anti-PVRIG treatment antibody is administered at a dosage of about 0.01 mg/kg to about 20 mg/kg of the anti-PVRIG antibody or about 0.01 mg/kg to about 10 mg/kg of the anti-PVRIG antibody.
72. The method according to any one of items 1 to 67, wherein the anti-PVRIG treatment antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg of the anti-PVRIG antibody.
73. The method according to any one of items 1 to 67, wherein the anti-PVRIG treatment antibody is administered 20 mg/kg every 4 weeks.
74. The method according to any one of items 1 to 73, wherein the cancer selected from the group consisting of prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, and/or fallopian tube cancer.
75. The method according to any one of items 1 to 74, wherein the anti-PVRIG antibody is administered in combination with an anti-PD-1 antibody.
76. The method according to any one of items 1 to 74, wherein the anti-PVRIG antibody is administered in combination with an anti-PD-L1 antibody.
77. The method according to any one of items 1 to 74, wherein the anti-PVRIG antibody is administered in combination with an anti-TIGIT antibody.
78. The method according to any one of items 1 to 74, wherein the anti-PVRIG antibody is administered in combination with an anti-PD-1 antibody and an anti-TIGIT antibody.
79. The method according to any one of items 1 to 74, wherein the anti-PVRIG antibody is administered in combination with an anti-PD-L1 antibody and an anti-TIGIT antibody.
80. The method according to item 75 or 78, wherein the anti-PD1 antibody is selected from the group consisting of nivolumab and pembrolizumab.
81. The method according to item 76 or 79, wherein the anti-PD-L1 antibody is selected from the group consisting of atezolizumab, avelumab, durvalumab, KN035(Envafolimab), and CK-301 (Cosibelimab).
82. The method according to item 77, the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.
83. The method according to item 77, the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.
84. The method according to any one of items 77 to 83, wherein the anti-TIGIT antibody is selected from the group consisting of CPA.9.083.H4(S241P) and CPA.9.086.H4(S241P).
85. The method according to any one of items 77 to 84, wherein the anti-TIGIT antibody comprises:
   a) a heavy chain comprising VH-CH1-hinge-CH2-CH3; and
   b) a light chain comprising VL-VC, wherein VC is either kappa or lambda.
86. The method according to item 85, wherein the sequence of the CH1-hinge-CH2-CH3 is selected from human IgG1, IgG2 and IgG4, and variants thereof.
87. The method according to any one of items 1 to 86, wherein the anti-PVRIG antibody is CHA.7.518.1.H4(S241P), the anti-TIGIT antibody is CPA.9.086.H4(S241P) and the anti-PD-1 antibody is nivolumab.
88. The method according to any one of items 1 to 86, wherein the anti-PVRIG antibody is CHA.7.518.1.H4(S241P), the anti-TIGIT antibody is CPA.9.083.H4(S241P) and the anti-PD-1 antibody is nivolumab.
89. The method according to any one of items 1 to 86, wherein the anti-PVRIG antibody is CHA.7.518.1.H4(S241P), the anti-TIGIT antibody is CPA.9.086.H4(S241P) and the anti-PD-1 antibody is pembrolizumab.
90. The method according to any one of items 1 to 86, wherein the anti-PVRIG antibody is CHA.7.518.1.H4(S241P), the anti-TIGIT antibody is CPA.9.083.H4(S241P) and the anti-PD-1 antibody is pembrolizumab.
91. The method according to any one of items 1 to 86, wherein the anti-PVRIG antibody is CHA.7.538.1.2.H4(S241P), the anti-TIGIT antibody is CPA.9.086.H4(S241P) and the anti-PD-1 antibody is nivolumab.
92. The method according to any one of items 1 to 86, wherein the anti-PVRIG antibody is CHA.7.538.1.2.H4(S241P), the anti-TIGIT antibody is CPA.9.083.H4(S241P) and the anti-PD-1 antibody is nivolumab.
93. The method according to any one of items 1 to 86, wherein the anti-PVRIG antibody is CHA.7.538.1.2.H4(S241P), the anti-TIGIT antibody is CPA.9.086.H4(S241P) and the anti-PD-1 antibody is pembrolizumab.
94. The method according to any one of items 1 to 86, wherein the anti-PVRIG antibody is CHA.7.538.1.2.H4(S241P), the anti-TIGIT antibody is CPA.9.083.H4(S241P) and the anti-PD-1 antibody is pembrolizumab.
95. The method according to any one of items 1 to 86, wherein the anti-PVRIG antibody is CHA.7.518.1.H4(S241P), the anti-TIGIT antibody is CPA.9.086.H4(S241P) and the anti-PD-L1 antibody is selected from the group consisting of atezolizumab, avelumab, durvalumab, KN035(Envafolimab), and CK-301 (Cosibelimab).
96. The method according to any one of items 1 to 86, wherein the anti-PVRIG antibody is CHA.7.518.1.H4(S241P), the anti-TIGIT antibody is CPA.9.083.H4(S241P) and the anti-PD-L1 antibody is selected from the group consisting of atezolizumab, avelumab, durvalumab, KN035(Envafolimab), and CK-301 (Cosibelimab).
97. The method according to any one of items 1 to 86, wherein the anti-PVRIG antibody is CHA.7.538.1.2.H4(S241P), the anti-TIGIT antibody is CPA.9.086.H4(S241P) and the anti-PD-L1 antibody is selected from the group consisting of atezolizumab, avelumab, durvalumab, KN035(Envafolimab), and CK-301 (Cosibelimab).
98. The method according to any one of items 1 to 86, wherein the anti-PVRIG antibody is CHA.7.538.1.2.H4(S241P), the anti-TIGIT antibody is CPA.9.083.H4(S241P) and the anti-PD-L1 antibody is selected from the group consisting of atezolizumab, avelumab, durvalumab, KN035(Envafolimab), and CK-301 (Cosibelimab).
99. An agent for use in a method, including a method of treatment, wherein the agent is capable of blocking the interaction between PVRIG and PVRL2.
100. The method according to item 99, wherein the agent capable of blocking the interaction between PVRIG and PVRL2 comprises an anti-PVRIG antibody.
101. The method according to any one of items 99 to 100, wherein the agent capable of blocking the interaction between PVRIG and PVRL2 increases the interaction between PVRL2 with DNAM-1 by:
   i) reducing the competition between PVRIG and DNAM-1 for binding to PVRL2 by between about 1-fold to about 10-fold.
102. The method according to any one of items 99 to 101, wherein the blocking reduces the competition between PVRIG and DNAM-1 for binding to PVRL2 and/or increases the interaction between PVRL2 and DNAM-1, as compared to a control or a patient that is not treated with the agent capable of blocking the interaction between PVRIG and PVRL2.
103. A method for determining a cancer patient population for treatment with an anti-PVRIG antibody, the method comprising:
   (a) detecting the presence of DNAM-1 and/or PVRIG in a biological sample from the cancer patient;
   (b) quantitating the measurement of the level of DNAM-1 and/or PVRIG; and
   (c) treating the cancer patient with an anti-PVRIG antibody when PVRIG in (a) as quantitated in step (b) are present and/or present at an increased level as compared to a control or a patient that does not have detectable levels of DNAM-1 and/or PVRIG.
104. A method for predicting or determining the efficacy of treatment for a cancer patient with an anti-PVRIG treatment antibody, the method comprising:
   (a) measuring the level of DNAM-1 and/or PVRIG;
   (b) quantitating the measurement of the level of DNAM-1 and/or PVRIG; and
   (c) correlating the level of the DNAM-1 and/or PVRIG with the efficacy of treatment, wherein any of DNAM-1 and/or PVRIG being present and/or being present at an increased level as compared to a control or a patient that does not have detectable levels of DNAM-1 and/or PVRIG, is indicative of treatment efficacy for treatment with an anti-PVRIG treatment antibody.
105. The method according to item 104, wherein the method further comprises:
   (d) treating the cancer patient with an anti-PVRIG antibody when DNAM-1 and/or PVRIG in (a) are present at an increased level as compared to a control or a patient that does not have detectable levels of the cells.
106. The method of items 103 to 105, wherein the biological sample comprises immune cells.
107. The method according to any one of items 106, wherein the immune cells are selected from the group consisting of NK cells, NKT cells, gamma-delta T cells, T cells, CD4 positive T cells, and CD8 positive T cells.
108. The method according to item 106, wherein the immune cells are NK cells, NKT cells, and/or gamma-delta T cells.
109. The method according to item 106, wherein the immune cells are selected from the group consisting of CD4 positive T cells and CD8 positive T cells.
110. The method according to item 109, wherein the T cells are selected from the group consisting of TSCM, TRM, naive, exhausted, cycling, memory, effector CD8 positive cells, and effector CD4 positive T cells.
111. The method according to any one of items 103 to 110, wherein the immune cells co-express PVRIG and DNAM-1.
112. A method for predicting or determining the efficacy of treatment or determining a population for treatment with an anti-PVRIG treatment antibody when the immune cells that express DNAM-1 and/or PVRIG comprise at least 0.1%, at least 0.5%, at least 1%, at least 1.5%, at least 2%, at least 2.5%, at least 3%, at least 3.5%, at least 4%, at least 4.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85% of the total immune cells in the biological sample is indicative of treatment efficacy and/or indicative for treatment with an anti-PVRIG antibody.
113. A method for predicting or determining the efficacy of treatment or determining a population for treatment with an anti-PVRIG treatment antibody when the T cells, NK cells, and NKT cells in the immune cells that express DNAM-1 and/or PVRIG comprise at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total T cells, NK cells, and NKT cells in the biological sample is indicative of treatment efficacy and/or indicative for treatment with an anti-PVRIG antibody.
114. The method according to any one of items 110 to 113, wherein the presence of and/or an increased level as compared to an untreated control and/or to a control prior to treatment of DNAM-1 and/or PVRIG, is indicative and/or predictive of anti-PVRIG treatment efficacy.
115. The method according to any one of items 103 to 114, wherein the biological sample is obtained from a tumor, tumor microenvironment, and/or peripheral blood from the cancer patient.
116. A method for determining a cancer patient population for treatment with an anti-PVRIG treatment antibody, the method comprising:
   (a) measuring the level of PVRIG and/or DNAM-1 expression in a biological sample obtained from a cancer and/or tumor microenvironment and/or peripheral blood in the cancer patient,
   (b) quantitating the measurement of the level of PVRIG and/or DNAM-1 expression in the biological sample, and
   (c) treating the cancer patient with an anti-PVRIG antibody wherein PVRIG and/or DNAM-1 is expressed and/or expressed at an increased level as compared to a control or a patient that does not have detectable levels of the PVRIG and/or DNAM-1 in the biological sample.
117. A method for predicting or determining the efficacy of treatment for a cancer patient with an anti-PVRIG treatment antibody, the method comprising:
   (a) measuring the level of PVRIG and/or DNAM-1 expression in a biological sample obtained from a cancer and/or tumor microenvironment and/or peripheral blood in the cancer patient,
   (b) quantitating the measurement of the level of PVRIG and/or DNAM-1 expression in the biological sample, and
   (c) correlating the level of PVRIG and/or DNAM-1 with the efficacy of treatment, wherein PVRIG and/or DNAM-1 is expressed and/or expressed at an increased level as compared to a control or a patient that does not have detectable levels of the PVRIG and/or DNAM-1, is indicative of treatment efficacy for treatment with an anti-PVRIG treatment antibody.
118. A method for altering the regimen of treatment for a patient with cancer, the method comprising:
   (a) measuring the level of PVRIG and/or DNAM-1 expression in a biological sample obtained from a cancer and/or tumor microenvironment and/or peripheral blood in the cancer patient,
   (b) quantitating the measurement of the level of PVRIG and/or DNAM-1 expression in the biological sample, wherein PVRIG and/or DNAM-1 being expressed and/or being expressed at an increased level as compared to a control or a patient that does not have detectable levels of the PVRIG and/or DNAM-1 is indicative of treatment efficacy,
   (c) correlating the level of PVRIG and/or DNAM-1 expression with the efficacy of treatment, wherein a high level of PVRIG and/or DNAM-1 expression is indicative of altering the treatment regimen for treatment with anti-PVRIG treatment antibody, and
   (d) altering the treatment regimen to include an anti-PVRIG antibody when a moderate or high level of PVRIG and/or DNAM-1 expression is measured.
119. The method according to any one of items 99 to 118, wherein the anti-PVRIG antibody comprises the vhCDR1, vhCDR2, vhCDR3, vlCDR1, vlCDR2, and vlCDR3 sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.520.1, CHA.7.520.2, CHA.7.522, CHA.7.524, CHA.7.526, CHA.7.527, CHA.7.528, CHA.7.530, CHA.7.534, CHA.7.535, CHA.7.537, CHA.7.538.1.2.H4(S241P), CHA.7.538.1, CHA.7.538.2, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.
120. The method according to any one of items 99 to 118, wherein the anti-PVRIG antibody comprises the variable heavy domain and the variable light domain sequences from an antibody selected from the group consisting of CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.518.1, CHA.7.518.2, CHA.7.518.3, CHA.7.518.4, CHA.7.518.5, CHA.7.520.1, CHA.7.520.2, CHA.7.522, CHA.7.524, CHA.7.524.1, CHA.7.524.2, CHA.7.524.3, CHA.7.524.4, CHA.7.526, CHA.7.527, CHA.7.528, CHA.7.530, CHA.7.530.1, CHA.7.530.2, CHA.7.530.3, CHA.7.530.4, CHA.7.530.5, CHA.7.534, CHA.7.535, CHA.7.537, CHA.7.538.1.2.H4(S241P), CHA.7.538.1, CHA.7.538.1.1, CHA.7.538.1.2, CHA.7.538.1.3, CHA.7.538.1.4, CHA.7.538.2, CHA.7.538.2.1, CHA.7.538.2.2, CHA.7.538.2.3, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050.
121. The method according to any one of items 99 to 118, wherein the anti-PVRIG treatment antibody comprises a heavy chain variable domain from the heavy chain of CHA.7.518.1.H4(S241P) (SEQ ID NO:8) and a light chain variable domain from the light chain of CHA.7.518.1.H4(S241P) (SEQ ID NO:13).
122. The method according to any one of items 99 to 118, wherein the anti-PVRIG treatment antibody comprises a heavy chain variable domain from the heavy chain of CHA.7.538.1.2.H4(S241P) (SEQ ID NO:266) and a light chain variable domain from the light chain of CHA.7.538.1.2.H4(S241P) (SEQ ID NO:271).
123. The method according to any one of items 99 to 122, wherein the PVRIG and/or DNAM-1 expression is determined using single-cell resolution analysis.
124. The method according to 123, wherein the single-cell resolution analysis includes RNAseq, immunohistochemistry (IHC), multiplex immunohistochemistry (mIHC) and/or immunofluorescence (IF), flow cytometry (e.g., FACS) and mass cytometry (e.g., CyTOF), as well as combinations thereof.
125. The method according to any one of items 99 to 124, wherein the PVRIG and/or DNAM-1 expression is determined using immunohistochemistry.
126. The method according to any one of items 123 to 126, wherein an anti-PVRIG antibody is used for the single-cell resolution analysis and/or immunohistochemistry, and wherein the anti-PVRIG antibody is AB-635 PVRIG Ab (6D8-1 clone).
127. The method according to any one of items 123 to 126, wherein an anti-PVRIG antibody is used for the single-cell resolution analysis and/or immunohistochemistry, and wherein the anti-PVRIG antibody is 6D8-1 (heavy chain SEQ ID NO:659 and light chain SEQ ID NO:663).
128. The method according to any one of items 123 to 126, wherein an anti-PVRIG antibody is used for the single-cell resolution analysis and/or immunohistochemistry, and wherein the PVRIG antibody used for the single-cell resolution analysis and/or immunohistochemistry comprises:
   i) a heavy chain variable domain comprising the vhCDR1, vhCDR2, and vhCDR3 from the heavy chain of 6D8-1 (SEQ ID NO:659), and
   ii) a light chain variable domain comprising the vlCDR1, vlCDR2, and vlCDR3 from the light chain of 6D8-1 (SEQ ID NO:663).
129. The method according to any one of items 123 to 128, wherein an anti-DNAM-1 antibody is used for the single-cell resolution analysis and/or immunohistochemistry, and wherein the DNAM-1 antibody used for the immunohistochemistry analysis is selected from the group consisting of DNAM-1/CD226 (E8L9G) XP^{®} Rabbit mAb #66631, Mouse Anti-CD226 Recombinant Antibody (clone MM0248-1X20), Cluster of Differentiation 226 (CD226) Antibody abx171784, and Recombinant Anti-CD226 antibody [EPR20710] (ab212077).
130. The method according to any one of items 99 to 129, wherein the DNAM-1 expression level is categorized as strong, moderate, weak, or negative.
131. The method according to any one of items 99 to 129, wherein the DNAM-1 expression is categorized as strong or moderate.
132. The method according to any one of items 99 to 129, wherein the DNAM-1 expression level is categorized as 0-no signal, 1-low, 2-medium, 3- high.
133. The method according to any one of items 99 to 129, wherein the DNAM-1 expression is categorized as 2-medium or 3- high.
134. The method according to any one of items 99 to 133, wherein a cancer patient having immune cells in the tumor, tumor microenvironment, and/or peripheral blood having at least 3%, 5%, 10%, 20%, 30%, 40%, 50%, 60%, 70%, 80%, 90%, or 100% DNAM-1 expression exhibits a higher probability to respond the anti-PVRIG antibody treatment as compared to a cancer patient with lower DNAM-1 expression.
135. The method according to any one of items 99 to 133, wherein a cancer patient having CD4 positive T cells and/or CD8 positive T cells in the tumor, tumor microenvironment, and/or peripheral blood having at least 3%, 5%, 10%, 20%, 30%, 40%, 50%, 60%, 70%, 80%, 90%, or 100% DNAM-1 expression exhibits a higher probability to respond the anti-PVRIG antibody treatment as compared to a cancer patient with lower DNAM-1 expression
136. The method according to any one of items 99 to 133, wherein a cancer patient having at least 3%, 5%, 10%, 20%, 30%, 40%, 50%, 60%, 70%, 80%, 90%, or 100% of CD4 positive T cells and/or CD8 positive T cells in the tumor, tumor microenvironment, and/or peripheral blood expressing PVRIG and DNAM-1 exhibits a higher probability to respond to the anti-PVRIG antibody treatment as compared to a cancer patient with lower PVRIG and/or DNAM-1 expression.
137. The method according to any one of items 99 to 133, wherein a cancer patient having at least 3%, 5%, 10%, 20%, 30%, 40%, 50%, 60%, 70%, 80%, 90%, or 100% of effector CD8 and/or CD4 positive T cells in the tumor, tumor microenvironment, and/or peripheral blood expressing PVRIG and DNAM-1 exhibits a higher probability to respond to the anti-PVRIG antibody treatment as compared to cancer patient with lower PVRIG and/or DNAM-1 expression.
138. The method according to any one of items 99 to 133, wherein a cancer patient having more than 2 copies of DNAM-1 in cells obtained from the tumor, tumor microenvironment, and/or peripheral blood exhibits a higher probability to respond to the anti-PVRIG antibody treatment as compared to cancer patient with only 2 copies of DNAM-1 in cells obtained from the tumor, tumor microenvironment, and/or peripheral blood.
139. The method according to any one of items 134 to 138, wherein the immune cells are NK cells, NKT cells, and/or gamma-delta T cells.
140. The method according to any one of items 134 to 138, wherein the immune cells are CD4 positive T cells and/or CD8 positive T cells.
141. The method according to any one of items 134 to 138, wherein the T cells are selected from the group consisting of TSCM, TRM, naive, exhausted, cycling, memory, effector CD8 positive T cells, and effector CD4 positive T cells.
142. The method according to any one of items 99 to 141, wherein the anti-PVRIG treatment antibody is administered as a stable liquid pharmaceutical formulation of the anti-PVRIG antibody comprising:
   (a) an anti-PVRIG antibody, wherein the anti-PVRIG antibody comprises:
      i) a heavy chain variable domain comprising the vhCDR1, vhCDR2, and vhCDR3 from the heavy chain of CHA.7.518.1.H4(S241P) (SEQ ID NO:8), and
      ii) a light chain variable domain comprising the vlCDR1, vlCDR2, and vlCDR3 from the light chain of CHA.7.518.1.H4(S241P) (SEQ ID NO:13);
   (b) from 10 mM to 100 mM histidine;
   (c) from 30 mM to 100 mM NaCl;
   (d) from 20 mM to 150 mM L-Arginine; and
   (e) from 0.005% to 0.1% w/v polysorbate 80,
   wherein the composition has a pH from 5.5 to 7.0.
143. The method according to any one of items 99 to 142, wherein the anti-PVRIG treatment antibody comprises a CH1-hinge-CH2-CH3 sequence of IgG4 (SEQ ID NO:657 or SEQ ID NO:658), wherein the hinge region optionally comprises mutations.
144. The method according to any one of items 99 to 143, wherein the anti-PVRIG antibody comprises a CH1-hinge-CH2-CH3 region from IgG1, IgG2, IgG3, or IgG4, wherein the hinge region optionally comprises mutations.
145. The method according to any one of items 99 to 144, wherein the heavy chain variable domain of the anti-PVRIG antibody is from the heavy chain of CHA.7.518.1.H4(S241P) (SEQ ID NO:8) and the light chain variable domain of the anti-PVRIG antibody is from the light chain of CHA.7.518.1.H4(S241P) (SEQ ID NO:13).
146. The method according to any one of items 99 to 145, wherein the anti-PVRIG antibody comprises a CL region of human kappa 2 light chain.
147. The method according to any one of items 142 to 146, wherein the stable liquid formulation comprises from 10 mM to 80 mM histidine, from 15 mM to 70 mM histidine, from 20 mM to 60 mM histidine, from 20 mM to 50 mM histidine, or from 20 mM to 30 mM histidine.
148. The method according to any one of items 142 to 147, wherein the pharmaceutical formulation comprises about 25 mM histidine.
149. The method according to any one of items 142 to 148, wherein the pharmaceutical formulation comprises from 30 mM to 100 mM NaCl, from 30 mM to 90 mM NaCl, from 40 mM to 80 mM NaCl, from 30 mM to 70 mM histidine, or from 45 mM to 70 mM NaCl.
150. The method according to any one of items 142 to 149, wherein the pharmaceutical formulation comprises about 60 mM NaCl.
151. The method according to any one of items 142 to 150, wherein the pharmaceutical formulation comprises from 20 mM to 140 mM L-arginine, from 30 mM to 140 mM L-arginine, from 40 mM to 130 mM L-arginine, from 50 mM to 120 mM L-arginine, from 60 mM to 110 mM L-arginine, from 70 mM to 110 mM L-arginine, from 80 mM to 110 mM L-arginine, or from 90 mM to 110 mM L-arginine.
152. The method according to any one of items 142 to 151, wherein the pharmaceutical formulation comprises about 100 mM L-arginine.
153. The method according to any one of items 142 to 152, wherein the pharmaceutical formulation comprises from 0.006% to 0.1% w/v polysorbate 80, from 0.007% to 0.09% w/v polysorbate 80, from 0.008% to 0.08% w/v polysorbate 80, from 0.009% to 0.09% w/v polysorbate 80, from 0.01% to 0.08% w/v polysorbate 80, from 0.01% to 0.07% w/v polysorbate 80, from 0.01% to 0.07% w/v polysorbate 80, or from 0.01% to 0.06% w/v polysorbate 80, or from 0.009% to 0.05% w/v polysorbate 80.
154. The method according to any one of items 142 to 153, wherein the pharmaceutical formulation comprises about 0.01% polysorbate 80.
155. The method according to any one of items 142 to 154, wherein the pH is from 6 to 7.0.
156. The method according to any one of items 142 to 155, wherein the pH is from 6.3 to 6.8.
157. The method according to any one of items 142 to 156, wherein the pH is 6.5 +/- 0.2.
158. The method according to any one of items 142 to 157, wherein the anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL.
159. The method according to any one of items 142 to 158, wherein the anti-PVRIG antibody is at a concentration of about 20 mg/mL.
160. The method according to any one of items 142 to 159, wherein the anti-PVRIG antibody formulation comprises:
   a) a heavy chain comprising:
      i) a VH-CH1-hinge-CH2-CH3, wherein the VH is from CHA.7.518.1.H4(S241P) (SEQ ID NO:4) and wherein the CH1-hinge-CH2-CH3 region is from IgG4; and
   b) a light chain comprising:
      i) a VL-CL, wherein the VL from CHA.7.518.1.H4(S241P) (SEQ ID NO:9) and wherein the CL region is from human kappa 2 light chain.
161. The method according to item 160, wherein the hinge region optionally comprises mutations.
162. The method according to item 160, wherein the hinge region optionally comprises mutations.
163. The method according to any one of items 100 to 162, wherein the anti-PVRIG antibody formulation comprises:
   i) a heavy chain comprising the heavy chain from CHA.7.518.1.H4(S241P) (SEQ ID NO:8); and
   ii) a light chain comprising the light chain from CHA.7.518.1.H4(S241P) (SEQ ID NO: 13).
164. The method according to any one of items 100 to 163, the anti-PVRIG antibody formulation comprising:
   (a) an anti-PVRIG antibody, wherein the anti-PVRIG antibody comprises:
      i) a heavy chain variable domain comprising the vhCDR1, vhCDR2, and vhCDR3 from the heavy chain of CHA.7.518.1.H4(S241P) (SEQ ID NO:8), and
      ii) a light chain variable domain comprising the vlCDR1, vlCDR2, and vlCDR3 from the light chain of CHA.7.518.1.H4(S241P) (SEQ ID NO:13);
   (b) about 25 mM histidine;
   (c) about 60 mM NaCl;
   (d) about 100 mM L-Arginine; and
   (e) about 0.01% % w/v polysorbate 80,
   wherein the composition has a pH from 6.5 +/- 0.2.
165. The method according to any one of items 100 to 163, the anti-PVRIG antibody formulation comprising:
   (a) an anti-PVRIG antibody, wherein the anti-PVRIG antibody comprises:
      i) a heavy chain comprising the heavy chain from CHA.7.518.1.H4(S241P) (SEQ ID NO:8); and
      ii) a light chain comprising the light chain from CHA.7.518.1.H4(S241P) (SEQ ID NO: 13);
   (b) about 25 mM histidine;
   (c) about 60 mM NaCl;
   (d) about 100 mM L-Arginine; and
   (e) about 0.01% % w/v polysorbate 80,
   wherein the composition has a pH from 6.5 +/- 0.2.
166. The method according to any one of items 100 to 165, wherein the anti-PVRIG treatment antibody is administered at a dosage of about 0.01 mg/kg to about 20 mg/kg of the anti-PVRIG antibody or about 0.01 mg/kg to about 10 mg/kg of the anti-PVRIG antibody.
167. The method according to any one of items 100 to 165, wherein the anti-PVRIG treatment antibody is administered at a dosage of about 0.01 mg/kg, 0.03 mg/kg, 0.1 mg/kg, 0.3 mg/kg, 1 mg/kg, 3 mg/kg, 10 mg/kg, or 20 mg/kg of the anti-PVRIG antibody.
168. The method according to any one of items 100 to 167, wherein the anti-PVRIG treatment antibody is administered 20 mg/kg every 4 weeks.
169. The method according to any one of items 99 to 168, wherein the cancer is selected from the group consisting of prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, and/or fallopian tube cancer.
170. The method according to any one of items 100 to 169, wherein the anti-PVRIG antibody is administered in combination with an anti-PD-1 antibody.
171. The method according to any one of items 100 to 169, wherein the anti-PVRIG antibody is administered in combination with an anti-PD-L1 antibody.
172. The method according to any one of items 100 to 169, wherein the anti-PVRIG antibody is administered in combination with an anti-TIGIT antibody.
173. The method according to any one of items 100 to 169, wherein the anti-PVRIG antibody is administered in combination with an anti-PD-1 antibody and an anti-TIGIT antibody.
174. The method according to any one of items 100 to 169, wherein the anti-PVRIG antibody is administered in combination with an anti-PD-L1 antibody and an anti-TIGIT antibody.
175. The method according items 170 or 173, wherein the anti-PD1 antibody is selected from the group consisting of nivolumab and pembrolizumab.
176. The method according items 171 or 174, wherein the anti-PD-L1 antibody is selected from the group consisting of atezolizumab, avelumab, durvalumab, KN035(Envafolimab), and CK-301 (Cosibelimab).
177. The method according to item 172, wherein the anti-TIGIT antibody comprises the vhCDR1, vhCDR2, vhCDR3, vlCDR1, vlCDR2, and vlCDR3 sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.
178. The method according to item 172, wherein the anti-TIGIT antibody comprises the variable heavy domain and the variable light domain sequences from an antibody selected from the group consisting of CPA.9.083.H4(S241P), CPA.9.086.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7, and CHA.9.541.8.
179. The method according to any one of items 172 or 177 to 178, wherein the anti-TIGIT antibody is selected from the group consisting of CPA.9.083.H4(S241P) and CPA.9.086.H4(S241P).
180. The method according to any one of items 172 or 177 to 179, wherein the anti-TIGIT antibody comprises:
   a) a heavy chain comprising VH-CH1-hinge-CH2-CH3; and
   b) a light chain comprising VL-VC, wherein VC is either kappa or lambda.
181. The method according to item 180, wherein the sequence of the CH1-hinge-CH2-CH3 is selected from human IgG1, IgG2 and IgG4, and variants thereof.
182. The method according to any one of items 100 to 181, wherein the anti-PVRIG antibody is CHA.7.518.1.H4(S241P), the anti-TIGIT antibody is CPA.9.086.H4(S241P) and the anti-PD-1 antibody is nivolumab.
183. The method according to any one of items 100 to 181, wherein the anti-PVRIG antibody is CHA.7.518.1.H4(S241P), the anti-TIGIT antibody is CPA.9.083.H4(S241P) and the anti-PD-1 antibody is nivolumab.
184. The method according to any one of items 100 to 181, wherein the anti-PVRIG antibody is CHA.7.518.1.H4(S241P), the anti-TIGIT antibody is CPA.9.086.H4(S241P) and the anti-PD-1 antibody is pembrolizumab.
185. The method according to any one of items 100 to 181, wherein the anti-PVRIG antibody is CHA.7.518.1.H4(S241P), the anti-TIGIT antibody is CPA.9.083.H4(S241P) and the anti-PD-1 antibody is pembrolizumab.
186. The method according to any one of items 100 to 181, wherein the anti-PVRIG antibody is CHA.7.538.1.2.H4(S241P), the anti-TIGIT antibody is CPA.9.086.H4(S241P) and the anti-PD-1 antibody is nivolumab.
187. The method according to any one of items 100 to 181, wherein the anti-PVRIG antibody is CHA.7.538.1.2.H4(S241P), the anti-TIGIT antibody is CPA.9.083.H4(S241P) and the anti-PD-1 antibody is nivolumab.
188. The method according to any one of items 100 to 181, wherein the anti-PVRIG antibody is CHA.7.538.1.2.H4(S241P), the anti-TIGIT antibody is CPA.9.086.H4(S241P) and the anti-PD-1 antibody is pembrolizumab.
189. The method according to any one of items 100 to 181, wherein the anti-PVRIG antibody is CHA.7.538.1.2.H4(S241P), the anti-TIGIT antibody is CPA.9.083.H4(S241P) and the anti-PD-1 antibody is pembrolizumab.
190. The method according to any one of items 100 to 181, wherein the anti-PVRIG antibody is CHA.7.518.1.H4(S241P), the anti-TIGIT antibody is CPA.9.086.H4(S241P) and the anti-PD-L1 antibody is selected from the group consisting of atezolizumab, avelumab, durvalumab, KN035(Envafolimab), and CK-301 (Cosibelimab).
191. The method according to any one of items 100 to 181, wherein the anti-PVRIG antibody is CHA.7.518.1.H4(S241P), the anti-TIGIT antibody is CPA.9.083.H4(S241P) and the anti-PD-L1 antibody is selected from the group consisting of atezolizumab, avelumab, durvalumab, KN035(Envafolimab), and CK-301 (Cosibelimab).
192. The method according to any one of items 100 to 181, wherein the anti-PVRIG antibody is CHA.7.538.1.2.H4(S241P), the anti-TIGIT antibody is CPA.9.086.H4(S241P) and the anti-PD-L1 antibody is selected from the group consisting of atezolizumab, avelumab, durvalumab, KN035(Envafolimab), and CK-301 (Cosibelimab).
193. The method according to any one of items 100 to 181, wherein the anti-PVRIG antibody is CHA.7.538.1.2.H4(S241P), the anti-TIGIT antibody is CPA.9.083.H4(S241P) and the anti-PD-L1 antibody is selected from the group consisting of atezolizumab, avelumab, durvalumab, KN035(Envafolimab), and CK-301 (Cosibelimab).
194. The method according to any one of the preceding items, wherein the activated DC cells, DC1, and/or DC2 express CXCL10.
195. The method according to any one of the preceding items, wherein the early memory CD8 T cells include CD8 T cells expressing CXCR3.

### EXAMPLES

The embodiments encompassed herein are now described with reference to the following examples. These examples are provided for the purpose of illustration only and the disclosure encompassed herein should in no way be construed as being limited to these examples, but rather should be construed to encompass any and all variations which become evident as a result of the teachings provided herein.

### EXAMPLE 1: ASSESSMENT OF PVRIG AND/OR PVRL2 EXPRESSION USING IHC

### Determination of PVRIG/PVRL2 expression using immunohistochemistry (IHC)

PVRIG and/or PVRL2 expression can be determined, for example, using IHC, Immunofluorescence, RNAscope, etc., and used to identify the patient who is predicted to have an increase likelihood of being responsive to treatment with an anti-PVRIG antibody, relative to a patient who does not express the biomarker at the same level and or relative to a patient that does not have the presence of the biomarker.

Antibodies used for IHC included:
- AB-635 PVRIG Ab (6D8-1 clone, by GenScript)
- The CST PVRL2 Ab, Nectin-2/CD112 (D8D3F) XP^{®} Rabbit mAb, is rabbit monoclonal Ab; cat number 95333.

### Monoclonal antibody sequencing of hybridoma 6D8-1

Antibody sequences of 6D8-1 are listed as below:
Heavy chain: DNA sequence (414 bp)
Leader sequence-FF1-CDR1-FF2-CDR2-FR3-CDR3-FR4 
GACGTGAAGTTCGTGGAGTCTGGGGGAGGCTTAGTGAAGGTTGG
AGGGTCCCTGAAACTCTCCTGTGCAGCCTCTGGATTCACTTTCAGTACCTA
TTACATGTCTTGGGTTCGCCAGACTCCAGAGAAGAGGCTGGAGTTGGTCG
CAGTCATTAATCCTACTGGTGGTAGAACCGACTTTCTAGACAGTGTGAGG
GGCCGATTCACCATTTCCAGAGACAATGCCAAGAACACCCTGTACCTGCA
AATGACCAGTCTGAAGTCTGAGGACACAGCCTTGTATCACTGTGCAAGAC
 
GGGGGGAGTTGGGACGGGCTCTGGACTACTGGGGTCAAGGGACCTCAGTC
ACCGTCTCCTCA (SEQ ID NO:1317)
 Heavy chain: Amino acids sequence (138 aa)
Leader sequence-FR1-CDR1-FR2-CDR2-FR3-CDR3-FR4 
DVKFVESGGGLVKVGGSLKLSCAASGFTFSTYY
MSWVRQTPEKRLELVAVINPTGGRTDFLDSVRGRFTISRDNAKNTLYLQMTS
LKSEDTALYHCARRGELGRALDYWGQGTSVTVSS (SEQ ID NO:1318)
 Light chain: DNA sequence (393 bp)
Leader sequence-FR1-CDR1-FR2-CDR2-FR3-CDR3-FR4 
AACATTGTGCTGACCCAATTTCCAGCTTCTTTGGCTGTGTC
TCTAGGACAGAGGGCCTCCATATCCTGCAGAGCCAGTGAAAGTGTTGATA
GTTATGGCTATAGTTTTATTCACTGGTACCAGCAGAAGCCAGGACAGCCA
CCCAAACTCCTCATCTATCTTGCATCCAGCCTAGAATCTGGGGTCCCTGCC
AGGTTCAGTGGCAGTGGGTCTAGGACAGACTTCACCCTCACCATTGATCCT
GTGGAGGCTAATGATGCTGCAACCTATTACTGTCAGCAGAATAATGGGGA
TCCGTGGACGTTCGGTGGAGGCACCAAGCTGGAAATCAAA (SEQ ID
NO:1319)
 Light chain: Amino acids sequence (131 aa)
Leader sequence-FR1-CDR1-FR2-CDR2-FR3-CDR3-FR4

NIVLTQFPASLAVSLGQRASISCRASESVDSYG
YSFIHWYQQKPGQPPKLLIYLASSLESGVPARFSGSGSRTDFTLTIDPVEANDA
ATYYCQQNNGDPWTFGGGTKLEIK (SEQ ID NO:1320)

**Table 4: Sequence Information**

| Sequence | Clones sequenced | Clones with >99% sequence identity |
|---|---|---|
| VH | 5 | 5 |
| VL | 5 | 5 |

Table 4: IMGT Analysis of V(D)J Junctions.

**Table 5**

| **Sequence** | **V-GENE and allele** | **Functio nality** | **V-REGION identity % (nt)** | **J-GENE and allele** | **D-GENE and allele** | **AA Junction** | **Junction frame** |
|---|---|---|---|---|---|---|---|
| VH | Musmus IGHV5-6-2^{∗}0=1 F | product ive | 94.44% (272/288 nt) | Musmus IGHJ4^{∗}01 F | Musmus IGHD3-3^{∗}01 F | CARRGELGR ALDYW | in-frame |
| VL | Musmus IGKV3-10^{∗}01 F | product ive | 96.56% (281/291 nt) | MusmusIGKJ1^{∗}0 1 F | - | CQQNNGDP WTF | in-frame |

### Materials

- Hybridoma cells provided by GenScript;
- TRIzol^{®} Reagent (Ambion, Cat. No. : 15596-026);
- PrimeScriptTM 1st Strand cDNA Synthesis Kit (Takara, Cat. No. : 6110A).

### Methods

- Total RNA was isolated from the hybridoma cells following the technical manual of TRIzol^{®} Reagent. Total RNA was then reverse transcribed into cDNA using isotype-specific anti-sense primers or universal primers following the technical manual of PrimeScriptTM 1st Strand cDNA Synthesis Kit. The antibody fragments of VH and VL were amplified according to the standard operating procedure (SOP) of rapid amplification of cDNA ends (RACE) of GenScript. Amplified antibody fragments were cloned into a standard cloning vector separately. Colony PCR was performed to screen for clones with inserts of correct sizes. No less than five colonies with inserts of correct sizes were sequenced for each fragment. The sequences of different clones were aligned and the consensus sequence of these clones was provided.

### Generation of anti-PVRIG antibody

Generation of mouse monoclonal antibodies against human PVRIG protein was performed at Genscript (USA).

Antibodies against human PVRIG protein were raised by immunizing group of 5 Balbc mice with protein fragment comprised from the intracellular domain (ICD) of PVRIG. The stages included the immunization, cell fusion and screening, subcloning and Abs production and purification.

Abs binding was tested on cells ectopically expressing human PVRIG as well as on several cell lines endogenously expressing PVRIG using deferent methods, western blot, ELISA and IHC.

### Immunohistochemistry (IHC) staining using anti-PVRIG antibody

FFPEs slide tissues were treated with an antigen retrieval buffer (Trilogy Buffer), the tissues then stained with 3ug/ml of mouse anti-human PVRIG monoclonal antibody, Clone AB-635 and a matched universal negative control antibody. An anti-mouse secondary antibody was used to detect anti-human PVRIG Ab or isotype bound to the tissue, and an HRP-polymer detection reagent was added for signal amplification.

### Immunohistochemistry (IHC) staining using anti-PVRL2 antibody

FFPEs slide tissues were treated with an antigen retrieval buffer (Diva Decloaker, Heat-Induced Epitope Retrieval pH 6.2 Citrate-based buffer), the tissues then stained with 0.5ug/ml of rabbit anti-human PVRL2 monoclonal antibody, Clone D8D3F and a matched universal negative control antibody. An anti-rabbit secondary antibody was used to detect anti-human PVRL2 Ab or isotype bound to the tissue, and an HRP-polymer detection reagent was added for signal amplification.

### PVRIG Diagnostic Assessment IHC Scores

Absence of any discernible PVRIG and/or PVRL2 staining IHC 0, or presence of discernible PVRIG and/or PVRL2 staining of any intensity in tumor-infiltrating immune cells identified or associated with intra/peri tumoral, and/or discernible staining in tumor immune aggregates and/or tertiary lymphoid structures.

### Scoring PVRIG Expression by IHC

The presence or absence of PVRIG expression in tumor specimens was evaluated as follows:

We used an anti-PVRIG-specific antibody (described in methods section), that can detect PVRIG in human formalin-fixed, paraffin-embedded (FFPE) tissues by IHC.

To measure and quantify relative expression of PVRIG in tumor samples, a PVRIG IHC scoring system was used (0-no signal, 1-low, 2-medium, 3- high) to measure PVRIG specific signal in tumor cells and tumor infiltrating immune cells, immune aggregates, and TLSs (Table 5 herein). Immune cells are defined as cells with lymphoid and/or macrophage/histiocyte morphology.

There was a wide dynamic range of PVRIG staining intensities in tumor tissues. The signal was also classified as strong, moderate, weak, or negative staining. PVRIG positive samples demonstrate primarily membranous staining in infiltrating immune cells, immune aggregates, and TLSs. PVRIG staining was observed with variable intensity from weak with fine, light-brown membranes to strong with dark-brown thick membranes easily recognized at low magnification. Representative PVRIG positive tumor samples include: ovary cancer, in which immune infiltrating cells and immune aggregates and TLSs showing clear staining for PVRIG in lymphoid cells, based on morphology (data not shown);

The staining in positive cases tends to be focal with respect to spatial distribution and intensity. The percentages immune cells showing staining of any intensity were visually estimated and used to determine PVRIG status (Table 6). An isotype negative control was used to evaluate the presence of background in test samples.

**Table 6**

| PVRL2 | | | | | | | | | | | PVRIG | | | | |
|---|---|---|---|---|---|---|---|---|---|---|---|---|---|---|---|
| | tumor score | | | | immune cell present | immune score | | | | Expression in TLS | %(aboundent/ rare) | immune score | | | |
| | 0 | 1 | 2 | 3 | | 0 | 1 | 2 | 3 | | | 0 | 1 | 2 | 3 |
| Endometrial | | 3 | 5 | 2 | 9/10(positive) | 1 | 4 | 4 | | NR | 6/10 (2 aboundent/4 rare) | 3 | 4 | 2 | |
| NSCLC | | 1 | 6 | 3 | 9/10(positive) | | 3 | 5 | 1 | NR | 7/9(2 aboundent/5 rare) | 2 | 5 | 2 | |
| Carcinoma | | 1 | 5 | 4 | 6/9 (positive) | | 2 | 3 | 1 | 3/10 | 4/6(4 **aboundent)** | 2 | | 4 | |
| CRC metastasis | | 5 | | 2 | 7/7 (positive) | | 4 | 2 | 1 | 5/7 | 6/7(1 aboundent/ 5 rare) | 1 | 5 | 1 | |
| CRC | | 1 | 2 | | 3/3 (positive) | | 2 | | 1 | 0/3 | 2/3(1 aboundent/1 rare) | 1 | 1 | 1 | |
| TNBC | | 1 | 1 | 3 | 5/5(positive) | | 1 | 4 | | 5/5 | 4/5 (2 aboundent/2 rare) | 1 | 2 | 2 | |
| Breast metastasis | | | 2 | 3 | 5/5(positive) | | 1 | 1 | 3 | 4/5 | 3/5(2 aboundent/1 rare) | 2 | 1 | 1 | 1 |

### PVRL2 Diagnostic Assessment IHC Scores

Absence of any discernible PVRL2 staining IHC 0; or presence of discernible PVRL2 staining of any intensity in tumor-infiltrating immune cells identified or associated with intratumoral, and/or contiguous peri-tumoral desmoplastic stroma and/or discernible staining in tumor immune aggregates and/or tertiary lymphoid structures, and/or discernible staining on endothelial cells

### Scoring PVRL2 Expression by IHC

The presence or absence of PVRL2 expression in tumor specimens was evaluated using anti-PVRL2 -specific antibody (CAT # 95333, Cell Signaling Technology) that can detect PVRL2 in human formalin- fixed, paraffin-embedded (FFPE) tissues by IHC. To measure and quantify relative expression of PVRL2 in tumor samples, a PVRL2 IHC scoring system was used (0-no signal, 1-low, 2-medium, 3- high) to measure PVRL2 specific signal in tumor cells and tumor infiltrating immune cells, immune aggregates, TLSs and endothelial cells (Table 5 herein). Immune cells are defined as cells with lymphoid and/or macrophage/histiocyte morphology.

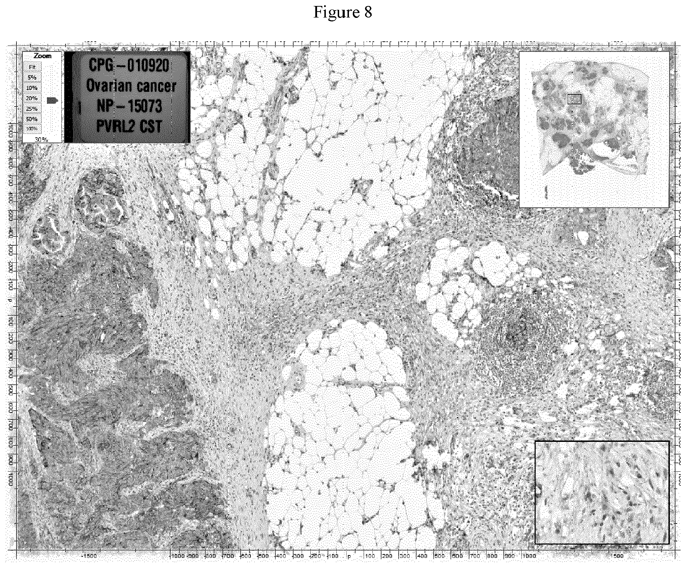

There was a wide dynamic range of PVRL2 staining intensities in tumor tissues. The signal was also classified as strong, moderate, weak, or negative staining. PVRL2 positive samples demonstrate primarily membranous staining in tumor cells and/or infiltrating immune cells, immune aggregates, TLSs and endothelial cells. PVRL2 staining was observed with variable intensity from weak with fine, light-brown membranes to strong with dark- brown thick membranes easily recognized at low magnification. As illustrated in Figure 8, representative PVRL2 positive tumor samples is shown: Ovary cancer, in which most tumor cells are strongly positive for PVRL2, as well as immune infiltrating cells and immune aggregates TLSs and endothelial cells showing a membranous staining (100× magnification).

The staining in positive cases tends to be focal with respect to spatial distribution and intensity. The percentages of tumor or immune cells showing staining of any intensity were visually estimated and used to determine PVRL2 status (Table 5). An isotype negative control was used to evaluate the presence of background in test samples.

In some cases, the PVRL2 positive status comprised the presence of discernible PVRL2 staining of any intensity in either tumor cells or tumor infiltrating immune cells in up to 50% of tumor area occupied by tumor cells, associated intratumoral, and contiguous peri-tumoral desmoplastic stroma. Thus, PVRL2 positive staining includes as high as 50% of tumor cells or tumor infiltrating immune cells showing staining of any intensity.

### EXAMPLE 2: PVRIG AS BIOMARKER FOR COMPANION DIAGNOSTICS: IHC

In the IHC results presented herein, there was a wide dynamic range of PVRIG staining intensities in tumor tissues tested ((endometrial carcinoma, NSCLC, ovarian carcinoma, primary and metastatic colon carcinoma and primary and metastatic breast carcinoma). The signal was also classified as strong, moderate, weak, or negative staining. PVRIG positive samples demonstrated primarily membranous staining in infiltrating immune cells, immune aggregates, and TLSs. PVRIG staining was observed with variable intensity from weak with fine, light-brown membranes to strong with dark- brown thick membranes easily recognized at low magnification. There was no correlation between tumor type or stage with intensity of PVRIG expression.

The presence or absence of PVRIG expression in tumor specimens was evaluated using anti-PVRIG -specific antibody that can detect PVRIG in human formalin-fixed, paraffin-embedded (FFPE) tissues by IHC. To measure and quantify relative expression of PVRIG in tumor samples, a PVRIG IHC scoring system was developed to measure PVRIG specific signal in tumor infiltrating immune cells. Immune cells were defined as cells with lymphoid and/or myeloid morphology. Infiltrating immune cell staining was defined as the percent of the total tumor area occupied by immune cells that show staining of any intensity. The total tumor area encompassed the malignant cells as well as tumor-associated stroma, including areas of immune infiltrates immediately adj acent to and contiguous with the main tumor mass. In addition, infiltrating immune cell staining was defined as the percent of all tumor infiltrating immune cells. Table 7, below.

**Table 7**

| PVRIG status | | Staining criteria | Predicted response |
|---|---|---|---|
| negative | | 0% membrane | No to minimal |
| Low positive | | >5% immune infiltrating cells at any intensity membrane or cytoplasmatic | likely |
| Low positive | | Any positive immune infiltrating cells in TLS or lymphoid aggregates at any intensity membrane or cytoplasmatic | likely |
| Strong positive | | Combination of the two criteria above | Very likely |

IHC Scoring instructions:
- Score viable tumor cells exhibiting complete circumferential or partial linear plasma membrane, and intensity (H-score)
- The percent of tumor area occupied by any tumor-associated immune cells (Immune Cells Present, ICP) was used to determine IC+, which was the percent area of ICP exhibiting PVRL2/PVRIG positive immune cell staining
- Number of TLS or/and immune aggregates (totalIA), totalIA+ percent of cells positive for PVRL2/PVRIG
- Average expression intensity of endothelial cells in the tumor area

### EXAMPLE 3: PVRL2 AS BIOMARKER FOR COMPANION DIAGNOSTICS: IHC

In the IHC results presented herein, there was a wide dynamic range of PVRL2 staining intensities in tumor tissues tested (endometrial carcinoma, NSCLC, ovarian carcinoma, primary and metastatic colon carcinoma and primary and metastatic breast carcinoma). The signal was also classified as strong, moderate, weak, or negative staining. PVRL2 positive samples demonstrated primarily membranous staining in tumor cells and/or infiltrating immune cells, immune aggregates, TLSs and endothelial cells. PVRL2 staining was observed with variable intensity from weak with fine, light-brown membranes to strong with dark- brown thick membranes easily recognized at low magnification.

The presence or absence of PVRL2 expression in tumor specimens was evaluated using anti-PVRL2-specific antibody that can detect PVRL2 in human formalin- fixed, paraffin-embedded (FFPE) tissues by IHC. To measure and quantify relative expression of PVRL2 in tumor samples, a PVRL2 IHC scoring system was developed to measure PVRL2 specific signal in tumor cells, endothelial cells and tumor infiltrating immune cells. Immune cells are defined as cells with lymphoid and/or myeloid morphology.

Tumor cell staining is expressed as the percent of all tumor cells showing membranous staining of any intensity. Endothelial cell and Infiltrating immune cell staining is defined as the percent of the total tumor area occupied by endothelial and/or immune cells that show staining of any intensity. The total tumor area encompassed the malignant cells as well as tumor-associated stroma, including areas of immune infiltrates immediately adjacent to and contiguous with the main tumor mass. In addition, infiltrating immune cell staining is defined as the percent of all tumor infiltrating immune cells. Table 8, below.

**Table 8**

| PVRL2 status | Staining criteria | Predicted response |
|---|---|---|
| negative | 0% membrane | No to minimal |
| Tumor Positivity (TS) | >20% tumor membrane staining at +1 IHC score | likely |
| Immune Positivity (IS) | >20% immune infiltrating cells at any intensity membrane or cytoplasmatic | likely |
| Endothelial Positivity (ES) | >20% endothelial cells at any intensity membrane or cytoplasmatic | likely |
| Total Positive (ToP = TS+IS+ES) | Any combination of two Positivity scores | Very likely |

### IHC Scoring instructions:

- Score viable tumor cells exhibiting complete circumferential or partial linear plasma membrane, and intensity (H-score)
- The percent of tumor area occupied by any tumor-associated immune cells (Immune Cells Present, ICP) is used to determine IC+, which is the percent area of ICP exhibiting PVRL2/PVRIG positive immune cell staining
- Number of TLS or/and immune aggregates (totaIA), totalIA+ percent of cells positive for PVRL2/PVRIG
- Average expression intensity of endothelial cells in the tumor area

### EXAMPLE 4: SINGLE CELL ANALYSIS OF PVRL2 AND PVRIG EXPRESSION

### EXAMPLE 4A: ANALYSIS OF PVRIG EXPRESSION IN IMMUNE SUBSETS

There is growing evidence of importance of TSCM cells in immunotherapy (Sade-Feldman M, et al., Cell. 2018 Nov 1;175(4):998-1013.e20. doi: 10.1016/j.cell.2018.10.03 8.).

### Methods of evaluating PVRIG expression on TSCM (Stem Cell-Like Memory T-Cells)

- Supervised Clustering
- Enriched Populations
- Un-supervised clustering (not shown)
- Selection of positive Cells in scRNA (e.g., TCF7+PD-1+)

### TSCM cluster is enriched for +-/ markers associated with TSCM

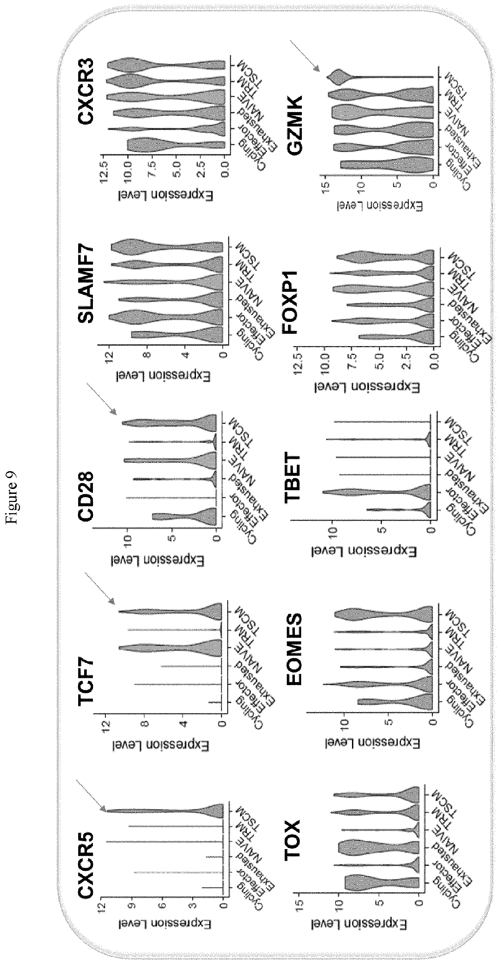

Figure 9. CD8+ TILS NSCLC scRNA (GSE99254) representative data (supervised approach)

### Inhibitory Receptor Expression in CD8+ Cells

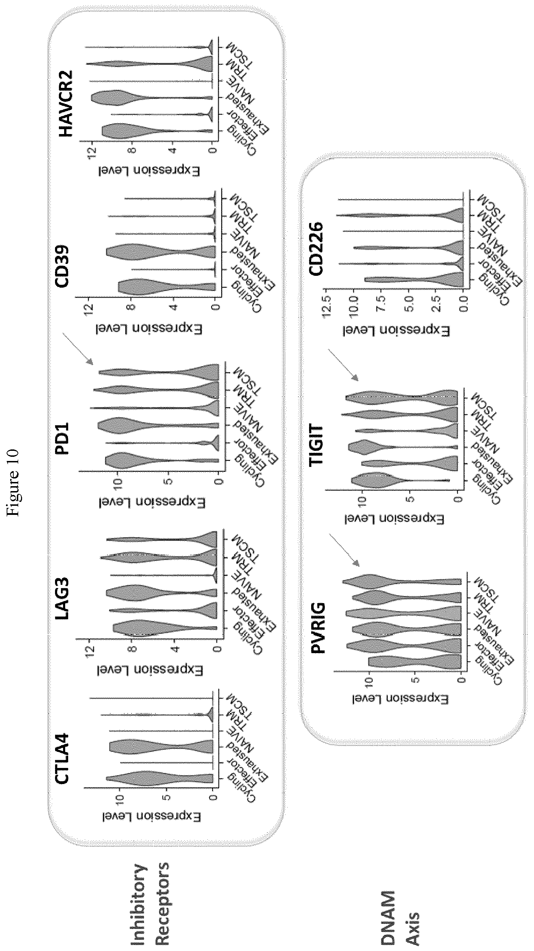

Figure 10. NSCLC scRNA (GSE99254) Representative Data (supervised approach).

### TSCM Cells are Preferentially Located in Lymph Nodes (Supervised Approach)

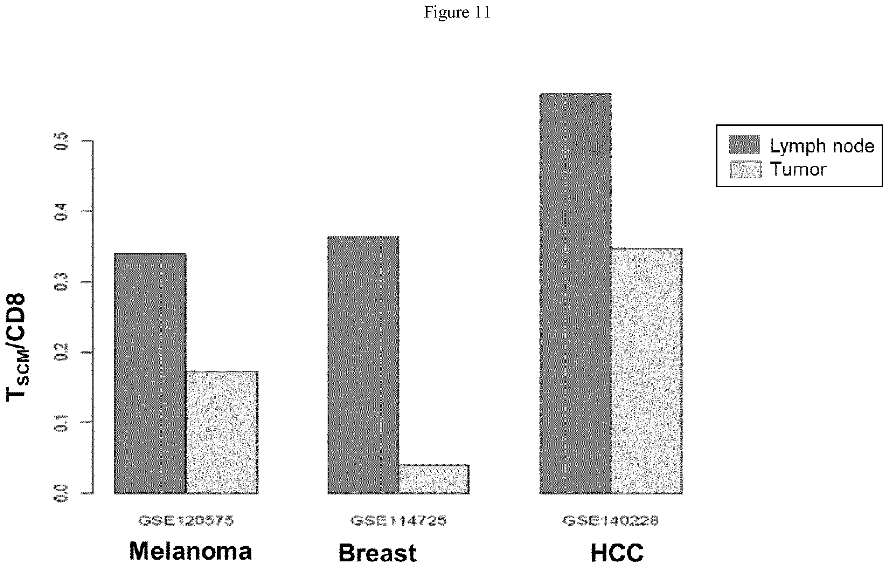

Figure 11. Fraction of TSCM in CD8 cells in lymph node vs. tumor.

### Fraction of TSCM CD8+ Cells is Predictive of Response to PD-1

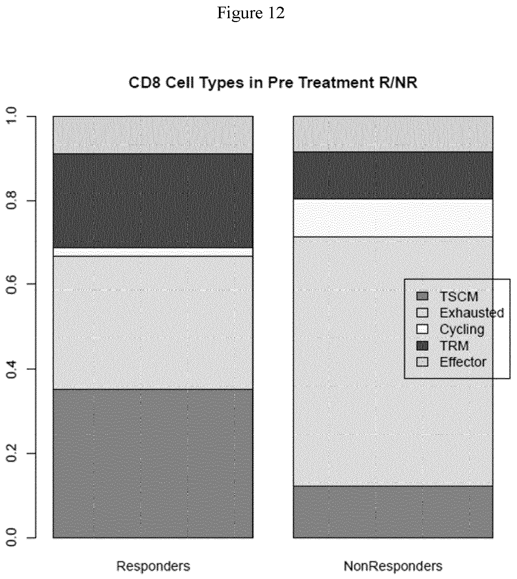

Figure 12. CD8 cell types pre-treatment responder vs. non-responders.

### PVRIG Expression in Enriched Populations Supports TSCM Expression Profile (NGS)

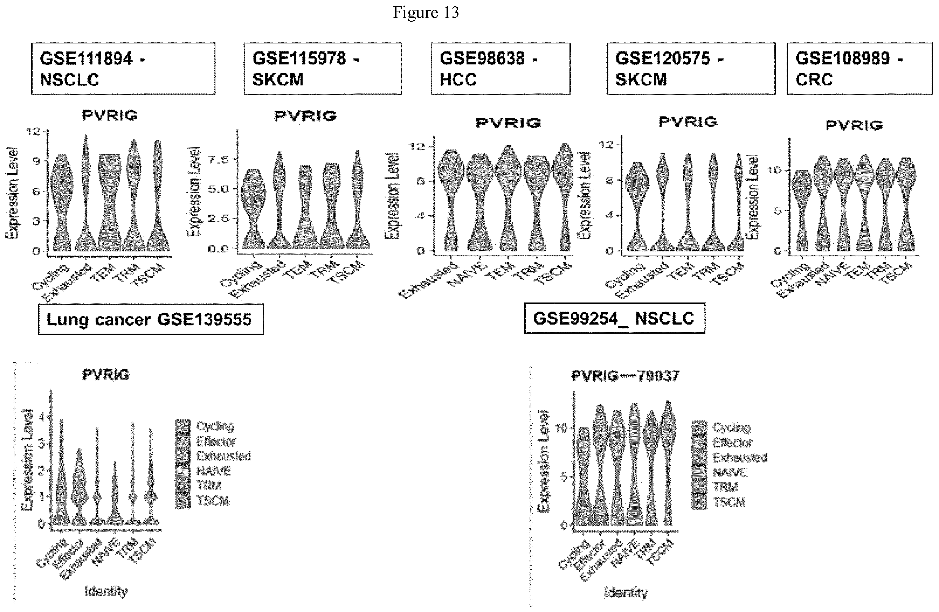

Figure 13. RNA Expression of DNAM-1 Axis in Stem-Like vs. Terminally differentiated CD8 Cells in Bladder Cancer Patients. The figure presents expression of PVRIG on Stem-like cells, which are important for (i) stem-like CD8 T cell acts as a precursor to generate a terminally differentiated effector population (ii) associated with improved prognosis upon checkpoint blockade (Data source: https://www.nature.com/articles/s415 86-019-183 6-5)

### PVRIG expression in TSCM (Stem Cell-Like Memory T-Cells)

This example describes a PCA based method of clustering of CD8 cells into various cell states and analyzing PVRIG expression among these cell states.

Six major CD8+ T cell states were identified across various scRNA studies which included: Naive, TSCM, TRM, Effector, ExCell states were classified based on the expression of previously identified markers including: naive (CXCR5, TCF7, IL7R, CCR7, SELL, CD28, SATB1), T effector (FGFBP2, CX3CR1, SORL1), Tissue resident memory (ITGAE, ZNF683, ITGA1), Exhausted (HAVCR2, ENTPD1, GZMB, LAYN, CD38, VCAM1), Cycling (Top2a, KI67) .

Naïve CD8 cells and TSCM both had high overlapping expression of markers associated with early differentiation state of CD8 cells. In order to generate a signature to allow for the separation of TSCM from naive cells we compared the expression profile of CD8 cells with high expression of a naive signature from peripheral blood in comparison to CD8 cells in tumor samples and CD8 cells with high expression of a naive signature from antigen specific CD8+ PMEL cells compared to CD8+ WT cells. We identified a series of recurring genes with expression in states of increases antigen exposure across different studies and associated these with antigen exposure and an early CD8 differentiation state characteristic of TSCM CD8+ cells. Gene used to separate CD8+ TSCM from Naive CD8+ included: CCL4, CCL5, CD69, CST7, FOS, FOSB, JUN, PPP1R15A, RGS1, SLC2A3, SRGN, TNFAIP3, BTG2, DUSP1, DUSP2, IER2, JUNB, NR4A2, RGS2, CD44, GZMB, HLA-DRB1, ITGA1, IFNG, JUND, PTPN22, FASLG, GZMA, GZMK, and/or IL2RB (*i*.*e*., TSCM signature).

Naieve CD8+ signature include ACTN1, C1orf162, CCR7, IL7R, LDHB, LDLRAP1, LEF1, LINC00861, MAL, MYC, NELL2, NOSIP,PLAC8, RASGRP",S1PR1, SELL, SERINC5, STK38, TCF7, TRABD2A, and/or LTB.

### PCA based method

Description of PCA based method can be found in Jolliffe. I. and Cadima J. (https://doi.org/10.1098/rsta.2015.0202).

Large datasets are increasingly common and are oftentimes difficult to process and interpret. Principal component analysis (PCA) is an approach for reducing the dimensionality of such datasets, enhancing interpretability while minimizing the possibility of loss of content of data. To this end the approach generates new uncorrelated variables that successively maximize variance. Identifying such new variables, the principal components, reduces to solve an eigenvalue/eigenvector problem, and the new variables are defined by the dataset at hand, not a priori, thereby making PCA a versatile data analysis tool.

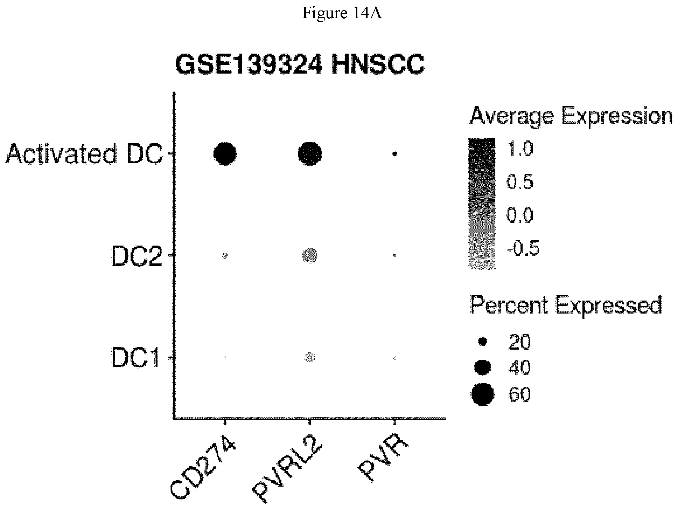

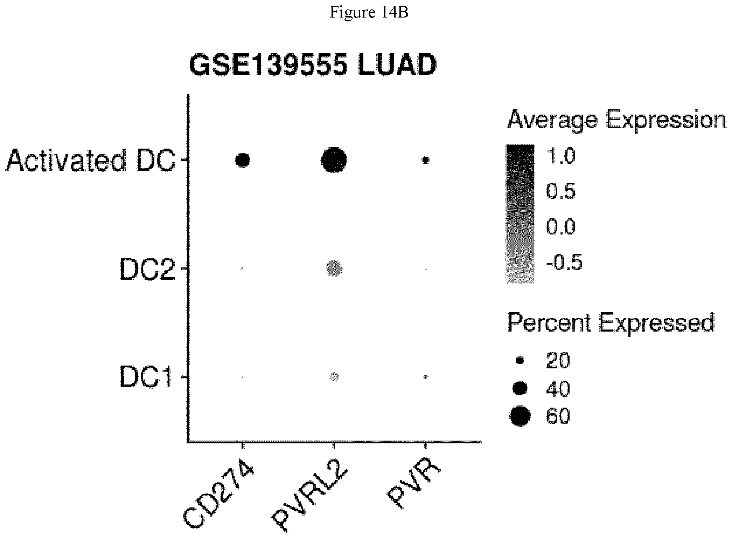

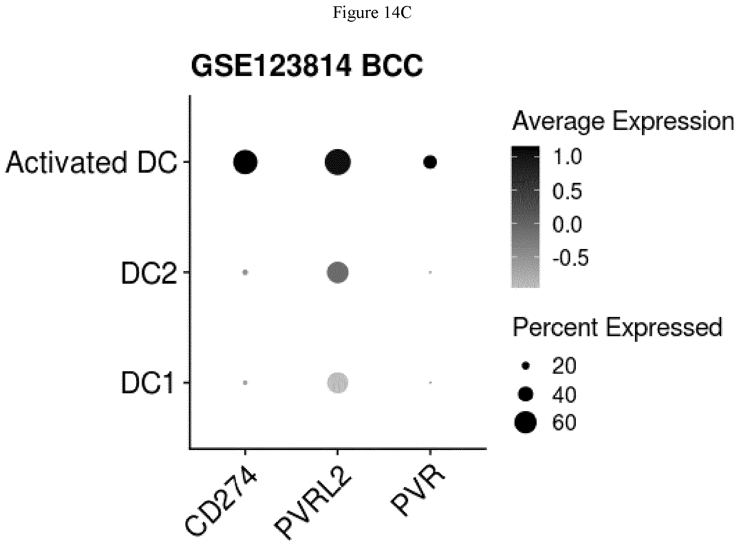

Figure 14. Expression profiles of PVRIG across data sets of various types of tumor.

Results of the PCA based analysis showed that PVRIG expression is expressed across different CD8 cell states (TEM, TRM, cycling, TSCM, naive, Exhausted) observed in scRNA studies across tumor indications. In contrast to PD-1 and other immune checkpoints whose expression profile is upregulated upon exhaustion, PVRIG expression was observed to be more uniform across different CD8 states.

Table 9. List of datasets used for CD8 cell subpopulation analysis, and their source studies.

**Table 9**

| **Studies** | **Tissue** | **Disease** | **GEO** | **Species** | **Treatment** |
|---|---|---|---|---|---|
| Defining T cell states associated with response to checkpoint immunotherapy in melanoma | Skin | Melanoma | GSE120575 | human | -aPD1 -aCTLA4 |
| Single-cell Map of Diverse Immune Phenotypes in the Breast Tumor Microenvironment 3' RNA Sequencing | Breast | Breast Cancer | GSE114725 | human | |
| Landscape and Dynamics of Single Immune Cells in Hepatocellular Carcinoma | Liver | Hepatocellular carcinoma | GSE140228 | human | |
| Single-cell transcriptomic analysis of tissue resident memory T cells in human lung cancer | Lung | Lung Cancer | GSE111894 | human | |
| Single-cell RNA-seq of melanoma ecosystems reveals sources of T cells exclusion linked to immunotherapy clinical outcomes | Skin | Melanoma | GSE115978 | human | -aPD1 |
| Clonal replacement of tumor-specific T cells following PD-1 blockade | Skin | Squamous cell carcinoma tumor | GSE123813_SCC | human | -aPD1 |
| Landscape of infiltrating T cells in liver cancer revealed by single-cell sequencing | Liver | Hepatocellular carcinoma | GSE98638 | human | |
| Lineage tracking reveals dynamic relationships of T cells in colorectal cancer | Colorectal | Colorectal cancer | GSE108989 | human | |
| T cell landscape of non-small cell lung cancer revealed by deep single-cell RNA sequencing | Lung | Non Small Cell Lung carcinoma | GSE99254 | human | |
| Single cell 3' RNA sequencing of 6 Belgian colorectal cancer patients | Colorectal | Colorectal cancer | GSE144735 | human | |
| Peripheral clonal expansion of T lymphocytes associates with tumour infiltration and response to cancer immunotherapy | Lung | Lung Adenocarcinoma and Large cell neuroendocrine carcinoma | GSE139555_LUNG_TUMOR | human | Pre treatment |

### PVRIG as biomarker, based on expression in various cd8 cells subtypes

This example provides a method of determining the likelihood of response of a human subject having cancer to a PVRIG inhibitor, comprising determining the level of PVRIG expressing various CD8 cells subtypes in tumor, tumor microenvironment, and/or peripheral blood.

**Table 10**

| **% NonZero Expressio n in CD8 cells** | **CD8-% PVRIG expres sing cells** | **CD8-num ber of cells** | **TSCM-% PVRIG expres sing cells** | **TSC M-num ber of cells** | **Naive-% PVRIG expres sing cells** | **Naiv e-num ber of cells** | **Exhau sted-% PVRIG expres sing cells** | **Exhau sted-numb er of cells** | TRM-**% PVRIG expre ssing cells** | **TRM-numb er of cells** | **Effect or-% PVRIG expre ssing cells** | **Effec tor-num ber of cells** |
|---|---|---|---|---|---|---|---|---|---|---|---|---|
| GSE13955 5_Lung_T UMOR_Si ngleCellLu ngCancer | 44 | 158 80 | 56 | 4154 | 35 | 1447 | 39 | 8714 | 31 | 59 | 72 | 554 |
| GSE14677 1_SS2_TU MOR_Sing leCellCRC | 68 | 117 9 | 61 | 462 | 63 | 187 | 77 | 392 | 8 | 5 | 6 | 53 |
| GSE11597 8_SingleC ellMelano ma | 54 | 163 9 | 47 | 182 | 53 | 1260 | 59 | 82 | NA | NA | 47 | 17 |
| GSE12057 5_SingleC ellMelano ma | 40 | 646 1 | 36 | 2462 | 44 | 2897 | 40 | 1915 | 28 | 117 | 34 | 259 |
| GSE12381 3_SCC_Sin gleCellSCC | 2 | 146 04 | 2 | 5918 | 2 | 3299 | 3 | 1054 | 0 | 402 | 3 | 79 |

Based on Table 10 above, cancer patients, having at least 10% of CD8 cells in the tumor, tumor microenvironment, and/or peripheral blood expressing PVRIG, had a higher probability to respond to the anti-PVRIG treatment.

Cancer patients, having at least 10% of TSCM cells in the tumor, tumor microenvironment, and/or peripheral blood expressing PVRIG, had a higher probability to respond to the anti-PVRIG treatment.

Cancer patients, having at least 10% of Naive CD8 cells in the tumor, tumor microenvironment, and/or peripheral blood expressing PVRIG, had a higher probability to respond to the anti-PVRIG treatment.

Cancer patients, having at least 10% of exhausted CD8 cells in the tumor, tumor microenvironment, and/or peripheral blood expressing PVRIG, had a higher probability to respond to the anti-PVRIG treatment.

Cancer patients, having at least 5% of TRM cells in the tumor, tumor microenvironment, and/or peripheral blood expressing PVRIG, had a higher probability to respond to the anti-PVRIG treatment.

Cancer patients, having at least 5% of Effector cells in the tumor, tumor microenvironment, and/or peripheral blood expressing PVRIG, had a higher probability to respond to the anti-PVRIG treatment.

**Table 11**

| **CD8 cell type** | **Cycling** | **Exhausted** | **Naive** | **Effector** | **TRM** | **TSCM** |
|---|---|---|---|---|---|---|
| **GSE120575** | 3.81% | 37.37% | 1.81% | 4.01% | 7.29% | 23.30% |
| **GSE114725** | 0.12% | 0.13% | 5.25% | 43.23% | 29.17% | 5.09% |
| **GSE140228** | 2.29% | 6.18% | 12.37% | 19.25% | 22.79% | 33.15% |
| **GSE111894** | 3.41% | 23.62% | 0.00% | 2.03% | 46.59% | 15.04% |
| **GSE115978** | 4.39% | 76.88% | 0.00% | 1.04% | 5.00% | 11.10% |
| **GSE123813_SCC** | 0.95% | 22.59% | 2.75% | 0.54% | 7.22% | 40.52% |
| **GSE98638** | 0.00% | 30.50% | 6.31% | 14.29% | 19.95% | 14.93% |
| **GSE108989** | 5.20% | 15.72% | 4.05% | 5.26% | 46.19% | 22.25% |
| **GSE99254** | 1.24% | 4.49% | 5.41% | 11.50% | 46.20% | 16.77% |
| **GSE144735** | 1.24% | 4.52% | 5.44% | 11.57% | 46.47% | 16.87% |
| **GSE139555_LUNG_TUMOR** | 0.69% | 9.11% | 0.37% | 3.49% | 54.87% | 26.16% |

Based on Table 11, cancer patients that have the presence or presense at an increased level as compared to a control or a patient that does not have the presence of any of: TSCM, TRM, naive, exhausted, cycling, and effector CD8 positive T cells, expressing PVRIG, in a biological sample obtained from a tumor, tumor microenvironment, and/or peripheral blood, have a higher probability to respond the anti-PVRIG treatment.

Cancer patients that have the presence of any of: TSCM, TRM, naive, exhausted, cycling, and effector CD8 positive T cells, expressing PVRIG, at least 0.5%, at least 1%, at least 1.5%, at least 2%, at least 2.5%, at least 3%, at least 3.5%, at least 4%, at least 4.5%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, or at least 85%, out of total CD8 cells in a biological sample obtained from a tumor, tumor microenvironment, and/or peripheral blood, have a higher probability to respond the anti-PVRIG treatment.

### EXAMPLE 4B: INHIBITORY LIGAND EXPRESSION ON ACTIVATED DENDRITIC CELLS

### ScRNA studies identify a dendritic cell (DC) cluster with an activated phenotype

Different studies have classified clusters as mRegDC, LAMP3+ DC, and activated DC. Common signature that have been published includes LAMP3, CD80, CD83, CCR7, CCL19, FSCN1, CD40, and PDL1. For example, Maier et al., Nature, 2020, reports that subsets of both DC1 and DC2 express the mRegDC signature. mRegDCs express the highest level of MHC II, IFNy, and IL-12, and are the most efficient activators of CD8+ cells. mRegDC signature is enriched in the DLN. And antigen uptake is associated with mRegDC program induction. Another article, Zhang et al., Cell, 2019, reports that cDC1, cDC2, pDCs may all contain LAMP3+ DCs. LAMP3+ is a maturation maker of multiple DC subsets. LAMP3+ DCs exhibits the highest migration score and migrate to lymph nodes. LAMP3+ is induced upon stimulation of DCs with LPS and IFNy and Poly I:C. LAMP3+ DCs have larger interaction networks compared to other DC subsets.

### PVR/PVRL2/PD-L1 are Co-expressed in `Activated' Dendritic Cells in Multiple scRNA Datasets

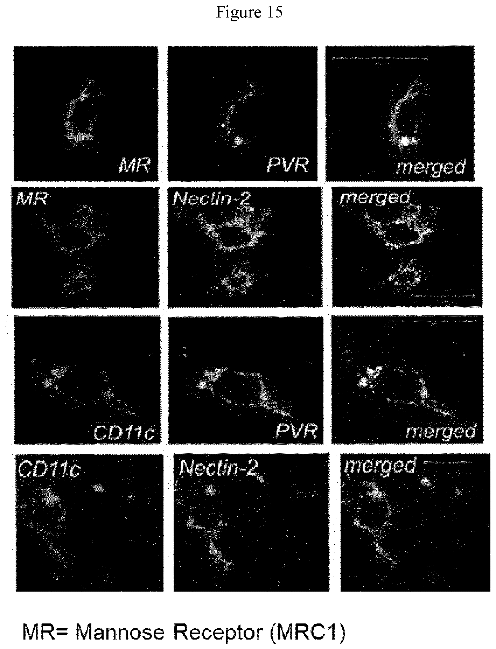

Figure 15. Expression of PVRL2, PD-L1, and PVR in various DC populations of HNSCC, LUAD, and BCC subjects.

### Validation of expression profile on "mature" DCs

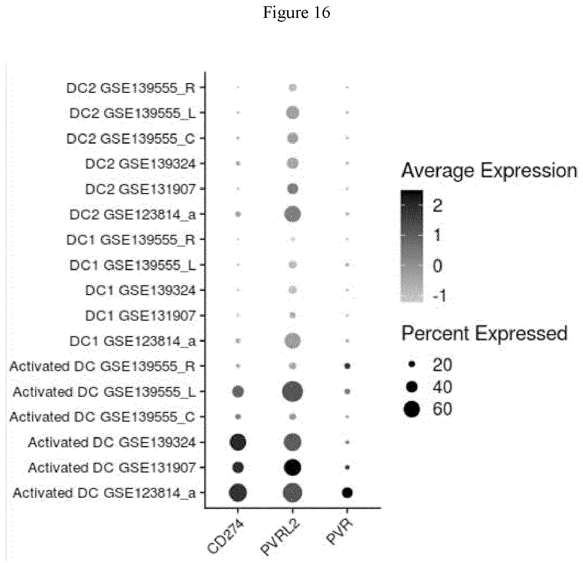

Figure 16. PVR & PVRL2 are expressed by myeloid cells in lymph nodes.

### PVRL2 expression in DCs

Data were analyzed using the Seurat package. Seurat is an R package designed for QC, analysis, and exploration of single-cell RNA-seq data. Seurat aims to enable users to identify and interpret sources of heterogeneity from single-cell transcriptomic measurements, and to integrate diverse types of single-cell data. Detailed description of Seurat can be found in Butler et al., Nature Biotechnology 2018, and Stuart, Butler, et al., Cell 2019.

Dendritic cell subpopulation were identified based on the expression of markers associated with DC subsets in various studies and which were recurrent across various studies. Four Dendritic Cell populations were commonly identified across scRNA studies which matched the profile of pDC, DC1, DC2 and activated DCs previously identified in various studies.

A conventional Dendritic Cell 1 (DC1) population was identified based on the expression of markers including: XCR1, Clec9a, CADM1, BATF3, IRF8, and/or CD141.

A conventional Dendritic Cell 2 (DC2) population was identified based on the expression of markers including: CD1c, FCER1A, Clec10a, CD11c, CD207, CD141, IRF4, and/or clec10a.

A conventional plasmacytoid Dendritic Cell (pDC) population was identified based on the expression of markers including: CD1c, FCER1A, IRF8, IRF4 LILRB4, and/or LILRA4.

An activated Dendritic Cell (activated Dendritic cell) population was identified based on the expression of markers including: BIRC3, CCR7, LAMP3, FSCN1, CD83, IDO1, and/or CCL22.

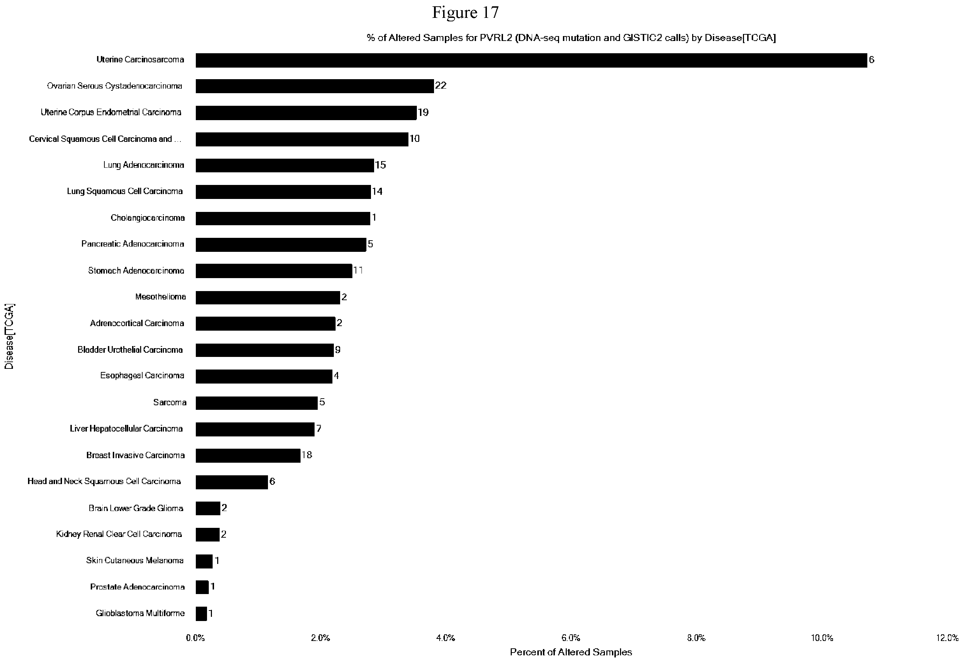

The Seurat based analysis of PVRL2 expression in dendritic cells subsets showed that PVRL2 expression is elevated in DCs with an activated phenotype when compared to DC1 and DC2 populations. Additionally, PVRL2 expression appears to be more pronounced on the DC2 subset of dendritic cells in comparison to DC1. PDL-1 and PVR expression is restricted to activated DC. See Figures 15 and 17.

Table 12. List of datasets used for Dendritic cell subpopulation analysis, and their source studies.

**Table 12**

| **Studies** | **Tissue** | **Disease** | **GEO** | **Treatment** |
|---|---|---|---|---|
| Clonal replacement of tumor-specific T cells following PD-1 blockade (basal cell carcinoma) | Skin | BCC | GSE123814_a | PD-1 |
| Immune Landscape of Viral- and Carcinogen-Driven Head and Neck Cancer | Head & Neck | HNSCC | GSE139324 | |
| Peripheral clonal expansion of T lymphocytes associates with tumour infiltration and response to cancer immunotherapy | Lung, Kidney, Colon | LUAD, ccRCC, CRC | GSE139555_L | |
| | | | GSE139555_R | |
| | | | GSE139555_C | PD-1 |
| Single-cell RNA sequencing demonstrates the molecular and cellular reprogramming of metastatic lung adenocarcinoma | Lung | NSCLC | GSE131907 | |

### Single cell analysis Suumary and Conclusions

PVRIG/TIGIT/PD-1 are expressed in CD8+ T Stem Cell Memory Cells.

PVRL2, PVR & PD-L1 are all regulated in activated DC subset across multiple scRNA datasets (n=8).

PVRL2 is expressed on endothelial cells in TLS & in lymph nodes (IHC).

CHA.7.518.1.H4(S241P) blockade could potentially mediate an interaction between activated DCs & Memory CD8+ cells in the tumor bed, lymph node and endothelial cells.

Potential mechanism which could lead to increase T cell priming and infiltration into less `inflamed' tumors.

As PVR, PDL1 & PVRL2 are induced upon activated DCs triple blockade of PVRIG/TIGIT/PD1 may be required for full antigen priming of CD8+ memory cells by activated DCs.

Expression of PVRIG and/or PVRL2 on specific immune cell subpopulations such as CD8 memory cells or activated DCs may be used to identify patients more likely to respond to PVRIG blockade.

### PVRL2 as biomarker, based on dendritic cells

Increased densities of populations of dendritic cells in tumor and tumor microenvironment have been associated with better clinical outcome. In experimental models, dendritic cells have been shown to contribute to the success of cancer immunotherapies, for example with the immune checkpoint blocker anti-PD-1 (Moynihan KD, et al., 2016. Nat Med. 22 (12): 1402-1410; Garris CS, et al., 2018. Immunity. 49 (6): 1148-1161).

We present herein that PVRL2 expression in various subtypes of DCs can serve as biomarker associated with better clinical outcome of anti-PVRIG treatment of cancer.

Based on Table 13 below, cancer patients having dendritic cells in the tumor, tumor microenvironment, and/or peripheral blood, with at least 10% PVRL2 expression, had a higher probability to respond the anti-PVRIG treatment.

Cancer patients having activated dendritic cells in the tumor, tumor microenvironment, and/or peripheral blood, with at least 10% PVRL2 expression, had a higher probability to respond the anti-PVRIG treatment.

Cancer patients having DC1 in the tumor, tumor microenvironment, and/or peripheral blood, with at least 5% PVRL2 expression, had a higher probability to respond the anti-PVRIG treatment.

Cancer patients having DC2 in the tumor, tumor microenvironment, and/or peripheral blood, with at least 10% PVRL2 expression, had a higher probability to respond the anti-PVRIG treatment.

**Table 13**

| | % PVRL2 expressing cells | | | |
|---|---|---|---|---|
| Data set | Activated DC | DC1 | DC2 | All DC |
| GSE123814_a | 70 | 56.9 | 58.3 | 60.8 |
| GSE131907 | 61.2 | 15.5 | 35.2 | 37.5 |
| GSE139555_R | 20.7 | 9.2 | 24.0 | 18.8 |
| GSE139555_C | 19.1 | NA | 35.8 | 29.6 |
| GSE139324 | 62.6 | 24.4 | 38 | 39.7 |
| GSE139555_L | 75.7 | 23.9 | 45.4 | 43 |

Based on Table 14 below, cancer patients that have the presence or presense at an increased level as compared to a control or a patient that does not have the presence of any of: Activated DC cells, DC1, and DC2, expressing PVRL2, in a biological sample obtained from a tumor, tumor microenvironment, and/or peripheral blood, have a higher probability to respond the anti-PVRIG treatment.

Cancer patients that have the presence of any of: Activated DC cells, DC1, and DC2, expressing PVRL2, at least 0.1%, at least 0.2%, at least 0.3%, at least 0.4%, at least 0.5%, at least 0.6%, at least 0.7%, at least 0.8%, at least 0.9%, at least 1%, at least 1.5%, at least 2%, at least 2.5%, at least 3%, at least 3.5%, at least 4%, at least 4.5%, or at least 5%, at least 10%, at least 15%, at least 20%, at least 25%, or at least 30%, out of total myeloid cells in a biological sample obtained from a tumor, tumor microenvironment, and/or peripheral blood, have a higher probability to respond the anti-PVRIG treatment.

**Table 14**

| | % out of myeloid cells | | | |
|---|---|---|---|---|
| Data set | Activated DC | DC1 | DC2 | |
| GSE123814_a | 8.91 | 8.93 | 19.08 | |
| GSE131907 | 1.00 | 0.66 | 3.90 | |
| GSE139555_R | 0.12 | 0.79 | 1.44 | |
| GSE139555_C | 14.96 | NA | 25.38 | |
| GSE139324 | 1.18 | 0.98 | 7.15 | |
| GSE139555_L | 1.60 | 4.86 | 16.78 | |

### EXAMPLE 5: AMPLIFICATION

### Background

PD-1 ligands, PD-L1 and PD-L2, are key targets of chromosome 9p24.1 amplification (both genes are located in the same chromosomal locus), a recurrent genetic abnormality in the nodular-sclerosis type of Hodgkin's lymphoma, in which -60% of patients harbor this amplification (Green et al., 2010). The 9p24.1 amplification results in high PDL1 and PD-L2 expression which is attributed to the high response rate of Hodgkin's lymphoma response to anti-PD1 therapy (Ansell et al., 2015). 9p24.1 amplification is rarely found in solid tumors, 0.7% in a cohort of 118187 tumor samples, the amplification is seen across tumor types (Goodman et al., 2018). Among patients treated with either PD-1 or PDL1 blocking mAbs the response rate in patients with the amplification was 66% (Goodman et al., 2018). In addition, in a recent publication the authors concluded that PDL1 copy number alteration could be an important predictive marker for PD-L1 inhibitor efficacy in breast cancer (Bachelot et al., 2020).

### Analysis and Results

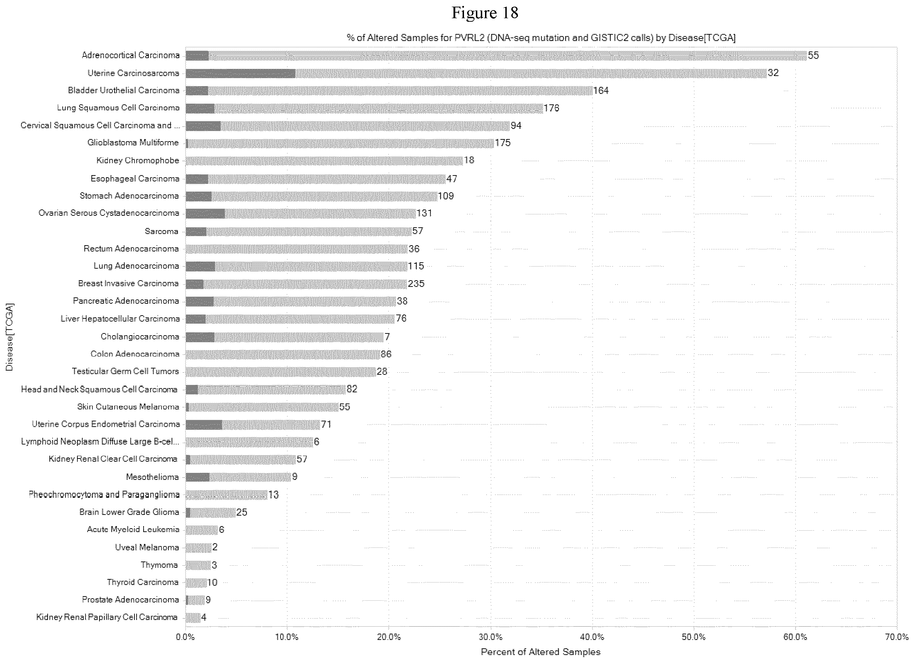

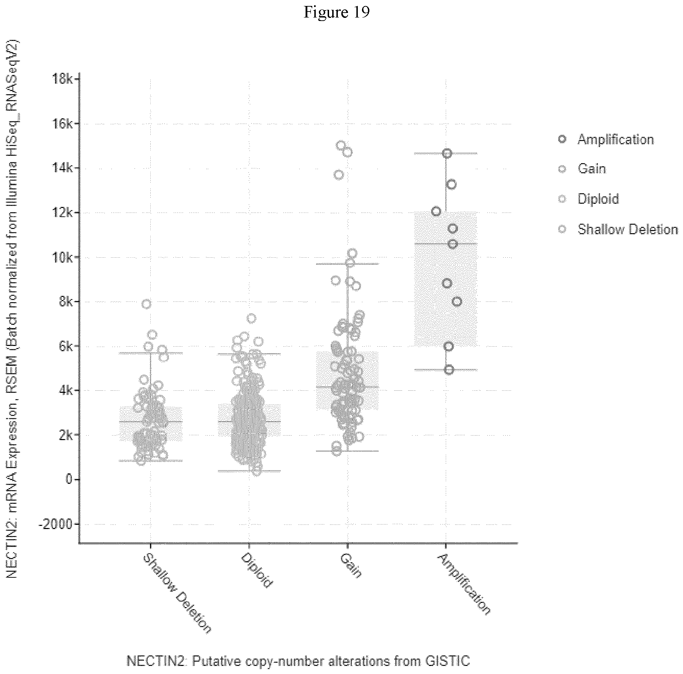

Similar to PD-1 ligands, the DNAM-1/TIGIT/PVRIG ligands PVR and PVRL2 reside in the same genomic locus (19q13.3). Using the TCGA cBioPortal (https://www.cbioportal.org/) and Omicsoft^{tm} tools exploring PVRL2 and PVR genomic gain (3-4 copies) or amplification (>4 copies), we found that -1-2% of all TCGA samples have a gene amplification of PVRL2 and/or PVR. Uterine carcinosarcoma has the highest rate of amplification (>10%, Figure 18 - PVRL2 as example). When examining genomic gain there are -40% of TCGA samples positive for the gain. In order to validate that the amplification/gain is accompanied also by mRNA higher expression in Figure 19 the correlation between PVRL2 expression to genomic status shows that there is a positive correlation between copy number and PVRL2 expression, gastric cancer as an example.

Similar correlation between PVRL2 copy number and PVRL2 expression by mRNA were found in cell lines (data not shown).

### Conclusions

As there is a positive correlation between PVRL2 expression level and the activity of (CHA.7.518.1.H4(S241P)) anti-PVRIG blocking mAb, we propose that tumors with amplification of PVRL2 genomic locus will be highly responsive to CHA.7.518.1.H4(S241P) treatment. The detection could be done using whole genome sequencing, exome sequencing, FISH, CNA probes or other molecular genetics methods.

### EXAMPLE 6: PVRL2 AS BIOMARKER FOR PVRIG AB TREATMENT EFFICIENCY: INDICATIVE RESULTS

### High PVRL2 Expression Leads to Monotherapy Activity for anti-PVRIG antibody CHA.7.518.1.H4(S241P)

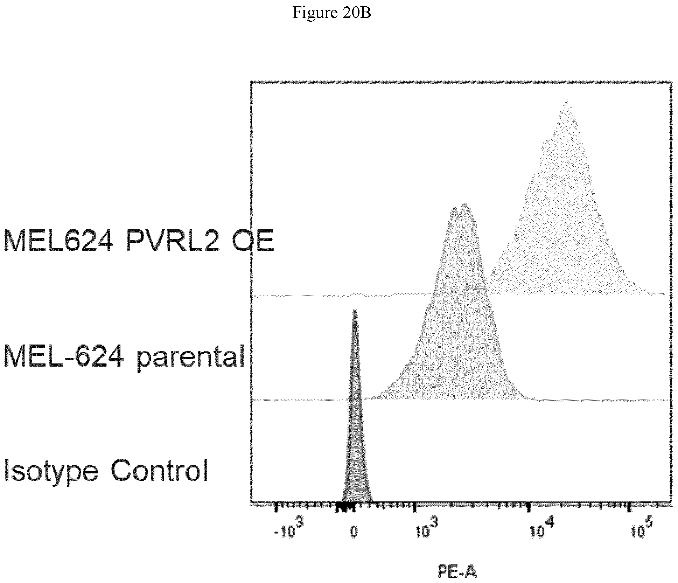

PVRL2 expression on Mel624 tumor cells and MEL624 cells stably transduced with a human PVRL2 (MEL624 PVRL2 OE) is depicted in Figure 20B, demonstrating higher PVRL2 expression levels in MEL624 PVRL2 OE cells than in the parental MEL624 or in the isotype control.

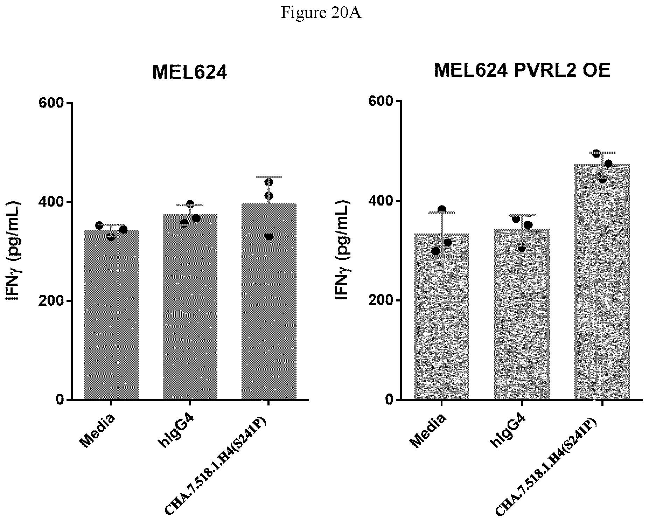

Figure 20A presents the results of CMV pp65 assay on MEL624 parental vs. MEL624 PVRL2 OE cells, treated with CHA.7.518.1.H4(S241P) (as opposed to the isotype control). The results demonstrate higher amount of human interferon gamma (IFNy) in MEL624 PVRL2 OE cells vs. the parental MEL624 cells, corresponding to higher activity of CHA.7.518.1.H4(S241P) on cells having higher PVRL2 expression.

These data indicate CHA.7.518.1.H4(S241P) may have a monotherapy activity on tumors with high PVRL2 expression

### CMV Tumor cell line assay

CMV pp65_{(495 - 503)} reactive T cells were expanded by thawing CMV seropositive donors (Conversant) and 2×10⁶ cells/ml were resuspended in RPMI medium (Gibco) supplemented with 1% glutamax (Gibco), 1% non-essential amino acids (NEAA), penicillin/streptomycin (Gibco), 10% human AB serum (Corning), 1 µg/ml CMV pp65₍₄₉₅₋₅₀₃₎ (Anaspec), 2 ng/ml IL-2 (R&D), and 10 ng/ml IL-7 (R&D). PBMCs were cultured for eight days with IL-2 and IL-7 replenished at day three and day five. At day eight, cells were harvested, and re-plated in low dose IL-2 (100 U/ml) at 2 million/ml in complete RPMI media for two days. At day eleven, cells are phenotyped for CD8⁺ T cell purity and CMV pp65₍₄₉₅₋₅₀₃₎ reactivity. Cells were stained with 0.25 µL of anti-CD3 (clone: OKT3)-allophycocyanin seven (APC-Cy7; Biolegend), 0.25 µL of anti-CD8 (clone: H1T8a)-Alexa Fluor (AF) 488 (Biolegend), a combined cocktail of 0.125 µL of anti-CD14 (clone: HCD14)-peridinin chlorophyll protein (PerCP-Cy5.5), 0.5 µl of anti-CD19 (clone:HIBCD14) PerCP-Cy5.5, 0.5 µL of anti-CD56 (clone:HCD56)-PerCP-Cy5.5(Biolegend), 1.25 µL of anti-TIGIT (clone:MBSA43)-allophycocyanin (APC;e-Bioscience) or 1.25 µL of IgG4 (Compugen)-isotype control (APC:Biolegend), 1.25 µL of CHA.7.518.1.H4(S241P)-AF-647 (Compugen, or 1.25 µL of IgG4-AF647 isotype control (Compugen), and 0.5 µL of anti-PD-1 (clone: EH12.2H7)-Brilliant Violet 421 (BV421;Biolegend) or 1.25 µL of IgG1 (clone: MOPC21) BV421 (Biolegend). To assess the frequency of tetramer-reactive CD8⁺ T cells, unlabeled PBMCs were stained with 10 µL of iTAg Tetramer - HLA-A*02:01 CMV pp65₍₄₉₅₋₅₀₃₎ (NLVPMVATV) - phycoerythrin (MBL-BION) for 30 min at room temperature. Cells were washed with PBS/1% BSA/0.01% sodium azide buffer, data were acquired using a Fortessa flow cytometer (BD Biosciences), and analyzed using FlowJo (Treestar).

Human CMV-Specific CD8+ T cell Co-Culture Assay with Human PVR- and Human PVRL2 (CD112)-Expressing Melanoma Cell Lines: The effect of CHA.7.518.1.H4(S241P), an anti-human PVRIG antibody, on antigen-specific cytokine secretion was assessed by an in vitro co-culture assay with human CMV-specific CD8⁺ T cells. The target cell line used in the assay was the HLA-A2+ melanoma cell line, Mel624, or MEL624 PVRL2 OE, which stably expressed human PVRL2 through lentiviral transduction (System Biosciences). The cells were pulsed with the CMV pp65 peptide at 0.0033 µg/ml or 0.001 µg/ml at 37 degree. C. for 1 hour. Cells were then washed and plated at 50,000 cells/well. CHA.7.518.1.H4(S241P) antibody, or an isotype antibody control hIgG4, were added to the culture at 10 µg/ml. Human CMV-specific CD8⁺ T cells from three different donors were expanded. 50,000 human CD8+ T cells were added to each well. Co-cultures were incubated at 37 degree. C. for 24 hours. After the incubation, plates were centrifuged at 1200 rpm for 1 minute and the supernatant was collected. The amount of human interferon gamma (IFNy) in the co-culture supernatant was measured by flow cytometry using a cytometric bead assay (BD).

### CHO-S OKT3 assay

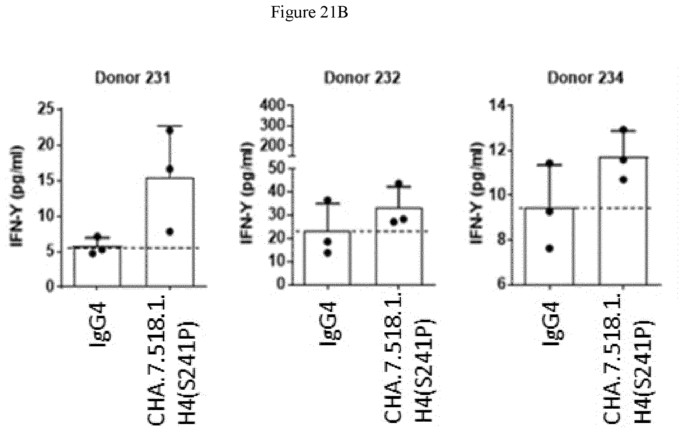

The effect of CHA.7.518.1.H4(S241P), a non-blocking control antibody, and anti-DNAM-1 on CD4+ T cells from a healthy donor activated by CHO-S OKT3 cells or CHO-S OKT3 hPVRL2 cells is shown in Figure 21A. The anti-DNAM-1 antibody, which blocks the interaction of PVRL2 with DNAM-1, only decreased CD4+ T cell proliferation when T cells were co-cultured with the hPVRL2-expressing CHO-S OKT3 cells, serving as a positive control in the assay. The dashed line indicates the percentage of CFSElo CD4+ T cells after treatment with either the human IgG4 or the mouse IgG1 isotype antibodies. Each dot represents a technical replicate, average + standard deviation is shown, data are representative of at least 2 trials. Figure 21B shows IFN-y production by three different human CD8⁺ T cell donors (Donors 231, 232, and 234) when co-cultured with the CHO-S OKT3 hPVRL2 cells and the indicated antibody.

The effects of CHA.7.518.1.H4(S241P) are dependent on the presence of hPVRL2 on CHO hPVRL2 cells as no effect was observed in the absence of PVRL2, suggesting that PVRL2 expression levels are indicative of treatment efficiency of patients with CHA.7.518.1.H4(S241P), as shown in Figures 21A and 21B.

The CHO-S OKT3 assay was utilized to determine whether CHA.7.518.1.H4(S241P) could enhance CD4+ and CD8+ T cell proliferation, and cytokine secretion. The target cells used in the co-culture assay were the Chinese hamster ovary cell line, CHO-S (ATCC), either stably overexpressing the single chain variable fragment of the anti-human CD3 antibody Clone OKT3 (abbreviated as OKT3), or stably overexpressing both OKT3 and human PVRL2 (abbreviated as hPVRL2). CHO-S OKT3 parental cells were grown in serum-free CD-CHO medium (Gibco) supplemented with 40mM glutamax (Gibco), penicillin/streptomycin (Gibco), and 6 µg/ml puromycin (Gibco). CHO-S OKT3 hPVRL2 cells were grown in the same CD-CHO medium as the parental cells, but also supplemented with 600 µg/ml hygromycin B (Gibco). Primary CD4+ or CD8+ T cells were isolated from healthy human donors using the RosetteSep^{™} human CD4+ T cell enrichment cocktail (Stemcell Technologies) or using the human CD8+ microbeads (Miltenyi Biotec), respectively, and frozen in liquid nitrogen. On the day of the coculture assay, CD4+ or CD8+ T cells were thawed, counted, and labeled with 1µM CFSE (Life Technologies) for 10 minutes at 37°C. Following this incubation, T cells were washed and resuspended in complete medium containing RPMI (Gibco), supplemented with 10% heatinactivated FBS, glutamax, penicillin/streptomycin, non-essential amino acids (Gibco), sodium pyruvate (Gibco), and 50µM β-mercaptoethanol (Gibco). To inhibit proliferation of the target cells, CHO-S OKT3 and CHO-S OKT3 hPVRL2 cells were harvested from culture, and treated with mitomycin C (Sigma-Aldrich) for 1 hour at 37°C with periodic mixing. After the incubation, the target cells were thoroughly washed, counted, and resuspended in complete RPMI medium. The assay was set up with a 5:1 ratio of T cells (100,000) to target cells (20,000). The target cells, T cells, and 10 µg/ml of each antibody treatment were added together in a 96-well U-bottom plate (Costar), and incubated for either 3 days (CD8+ T cells), or 5 days (CD4+ T cells) at 37°C. The antibody treatments included CHA.7.518.1.H4(S241P) antibody, a non-blocking control antibody to PVRIG, mouse-anti-DNAM-1 IgG1 (Clone 11A8, Biolegend), a mouse anti-human TIGIT IgG1 (Clone MBSA43, BioLegend), mouse IgG1 isotype control (Clone MOPC21, BioLegend), and a human IgG4 isotype control. For antibody dose-titrations, 3-fold dilutions from 66nM to 0.264nM of the CHA.7.518.1.H4(S241P) antibody and the respective isotype control antibody were utilized. For dose response experiments, data were analyzed by non-linear regression and fit to either a "One site specific binding" or "One site - Total and nonspecific binding" model using GraphPad Prism. After the 3-day or 5-day incubation period, co-culture supernatants were analyzed for secreted cytokines, including IFNy, with the cytometric bead array (CBA) human Th1/Th2/Th17 cytokine kit (BD Biosciences), or with the LEGENDplex^{™} Human Th cytokine kit (BioLegend). T cell proliferation was measured by staining CD4+ or CD8+ T cells with the LIVE/DEAD fixable aqua dead cell stain kit (ThermoFisher Scientific), anti-CD4 antibody (Clone RPA-T4, BioLegend), and anti-CD8 antibody (Clone HIT8a, BioLegend). Data was acquired using a FACS Canto II (BD Biosciences), and analyzed using FlowJo (Treestar) software to determine the percentage of live, CFSE low proliferating CD4+ or CD8+ T cells.

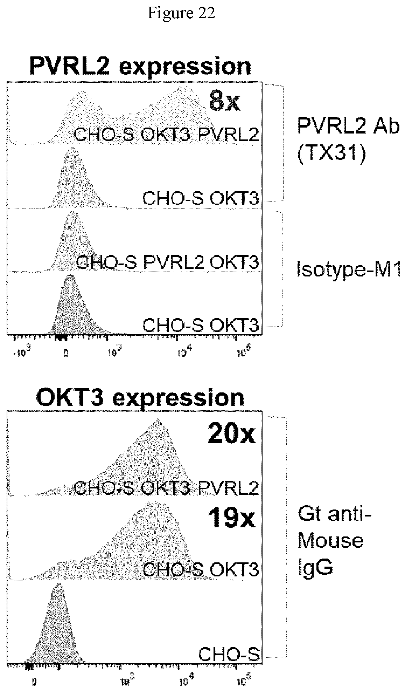

PVRL2 expression and anti-CD3 (OKT3 clone) on CHO-S cells stably transduced with anti-CD3 (OKT3) and a human PVRL2 (CHO-S OKT3 PVRL2 OE), is depicted in Figure 22, demonstrating higher PVRL2 expression levels in CHO-S PVRL2 OE cells than in the parental CHO-S OKT3.

### EXAMPLE 7: PVRIG PROTEIN EXPRESSION IN TSCM

PVRIG protein expression on several lymphoid populations was tested in fresh human tumor samples from different cancer types.

Fresh human tumor and normal adjacent tissue samples from colorectal and ovarian cancers were harvested into RPMI and RPMI+1% HEPES respectively. The samples were cut into small pieces and transferred to GentleMACS C Tubes (Miltenyi) and digested using the enzyme mix - Tumor Dissociation Kit from Miltenyi, (130-095-929) in the GentleMACS machine according to manufacturer's instructions. Cells were filtered through 70uM filter, and red blood cells were eliminated using RBC lysis buffer (BD 555899). Single cells were stained with CD45-BUV395 (clone: HI30; BD bioscience ), CD8-BV785 (clone: SK1; BioLegend), CD3-APC cy7 (clone: HIT3a; BioLegend), CHA.7.518.1.H4(S241P)-AF-647 (anti PVRIG antibody CHA.7.518.1.H4(S241P) (Compugen) labeled with fluorescent dyes AF647), or IgG4-AF647 isotype control (labeled with fluorescent dyes AF647), TIM3-PE cy7 (clone: F38-2E2; BioLegend)), CD28-BV650 (clone: CD28.2; BioLegend), CXCR5 - BB515 (clone: RF8B2; BD bioscience ), PD-1 - BV421 (clone: EH12.2H7; BioLegend), TIGIT- percp e710 (clone: MBSA43; e-Bioscience), CD69-BV711 (clone: FN50; BioLegend). Antibodies to lineage markers (CD45, CD3 and CD8) were used at 3µg/ml final concentration. Other antibodies were used at concentration of 5µg/ml. 2×10⁶ cells were stained with Zombie aqua (ThemnoFisher) for live/dead cells. Afterward, cells were blocked with a cocktail of anti-CD32 (e-Bioscience), anti-CD16 (BioLegend) and anti-CD64 (BioLegend). Samples were washed with FACS buffer (1%BSA, 0.1% sodium azide, in PBS) and stained with antibodies for 30 min at 4°C. Finally, cells were washed and acquired on the BD Fortessa flow cytometer and further analyzed using the FlowJo software (TreeStar). Gating strategy for the populations:
- CD8⁺ T cells: CD45⁺CD3⁺CD8⁺
- CD4⁺ T cells: CD45⁺CD3⁺CD8⁻
- Tscm: CD45⁺CD3⁺CD8⁺TIM3⁻CXCR5⁺
- CD45⁺CD3⁺CD8⁺TIM3⁻PD1⁺CD28⁺

### Results

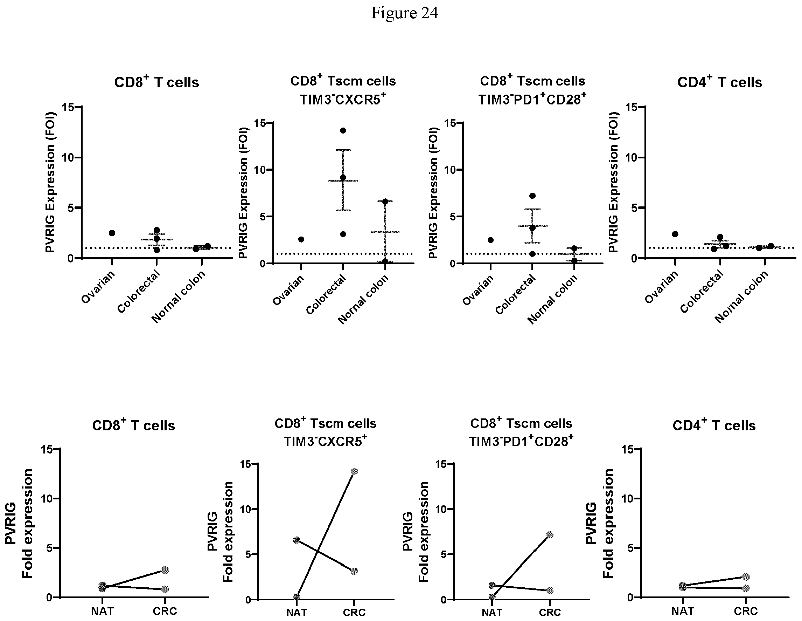

CD8⁺ Tscm cells in human colorectal and ovarian cancer samples were defined using two gating approaches: TIM3⁻CXCR5⁺ and TIM3⁻PD1⁺CD28⁺. As seen in Figure 24A, samples were grouped based on the tumor type. Each dot in a column represents a sample. In the colorectal tumor samples tested, the expression of PVRIG was higher in the Tscm T cells compared to total CD8⁺ or CD4⁺ T cells. In the ovarian tumor sample PVRIG levels were similar among the populations tested.

In addition, comparing PVRIG expression in matched tumor and normal adjacent tissue (NAT) samples revealed higher expression in the tumor tissue in 1 of 2 colorectal samples tested in all the populations.

Patients presenting with higher PVRIG expression in TSCM T cells, benefit from a more efficient response to anti PVRIG treatment.

There is a growing appreciation for the potential role of stem-like memory T cells in cancer biology, and while recent evidence suggests that these cells express TIGIT and PD-1, our work now shows that they also express PVRIG. Furthermore, our data show that PVRIG's ligand, PVRL2, is expressed at high levels in both dendritic cells and tertiary lymphoid structures, as well as in PD-L1low or less-inflamed tumors. The data in the examples provided in the present applicatoin suggest that CHA.7.518.1.H4(S241P), may enhance T cell proliferation, activation and infiltration through activity in these important cell populations, even into tumors where current checkpoint blockade has not proven successful.

The data in the present examples support the role PVRIG represents an untapped immune checkpoint, suggesting a more significant role withing the DNAM-1 pathway and that targeting this pathway have the potential to provide a new treatment option, as monotherapy or in combination with other immune checkpoints, for both inflamed and non-inflamed tumors. Furthermore, these data reinforce that the simultaneous triple blockade of PVRIG, TIGIT, and PD-1 has the potential to expand the reach of cancer immunotherapies to new patient populations and cancer indications currently unresponsive or refractory to existing treatments.

PVRIG is expressed on Stem-like memory T-cells (TSCM), the cells that give rise to differentiated cytotoxic effector T cells, mediating direct anti-tumor effects in the tumor microenvironment.

PVRL2 and PVR, the ligands for PVRIG and TIGIT, respectively, are expressed in both PD-L1low and PD-L1high tumor types.

PVRL2 has abundant expression across various dendritic cell (DC) types, which are associated with efficient T cell activation, and in Tertiary Lymphoid Structures, structures in the tumor bed where local T cell priming occurs.

Cumulatively, the data presented suggest that CHA.7.518.1.H4(S241P) blockade could potentially mediate an interaction between DCs & TSCM cells in the tumor bed and lymphoid organs. This potential mechanism could lead to increase T cell priming and infiltration into less inflamed tumors.

### EXAMPLE 8: TIGIT-PDCD1 IN CD8 T CELLS

### PVRIG & TIGIT are not redundant in CD8 T cells

Single cell analysis reveald that PDCD1 and TIGIT have mRNA expression patterns similar to markers of exhausted CD8 T cells, whereas PVRIG is different and closer to markers of transitional-memory T cells (equals early memory T cells).

Four high quality datasets were used in the analysis (high number of genes (>2200) per CD8 T cells).

The analysis was focusing on CD8 T cells since they are the main cells that fight tumors.

PVRIG clusters with early differentiation/memory genes, unlike other ICPS that cluster with exhausted genes, in CD8+ T cells.

Separated cells from a certain SC (Single Cell) experiment by their gene expression profile. This can be done using the Principal Component Analysis (PCA)-based method (see, the Word Wide Web at builtin.com/data-science/step-step-explanation-principal-component-analysis).

This was used to label cells, by different cell types labels, in SC experiments. This was done in a nested manner. First, separated into the main cell types: "Immune", "Stromal", "Epithelial". Then separated each subgroup into its main cell types, for example, for "Immune", "TCell", "BCell", "Myeloid", "NK", "Plasma Cell". Separated into more nested subgroups. In the analysis genes were separate based on the cells in which they were expressed. Many gene markers were reviewed and over 1000 variable genes examined. Each point in the plots shown in the Figures is a gene.

Genes that are markers for particular CD8 cell types are described below:
- Naive: TCF7, CCR7, IL7R, LEF1, SELL, LTB
- Exhausted: TIM3, ENTPD1, LAYN, CD38, LAG3, VCAM1, TOX
- Transitional Memory (early memory) : GZMK, SH2D1A, EOMES, DTHD1, SLAMF7, FCRL3,CD28
- Tissue resident Memory: XCL1, XCL2, ITGAE, ITGA1, ZNF683, CXCR6
- Circulating memory: NR4A1, NR4A2, NR4A3, KLF2
- Cytotoxic: FCGR3A, FGFBP2, CX3CR1, KLRG1, LILRB1
- Cycling: TOP2A, MKI67

For this analysis, only CD8 cells were used to separate the genes. For the analysis, 4 different datasets that have many CD8 T cells were used. Specific markers were used to differentiate between various CD8 cell types.

**Table 15**

| | | |
|---|---|---|
| T cell landscape of NSCLCrevealed by deep ssRNA sequencing | NSCLC | GSE99254 |
| Lineage tracking reveals dynamic relationships of T cells in colorectal cancer | Colorectal cancer | GSE108989 |
| Defining T Cell States Associated with Response to Checkpoint Immunotherapy in Melanoma | Melanoma | GSE120575 |
| Landscape of infiltrating T cells in liver cancer revealed by single cell sequencing | Liver cancer | GSE98638 |

### Unsupervised Correlation Analysis

The heatmaps was based on the average of Spearman correlation coefficients between genes across **13 datasets** (*see,* Figure 38).

Spearman correlations were calculated after generating automatic clusters (using Seurat R package) and calculating the average expression level per cluster for each gene.

As can be seen from the results below, PVRIG, GZMK, and EOMES form a transitional cluster that was lowly correlated with both exhausted and naive cluster, which are anti-correlated with each other. *See,* Figure 39.

### Cosine Similarity

Cosine similarity was calculated between all gene pairs based on their expression levels in CD8 T cells of GSE99254. Cosine similarity was used since it better handles the zero-inflated single cell data compared to the classical Pearson correlation.

A heatmap was drawn using the pheatmap R package (see, the World Wide Web at cran.r-project.org/web/packages/pheatmap/pheatmap.pdf).

The results reveal that the five cell states (naive, exhausted, early memory, tissue resident memory, and cytotoxic) of CD8 T cells are nicely separated. The only exception is CD28, which is clustered with markers of naive CD8 T cells.

The results also reveal that PDCD1, TIGIT and CTLA4 are clustered with markers of exhausted CD8 T cells, whereas PVRIG is clustered with markers of Early Memory CD8 T cells.

### Summary

The marker gene groups are pretty much separated and clustered (dots with the same color are often clustered together).

As expected, in most of the plots, we find PDCD1 clusters with the CD8 Exhausted gene markers.

In most of the plots TIGIT and PDCD1 are close one to the other and cluster with the CD8 Exhausted gene markers.

Interestingly, in many cases PVRIG clusters with the CD8 Transitional Memory genes markers, supporting the different mechanism of action of PVRIG as compared to TIGIT and PD1, as well as the potential of CD8 Transitional Memory cells levels expressing PVRIG, to serve as biomarkers for patient population determination for treatment with an anti-PVRIG antibody and predicting the efficacy of treatment with an anti-PVRIG treatment antibody.

### EXAMPLE 9: DNAM-1 AXIS PATHWAY

DNAM-1 axis plays an essential role in tumor immunology. PVRIG binds PVRL2 as a functional ligand (TIGIT binding to PVRL2 is of low affinity). DNAM-1 axis comprises two parallel dominant complementary inhibitory pathways: PVRIG and TIGIT/CD96. TIGIT and PVRIG deliver direct inhibitory signal into T and NK cells. TIGIT/PVRIG has higher affinity to PVR/PVRL2 than DNAM-1 (decoy effect).

PD-1 inhibition blocks DNAM-1 (CD226) dephosphorylation and inactivation. DNAM-1 KO or inhibition reverses a-PD-1 tumor growth inhibition.

Potential intersection between PVRIG/TIGIT and PD-1 Pathways support combination approach to overcome immunotherapy resistance. Different tumor types may respond to different combinations depending on dominance of the pathways. Synergistic T cell activation with PVRIG, PD-1 and TIGIT blockade was shown in Whelan, et al., Cancer Immunol Res., 7(2):257-268, 2019 (incorporated by reference herein).

Growing Evidence of T stem-like Cells Importance in immunooncology. Anti-PD-L1 expands a key population of PD-1-positive T_{SCM} which also express TIGIT. TIGIT and PD-1 co-blockade might enable optimal T_{SCM} activation and DNAM-1 co-stimulation.

### Summary

PVRIG is a novel checkpoint in the DNAM-1 axis, co-expressed with PD-1 and TIGIT in T_{SCM} and exhausted T cells but had a differentiated dominant expression on early differentiated cells (T_{SCM} - like).

PVRIG blockade showed synergistic activity with TIGIT and PD-1 blockade in pre-clinical studies.

PVRL2 and PVR were expressed in PD-L1^{low} and PD-L1^{high} tumor types. PVR and PVRL2 were expressed in inflamed and non-inflamed tumors. PVR/PVRL2 on tumor cells induced by: Genotoxic stress (DNA damage, oxidative stress). Tumorigenesis (loss of contact inhibition/increased invasiveness). PVRIG+TIGIT blockade may address PD-L1_{low} non-inflamed indications. PVR/PVRL2 on tumor cells was not modulated by IFN-γ, as provided in Whelan, et al., Cancer Immunol Res. 2019 (incorporated by reference herein) and PVR/PVRL2 was commonly expressed in PD-L1 negative tumors (*see,* SITC, November 2017, Whelan, *et al.,* poster presentation).

PVRL2 had abundant expression across DC types and in TLS.

CHA.7.518.1.H4(S241P) blockade could potentially mediate an interaction between DCs and T_{SCM} in the tumor bed (TLS) and lymphoid organs, a mechanism which could lead to increased T cell priming and infiltration into less 'inflamed' tumors.

Preliminary indication for CHA.7.518.1.H4(S241P) antitumor activity in patients with PVRL2+PD-L1^{low} tumors.

Early clinical data showed increased peripheral immune cell proliferation and serum IFNy, suggesting immune activation by CHA.7.518.1.H4(S241P) single agent.

### EXAMPLE 10: NOVEL DNAM-1 AXIS MEMBER, PVRIG, IS POTENTIALLY A DOMINANT CHECKPOINT INVOLVED IN STEM-LIKE MEMORY T CELLS-DENDRITIC CELL INTERACTION

### Background

T cell accumulation in tumors is a prerequisite for response to cancer immunotherapy. Recent studies highlighted the importance of an early-memory (stem-like) T cell sub-population, that can self-renew and differentiate into effector cells, and of DCs, which are essential for T-cell expansion following checkpoint blockade. PVRIG is a novel inhibitory receptor that competes with the co-activating receptor DNAM-1, for the binding of a shared ligand, PVRL2.

T-cell infiltration and persistence in tumors is a prerequisite for response to cancer immunotherapy. Recent studies highlighted the importance of an early-memory (stem-like) T-cell sub-population, that can self-renew and differentiate into effector cells, and of dendritic cells (DCs), which are essential for T-cell priming and expansion following checkpoint blockade [1, 2]. PVRIG is a novel inhibitory receptor that competes with the co-activating receptor DNAM-1, for the binding of a shared ligand, PVRL2. PVRIG expression is induced on T and NK tumor infiltrating cells, whereas PVRL2 is expressed on tumor, endothelial and myeloid cells in the tumor micro-environment (TME) [3]. We investigated the expression of PVRIG and PVRL2 across TME immune subpopulations.

### Methods

Publicly available TME scRNA sequencing datasets were analyzed for the expression of PVRIG and PVRL2 across immune subsets. Unsupervised principal component analysis with genes used as entries, and hierarchical co-expression pattern among genes known to be expressed on naive, memory, and exhausted CD8+ T-cells was performed. Observations were validated by evaluating PVRIG and PVRL2 expression by flow-cytometry and immunohistochemistry across a variety of tumor indications.

### Results

Across scRNA datasets, PVRIG, like TIGIT and PD-1, was expressed by both stem-like (TCF1+PD1+) and exhausted (TIM3+CD39+) CD8+ T-cells. High resolution unsupervised scRNA gene co-expression analysis revealed that while TIGIT is strongly correlated with PD-1, CTLA-4, and other markers of exhausted T-cells, PVRIG uniquely clusters with markers of early memory T-cells (Figure 49A-49B). Accordingly, PVRIG protein expression was increased on CD28+ early-memory T-cells across indications (Figure 50).

RNA expression data also revealed that PVRL2 is more abundantly expressed across DC-subtypes compared to PD-L1 and PVR (ligand of TIGIT, Figure 51). Flow cytometry confirmed dominant PVRL2 expression on DC subtypes across tumor indications. Immunohistochemistry analysis identified PVRL2 expression in Tertiary Lymphoid Structures (Figure 52).

### Conclusions

PVRIG is expressed on stem-like and exhausted T cells but has a unique dominant expression on early memory cells. PVRIG is also co-expressed with PD-1 and TIGIT on stem-like and exhausted T cells but has a unique dominant expression on early memory cells. PVRL2 is abundantly expressed across DC-types. PVRIG blockade could therefore enhance memory T-cells activation by DCs, resulting in their increased expansion and differentiation.

### References:

1. Jansen, C.S., Prokhnevska, N., Master, V.A. et al. An intra-tumoral niche maintains and differentiates stem-like CD8 T cells. Nature 2019;576;465-470.
2. Held W, et al. Intratumoral CD8+ T cells with stem cell-like properties: implications for cancer immunotherapy. Sci Transl Med. 2019;11(515):eaay6863
3. Whelan S, Ophir E, Kotturi MF, Levy O, Ganguly S, Leung L, et al. PVRIG and PVRL2 are induced in cancer and inhibit CD8+ T-cell function. Cancer Immunol Res. 2019;7:257-68.

### EXAMPLE 11: PVRL2 EXPRESSION ON VARIOUS CELL TYPES

PVRL2 expression can be determined, for example, using immunohistochemistry (IHC), Immunofluorescence, RNAscope, single cell RNA (scRNA), etc. Figure 14 shows the results of using scRNA in mapping the gene expression of PVRL2 in different types of cells in patients with tumors, healthy patients, or patients with inflamed tissues. As shown in Figure 59, PVRL2 is overexpressed by endothelial cells and enterocytes in healthy patients and patients with an inflamed gastrointestinal (GI) tract.

The data presented in Figures 58-59 were analyzed using the Seurat package. Seurat is an R package designed for QC, analysis, and exploration of single-cell RNA-seq data. Seurat aims to enable users to identify and interpret sources of heterogeneity from single-cell transcriptomic measurements, and to integrate diverse types of single-cell data. (A detailed description of Seurat can be found in Butler et al., Nature Biotechnology 2018, and Stuart, Butler, et al., Cell 2019.)

Figures 58-59 present normalized expression after Seurat pipeline: 1=1 standard deviation above mean.

### EXAMPLE 12: EXPLORING THE IMMUNE-TUMOR MICROENVIRONMENT USING HIGH RESOLUTION SINGLE-CELL SPATIAL TRANSCRIPTOMICS: LEVERAGING SINGLE CELL SPATIAL TRANSCRIPTOMICS TO GAIN IN-DEPTH UNDERSTANDING OF THE TUMOR MICROENVIRONMENT

Combining computational expertise with cutting-edge single cell technology Compugen showed location of PVRIG at the sites of T cell priming in the tumor microenvironment.

Preliminary findings further support the rationale for blocking PVRIG to address immunotherapy resistance in inflamed and less inflamed tumors.

Approach provides additional armamentarium to discover novel targets and gain biological insights for development of novel immunotherapies.

The computational platform is the cornerstone of our drug discovery and development capabilities. The biology of the TME is complex, and an in-depth understanding is required to develop novel cancer immunotherapies. This example supports successfully employing high resolution single cell spatial mapping of immune cells to decipher this complexity. Utilizing computational immuno-oncology biology and cutting-edge technology provides an unprecedented view into the composition and spatial localization of individual cells in the TME. Initial findings further suggest the presence of the DNAM-1 pathway including PVRIG, is located at the sites of T cell priming, including the tertiary lymphoid structures. This supports previous findings and further supports the rationale to block PVRIG to address immunotherapy resistance in both inflamed and less inflamed tumors. The ability to study cancer at the spatially resolved single-cell level is expanding our understanding of the complex interactions in the TME and opens the door to new therapeutic approaches.

The analytics approach integrates proprietary omics data, such as proteomics and spatial single-cell transcriptomics with public domain genomics and clinical metadata towards a machine learning based discovery of novel IO-specific targets, biomarkers and mechanism of action.

The computational-driven hypotheses are then rapidly tested and validated by an internal wet-lab experimental group. The validated information is then integrated back into the discovery cycle, providing an additional layer of proprietary data which is being utilized to further optimize the computational predictive models. This tight integration of computational prediction with experimental validation has proved to be essential in its IO discoveries, clinical-stage programs, and pipeline progression.

### Background:

Cancer immunotherapies manipulate the immune system to repropagate anti-tumor response. Development of such treatments relies on the understanding of the interactions between tumor, stroma and immune cells found in the tumor microenvironment (TME); which are often based on direct cell-cell interactions and local secretion of factors. Therefore, high resolution spatial mapping of immune cells and adjacent cells is highly valuable resource for deciphering nature of interactions in the TME for the development of novel anticancer treatments. Here we harness advancements in MERFISH technology to detect the expression of 350 distinct mRNA transcripts at sub-cellular resolution directly on Ovarian & Colorectal tumor tissue sections.

### Methods:

MERFISH assay was conducted on FFPE or frozen sections using the Vizgen Inc protocol and images were captured using 60x microscope. Images were decoded to RNA spots with xyz and gene id using Vizgen's Merlin software. Single-cell analysis was conducted using Scanpy after filtering cells based on size and quality. Probe and spatial single-cell visualizations were done using custom code, MERSCOPE-viewer or Scanpy. Cellular neighborhood and gene-gene correlation was calculated using custom code. Ligand-receptor analysis was conducted using Squidpy.

### Conclusion:

High-resolution Spatial Transcriptomics allows in-depth profiling of the TME at the single-cell level. We have used this method to reaffirm aspects of known biology of the TME, such as association of CXCL10+ Activated DCs and CXCR3+ T-cells. We also demonstrated the presence of DNAM-1 pathway members in TLS regions. The presented methods could be used to uncover new biological findings that would enhance the understanding of the TME and possibly support the development of new cancer immunotherapy treatments.

The examples set forth above are provided to give those of ordinary skill in the art a complete disclosure and description of how to make and use the embodiments of the compositions, systems and methods of the invention, and are not intended to limit the scope of what the inventors regard as their invention. Modifications of the above-described modes for carrying out the invention that are obvious to persons of skill in the art are intended to be within the scope of the following claims. All patents and publications mentioned in the specification are indicative of the levels of skill of those skilled in the art to which the invention pertains.

All headings and section designations are used for clarity and reference purposes only and are not to be considered limiting in any way. For example, those of skill in the art will appreciate the usefulness of combining various aspects from different headings and sections as appropriate according to the spirit and scope of the invention described herein.

All references cited herein are hereby incorporated by reference herein in their entireties and for all purposes to the same extent as if each individual publication or patent or patent application was specifically and individually indicated to be incorporated by reference in its entirety for all purposes.

Many modifications and variations of this application can be made without departing from its spirit and scope, as will be apparent to those skilled in the art. The specific embodiments and examples described herein are offered by way of example only, and the application is to be limited only by the terms of the appended claims, along with the full scope of equivalents to which the claims are entitled.

## Claims

1. An anti-PVRIG antibody for use in treating cancer in a cancer patient population, wherein the cancer patient population is determined by a method for determining a cancer patient population for treatment with an anti-PVRIG antibody, the method comprising:
(a) detecting the presence in a biological sample from the cancer patient one or more cellular components selected from the group consisting of:
(i) TSCM, TRM, naive, exhausted, cycling, and effector CD8 positive T cells, that express PVRIG; and/or
(ii) activated DC cells, DC1, and/or DC2 that express PVRL2,
(b) quantitating the measurement of the level of components selected from the group consisting of:
(i) TSCM, TRM, naive, exhausted, cycling, and effector CD8 positive T cells, that express PVRIG; and/or
(ii) activated DC cells, DC1, and/or DC2 that express PVRL2; and
(c) treating the cancer patient with the anti-PVRIG antibody when one or more cellular components in (a) as quantitated in step (b) are present at an increased level as compared to a control or a patient that does not have detectable levels of the cells.

2. An anti-PVRIG antibody for use in treating cancer in a cancer patient population, wherein the cancer patient population is determined by a method for determining a cancer patient population for treatment with an anti-PVRIG antibody, the method comprising:
(a) detecting the presence in a biological sample from the cancer patient early memory CD8 T cells;
(b) quantitating the measurement of the level of early memory CD8 T cells;
(c) treating the cancer patient with the anti-PVRIG antibody when the level of early memory CD8 T cells are present at an increased level as compared to a control or a patient that does not have detectable levels of the cells.

3. The anti-PVRIG antibody for use according to any one of claims 1 to 2, wherein the anti-PVRIG treatment antibody comprises a heavy chain variable domain from the heavy chain of CHA.7.518.1.H4(S241P) (SEQ ID NO:8) and a light chain variable domain from the light chain of CHA.7.518.1.H4(S241P) (SEQ ID NO:13).

4. The anti-PVRIG antibody for use according to any one of claims 1 to 3, wherein the PVRIG and/or PVRL2 expression is determined using single-cell resolution analysis, wherein the single-cell resolution analysis includes RNAseq, immunohistochemistry (IHC), multiplex immunohistochemistry (mIHC) and/or immunofluorescence (IF), flow cytometry (e.g., FACS) and mass cytometry (e.g., CyTOF), as well as combinations thereof.

5. The anti-PVRIG antibody for use according to claim 4, wherein the PVRIG antibody used for the single-cell resolution analysis and/or immunohistochemistry comprises:
i) a heavy chain variable domain comprising the vhCDR1, vhCDR2, and vhCDR3 from the heavy chain of 6D8-1 (SEQ ID NO:659), and
ii) a light chain variable domain comprising the vlCDR1, vlCDR2, and vlCDR3 from the light chain of 6D8-1 (SEQ ID NO:663).

6. The anti-PVRIG antibody for use according to any one of claims 1 to 5, wherein the anti-PVRIG treatment antibody is administered as a stable liquid pharmaceutical formulation of the anti-PVRIG antibody comprising:
(a) an anti-PVRIG antibody, wherein the anti-PVRIG antibody comprises:
i) a heavy chain variable domain comprising the vhCDR1, vhCDR2, and vhCDR3 from the heavy chain of CHA.7.518.1.H4(S241P) (SEQ ID NO:8), and
ii) a light chain variable domain comprising the vlCDR1, vlCDR2, and vlCDR3 from the light chain of CHA.7.518.1.H4(S241P) (SEQ ID NO:13);
(b) from 10 mM to 100 mM histidine;
(c) from 30 mM to 100 mM NaCl;
(d) from 20 mM to 150 mM L-Arginine; and
(e) from 0.005% to 0.1% w/v polysorbate 80,
wherein the composition has a pH from 5.5 to 7.0.

7. The anti-PVRIG antibody for use according to any one of claims 1 to 6, wherein the anti-PVRIG treatment antibody comprises a CH1-hinge-CH2-CH3 sequence of IgG4 (SEQ ID NO:657 or SEQ ID NO:658), wherein the hinge region optionally comprises mutations.

8. The anti-PVRIG antibody for use according to any one of claims 6 to 7, wherein the anti-PVRIG antibody is at a concentration of from 10 mg/mL to 40 mg/mL, 15 mg/mL to 40 mg/mL, 15 mg/mL to 30 mg/mL, 10 mg/mL to 25 mg/mL, or 15 mg/mL to 25 mg/mL.

9. The anti-PVRIG antibody for use according to any one of claims 6 to 8, wherein the anti-PVRIG antibody formulation comprises:
a) a heavy chain comprising:
i) a VH-CH1-hinge-CH2-CH3, wherein the VH is from CHA.7.518.1.H4(S241P) (SEQ ID NO:4) and wherein the CH1-hinge-CH2-CH3 region is from IgG4; and
b) a light chain comprising:
i) a VL-CL, wherein the VL from CHA.7.518.1.H4(S241P) (SEQ ID NO:9) and wherein the CL region is from human kappa 2 light chain.

10. The anti-PVRIG antibody for use according to any one of claims 1 to 9, wherein the anti-PVRIG treatment antibody is administered at a dosage of about 0.01 mg/kg to about 20 mg/kg of the anti-PVRIG antibody or about 0.01 mg/kg to about 10 mg/kg of the anti-PVRIG antibody.

11. The anti-PVRIG antibody for use according to any one of claims 1 to 10, wherein the anti-PVRIG antibody is administered in combination with an anti-PD-1 antibody.

12. The anti-PVRIG antibody for use according to any one of claims 1 to 11, wherein the anti-PVRIG antibody is administered in combination with an anti-TIGIT antibody.

13. The anti-PVRIG antibody for use according to any one of claims 1 to 12, wherein the anti-PVRIG antibody is administered in combination with an anti-PD-1 antibody and an anti-TIGIT antibody.

14. The anti-PVRIG antibody for use according to any one of claims 1 and 3 to 13, wherein the activated DC cells, DC1, and/or DC2 express CXCL10.

15. The anti-PVRIG antibody for use according to any one of claims 2 to 13, wherein the early memory CD8 T cells include CD8 T cells expressing CXCR3.

16. The method according to any one of claims 1 to 15, wherein the cancer selected from the group consisting of prostate cancer, liver cancer (HCC), colorectal cancer (CRC), colorectal cancer MSS (MSS-CRC; including refractory MSS colorectal), CRC (MSS unknown), ovarian cancer (including ovarian carcinoma), endometrial cancer (including endometrial carcinoma), breast cancer, pancreatic cancer, stomach cancer, cervical cancer, head and neck cancer, thyroid cancer, testis cancer, urothelial cancer, lung cancer, melanoma, non-melanoma skin cancer (squamous and basal cell carcinoma), glioma, renal cell cancer (RCC), renal cell carcinoma (RCC), lymphoma (non-Hodgkins' lymphoma (NHL) and Hodgkin's lymphoma (HD)), Acute myeloid leukemia (AML), T cell Acute Lymphoblastic Leukemia (T-ALL), Diffuse Large B cell lymphoma, testicular germ cell tumors, mesothelioma, esophageal cancer, triple negative breast cancer, Merkel Cells cancer, MSI-high cancer, KRAS mutant tumors, adult T-cell leukemia/lymphoma, pleural mesothelioma, anal SCC, neuroendocrine lung cancer (including neuroendocrine lung carcinoma), NSCLC, NSCL (large cell), NSCLC large cell, NSCLC squamous cell, cervical SCC, malignant melanoma, pancreatic cancer, pancreatic adenocarcinoma, adenoid cystic cancer (including adenoid cystic carcinoma), primary peritoneal cancer, microsatellite stable primary peritoneal cancer, platinum resistant microsatellite stable primary peritoneal cancer, Myelodysplastic syndromes (MDS), HNSCC, PD1 refractory or relapsing cancer, gastroesophageal junction cancer, gastric cancer, and/or fallopian tube cancer.
